(19) Europäisches Patentamt
European Patent Office
Office européen des brevets

(11) **EP 4 692 095 A1**

(12) # EUROPEAN PATENT APPLICATION
published in accordance with Art. 153(4) EPC


(43) Date of publication:
**11.02.2026 Bulletin 2026/07**

(21) Application number: **24778233.7**

(22) Date of filing: **29.03.2024**

(51) International Patent Classification (IPC):
***C07D 498/16*** (2006.01) ***C07D 267/22*** (2006.01)
***C07D 267/16*** (2006.01) ***A61K 31/33*** (2006.01)
***A61K 31/55*** (2006.01) ***A61P 35/02*** (2006.01)

(52) Cooperative Patent Classification (CPC):
**A61K 31/33; A61K 31/55; A61P 35/02; C07D 267/16; C07D 267/22; C07D 498/16**

(86) International application number:
**PCT/CN2024/084821**

(87) International publication number:
**WO 2024/199447 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.03.2023 CN 202310335335**



(71) Applicants:
- **Jiangsu Hansoh Pharmaceutical Group Co., Ltd.**
  **Lianyungang, Jiangsu 222047 (CN)**
- **Shanghai Hansoh Biomedical Co., Ltd.**
  **Shanghai 201203 (CN)**

(72) Inventors:
- **LI, Yuanyuan**
  **Shanghai 201203 (CN)**
- **GUO, Linsong**
  **Shanghai 201203 (CN)**

(74) Representative: **Cooley (UK) LLP**
**22 Bishopsgate**
**London EC2N 4BQ (GB)**


(54) **SALT CRYSTAL FORM OF QUATERNARY FUSED RING COMPOUND, AND PREPARATION METHOD THEREFOR AND USE THEREOF**


(57) The present invention relates to a salt crystal form of a quaternary fused ring compound, and a preparation method therefor and the use thereof. Specifically, the present invention relates to a salt crystal form of the quaternary fused ring compound of a compound as represented by formula (I), a preparation method therefor, a pharmaceutical 5 composition comprising a therapeutically effective amount of the compound or the crystal form thereof, and the use thereof as a tyrosine kinase inhibitor drug.


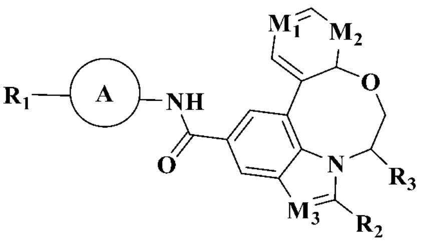

(I)

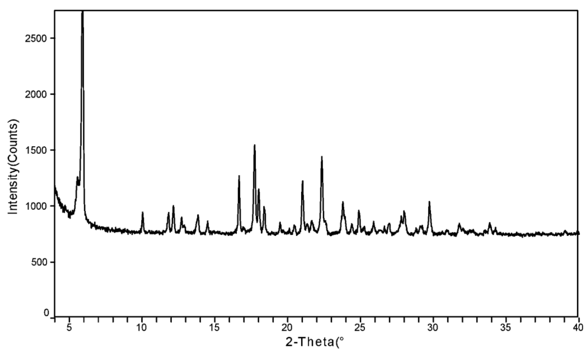


FIG. 1

EP 4 692 095 A1

**Description**

## FIELD OF THE INVENTION

The present invention belongs to the field of medicines, and specifically relates to a salt crystal form of a quaternary fused ring compound, a preparation method therefor, and use thereof.

## BACKGROUND

The tyrosine kinase activity of ABL1 protein is usually strictly regulated, and the N-terminal cap region of the SH3 domain plays an important role here. One regulatory mechanism involves myristoylation of the N-terminal cap glycine-2 residue, followed by interaction with the myristate binding site in the SH1 catalytic domain. A marker of chronic myeloid leukemia (CML) is the Philadelphia chromosome (Ph), which is formed by the reciprocal translocation of the t(9,22) chromosomes in hematopoietic stem cells. This chromosome carries oncogene BCR-ABL1, which encodes a chimeric protein BCR-ABL1 lacking an N-terminal cap and having a constitutively activated tyrosine kinase domain.

Although drugs such as imatinib, nilotinib, and dasatinib that inhibit the tyrosine kinase activity of BCR-ABL1 via an ATP-competitive mechanism may be effective in treating CML, some patients relapse due to the emergence of drug-resistant clones. For example, small molecules or combinations thereof can be used to inhibit the activity of BCR-ABL 1 and BCR-ABL 1 mutants via an ATP binding site, a myristoyl binding site, or a combination of the both sites.

Patent PCT/CN2022/122536 protects a class of quaternary fused ring compounds. In view of the importance of study on a salt form and a crystal form of a drug for a compound in clinical research, and in order to improve the product solubility and solid stability, reduce the storage costs, prolong the product life cycles, and enhance the product bioavailability, the present invention comprehensively studies salt crystal forms of the above compounds.

## SUMMARY OF THE INVENTION

All contents involved in patent PCT/CN2022/122536 are incorporated herein by reference.

An objective of the present invention is to provide a crystal form of an acid salt of a compound represented by formula (I),

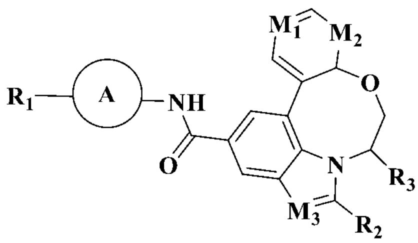

(I)

Wherein:

ring A is $C_{6-10}$ aryl or 5-6-membered heteroaryl;
$R_1$ is selected from $C_{1-3}$ alkyl, $C_{1-3}$ deuteroalkyl, $C_{1-3}$ haloalkyl, $C_{1-3}$ hydroxyalkyl, $C_{1-3}$ alkoxy, $C_{13}$ alkylthio, or $C_{1-3}$ haloalkoxy;
$M_1$ is selected from N or CH;
$M_2$ is selected from NH or $CH_2$;
$M_3$ is selected from N or CH;
$R_2$ or $R_3$ is each independently selected from $C_{1-3}$ alkyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, or $C_{1-3}$ haloalkoxy; and
the acid salt is selected from an ethylsulfonate, a mesylate, a sulfate, a hydrochloride, a p-tosylate, a besylate, an isethionate, or a 1,5-napadisylate.

In a further preferred embodiment of the present invention, the compound represented by formula (I) is further as shown in general formula (I-a):

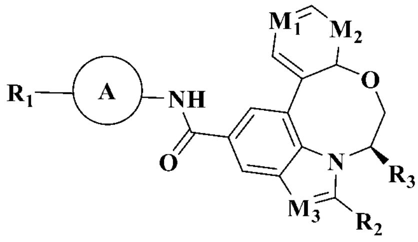

(I-a)

In a further preferred embodiment of the present invention, ring A is phenyl or pyridyl;

$R_1$ is selected from $C_{1-3}$ alkyl, $C_{1-3}$ haloalkyl, $C_{1-3}$ alkoxy, or $C_{1-3}$ haloalkoxy;
$M_1$ is N;
$M_2$ is NH;
$M_3$ is N; and
$R_2$ or $R_3$ is each independently selected from hydrogen, $C_{1-3}$ alkyl, or $C_{1-3}$ haloalkyl.

In a further preferred embodiment of the present invention, $R_1$ is selected from $-CF_2$, $-CF_3$, $-CF_2Cl$, $-OCF_2$, $-OCF_3$, or $-OCF_2Cl$; and
$R_2$ or $R_3$ is each independently selected from $-CH_3$, $-CH_2CH_3$, $-CF_2$, $-CF_3$, or $-CF_2Cl$.

In a further preferred embodiment of the present invention, a specific structure of the compound represented by formula (I) is as follows:

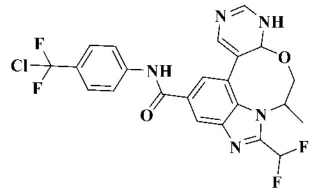
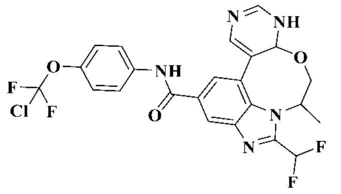
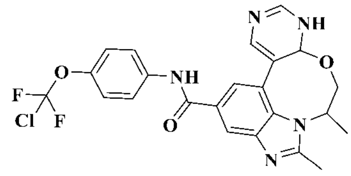

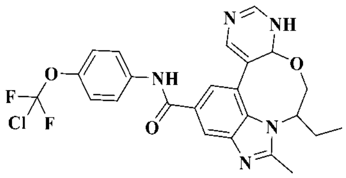
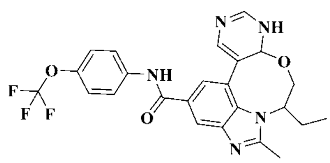
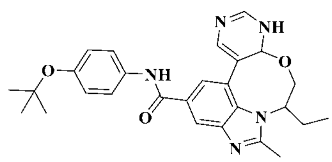

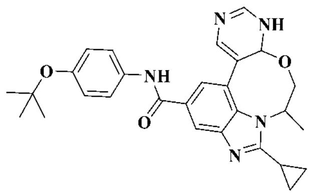
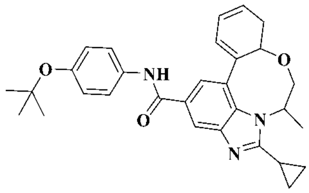
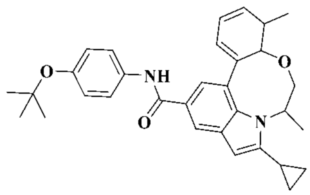

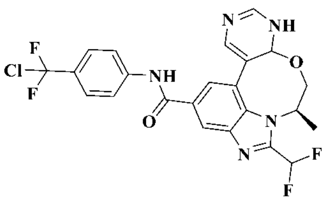
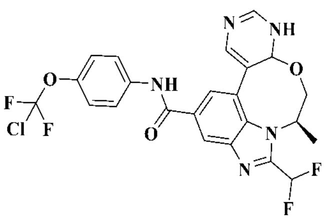
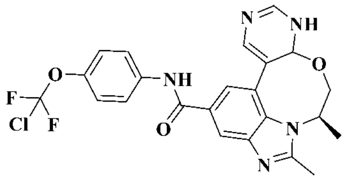

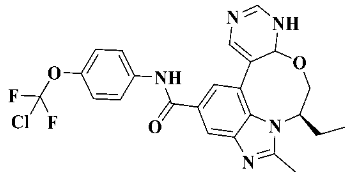 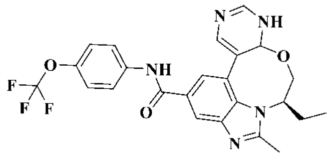 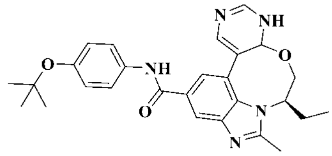

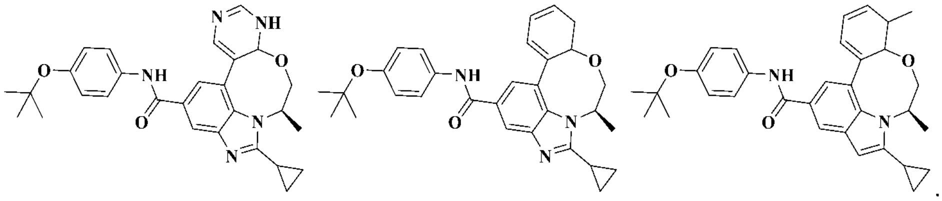.

In a further preferred embodiment of the present invention, the crystal form is a crystal form of a hydrobromide, a crystal form of a hydrochloride, a crystal form of a sulfate, a crystal form of a p-tosylate, a crystal form of a mesylate, a crystal form of a besylate, a crystal form of an oxalate, a crystal form of an acetate, a crystal form of an ethylsulfonate, a crystal form of a maleate, a crystal form of a phosphate, a crystal form of a fumarate, a crystal form of a succinate, a crystal form of a malonate, a crystal form of an adipate, a crystal form of a malate, a crystal form of a tartrate, a crystal form of a 1,5-napadisylate, a crystal form of an isethionate, a crystal form of a citrate, a crystal form of a hippurate, a crystal form of a lactate, a crystal form of a benzoate, a crystal form of a palmitate, or a crystal form of a salicylate;

preferably the crystal form of the hydrobromide, the crystal form of the ethylsulfonate, the crystal form of the mesylate, the crystal form of the sulfate, the crystal form of the hydrochloride, the crystal form of the p-tosylate, the crystal form of the besylate, the crystal form of the isethionate, the crystal form of the 1,5-napadisylate, the crystal form of the maleate, the crystal form of the fumarate, the crystal form of the succinate, the crystal form of the malate, or the crystal form of the tartrate; and
more preferably the crystal form of the ethylsulfonate, the crystal form of the mesylate, the crystal form of the sulfate, the crystal form of the hydrochloride, the crystal form of the p-tosylate, the crystal form of the besylate, the crystal form of the isethionate, or the crystal form of the 1,5-napadisylate.

In a further preferred embodiment of the present invention, the crystal form of the acid salt is a crystal form A of an ethylsulfonate, a crystal form B of the ethylsulfonate, a crystal form A of a mesylate, a crystal form B of the mesylate, a crystal form A of a sulfate, a crystal form B of the sulfate, a crystal form C of the sulfate, a crystal form D of the sulfate, a crystal form E of the sulfate, a crystal form F of the sulfate, a crystal form G of the sulfate, a crystal form A of a hydrochloride, a crystal form B of the hydrochloride, a crystal form C of the hydrochloride, a crystal form A of a p-tosylate, a crystal form B of the p-tosylate, a crystal form C of the p-tosylate, a crystal form D of the p-tosylate, a crystal form E of the p-tosylate, a crystal form A of a besylate, a crystal form B of the besylate, a crystal form C of the besylate, a crystal form A of an isethionate, a crystal form B of the isethionate, or a crystal form A of a 1,5-napadisylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein:

an X-ray powder diffraction pattern of the crystal form A of the ethylsulfonate has a diffraction peak at 5.9±0.2°; or a diffraction peak at 17.7±0.2°; or a diffraction peak at 22.3±0.2°; or a diffraction peak at 16.7±0.2°; or a diffraction peak at 21.0±0.2°; or a diffraction peak at 18.0±0.2°; or a diffraction peak at 5.6±0.2°; or a diffraction peak at 29.7±40.2°; or a diffraction peak at 23.8±0.2°; or a diffraction peak at 12.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;
an X-ray powder diffraction pattern of the crystal form B of the ethylsulfonate has a diffraction peak at 5.6±0.2°; or a diffraction peak at 16.5±0.2°; or a diffraction peak at 8.4±0.2°; or a diffraction peak at 10.0±0.2°; or a diffraction peak at 17.6±0.2°; or a diffraction peak at 23.7±0.2°; or a diffraction peak at 27.7±0.2°; or a diffraction peak at 15.2±0.2°; or a diffraction peak at 28.9±0.2°; or a diffraction peak at 12.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;
an X-ray powder diffraction pattern of the crystal form A of the mesylate has a diffraction peak at 6.0±0.2°; or a diffraction peak at 17.8±0.2°; or a diffraction peak at 21.4±0.2°; or a diffraction peak at 16.5±0.2°; or a diffraction peak at 22.4±0.2°; or a diffraction peak at 12.2±0.2°; or a diffraction peak at 24.3±0.2°; or a diffraction peak at 23.5±0.2°;

or a diffraction peak at 29.8±0.2°; or a diffraction peak at 19.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the mesylate has a diffraction peak at 6.0±0.2°; or a diffraction peak at 18.0±0.2°; or a diffraction peak at 22.6±0.2°; or a diffraction peak at 30.1±0.2°; or a diffraction peak at 6.6±0.2°; or a diffraction peak at 12.2±0.2°; or a diffraction peak at 13.2±0.2°; or a diffraction peak at 15.5±0.2°; or a diffraction peak at 21.4±0.2°; or a diffraction peak at 24.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form A of the sulfate has a diffraction peak at 5.8±0.2°; or a diffraction peak at 21.6±0.2°; or a diffraction peak at 17.6±0.2°; or a diffraction peak at 19.7±0.2°; or a diffraction peak at 16.5 ±0.2°; or a diffraction peak at 12.0±0.2°; or a diffraction peak at 12.3±0.2°; or a diffraction peak at 17.2±0.2°; or a diffraction peak at 13.6±0.2°; or a diffraction peak at 25.9±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the sulfate has a diffraction peak at 5.7±0.2°; or a diffraction peak at 16.9±0.2°; or a diffraction peak at 17.4±0.2°; or a diffraction peak at 22.5±0.2°; or a diffraction peak at 19.3 ±0.2°; or a diffraction peak at 9.9±0.2°; or a diffraction peak at 20.1±0.2°; or a diffraction peak at 13.8±0.2°; or a diffraction peak at 11.1±0.2°; or a diffraction peak at 18.6±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the sulfate has a diffraction peak at 5.6±0.2°; or a diffraction peak at 16.7±0.2°; or a diffraction peak at 8.3±0.2°; or a diffraction peak at 12.7±0.2°; or a diffraction peak at 15.3 ±0.2°; or a diffraction peak at 17.6±0.2°; or a diffraction peak at 10.0±0.2°; or a diffraction peak at 15.5±0.2°; or a diffraction peak at 13.1±0.2°; or a diffraction peak at 21.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form D of the sulfate has a diffraction peak at 17.3±0.2°; or a diffraction peak at 24.3±0.2°; or a diffraction peak at 20.6±0.2°; or a diffraction peak at 26.2±0.2°; or a diffraction peak at 22.1±0.2°; or a diffraction peak at 18.6±0.2°; or a diffraction peak at 15.1±0.2°; or a diffraction peak at 12.9±0.2°; or a diffraction peak at 25.9±0.2°; or a diffraction peak at 18.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form E of the sulfate has a diffraction peak at 5.8±0.2°; or a diffraction peak at 17.2±0.2°; or a diffraction peak at 9.8±0.2°; or a diffraction peak at 13.8±0.2°; or a diffraction peak at 20.0 ±0.2°; or a diffraction peak at 22.6±0.2°; or a diffraction peak at 19.2±0.2°; or a diffraction peak at 22.2±0.2°; or a diffraction peak at 11.1±0.2°; or a diffraction peak at 26.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form F of the sulfate has a diffraction peak at 6.0±0.2°; or a diffraction peak at 16.0±0.2°; or a diffraction peak at 22.4±0.2°; or a diffraction peak at 17.3±0.2°; or a diffraction peak at 20.0 ±0.2°; or a diffraction peak at 18.5±0.2°; or a diffraction peak at 20.5±0.2°; or a diffraction peak at 14.4±0.2°; or a diffraction peak at 24.9±0.2°; or a diffraction peak at 24.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form G of the sulfate has a diffraction peak at 5.9±0.2°; or a diffraction peak at 16.7±0.2°; or a diffraction peak at 17.6±0.2°; or a diffraction peak at 5.6±0.2°; or a diffraction peak at 16.9 ±0.2°; or a diffraction peak at 22.2±0.2°; or a diffraction peak at 29.5±0.2°; or a diffraction peak at 27.7±0.2°; or a diffraction peak at 25.1±0.2°; or a diffraction peak at 10.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form A of the hydrochloride has a diffraction peak at 22.4±0.2°; or a diffraction peak at 14.0±0.2°; or a diffraction peak at 17.1±0.2°; or a diffraction peak at 6.2±0.2°; or a diffraction peak at 19.4±0.2°; or a diffraction peak at 25.2±0.2°; or a diffraction peak at 17.5±0.2°; or a diffraction peak at 21.6±0.2°; or a diffraction peak at 19.8±0.2°; or a diffraction peak at 23.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the hydrochloride has a diffraction peak at 6.7±0.2°; or a

diffraction peak at 27.0±0.2°; or a diffraction peak at 23.4±0.2°; or a diffraction peak at 13.4±0.2°; or a diffraction peak at 11.0±0.2°; or a diffraction peak at 24.1±0.2°; or a diffraction peak at 15.6±0.2°; or a diffraction peak at 4.5±0.2°; or a diffraction peak at 20.0±0.2°; or a diffraction peak at 10.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the hydrochloride has a diffraction peak at 16.5±0.2°; or a diffraction peak at 20.4±0.2°; or a diffraction peak at 22.2±0.2°; or a diffraction peak at 9.7±0.2°; or a diffraction peak at 17.8±0.2°; or a diffraction peak at 5.3±0.2°; or a diffraction peak at 17.5±0.2°; or a diffraction peak at 6.0±0.2°; or a diffraction peak at 14.3±0.2°; or a diffraction peak at 21.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form A of the p-tosylate has a diffraction peak at 16.8±0.2°; or a diffraction peak at 19.9±0.2°; or a diffraction peak at 5.7±0.2°; or a diffraction peak at 22.5±0.2°; or a diffraction peak at 21.8±0.2°; or a diffraction peak at 24.9±0.2°; or a diffraction peak at 22.3±0.2°; or a diffraction peak at 20.8±0.2°; or a diffraction peak at 26.6±0.2°; or a diffraction peak at 12.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the p-tosylate has a diffraction peak at 5.5±0.2°; or a diffraction peak at 19.9±0.2°; or a diffraction peak at 13.2±0.2°; or a diffraction peak at 21.9±0.2°; or a diffraction peak at 28.1±0.2°; or a diffraction peak at 14.1±0.2°; or a diffraction peak at 10.9±0.2°; or a diffraction peak at 17.6±0.2°; or a diffraction peak at 9.5±0.2°; or a diffraction peak at 20.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the p-tosylate has a diffraction peak at 5.8±0.2°; or a diffraction peak at 17.3±0.2°; or a diffraction peak at 16.7±0.2°; or a diffraction peak at 22.0±0.2°; or a diffraction peak at 19.6±0.2°; or a diffraction peak at 23.1±0.2°; or a diffraction peak at 22.4±0.2°; or a diffraction peak at 20.1±0.2°; or a diffraction peak at 29.0±0.2°; or a diffraction peak at 12.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form D of the p-tosylate has a diffraction peak at 4.9±0.2°; or a diffraction peak at 5.74±0.2°; or a diffraction peak at 17.2±0.2°; or a diffraction peak at 22.0±0.2°; or a diffraction peak at 19.5±0.2°; or a diffraction peak at 28.9±0.2°; or a diffraction peak at 25.5±0.2°; or a diffraction peak at 12.7±0.2°; or a diffraction peak at 14.8±0.2°; or a diffraction peak at 23.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form E of the p-tosylate has a diffraction peak at 5.4±0.2°; or a diffraction peak at 16.1±0.2°; or a diffraction peak at 9.9±0.2°; or a diffraction peak at 16.7±0.2°; or a diffraction peak at 8.4±0.2°; or a diffraction peak at 23.1±0.2°; or a diffraction peak at 26.9±0.2°; or a diffraction peak at 25.7±0.2°; or a diffraction peak at 25.2±0.2°; or a diffraction peak at 28.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form A of the besylate has a diffraction peak at 5.7±0.2°; or a diffraction peak at 17.2±0.2°; or a diffraction peak at 21.8±0.2°; or a diffraction peak at 5.5±0.2°; or a diffraction peak at 16.6±0.2°; or a diffraction peak at 23.0±0.2°; or a diffraction peak at 17.6±0.2°; or a diffraction peak at 20.3±0.2°; or a diffraction peak at 27.3±0.2°; or a diffraction peak at 28.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the besylate has a diffraction peak at 19.7±0.2°; or a diffraction peak at 17.4±0.2°; or a diffraction peak at 13.6±0.2°; or a diffraction peak at 22.6±0.2°; or a diffraction peak at 9.7±0.2°; or a diffraction peak at 5.7±0.2°; or a diffraction peak at 14.2±0.2°; or a diffraction peak at 29.1±0.2°; or a diffraction peak at 12.8±0.2°; or a diffraction peak at 23.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the besylate has a diffraction peak at 5.44±0.2°; or a diffraction peak at 16.6±0.2°; or a diffraction peak at 16.9±0.2°; or a diffraction peak at 15.0±0.2°; or a diffraction peak at 12.74±0.2°; or a diffraction peak at 19.4±0.2°; or a diffraction peak at 8.3±0.2°; or a diffraction peak at 20.9±0.2°; or a diffraction peak at 13.8±0.2°; or a diffraction peak at 9.9±0.2°; preferably comprises any 2-5, or 3-5, or 3-6, or 3-8, or 5-8, or 6-8 of the above diffraction peaks, and more preferably comprises any 6, 7, or 8 diffraction peaks selected

therefrom;

an X-ray powder diffraction pattern of the crystal form A of the isethionate has a diffraction peak at 5.4±0.2°; or a diffraction peak at 16.1±0.2°; or a diffraction peak at 20.9±0.2°; or a diffraction peak at 20.0±0.2°; or a diffraction peak at 25.2±0.2°; or a diffraction peak at 15.1±0.2°; or a diffraction peak at 16.7±0.2°; or a diffraction peak at 25.7±0.2°; or a diffraction peak at 12.7±0.2°; or a diffraction peak at 19.5±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the isethionate has a diffraction peak at 5.9±0.2°; or a diffraction peak at 16.7±0.2°; or a diffraction peak at 21.2±0.2°; or a diffraction peak at 19.5±0.2°; or a diffraction peak at 22.5±0.2°; or a diffraction peak at 10.0±0.2°; or a diffraction peak at 13.0±0.2°; or a diffraction peak at 24.3±0.2°; or a diffraction peak at 15.5±0.2°; or a diffraction peak at 17.5±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom; and

An X-ray powder diffraction pattern of the crystal form A of the 1,5-napadisylate has a diffraction peak at 21.3 ±0.2°; or a diffraction peak at 10.2±0.2°; or a diffraction peak at 9.5±0.2°; or a diffraction peak at 17.1±0.2°; or a diffraction peak at 9.9±0.2°; or a diffraction peak at 16.7±0.2°; or a diffraction peak at 25.8±0.2°; or a diffraction peak at 5.7±0.2°; or a diffraction peak at 8.0±0.2°; or a diffraction peak at 23.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom.

A further preferred embodiment of the present invention provides a crystal form A of an ethylsulfonate of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 2θ of 5.910.2°,17.710.2°, and22.340.2°, preferably comprises 2 diffraction peaks selected therefrom, more preferably comprises 3 diffraction peaks selected therefrom; optionally, further may comprise at least one diffraction peak at 2θ of 16.7+0.2°, 21.0+0.2°, 18.0+0.2°, 5.6+0.2°, and 29.740.2°, and preferably comprises 2, 3, 4, or 5 diffraction peaks selected therefrom.

For example, the X-ray powder diffraction pattern of the crystal form A of the ethylsulfonate has diffraction peaks at 20 of:

5.940.2° and 16.7±0.2°;
or 5.9+0.2°, 16.7+0.2°, and 21.0+0.2°;
or 17.7±0.2°, 16.7±0.2°, and 18.0±0.2°;
or 22.3±0.2°, 5.6±0.2°, and 29.7±0.2°;
or 5.9+0.2°, 16.7±0.2°, 5.6+0.2°, and 29.7±0.2°;
or 5.9+0.2°, 17.7±0.2°, 16.7+0.2°, 21.0±0.2°, 5.6+0.2°, and 29.7±0.2°;
or 22.3±0.2°, 16.7±0.2°, 21.0+0.2°, 18.0+0.2°, 5.6+0.2°, and 29.7±0.2°.

The X-ray powder diffraction pattern of the crystal form A of the ethylsulfonate optionally further comprises one or more diffraction peaks at 2θ of 23.8±0.2°, 12.2±0.2°, 18.4±0.2°, 28.0±0.2°, 24.9±0.2°, 10.0±0.2°, and 11.8±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the ethylsulfonate has diffraction peaks at 20 of:

5.9±0.2°, 17.7±0.2°, 22.3±0.2°, 16.7±0.2°, 21.0±0.2°, 18.0+0.2°, 23.8±0.2°, and 12.2±0.2°;
or 5.9±0.2°, 17.7±0.2°, 22.3±0.2°, 16.7±0.2°, 21.0±0.2°, 18.0+0.2°, 23.8±0.2°, 12.2±0.2°, 18.4±0.2°, and 28.0 ±0.2°.

The X-ray powder diffraction pattern of the crystal form A of the ethylsulfonate comprises one or more diffraction peaks at 2θ of 5.9+0.2°, 17.7+0.2°, 22.3±0.2°, 16.7±0.2°, 21.0+0.2°, 18.0+0.2°, 5.6+0.2°, 29.7±0.2°, 23.8±0.2°, 12.2 ±0.2°, 18.4±0.2°, 28.0±0.2°, 24.9±0.2°, 10.0±0.2°, and 11.8±0.2°; and preferably comprises diffraction peaks optionally at 4, 6, 8, or 10 positions selected therefrom.

For example, the X-ray powder diffraction pattern of the crystal form A of the ethylsulfonate has diffraction peaks at 20 of:

5.9±0.2°, 17.7±0.2°, 22.3±0.2°, 16.7±0.2°, 21.0±0.2°, 18.0+0.2°, 5.6+0.2°, and 29.7±0.2°;
or 5.9+0.2°, 22.3±0.2°, 21.0±0.2°, 18.0+0.2°, 5.6+0.2°, 29.7±0.2°, 23.8±0.2°, and 12.2±0.2°;
or 5.9+0.2°, 17.7±0.2°, 22.3±0.2°, 16.7±0.2°, 21.0±0.2°, 18.0+0.2°, 5.6+0.2°, 29.7±0.2°, 23.8±0.2°, and 12.2±0.2°;
or 17.7±0.2°, 16.7±0.2°, 5.6+0.2°, 29.7±0.2°, 23.8±0.2°, 12.2±0.2°, 18.4±0.2°, 28.0±0.2°, 24.9±0.2°, and 10.0 ±0.2°.

The X-ray powder diffraction pattern of the crystal form A of the ethylsulfonate comprises one or more diffraction peaks at 2θ of 5.9+0.2°, 17.7+0.2°, 22.3±0.2°, 16.7±0.2°, 21.0+0.2°, 18.0+0.2°, 5.6+0.2°, 29.7±0.2°, 23.8±0.2°, 12.2 ±0.2°, 18.4±0.2°, 28.0±0.2°, 24.9±0.2°, 10.0±0.2°, 11.8±0.2°, 13.8±0.2°, 27.8±0.2°, 12.7±0.2°, 22.6±0.2°, and 14.5 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the ethylsulfonate has diffraction peaks at 20 of:

5.9±0.2°, 22.3±0.2°, 21.0+0.2°, 18.0+0.2°, 5.6+0.2°, 29.7±0.2°, 23.8±0.2°, and 18.4±0.2°;
or 5.9+0.2°, 17.7±0.2°, 21.0±0.2°, 18.0+0.2°, 5.6+0.2°, 29.7±0.2°, 23.8±0.2°, and 12.2±0.2°;
or 22.3±0.2°, 16.7±0.2°, 21.0±0.2°, 18.0+0.2°, 5.6+0.2°, 29.7±0.2°, 23.8±0.2°, 12.2±0.2°, 18.4±0.2°, and 28.0 ±0.2°;
or 17.7±0.2°, 22.3±0.2°, 16.7+0.2°, 21.0+0.2°, 18.0±0.2°,5.6±0.2°, 29.7±0.2°, 23.8±0.2°, 12.2±0.2°, 18.4±0.2°, 28.0±0.2°, and 24.9±0.2°.

Most preferably, for the crystal form A of the ethylsulfonate, the number of acids is 1, its X-ray powder diffraction pattern uses Cu-Kα radiation, and its X-ray characteristic diffraction peaks represented by a 20 angle and an interplanar spacing d are as shown in Table 1.

Table 1

| Serial No. | XRPD data of crystal form A of ethylsulfonate | | | |
|---|---|---|---|---|
| | 2θ (±0.2°) | d value | Peak height | Ratio (I%) |
| 1 | 5.562 | 15.8748 | 368 | 15.1 |
| 2 | 5.907 | 14.9498 | 2432 | 100 |
| 3 | 10.041 | 8.8017 | 183 | 7.5 |
| 4 | 11.823 | 7.4789 | 173 | 7.1 |
| 5 | 12.151 | 7.2777 | 232 | 9.5 |
| 6 | 12.717 | 6.9552 | 129 | 5.3 |
| 7 | 13.834 | 6.3961 | 162 | 6.7 |
| 8 | 14.499 | 6.1041 | 107 | 4.4 |
| 9 | 16.668 | 5.3145 | 506 | 20.8 |
| 10 | 17.726 | 4.9995 | 781 | 32.1 |
| 11 | 18.009 | 4.9216 | 387 | 15.9 |
| 12 | 18.379 | 4.8234 | 214 | 8.8 |
| 13 | 21.027 | 4.2215 | 465 | 19.1 |
| 14 | 21.656 | 4.1003 | 104 | 4.3 |
| 15 | 22.347 | 3.975 | 683 | 28.1 |
| 16 | 22.570 | 3.9362 | 113 | 4.6 |
| 17 | 23.788 | 3.7374 | 278 | 11.4 |
| 18 | 24.885 | 3.5751 | 190 | 7.8 |
| 19 | 27.802 | 3.2062 | 159 | 6.5 |
| 20 | 27.987 | 3.1855 | 206 | 8.5 |
| 21 | 29.730 | 3.0025 | 284 | 11.7 |

(continued)

| Serial No. | XRPD data of crystal form A of ethylsulfonate | | | |
|---|---|---|---|---|
| | 2θ (±0.2°) | d value | Peak height | Ratio (I%) |
| 22 | 33.880 | 2.6436 | 107 | 4.4 |

Further preferably, the X-ray powder diffraction pattern of the crystal form A of the ethylsulfonate is as shown in FIG. 1; its DSC pattern is substantially as shown in FIG. 2; and its TGA pattern is substantially as shown in FIG. 3.

A further preferred embodiment of the present invention provides a crystal form B of an ethylsulfonate of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 5.6+0.2°, 16.5+0.2°, and 8.4+0.2°, preferably comprises 2 diffraction peaks selected therefrom, more preferably comprises 3 diffraction peaks selected therefrom; optionally, further may comprise at least one diffraction peak at 2θ of 10.0±0.2°, 17.6+0.2°, 23.7+0.2°, 27.7±0.2°, and 15.2±0.2°, and preferably comprises 2, 3, 4, or 5 diffraction peaks selected therefrom.

For example, the X-ray powder diffraction pattern of the crystal form B of the ethylsulfonate has diffraction peaks at 20 of:

5.6±0.2°, 10.0+0.2°, and 17.6±0.2°;
or 16.5+0.2°, 17.6±0.2°, and 23.7±0.2°;
or 8.4+0.2°, 27.7±0.2°, and 15.2±0.2°;
or 8.4+0.2°, 23.7±0.2°, 27.7±0.2°, and 15.2±0.2°;
or 8.4+0.2°, 10.0±0.2°, 17.6±0.2°, 23.7±0.2°, 27.7±0.2°, and 15.2±0.2°.

The X-ray powder diffraction pattern of the crystal form B of the ethylsulfonate optionally further comprises one or more diffraction peaks at 2θ of 28.9±0.2°, 12.8±0.2°, 13.8±0.2°, 21.1±0.2°, 11.8±0.2°, 18.6±0.2°, and 13.1±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the ethylsulfonate has diffraction peaks at 20 of:

5.6±0.2°, 16.5±0.2°, 8.4+0.2°, 10.0±0.2°, 17.6±0.2°, 23.7±0.2°, 28.9±0.2°, and 12.8±0.2°;
or 5.6+0.2°, 16.5±0.2°, 8.4+0.2°, 10.0±0.2°, 17.6+0.2°, 23.7±0.2°, 28.9±0.2°, 12.8±0.2°, and 13.8±0.2°;
or 5.6+0.2°, 16.5+0.2°, 8.4+0.2°, 10.0±0.2°, 17.6+0.2°, 23.7±0.2°, 28.9±0.2°, 12.8±0.2°, 13.8±0.2°, and 21.1 ±0.2°.

The X-ray powder diffraction pattern of the crystal form B of the ethylsulfonate comprises one or more diffraction peaks at 2θ of 5.6±0.2°, 16.5+0.2°, 8.4+0.2°, 10.0±0.2°, 17.6±0.2°, 23.7+0.2°, 27.7±0.2°, 15.2±0.2°, 28.9±0.2°, 12.8 ±0.2°, 13.8±0.2°, 21.1±0.2°, 11.8±0.2°, 18.6±0.2°, and 13.1±0.2°; and preferably comprises diffraction peaks optionally at 4, 6, 8, or 10 positions selected therefrom.

For example, the X-ray powder diffraction pattern of the crystal form B of the ethylsulfonate has diffraction peaks at 20 of:

5.6±0.2°, 16.5+0.2°, 8.4+0.2°, 10.0+0.2°, 17.6±0.2°, 23.7±0.2°, 27.7±0.2°, and 15.2±0.2°;
or 5.6±0.2°, 8.4+0.2°, 10.0+0.2°, 17.6+0.2°, 23.7±0.2°, 27.7±0.2°, 15.24±0.2°, and 12.8±0.2°;
or 5.6+0.2°, 16.5+0.2°, 8.4+0.2°, 10.0±0.2°, 17.6±0.2°, 23.7±0.2°, 27.7±0.2°, 15.24±0.2°, 28.94±0.2°, and 12.8 ±0.2°;
or 16.5±0.2°, 8.4+0.2°, 10.0±0.2°, 17.6+0.2°, 23.7+0.2°, 27.7±0.2°, 15.2±0.2°, 28.9±0.2°, 12.8±0.2°, and 13.8 ±0.2°.

The X-ray powder diffraction pattern of the crystal form B of the ethylsulfonate comprises one or more diffraction peaks at 2θ of 5.6±0.2°, 16.5+0.2°, 8.4+0.2°, 10.0±0.2°, 17.6+0.2°, 23.7±0.2°, 27.7±0.2°, 15.2±0.2°, 28.9±0.2°, 12.8 ±0.2°, 13.8±0.2°, 21.1±0.2°, 11.8±0.2°, 18.6±0.2°, 13.1±0.2°, 20.1±0.2°, 25.3±0.2°, 19.5±0.2°, 25.6±0.2°, and 26.3 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form B of the ethylsulfonate has diffraction peaks

at 2θ of:

5.6±0.2°, 8.4+0.2°, 10.0±0.2°, 17.6±0.2°, 23.7+0.2°, 27.7±0.2°, 15.2±0.2°, and 28.9±0.2°;
or 16.±0.2°, 10.0±0.2°, 17.6+0.2°, 23.7±0.2°, 27.7+0.2°, 15.2±0.2°, 28.9±0.2°, and 12.8±0.2°;
or 5.6+0.2°, 16.5±0.2°, 17.6±0.2°, 23.7±0.2°, 27.7±0.2°, 15.2±0.2°, 21.1±0.2°, and 11.8±0.2°;
or 16.5±0.2°, 8.4±0.2°, 23.7+0.2°, 27.7±0.2°, 15.2±0.2°, 28.9±0.2°, 21.1±0.2°, and 11.8±0.2°;
or 5.6+0.2°, 8.4+0.2°, 10.0±0.2°, 17.6±0.2°, 23.7±0.2°, 27.7±0.2°, 15.2±0.2°, 28.9±0.2°, 12.84±0.2°, and 13.8 ±0.2°;
or 16.5±0.2°, 8.4+0.2°, 17.6±0.2°, 23.7±0.2°, 27.7±0.2°, 15.2±0.2°, 28.9±0.2°, 13.8±0.2°, 21.1±0.2°, and 11.8 ±0.2°;
or 16.5+0.2°, 8.4±0.2°, 10.0±0.2°, 17.6+0.2°, 23.7±0.2°, 27.7±0.2°, 15.2±0.2°, 28.9±0.2°, 12.8±0.2°, 13.8±0.2°, 21.1±0.2°, and 11.8±0.2°.

Most preferably, its X-ray characteristic diffraction peaks represented by a 2θ angle and an interplanar spacing d using Cu-Kα radiation are as shown in Table 2.

Table 2

| Serial No. | XRPD data of crystal form B of ethylsulfonate | | | |
|---|---|---|---|---|
| | 2θ (±0.2°) | d value | Peak height | Ratio (I%) |
| 1 | 5.559 | 15.8831 | 6106 | 100 |
| 2 | 8.377 | 10.5458 | 428 | 7 |
| 3 | 10.020 | 8.8199 | 430 | 7 |
| 4 | 11.765 | 7.5159 | 174 | 2.8 |
| 5 | 12.780 | 6.9213 | 224 | 3.7 |
| 6 | 13.081 | 6.7625 | 123 | 2 |
| 7 | 13.834 | 6.3959 | 220 | 3.6 |
| 8 | 15.230 | 5.8127 | 325 | 5.3 |
| 9 | 16.529 | 5.3587 | 1377 | 22.6 |
| 10 | 17.603 | 5.0341 | 397 | 6.5 |
| 11 | 18.637 | 4.757 | 169 | 2.8 |
| 12 | 19.507 | 4.5468 | 113 | 1.9 |
| 13 | 20.080 | 4.4184 | 120 | 2 |
| 14 | 21.071 | 4.2128 | 221 | 3.6 |
| 15 | 22.344 | 3.9755 | 107 | 1.8 |
| 16 | 23.663 | 3.7568 | 374 | 6.1 |
| 17 | 25.308 | 3.5162 | 123 | 2 |
| 18 | 25.572 | 3.4805 | 114 | 1.9 |
| 19 | 26.261 | 3.3907 | 113 | 1.9 |
| 20 | 27.660 | 3.2223 | 345 | 5.7 |
| 21 | 28.938 | 3.0829 | 251 | 4.1 |
| 22 | 33.372 | 2.6827 | 75 | 1.2 |

Further preferably, the X-ray powder diffraction pattern of the crystal form B of the ethylsulfonate is substantially as shown in FIG. 4; its DSC pattern is substantially as shown in FIG. 5; and its TGA pattern is substantially as shown in FIG. 6.

A further preferred embodiment of the present invention provides a crystal form A of a mesylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction

pattern comprises at least one or more diffraction peaks at 2θ of 6.0±0.2°, 17.8±0.2°, and 21.4±0.2°, preferably comprises 2 diffraction peaks selected therefrom, more preferably comprises 3 diffraction peaks selected therefrom; optionally, further may comprise at least one diffraction peak at 2θ of 16.5+0.2°, 22.4±0.2°, 12.2±0.2°, 24.3±0.2°, and 23.5±0.2°, and preferably comprises 2, 3, 4, or 5 diffraction peaks selected therefrom.

For example, the X-ray powder diffraction pattern of the crystal form A of the mesylate has diffraction peaks at 2θ of:

6.0±0.2°, 17.8±0.2°, and 16.5±0.2°;
or 17.8±0.2°, 16.5±0.2°, and 22.4±0.2°;
or 21.4±0.2°, 16.5+0.2°, and 12.2±0.2°;
or 6.0±0.2°, 17.8±0.2°, 16.5+0.2°, and 22.4±0.2°;
or 17.8±0.2°, 16.5±0.2°, 22.4±0.2°, and 12.2±0.2°;
or 17.8±0.2°, 16.5±0.2°, 22.4±0.2°, 12.2±0.2°, 24.3±0.2°, and 23.5±0.2°;
or 21.4±0.2°, 16.5±0.2°, 22.4±0.2°, 12.2±0.2°, 24.3±0.2°, and 23.5±0.2°.

The X-ray powder diffraction pattern of the crystal form A of the mesylate optionally further comprises one or more diffraction peaks at 2θ of 29.8±0.2°, 19.8±0.2°, 16.7±0.2°, 25.8±0.2°, 14.8±0.2°, 28.0±0.2°, and 33.9±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the mesylate has diffraction peaks at 2θ of:

6.0±0.2°, 17.8±0.2°, 21.4±0.2°, 16.5±0.2°, 22.4±0.2°, 12.2±0.2°, 29.8±0.2°, and 19.8±0.2°;
or 6.0±0.2°, 17.8±0.2°, 21.4±0.2°, 16.5±0.2°, 22.4+0.2°, 12.2±0.2°, 29.8±0.2°, 19.8±0.2°, and 16.7±0.2°;
or 6.0±0.2°, 17.8±0.2°, 21.4±0.2°, 16.5±0.2°, 22.4±0.2°, 12.2±0.2°, 29.8±0.2°, 19.8±0.2°, 16.7+0.2°, and 25.8 ±0.2°.

The X-ray powder diffraction pattern of the crystal form A of the mesylate comprises one or more diffraction peaks at 2θ of 6.0±0.2°, 17.8±0.2°, 21.4±0.2°, 16.5±0.2°, 22.4±0.2°, 12.2±0.2°, 24.3±0.2°, 23.5±0.2°, 29.8±0.2°, 19.8 ±0.2°, 16.7±0.2°, 25.8±0.2°, 14.8±0.2°, 28.0±0.2°, and 33.9±0.2°; and preferably comprises diffraction peaks optionally at 4, 6, 8, or 10 positions selected therefrom.

For example, the X-ray powder diffraction pattern of the crystal form A of the mesylate has diffraction peaks at 2θ of:

17.8±0.2°, 21.4±0.2°, 16.5+0.2°, 22.4±0.2°, 12.2±0.2°, 24.3±0.2°, 23.5±0.2°, and 29.8±0.2°;
or 6.0±0.2°, 17.8±0.2°, 21.4±0.2°, 16.5±0.2°, 22.4±0.2°, 12.2±0.2°, 24.3±0.2°, 23.5±0.2°, 29.8±0.2°, and 19.8 ±0.2°;
or 17.8±0.2°, 21.4±0.2°, 16.5±0.2°, 22.4±0.2°, 12.2±0.2°, 24.3±0.2°, 23.5±0.2°, 29.8±0.2°, 19.8±0.2°, and 16.7 ±0.2°.

The X-ray powder diffraction pattern of the crystal form A of the mesylate comprises one or more diffraction peaks at 2θ of 6.0±0.2°, 17.8±0.2°, 21.4±0.2°, 16.5±0.2°, 22.4±0.2°, 12.2±0.2°, 24.3±0.2°, 23.5±0.2°, 29.8±0.2°, 19.8 ±0.2°, 16.7±0.2°, 25.8±0.2°, 14.8±0.2°, 28.0±0.2°, 33.9±0.2°, 22.0+0.2°, 10.7±0.2°, 23.7+0.2°, 20.7±0.2°, and 27.2 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the mesylate has diffraction peaks at 2θ of:

6.0±0.2°, 17.8±0.2°, 21.4±0.2°, 16.5±0.2°, 22.4±0.2°, 12.2±0.2°, 24.3±0.2°, and 23.5±0.2°;
or 6.0±0.2°, 21.4±0.2°, 16.5+0.2°, 22.4±0.2°, 12.2±0.2°, 24.3±0.2°, 23.5±0.2°, and 29.8±0.2°;
or 17.8±0.2°, 21.4±0.2°, 16.5±0.2°, 22.4±0.2°, 12.2±0.2°, 24.3±0.2°, 23.5±0.2°, and 29.8±0.2°;
or 6.0±0.2°, 17.8±0.2°, 21.4±0.2°, 22.4±0.2°, 12.2±0.2°, 24.3±0.2°, 23.5±0.2°, and 25.8±0.2°;
or 6.0±0.2°, 21.4±0.2°, 16.5+0.2°, 12.2±0.2°, 24.3±0.2°, 23.5±0.2°, 29.8±0.2°, 19.8±0.2°, 16.7+0.2°, and 25.8 ±0.2°;
or 21.4±0.2°, 16.5±0.2°, 22.4±0.2°, 12.2±0.2°, 24.3±0.2°, 23.5±0.2°, 29.8±0.2°, 19.8±0.2°, 16.7+0.2°, and 25.8 ±0.2°;
or 6.0±0.2°, 17.8±0.2°, 21.4+0.2°, 16.5±0.2°, 22.4±0.2°, 12.2±0.2°, 24.3±0.2°, 23.5±0.2°, 29.8+0.2°, 19.8±0.2°,

16.7±0.2°, and 25.8±0.2°;
or 17.8±0.2°, 21.4±0.2°, 16.5±0.2°, 22.4±0.2°, 12.2±0.2°, 24.3±0.2°, 23.5+0.2°, 16.7+0.2°, 25.8±0.2°, 14.8 ±0.2°, 28.0±0.2°, and 33.9±0.2°.

Most preferably, its X-ray characteristic diffraction peaks represented by a 2θ angle and an interplanar spacing d using Cu-Kα radiation are as shown in Table 3.

Table 3

| Serial No. | XRPD data of crystal form A of mesylate | | | |
|---|---|---|---|---|
| | 2θ (10.2°) | d value | Peak height | Ratio (I%) |
| 1 | 5.987 | 14.7488 | 3869 | 100 |
| 2 | 10.710 | 8.2536 | 154 | 4 |
| 3 | 12.208 | 7.2439 | 675 | 17.4 |
| 4 | 12.896 | 6.8591 | 127 | 3.3 |
| 5 | 13.528 | 6.5401 | 128 | 3.3 |
| 6 | 14.803 | 5.9795 | 248 | 6.4 |
| 7 | 16.486 | 5.3727 | 701 | 18.1 |
| 8 | 16.650 | 5.32 | 270 | 7 |
| 9 | 17.762 | 4.9894 | 1209 | 31.2 |
| 10 | 19.795 | 4.4812 | 288 | 7.4 |
| 11 | 20.703 | 4.2869 | 140 | 3.6 |
| 12 | 21.351 | 4.1581 | 878 | 22.7 |
| 13 | 21.962 | 4.0439 | 167 | 4.3 |
| 14 | 22.368 | 3.9714 | 694 | 17.9 |
| 15 | 23.503 | 3.782 | 334 | 8.6 |
| 16 | 23.746 | 3.744 | 152 | 3.9 |
| 17 | 24.295 | 3.6605 | 640 | 16.5 |
| 18 | 25.814 | 3.4484 | 269 | 7 |
| 19 | 27.196 | 3.2762 | 139 | 3.6 |
| 20 | 28.045 | 3.179 | 240 | 6.2 |
| 21 | 29.766 | 2.999 | 324 | 8.4 |
| 22 | 33.943 | 2.6389 | 221 | 5.7 |

Further preferably, the X-ray powder diffraction pattern of the crystal form A of the mesylate is substantially as shown in FIG. 7; its DSC pattern is substantially as shown in FIG. 8; and its TGA pattern is substantially as shown in FIG. 9.

A further preferred embodiment of the present invention provides a crystal form B of a mesylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 6.0±0.2°, 18.0+0.2°, and 22.6+0.2°, preferably comprises 2 diffraction peaks selected therefrom, more preferably comprises 3 diffraction peaks selected therefrom; optionally, further may comprise at least one diffraction peak at 2θ of 30.1±0.2°, 6.6±0.2°, 12.2±0.2°, 13.2±0.2°, and 15.5±0.2°, and preferably comprises 2, 3, 4, or 5 diffraction peaks selected therefrom.

For example, the X-ray powder diffraction pattern of the crystal form B of the mesylate has diffraction peaks at 2θ of:

18.0+0.2°, 12.2±0.2°, and 13.2±0.2°;
or 22.6±0.2°, 13.2±0.2°, and 15.5±0.2°;
or 6.0±0.2°, 30.1±0.2°, 12.2±0.2°, and 13.2±0.2°;

or 18.0+0.2°, 30.1±0.2°, 6.6±0.2°, and 12.2±0.2°;
or 6.0±0.2°, 30.1±0.2°, 6.6±0.2°, 12.2±0.2°, 13.2±0.2°, and 15.5±0.2°;
or 22.6±0.2°, 30.1±0.2°, 6.6±0.2°, 12.2±0.2°, 13.2±0.2°, and 15.5±0.2°.

The X-ray powder diffraction pattern of the crystal form B of the mesylate optionally further comprises one or more diffraction peaks at 2θ of 21.4±0.2°, 24.0±0.2°, 12.0±0.2°, 10.1±0.2°, 24.6±0.2°, 16.6±0.2°, and 19.8±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the mesylate has diffraction peaks at 20 of:

6.0±0.2°, 18.0+0.2°, 22.6±0.2°, 30.1±0.2°, 6.6±0.2°, 12.2±0.2°, 21.4±0.2°, and 24.0±0.2°;
or 6.0±0.2°, 18.0±0.2°°, 22.6+0.2°, 30.1±0.2°, 6.6±0.2°, 12.2±0.2°, 21.4±0.2°, 24.0±0.2°, and 12.0±0.2°;
or 6.0±0.2°, 18.0±0.2°°, 22.6+0.2°, 30.1±0.2°, 6.6±0.2°, 12.2±0.2°, 21.4±0.2°, 24.0±0.2°, 12.0±0.2°, and 10.1 ±0.2°.

The X-ray powder diffraction pattern of the crystal form B of the mesylate comprises one or more diffraction peaks at 2θ of 6.0±0.2°, 18.0+0.2°, 22.6±0.2°, 30.1±0.2°, 6.6±0.2°, 12.2±0.2°, 13.2±0.2°, 15.5±0.2°, 21.4±0.2°, 24.0±0.2°, 12.0±0.2°, 10.1±0.2°, 24.6±0.2°, 16.6±0.2°, and 19.8±0.2°; and preferably comprises diffraction peaks optionally at 4, 6, 8, or 10 positions selected therefrom.

For example, the X-ray powder diffraction pattern of the crystal form B of the mesylate has diffraction peaks at 20 of:

6.0±0.2°, 18.0+0.2°, 22.6±0.2°, 30.1±0.2°, 6.6±0.2°, 12.2±0.2°, 13.2±0.2°, and 15.5±0.2°;
or 18.0+0.2°, 22.6±0.2°, 30.1±0.2°, 6.6±0.2°, 12.2±0.2°, 13.2±0.2°, 15.5±0.2°, and 21.44±0.2°;
or 18.0+0.2°, 22.6±0.2°, 30.1±0.2°, 6.6±0.2°, 12.2±0.2°, 13.2±0.2°, 15.5±0.2°, 21.4±0.2°, 24.0±0.2°, and 12.0 ±0.2°.

The X-ray powder diffraction pattern of the crystal form B of the mesylate comprises one or more diffraction peaks at 2θ of 6.0±0.2°, 18.0+0.2°, 22.6±0.2°, 30.1±0.2°, 6.6±0.2°, 12.2±0.2°, 13.2±0.2°, 15.5±0.2°, 21.4±0.2°, 24.0±0.2°, 12.0±0.2°, 10.1±0.2°, 24.6±0.2°, 16.6±0.2°, 19.8±0.2°, 25.5+0.2°, 20.3±0.2°, 26.0+0.2°, 31.3±0.2°, and 28.4±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form B of the mesylate has diffraction peaks at 20 of:

6.0±0.2°, 18.0±0.2°°, 22.6±0.2°, 30.1±0.2°, 6.6±0.2°, 12.2±0.2°, 13.2±0.2°, and 15.5±0.2°;
or 6.0±0.2°, 22.6±0.2°, 30.1±0.2°, 6.6±0.2°, 12.2±0.2°, 13.2±0.2°, 15.5±0.2°, and 21.4±0.2°;
or 18.0±0.2°, 22.6±0.2°, 30.1±0.2°, 6.6±0.2°, 12.2±0.2°, 13.2±0.2°, 15.5±0.2°, 21.4±0.2°, 24.0±0.2°, and 12.0 ±0.2°;
or 6.0±0.2°, 18.0±0.2°, 22.6±0.2°, 30.1±0.2°, 6.6±0.2°, 12.2±0.2°, 13.2±0.2°, 15.5±0.2°, 21.4±0.2°, 24.0+0.2°, 12.0±0.2°, and 10.1±0.2°;
or 18.0±0.2°, 22.6±0.2°, 30.1±0.2°, 6.6±0.2°, 12.2±0.2°, 13.2±0.2°, 15.5±0.2°, 21.4±0.2°, 24.6±0.2°, 16.6 ±0.2°, 19.8±0.2°, and 25.5±0.2°.

Most preferably, its X-ray characteristic diffraction peaks represented by a 20 angle and an interplanar spacing d using Cu-Kα radiation are as shown in Table 4.

Table 4

| Serial No. | XRPD data of crystal form B of mesylate | | | |
|---|---|---|---|---|
| | 2θ (±0.2°) | d value | Peak height | Ratio (I%) |
| 1 | 6.004 | 14.7072 | 6799 | 100 |
| 2 | 6.615 | 13.351 | 342 | 5 |
| 3 | 10.124 | 8.7299 | 181 | 2.7 |
| 4 | 11.966 | 7.3902 | 211 | 3.1 |

(continued)

| Serial No. | XRPD data of crystal form B of mesylate | | | |
|---|---|---|---|---|
| | 2θ (±0.2°) | d value | Peak height | Ratio (I%) |
| 5 | 12.245 | 7.2219 | 316 | 4.6 |
| 6 | 13.223 | 6.69 | 306 | 4.5 |
| 7 | 15.496 | 5.7137 | 284 | 4.2 |
| 8 | 16.627 | 5.3274 | 163 | 2.4 |
| 9 | 16.874 | 5.2499 | 95 | 1.4 |
| 10 | 17.967 | 4.933 | 1456 | 21.4 |
| 11 | 19.834 | 4.4726 | 162 | 2.4 |
| 12 | 20.341 | 4.3623 | 131 | 1.9 |
| 13 | 21.416 | 4.1457 | 260 | 3.8 |
| 14 | 22.592 | 3.9325 | 869 | 12.8 |
| 15 | 24.012 | 3.7031 | 228 | 3.4 |
| 16 | 24.558 | 3.6219 | 169 | 2.5 |
| 17 | 25.470 | 3.4943 | 163 | 2.4 |
| 18 | 25.975 | 3.4275 | 122 | 1.8 |
| 19 | 28.406 | 3.1394 | 114 | 1.7 |
| 20 | 30.114 | 2.9651 | 399 | 5.9 |
| 21 | 31.330 | 2.8527 | 119 | 1.8 |
| 22 | 34.271 | 2.6144 | 108 | 1.6 |

Further preferably, the X-ray powder diffraction pattern of the crystal form B of the mesylate is substantially as shown in FIG. 10; and its DSC pattern is substantially as shown in FIG. 11.

A further preferred embodiment of the present invention provides a crystal form A of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 5.8±0.2°, 21.610.2°, and 17.6±0.2°, preferably comprises 2 diffraction peaks selected therefrom, more preferably comprises 3 diffraction peaks selected therefrom; optionally, further may comprise at least one diffraction peak at 2θ of 19.7±0.2°, 16.5±0.2°, 12.0±0.2°, 12.3±0.2°, and 17.2±0.2°, and preferably comprises 2, 3, 4, or 5 diffraction peaks selected therefrom.

For example, the X-ray powder diffraction pattern of the crystal form A of the sulfate has diffraction peaks at 2θ of:

17.6±0.2°, 12.3±0.2°, and 17.2±0.2°;
or 5.8±0.2°, 19.7±0.2°, 16.5±0.2°, and 12.0±0.2°;
or 21.6±0.2°, 16.5±0.2°, 12.0±0.2°, and 12.3±0.2°;
or 17.6±0.2°, 16.5±0.2°, 12.0±0.2°, and 12.3±0.2°;
or 5.8±0.2°, 19.7±0.2°, 16.5±0.2°, 12.0±0.2°, 12.3±0.2°, and 17.2±0.2°;
or 21.6±0.2°, 19.7±0.2°, 16.5±0.2°, 12.0±0.2°, 12.3±0.2°, and 17.2±0.2°;
or 17.6±0.2°, 19.7±0.2°, 16.5±0.2°, 12.0±0.2°, 12.3±0.2°, and 17.2±0.2°.

The X-ray powder diffraction pattern of the crystal form A of the sulfate optionally further comprises one or more diffraction peaks at 20 of 13.6±0.2°, 25.9±0.2°, 23.5±0.2°, 21.8±0.2°, 14.4±0.2°, 10.4±0.2°, and 24.7±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the sulfate has diffraction peaks at 2θ of:

5.8±0.2°, 21.6±0.2°, 17.6±0.2°, 19.7±0.2°, 16.5±0.2°, 12.0±0.2°, 13.6±0.2°, and 25.9±0.2°;
or 5.8±0.2°, 21.6±0.2°, 17.6±0.2°, 19.7±0.2°, 16.5±0.2°, 12.0±0.2°, 13.6+0.2°, 25.9±0.2°, and 23.5±0.2°;

or 5.8±0.2°, 21.6±0.2°, 17.6±0.2°, 19.7±0.2°, 16.5±0.2°, 12.0±0.2°, 13.6±0.2°, 25.9±0.2°, 23.5±0.2°, and 21.8 ±0.2°.

The X-ray powder diffraction pattern of the crystal form A of the sulfate comprises one or more diffraction peaks at 20 of 5.8±0.2°, 21.6±0.2°, 17.6±0.2°, 19.7±0.2°, 16.5±0.2°, 12.0±0.2°, 12.3±0.2°, 17.2±0.2°, 13.6±0.2°, 25.9±0.2°, 23.5±0.2°, 21.8±0.2°, 14.4±0.2°, 10.4±0.2°, and 24.7±0.2°; and preferably comprises diffraction peaks optionally at 4, 6, 8, or 10 positions selected therefrom.

For example, the X-ray powder diffraction pattern of the crystal form A of the sulfate has diffraction peaks at 2θ of:

5.8±0.2°, 21.6±0.2°, 17.6±0.2°, 19.7±0.2°, 16.5±0.2°, 12.0±0.2°, 12.3±0.2°, and 17.2±0.2°;
or 21.6±0.2°, 17.6±0.2°, 19.7±0.2°, 16.5±0.2°, 12.0±0.2°, 12.3±0.2°, 17.2±0.2°, and 13.6±0.2°;
or 5.8±0.2°, 21.6±0.2°, 17.6±0.2°, 19.7±0.2°, 16.5±0.2°, 12.0±0.2°, 12.3±0.2°, 17.2±0.2°, 13.6±0.2°, and 25.9 ±0.2°;
or 21.6±0.2°, 17.6±0.2°, 19.7±0.2°, 16.5±0.2°, 12.0±0.2°, 12.3±0.2°, 17.2±0.2°, 13.6±0.2°, 25.9±0.2°, and 23.5 ±0.2°.

The X-ray powder diffraction pattern of the crystal form A of the sulfate comprises one or more diffraction peaks at 20 of 5.8±0.2°, 21.6±0.2°, 17.6±0.2°, 19.7±0.2°, 16.5±0.2°, 12.0±0.2°, 12.3±0.2°, 17.2±0.2°, 13.6±0.2°, 25.9±0.2°, 23.5±0.2°, 21.8±0.2°, 14.4±0.2°, 10.4±0.2°, 24.7+0.2°, 27.3±0.2°, 24.5±0.2°, 20.7±0.2°, 9.8±0.2°, and 26.7±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the sulfate has diffraction peaks at 2θ of:

5.810.2°, 21.6±0.2°, 17.6±0.2°, 19.7±0.2°, 16.5±0.2°, 12.0±0.2°, 12.3±0.2°, and 17.2±0.2°;
or 21.6±0.2°, 17.6±0.2°, 19.7±0.2°, 16.5±0.2°, 12.0±0.2°, 12.3±0.2°, 17.2±0.2°, and 13.6±0.2°;
or 5.8±0.2°, 21.6±0.2°, 17.6±0.2°, 12.0±0.2°, 12.3±0.2°, 17.2±0.2°, 23.5±0.2°, and 21.8±0.2°;
or 5.8±0.2°, 17.6±0.2°, 16.5±0.2°, 12.3±0.2°, 13.6±0.2°, 23.5±0.2°, 21.8±0.2°, 10.4±0.2°, 24.7±0.2°, and 27.3 ±0.2°;
or 21.6±0.2°, 17.6±0.2°, 19.7±0.2°, 16.5±0.2°, 12.0±0.2°,12.3±0.2°, 17.2±0.2°, 13.6+0.2°, 25.9±0.2°, 23.5+0.2°, 21.8±0.2°, and 14.4±0.2°.

Most preferably, its X-ray characteristic diffraction peaks represented by a 20 angle and an interplanar spacing d using Cu-Kα radiation are as shown in Table 5.

Table 5

| Serial No. | XRPD data of crystal form A of sulfate | | | |
|---|---|---|---|---|
| | 2θ (±0.2°) | d value | Peak height | Ratio (I%) |
| 1 | 5.761 | 15.327 | 1969 | 100 |
| 2 | 9.836 | 8.9852 | 126 | 6.4 |
| 3 | 10.389 | 8.5083 | 223 | 11.3 |
| 4 | 11.969 | 7.3882 | 447 | 22.7 |
| 5 | 12.291 | 7.1954 | 444 | 22.5 |
| 6 | 12.777 | 6.9227 | 117 | 5.9 |
| 7 | 13.605 | 6.5029 | 336 | 17.1 |
| 8 | 14.403 | 6.1444 | 228 | 11.6 |
| 9 | 16.505 | 5.3665 | 553 | 28.1 |
| 10 | 17.199 | 5.1515 | 433 | 22 |
| 11 | 17.639 | 5.024 | 610 | 31 |
| 12 | 19.668 | 4.51 | 589 | 29.9 |
| 13 | 20.706 | 4.2861 | 142 | 7.2 |
| 14 | 21.553 | 4.1196 | 859 | 43.6 |

(continued)

| Serial No. | XRPD data of crystal form A of sulfate | | | |
|---|---|---|---|---|
| | 2θ (±0.2°) | d value | Peak height | Ratio (I%) |
| 15 | 21.777 | 4.0778 | 239 | 12.1 |
| 16 | 23.502 | 3.7822 | 281 | 14.3 |
| 17 | 24.474 | 3.6341 | 165 | 8.4 |
| 18 | 24.659 | 3.6073 | 200 | 10.2 |
| 19 | 25.873 | 3.4407 | 300 | 15.2 |
| 20 | 26.726 | 3.3328 | 120 | 6.1 |
| 21 | 27.273 | 3.2672 | 190 | 9.6 |
| 22 | 28.814 | 3.0959 | 99 | 5 |

Further preferably, the X-ray powder diffraction pattern of the crystal form A of the sulfate is substantially as shown in FIG. 17; its DSC pattern is substantially as shown in FIG. 18; and its TGA pattern is substantially as shown in FIG. 19.

A further preferred embodiment of the present invention provides a crystal form B of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 5.7±0.2°, 16.9±0.2°, and 17.4±0.2°, preferably comprises 2 diffraction peaks selected therefrom, more preferably comprises 3 diffraction peaks selected therefrom; optionally, further may comprise at least one diffraction peak at 2θ of 22.5±0.2°, 19.3±0.2°, 9.9±0.2°, 20.1±0.2°, and 13.8±0.2°, and preferably comprises 2, 3, 4, or 5 diffraction peaks selected therefrom.

For example, the X-ray powder diffraction pattern of the crystal form B of the sulfate has diffraction peaks at 20 of:

5.7±0.2°, 22.5±0.2°, and 19.3±0.2°;
or 16.9±0.2°, 22.5±0.2°, and 19.3±0.2°;
or 16.9±0.2°, 19.3±0.2°, 9.9+0.2°, and 20.1±0.2°;
or 17.4±0.2°, 22.5±0.2°, 19.3±0.2°, and 20.1±0.2°;
or 5.7±0.2°, 22.5±0.2°, 19.3±0.2°, 9.9±0.2°, 20.1±0.2°, and 13.8±0.2°;
or 16.9±0.2°, 17.4±0.2°, 22.5±0.2°, 19.3±0.2°, 9.9±0.2°, and 20.1±0.2°;
or 17.4±0.2°, 22.5±0.2°, 19.3±0.2°, 9.9±0.2°, 20.1±0.2°, and 13.8±0.2°.

The X-ray powder diffraction pattern of the crystal form B of the sulfate optionally further comprises one or more diffraction peaks at 20 of 11.1±0.2°, 18.6+0.2°, 27.2±0.2°, 26.8±0.2°, 24.2±0.2°, 25.4±0.2°, and 23.7±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the sulfate has diffraction peaks at 20 of:

5.7±0.2°, 16.9±0.2°, 17.4±0.2°, 22.5±0.2°, 19.3±0.2°, 9.9±0.2°, 11.1±0.2°, and 18.6±0.2°;
or 5.7±0.2°, 16.9±0.2°, 17.4±0.2°, 22.5±0.2°, 19.3±0.2°, 9.9±0.2°, 11.1±0.2°, 18.6±0.2°, and 27.2±0.2°;
or 5.7±0.2°, 16.9±0.2°, 17.4±0.2°, 22.5±0.2°, 19.3±0.2°, 9.9±0.2°, 11.1±0.2°, 18.6±0.2°, 27.2±0.2°, and 26.8 ±0.2°.

The X-ray powder diffraction pattern of the crystal form B of the sulfate comprises one or more diffraction peaks at 20 of 5.7±0.2°, 16.9±0.2°, 17.4±0.2°, 22.5±0.2°, 19.3±0.2°, 9.9±0.2°, 20.1±0.2°, 13.8±0.2°, 11.1±0.2°, 18.6±0.2°, 27.2±0.2°, 26.8±0.2°, 24.2±0.2°, 25.4+0.2°, and 23.7±0.2°; and preferably comprises diffraction peaks optionally at 4, 6, 8, or 10 positions selected therefrom.

For example, the X-ray powder diffraction pattern of the crystal form B of the sulfate has diffraction peaks at 20 of:

5.7±0.2°, 16.9±0.2°, 17.4±0.2°, 22.5±0.2°, 19.3±0.2°, 9.9±0.2°, 20.1±0.2°, and 13.8±0.2°;
or 16.9±0.2°, 17.4±0.2°, 22.5±0.2°, 19.3±0.2°, 9.9±0.2°, 20.1±0.2°, 13.8±0.2°, and 11.1±0.2°;
or 5.7±0.2°, 16.9±0.2°, 17.4±0.2°, 22.5±0.2°, 19.3±0.2°, 9.9±0.2°, 20.1±0.2°, 13.8±0.2°, 11.1±0.2°, and 18.6 ±0.2°;

or 16.9±0.2°, 17.4±0.2°, 22.5+0.2°, 19.3±0.2°, 9.9±0.2°, 20.1±0.2°, 13.8±0.2°, 11.1±0.2°, 18.6±0.2°, and 27.2 ±0.2°.

The X-ray powder diffraction pattern of the crystal form B of the sulfate comprises one or more diffraction peaks at 2θ of 5.7±0.2°, 16.9±0.2°, 17.4±0.2°, 22.5±0.2°, 19.3±0.2°, 9.9±0.2°, 20.1±0.2°, 13.8±0.2°, 11.1±0.2°, 18.6±0.2°, 27.2±0.2°, 26.8±0.2°, 24.2±0.2°, 25.4±0.2°, 23.74±0.2°, 28.54±0.2°, 29.1±0.2°, 15.4±0.2°, 20.8±0.2°, and 11.6 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form B of the sulfate has diffraction peaks at 2θ of:

5.7±0.2°, 16.9±0.2°, 17.4±0.2°, 22.5±0.2°, 19.3±0.2°, 9.9±0.2°, 20.1±0.2°, and 13.8±0.2°;
or 5.7±0.2°, 16.9±0.2°, 22.5±0.2°, 19.3±0.2°, 9.9±0.2°, 20.1±0.2°, 13.8±0.2°, and 11.1±0.2°;
or 16.9±0.2°, 17.4±0.2°, 19.3±0.2°, 20.1±0.2°, 11.1±0.2°, 27.2±0.2°, 24.2±0.2°, 25.4±0.2°, 23.7±0.2°, and 28.5 ±0.2°;
or 5.7±0.2°, 16.9±0.2°, 19.3±0.2°, 9.9±0.2°, 11.1±0.2°, 18.6±0.2°, 27.2±0.2°, 25.4±0.2°, 23.7±0.2°, and 28.5 ±0.2°;
or 16.9±0.2°, 17.410.2°, 22.5+0.2°, 19.3±0.2°, 9.9±0.2°, 20.1±0.2°, 13.8±0.2°, 11.1±0.2°, 18.6±0.2°, 24.2±0.2°, 25.4±0.2°, and 23.7±0.2°.

Most preferably, its X-ray characteristic diffraction peaks represented by a 2θ angle and an interplanar spacing d using Cu-Kα radiation are as shown in Table 6.

Table 6

| Serial No. | XRPD data of crystal form B of sulfate | | | |
|---|---|---|---|---|
| | 2θ (±0.2°) | d value | Peak height | Ratio (I%) |
| 1 | 5.682 | 15.5423 | 3465 | 100 |
| 2 | 9.940 | 8.8915 | 169 | 4.9 |
| 3 | 11.076 | 7.9819 | 112 | 3.2 |
| 4 | 11.601 | 7.6219 | 37 | 1.1 |
| 5 | 13.811 | 6.4066 | 114 | 3.3 |
| 6 | 15.391 | 5.7524 | 41 | 1.2 |
| 7 | 16.936 | 5.2308 | 424 | 12.2 |
| 8 | 17.401 | 5.092 | 251 | 7.2 |
| 9 | 18.560 | 4.7768 | 72 | 2.1 |
| 10 | 19.284 | 4.599 | 216 | 6.2 |
| 11 | 20.139 | 4.4056 | 129 | 3.7 |
| 12 | 20.763 | 4.2746 | 42 | 1.2 |
| 13 | 22.529 | 3.9434 | 219 | 6.3 |
| 14 | 23.741 | 3.7446 | 50 | 1.4 |
| 15 | 24.190 | 3.6762 | 59 | 1.7 |
| 16 | 25.439 | 3.4984 | 57 | 1.6 |
| 17 | 26.761 | 3.3286 | 64 | 1.8 |
| 18 | 27.218 | 3.2737 | 73 | 2.1 |
| 19 | 28.470 | 3.1325 | 50 | 1.4 |
| 20 | 29.108 | 3.0653 | 47 | 1.4 |

Further preferably, the X-ray powder diffraction pattern of the crystal form B of the sulfate is substantially as

shown in FIG. 20; its DSC pattern is substantially as shown in FIG. 21; and its TGA pattern is substantially as shown in FIG. 22.

A further preferred embodiment of the present invention provides a crystal form C of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 2θ of 5.6±0.2°, 16.7±0.2°, and 8.3±0.2°, preferably comprises 2 diffraction peaks selected therefrom, more preferably comprises 3 diffraction peaks selected therefrom; optionally, further may comprise at least one diffraction peak at 2θ of 12.7±0.2°, 15.3±0.2°, 17.6±0.2°, 10.0±0.2°, and 15.5±0.2°, and preferably comprises 2, 3, 4, or 5 diffraction peaks selected therefrom.

For example, the X-ray powder diffraction pattern of the crystal form C of the sulfate has diffraction peaks at 2θ of:

5.6±0.2° and 12.7±0.2°;
16.7±0.2°, 17.6±0.2°, and 15.5±0.2°;
or 8.3±0.2°, 17.6±0.2°, and 15.5±0.2°;
or 5.6±0.2°, 15.3±0.2°, 17.6±0.2°, and 15.5±0.2°;
or 5.6±0.2°, 12.7±0.2°, 15.3±0.2°, 17.6±0.2°, 10.0±0.2°, and 15.5±0.2°;
or 16.7±0.2°, 12.7±0.2°, 15.3±0.2°, 17.6±0.2°, 10.0±0.2°, and 15.5±0.2°;
or 8.3±0.2°, 12.7±0.2°, 15.3±0.2°, 17.6±0.2°, 10.0±0.2°, and 15.5±0.2°.

The X-ray powder diffraction pattern of the crystal form C of the sulfate optionally further comprises one or more diffraction peaks at 2θ of 13.1±0.2°, 21.0±0.2°, 25.7±0.2°, 25.3±0.2°, 19.5±0.2°, 20.1±0.2°, and 18.6±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form C of the sulfate has diffraction peaks at 2θ of:

5.6±0.2°, 16.7±0.2°, 8.3±0.2°, 12.7±0.2°, 15.3±0.2°, 17.6±0.2°, 13.1±0.2°, and 21.0±0.2°;
or 5.6±0.2°, 16.7±0.2°, 8.3±0.2°, 12.7±0.2°, 15.3±0.2°, 17.6±0.2°, 13.1±0.2°, 21.0±0.2°, and 25.7±0.2°;
or 5.6±0.2°, 16.7±0.2°, 8.3±0.2°, 12.7±0.2°, 15.3±0.2°, 17.6±0.2°, 13.1±0.2°, 21.0±0.2°, 25.7±0.2°, and 25.3 ±0.2°.

The X-ray powder diffraction pattern of the crystal form C of the sulfate comprises one or more diffraction peaks at 2θ of 5.6±0.2°, 16.7±0.2°, 8.3+0.2°, 12.7±0.2°, 15.3±0.2°, 17.6±0.2°, 10.0±0.2°, 15.5±0.2°, 13.1±0.2°, 21.0±0.2°, 25.7±0.2°, 25.3±0.2°, 19.5±0.2°, 20.1±0.2°, and 18.6±0.2°; and preferably comprises diffraction peaks optionally at 4, 6, 8, or 10 positions selected therefrom.

For example, the X-ray powder diffraction pattern of the crystal form C of the sulfate has diffraction peaks at 2θ of:

5.64±0.2°, 16.74±0.2°, 8.3±0.2°, 12.7±0.2°, 15.3±0.2°, 17.6±0.2°, 10.0±0.2°, and 15.5±0.2°;
or 16.7±0.2°, 8.3±0.2°, 12.7±0.2°, 15.3±0.2°, 17.6±0.2°, 10.0±0.2°, 15.5±0.2°, and 13.1±0.2°;
or 5.6±0.2°, 16.7±0.2°, 8.3±0.2°, 12.7±0.2°, 15.3±0.2°, 17.6±0.2°, 10.0±0.2°, 15.5±0.2°, 13.1±0.2°, and 21.0 ±0.2°;
or 16.7±0.2°, 8.3±0.2°, 12.7±0.2°, 15.3±0.2°, 17.6±0.2°, 10.0±0.2°, 15.5±0.2°, 13.1±0.2°, 21.0±0.2°, and 25.7 ±0.2°.

The X-ray powder diffraction pattern of the crystal form C of the sulfate comprises one or more diffraction peaks at 2θ of 5.6±0.2°, 16.7±0.2°, 8.3+0.2°, 12.7±0.2°, 15.3±0.2°, 17.6±0.2°, 10.0±0.2°, 15.5±0.2°, 13.1±0.2°, 21.0±0.2°, 25.7±0.2°, 25.3±0.2°, 19.5±0.2°, 20.1±0.2°, 18.6±0.2°, 13.9±0.2°, 23.8+0.2°, 22.3+0.2°, 33.7+0.2°, and 24.4±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form C of the sulfate has diffraction peaks at 2θ of:

5.6±0.2°, 16.7±0.2°, 8.3±0.2°, 12.7±0.2°, 15.3±0.2°, 17.6±0.2°, 10.0±0.2°, and 15.5±0.2°;
or 5.6±0.2°, 16.7±0.2°, 12.7±0.2°, 15.3±0.2°, 17.6±0.2°, 10.0±0.2°, 15.5±0.2°, and 13.1±0.2°;
or 5.6±0.2°, 16.7±0.2°, 8.3±0.2°, 12.7±0.2°, 15.3±0.2°, 17.6±0.2°, 10.0±0.2°, 15.5±0.2°, 13.1±0.2°, and 21.0 ±0.2°;
or 5.6±0.2°, 16.7±0.2°, 12.7±0.2°, 15.3±0.2°, 17.6±0.2°, 10.0±0.2°, 15.5±0.2°, 13.1±0.2°, 21.0±0.2°, and 25.7 ±0.2°;
or 16.7±0.2°, 8.3±0.2°, 15.3±0.2°, 10.0±0.2°, 15.5±0.2°, 13.1±0.2°, 21.0±0.2°, 25.7±0.2°, 25.3±0.2°, and 19.5 ±0.2°.

Most preferably, its X-ray characteristic diffraction peaks represented by a 2θ angle and an interplanar spacing d using Cu-Kα radiation are as shown in Table 7.

Table 7

| Serial No. | XRPD data of crystal form C of sulfate | | | |
|---|---|---|---|---|
| | 2θ (±0.2°) | d value | Peak height | Ratio (I%) |
| 1 | 5.583 | 15.816 | 1010 | 100 |
| 2 | 8.342 | 10.5901 | 346 | 34.3 |
| 3 | 10.002 | 8.8358 | 137 | 13.6 |
| 4 | 12.740 | 6.9428 | 206 | 20.4 |
| 5 | 13.063 | 6.7719 | 110 | 10.9 |
| 6 | 13.909 | 6.3615 | 87 | 8.6 |
| 7 | 15.251 | 5.8046 | 201 | 19.9 |
| 8 | 15.473 | 5.7219 | 118 | 11.7 |
| 9 | 16.670 | 5.3139 | 383 | 37.9 |
| 10 | 17.585 | 5.0392 | 183 | 18.1 |
| 11 | 18.616 | 4.7623 | 99 | 9.8 |
| 12 | 19.508 | 4.5467 | 105 | 10.4 |
| 13 | 20.135 | 4.4065 | 102 | 10.1 |
| 14 | 21.015 | 4.2239 | 109 | 10.8 |
| 15 | 22.265 | 3.9895 | 66 | 6.5 |
| 16 | 22.668 | 3.9194 | 47 | 4.7 |
| 17 | 23.845 | 3.7285 | 87 | 8.6 |
| 18 | 24.354 | 3.6518 | 51 | 5 |
| 19 | 25.263 | 3.5224 | 107 | 10.6 |
| 20 | 25.712 | 3.4619 | 109 | 10.8 |
| 21 | 26.259 | 3.391 | 45 | 4.5 |
| 22 | 33.720 | 2.6558 | 52 | 5.1 |

Further preferably, the X-ray powder diffraction pattern of the crystal form C of the sulfate is substantially as shown in FIG. 23; its DSC pattern is substantially as shown in FIG. 24; and its TGA pattern is substantially as shown in FIG. 25.

A further preferred embodiment of the present invention provides a crystal form D of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 2θ of 17.3±0.2°, 24.3±0.2°, and 20.6±0.2°, preferably comprises 2 diffraction peaks selected therefrom, more preferably comprises 3 diffraction peaks selected therefrom; optionally, further may comprise at least one diffraction peak at 2θ of 26.2±0.2°, 22.1±0.2°, 18.6±0.2°, 15.1±0.2°, and 12.9±0.2°, and preferably comprises 2, 3, 4, or 5 diffraction peaks selected therefrom.

For example, the X-ray powder diffraction pattern of the crystal form D of the sulfate has diffraction peaks at 2θ of:

17.3±0.2°, 26.2±0.2°, and 12.9±0.2°;
or 24.3±0.2°, 22.1±0.2°, and 18.6±0.2°;
or 17.3±0.2°, 26.2±0.2°, and 22.1±0.2°;
or 24.3±0.2°, 26.2±0.2°, 22.1±0.2°, and 18.6±0.2°;
or 17.3±0.2°, 26.2±0.2°, 22.1±0.2°, 18.6±0.2°, 15.1±0.2°, and 12.94±0.2°;
or 24.3±0.2°, 26.2±0.2°, 22.1±0.2°, 18.6±0.2°, 15.1±0.2°, and 12.94±0.2°;
or 20.6±0.2°, 26.2±0.2°, 22.1±0.2°, 18.6±0.2°, 15.1±0.2°, and 12.9±0.2°.

The X-ray powder diffraction pattern of the crystal form D of the sulfate optionally further comprises one or more diffraction peaks at 2θ of 25.9+0.2°, 18.0+0.2°, 25.8+0.2°, 22.9±0.2°, 29.1±0.2°, 6.8±0.2°, and 11.4±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form D of the sulfate has diffraction peaks at 2θ of:

17.3±0.2°, 24.3±0.2°, 20.6±0.2°, 26.2±0.2°, 22.1±0.2°, 18.6±0.2°, 25.9±0.2°, and 18.0±0.2°;
or 17.3±0.2°, 24.3±0.2°, 20.6±0.2°, 26.2±0.2°, 22.1+0.2°, 18.6±0.2°, 25.9±0.2°, 18.0±0.2°, and 25.8±0.2°;
or 17.3±0.2°, 24.3±0.2°, 20.6±0.2°, 26.2±0.2°, 22.1±0.2°, 18.6±0.2°, 25.9±0.2°, 18.0±0.2°, 25.8±0.2°, and 22.9 ±0.2°.

The X-ray powder diffraction pattern of the crystal form D of the sulfate comprises one or more diffraction peaks at 2θ of 17.3±0.2°, 24.3±0.2°, 20.6±0.2°, 26.2±0.2°, 22.1±0.2°, 18.6±0.2°, 15.1±0.2°, 12.9±0.2°, 25.9±0.2°, 18.0±0.2°, 25.8±0.2°, 22.9±0.2°, 29.1±0.2°, 6.8±0.2°, and 11.4±0.2°; and preferably comprises diffraction peaks optionally at 4, 6, 8, or 10 positions selected therefrom.

For example, the X-ray powder diffraction pattern of the crystal form D of the sulfate has diffraction peaks at 2θ of:

17.3±0.2°, 24.3±0.2°, 20.6±0.2°, 26.2±0.2°, 22.1±0.2°, 18.6±0.2°, 15.1±0.2°, and 12.9±0.2°;
or 24.3±0.2°, 20.6±0.2°, 26.2±0.2°, 22.1±0.2°, 18.6±0.2°, 15.1±0.2°, 12.9±0.2°, and 25.9±0.2°;
or 24.3±0.2°, 20.6±0.2°, 26.2±0.2°, 22.1±0.2°, 18.6±0.2°, 15.1±0.2°, 12.9±0.2°, 25.9±0.2°, 18.0±0.2°, and 25.8 ±0.2°.

The X-ray powder diffraction pattern of the crystal form D of the sulfate comprises one or more diffraction peaks at 2θ of 17.3±0.2°, 24.3±0.2°, 20.6±0.2°, 26.2±0.2°, 22.1±0.2°, 18.6±0.2°, 15.1±0.2°, 12.9±0.2°, 25.9±0.2°, 18.0±0.2°, 25.8±0.2°, 22.9±0.2°, 29.1±0.2°, 6.8±0.2°, 11.4±0.2°, 19.5±0.2°, 34.8±0.2°, 24.9±0.2°, 19.2±0.2°, and 26.6±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form D of the sulfate has diffraction peaks at 2θ of:

17.3±0.2°, 24.3±0.2°, 20.6±0.2°, 26.2±0.2°, 22.1±0.2°, 18.6±0.2°, 15.1±0.2°, and 12.9±0.2°;
or 17.3±0.2°, 24.3±0.2°, 26.2±0.2°, 22.1±0.2°, 18.6±0.2°, 15.1±0.2°, 12.9±0.2°, and 25.9±0.2°;
or 24.3±0.2°, 20.6±0.2°, 26.2±0.2°, 22.1±0.2°, 18.6±0.2°, 15.1±0.2°, 12.9±0.2°, and 25.9±0.2°;
or 24.3±0.2°, 20.6±0.2°, 22.1±0.2°, 18.6±0.2°, 15.1±0.2°, 12.9±0.2°, 25.9±0.2°, 18.0±0.2°, 25.8±0.2°, and 22.9 ±0.2°;
or 24.3±0.2°, 22.1±0.2°, 18.6±0.2°, 15.1±0.2°, 25.8±0.2°, 22.9±0.2°, 29.1±0.2°, 6.8±0.2°, 11.4±0.2°, and 19.5 ±0.2°;
or 20.6±0.2°, 26.2±0.2°, 22.1±0.2°, 18.6±0.2°, 15.1±0.2°, 12.9±0.2°, 25.9±0.2°, 18.0±0.2°, 25.8±0.2°, and 22.9 ±0.2°;
or 20.6±0.2°, 18.6±0.2°, 25.9±0.2°, 18.0±0.2°, 6.8±0.2°, 11.4±0.2°, 19.5±0.2°, 34.8±0.2°, 24.9±0.2°, and 19.2 ±40.2°.

Most preferably, its X-ray characteristic diffraction peaks represented by a 2θ angle and an interplanar spacing d using Cu-Kα radiation are as shown in Table 8.

Table 8

| Serial No. | XRPD data of crystal form D of sulfate | | | |
|---|---|---|---|---|
| | 2θ (±0.2°) | d value | Peak height | Ratio (I%) |
| 1 | 6.843 | 12.9065 | 172 | 20.5 |
| 2 | 11.361 | 7.7824 | 131 | 15.6 |
| 3 | 12.918 | 6.8473 | 309 | 36.9 |
| 5 | 13.931 | 6.3517 | 88 | 10.5 |
| 6 | 15.051 | 5.8816 | 372 | 44.4 |
| 7 | 17.274 | 5.1293 | 838 | 100 |
| 8 | 17.970 | 4.9322 | 233 | 27.8 |

(continued)

| Serial No. | XRPD data of crystal form D of sulfate | | | |
|---|---|---|---|---|
| | 2θ (±0.2°) | d value | Peak height | Ratio (I%) |
| 9 | 18.616 | 4.7624 | 409 | 48.8 |
| 10 | 19.227 | 4.6125 | 94 | 11.2 |
| 11 | 19.526 | 4.5425 | 119 | 14.2 |
| 12 | 20.605 | 4.307 | 645 | 77 |
| 13 | 22.062 | 4.0257 | 438 | 52.3 |
| 14 | 22.930 | 3.8752 | 198 | 23.6 |
| 15 | 24.296 | 3.6603 | 746 | 89 |
| 16 | 24.943 | 3.5669 | 104 | 12.4 |
| 17 | 25.794 | 3.4511 | 205 | 24.5 |
| 18 | 25.938 | 3.4323 | 284 | 33.9 |
| 19 | 26.220 | 3.396 | 485 | 57.9 |
| 20 | 26.604 | 3.3478 | 94 | 11.2 |
| 21 | 27.560 | 3.2339 | 88 | 10.5 |
| 22 | 29.098 | 3.0663 | 181 | 21.6 |

Further preferably, the X-ray powder diffraction pattern of the crystal form D of the sulfate is substantially as shown in FIG. 26; its DSC pattern is substantially as shown in FIG. 27; and its TGA pattern is substantially as shown in FIG. 28.

A further preferred embodiment of the present invention provides that an X-ray powder diffraction pattern of a crystal form E of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide comprises at least one or more diffraction peaks at 2θ of 5.8±0.2°, 17.2±0.2°, and 9.8±0.2°, preferably comprises 2 diffraction peaks selected therefrom, more preferably comprises 3 diffraction peaks selected therefrom; optionally, further may comprise at least one diffraction peak at 2θ of 13.8±0.2°, 20.0±0.2°, 22.6±0.2°, 19.2±0.2°, and 22.2±0.2°, and preferably comprises 2, 3, 4, or 5 diffraction peaks selected therefrom.

For example, the X-ray powder diffraction pattern of the crystal form E of the sulfate has diffraction peaks at 2θ of:

5.8±0.2°, 13.8±0.2°, and 22.6±0.2°;
or 17.2±0.2°, 20.0+0.2°, and 19.2±0.2°;
or 9.8±0.2°, 22.6±0.2°, and 22.2±0.2°;
or 5.8±0.2°, 13.8±0.2°, 20.0±0.2°, and 22.6±0.2°;
or 17.2±0.2°, 20.0+0.2°, 22.6±0.2°, and 19.24±0.2°;
or 5.8±0.2°, 13.8±0.2°, 20.0±0.2°, 22.6±0.2°, 19.2±0.2°, and 22.2±0.2°;
or 17.2±0.2°, 13.8±0.2°, 20.0+0.2°, 22.6±0.2°, 19.2±0.2°, and 22.2±0.2°;
or 9.8±0.2°, 13.8±0.2°, 20.0±0.2°, 22.6±0.2°, 19.2±0.2°, and 22.2±0.2°.

The X-ray powder diffraction pattern of the crystal form E of the sulfate optionally further comprises one or more diffraction peaks at 2θ of 11.1±0.2°, 26.2±0.2°, 24.3±0.2°, 20.6±0.2°, 18.6±0.2°, 19.7±0.2°, and 15.1±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form E of the sulfate has diffraction peaks at 2θ of:

5.8±0.2°, 17.2±0.2°, 9.8±0.2°, 13.8±0.2°, 20.0+0.2°, 22.6±0.2°, 11.1±0.2°, and 26.2±0.2°;
or 5.8±0.2°, 17.24±0.2°, 9.8±0.2°, 13.8±0.2°, 20.0+0.2°, 22.6±0.2°, 11.1±0.2°, 26.2±0.2°, and 24.3±0.2°;
or 5.8±0.2°, 17.2±0.2°, 9.8±0.2°, 13.8±0.2°, 20.0±0.2°, 22.6±0.2°, 11.1±0.2°, 26.2±0.2°, 24.3±0.2°, and 20.6 ±0.2°.

The X-ray powder diffraction pattern of the crystal form E of the sulfate comprises one or more diffraction peaks at

20 of 5.8±0.2°, 17.2±0.2°, 9.8±0.2°, 13.8±0.2°, 20.0+0.2°, 22.6±0.2°, 19.2±0.2°, 22.2±0.2°, 11.1±0.2°, 26.2±0.2°, 24.3±0.2°, 20.6±0.2°, 18.6±0.2°, 19.7±0.2°, and 15.1±0.2°; and preferably comprises diffraction peaks optionally at 4, 6, 8, or 10 positions selected therefrom.

For example, the X-ray powder diffraction pattern of the crystal form E of the sulfate has diffraction peaks at 2θ of:

5.8±0.2°, 17.2±0.2°, 9.8±0.2°, 13.8±0.2°, 20.0+0.2°, 22.6±0.2°, 19.2±0.2°, and 22.2±0.2°;
or 17.2±0.2°, 9.8±0.2°, 13.8±0.2°, 20.0±0.2°, 22.6±0.2°, 19.2±0.2°, 22.2±0.2°, and 11.1±0.2°;
or 5.8±0.2°, 17.2±0.2°, 9.8±0.2°, 13.8±0.2°, 20.0±0.2°, 22.6±0.2°, 19.2±0.2°, 22.2±0.2°, 11.1±0.2°, and 26.2 ±0.2°;
or 17.2±0.2°, 9.8±0.2°, 13.8+0.2°, 20.0+0.2°, 22.6+0.2°, 19.2±0.2°, 22.2±0.2°, 11.1±0.2°, 26.2±0.2°, and 24.3 ±0.2°.

The X-ray powder diffraction pattern of the crystal form E of the sulfate comprises one or more diffraction peaks at 20 of 5.8±0.2°, 17.2±0.2°, 9.8±0.2°, 13.8±0.2°, 20.0+0.2°, 22.6±0.2°, 19.2±0.2°, 22.2±0.2°, 11.1±0.2°, 26.2±0.2°, 24.3±0.2°, 20.6±0.2°, 18.6±0.2°, 19.7±0.2°, 15.1±0.2°, 29.1±0.2°, 12.1±0.2°, 21.3±0.2°, 12.9±0.2°, and 8.8±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form E of the sulfate has diffraction peaks at 2θ of:

5.8±0.2°, 17.2±0.2°, 9.8±0.2°, 13.8±0.2°, 20.0+0.2°, 22.6±0.2°, 19.2±0.2°, and 22.2±0.2°;
or 5.8±0.2°, 17.2±0.2°, 13.8±0.2°, 20.0±0.2°, 22.6±0.2°, 19.2±0.2°, 22.2±0.2°, and 11.1±0.2°;
or 5.8±0.2°, 13.8±0.2°, 20.0±0.2°, 22.2±0.2°, 11.1±0.2°, 26.2±0.2°, 24.3±0.2°, 20.6±0.2°, 18.6±0.2°, and 19.7 ±0.2°;
or 17.2±0.2°, 9.8±0.2°, 13.8+0.2°, 20.0+0.2°, 22.6+0.2°, 19.2±0.2°, 22.2±0.2°, 11.1±0.2°, 26.2±0.2°, and 24.3 ±0.2°;
or 17.2±0.2°, 9.8±0.2°, 20.0±0.2°, 19.2±0.2°, 22.2±0.2°, 26.2±0.2°, 24.3±0.2°, 20.6±0.2°, 18.6±0.2°, and 19.7 ±0.2°;
or 9.8±0.2°, 13.8±0.2°, 20.0±0.2°, 22.6±0.2°, 19.2±0.2°, 22.2±0.2°, 11.1±0.2°, 26.2±0.2°, 24.3±0.2°, and 20.6 ±0.2°;
or 9.8±0.2°, 13.8±0.2°, 19.2±0.2°, 22.2±0.2°, 26.2±0.2°, 24.3±0.2°, 20.6±0.2°, 12.1±0.2°, 21.3±0.2°, and 12.9 ±0.2°.

Most preferably, its X-ray characteristic diffraction peaks represented by a 20 angle and an interplanar spacing d using Cu-Kα radiation are as shown in Table 9.

Table 9

| Serial No. | XRPD data of crystal form E of sulfate | | | |
|---|---|---|---|---|
| | 20 (±0.2°) | d value | Peak height | Ratio (I%) |
| 1 | 5.762 | 15.3244 | 1718 | 100 |
| 2 | 8.790 | 10.0517 | 39 | 2.3 |
| 3 | 9.834 | 8.9864 | 175 | 10.2 |
| 4 | 11.116 | 7.953 | 112 | 6.5 |
| 5 | 11.562 | 7.6475 | 38 | 2.2 |
| 6 | 12.086 | 7.3168 | 54 | 3.1 |
| 7 | 12.860 | 6.8783 | 51 | 3 |
| 8 | 13.809 | 6.4077 | 149 | 8.7 |
| 9 | 15.066 | 5.8756 | 60 | 3.5 |
| 10 | 17.222 | 5.1447 | 570 | 33.2 |
| 11 | 18.618 | 4.7618 | 82 | 4.8 |
| 12 | 19.242 | 4.6088 | 132 | 7.7 |
| 13 | 19.691 | 4.5048 | 80 | 4.7 |

(continued)

| Serial No. | XRPD data of crystal form E of sulfate | | | |
|---|---|---|---|---|
| | 20 (±0.2°) | d value | Peak height | Ratio (I%) |
| 14 | 20.033 | 4.4286 | 143 | 8.3 |
| 15 | 20.600 | 4.308 | 87 | 5.1 |
| 16 | 21.277 | 4.1724 | 53 | 3.1 |
| 17 | 22.208 | 3.9996 | 122 | 7.1 |
| 18 | 22.550 | 3.9397 | 136 | 7.9 |
| 19 | 24.314 | 3.6577 | 89 | 5.2 |
| 20 | 26.178 | 3.4013 | 96 | 5.6 |
| 21 | 29.056 | 3.0707 | 58 | 3.4 |

Further preferably, the X-ray powder diffraction pattern of the crystal form E of the sulfate is substantially as shown in FIG. 29; its DSC pattern is substantially as shown in FIG. 30; and its TGA pattern is substantially as shown in FIG. 31.

A further preferred embodiment of the present invention provides a crystal form F of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 6.0±0.2°, 16.0±0.2°, and 22.4±0.2°, preferably comprises 2 diffraction peaks selected therefrom, more preferably comprises 3 diffraction peaks selected therefrom; optionally, further may comprise at least one diffraction peak at 2θ of 17.3+0.2°, 20.0+0.2°, 18.5±0.2°, 20.5±0.2°, and 14.4±0.2°, and preferably comprises 2, 3, 4, or 5 diffraction peaks selected therefrom.

For example, the X-ray powder diffraction pattern of the crystal form F of the sulfate has diffraction peaks at 20 of:

6.0±0.2°, 17.3±0.2°, and 14.4±0.2°;
or 16.0+0.2°, 20.0+0.2°, and 18.5±0.2°;
or 6.0±0.2°, 17.3±0.2°, 20.0±0.2°, and 18.5±0.2°;
or 16.0+0.2°, 20.0+0.2°, 18.5+0.2°, and 14.4±0.2°;
or 22.4±0.2°, 18.5±0.2°, 20.5±0.2°, and 14.4±0.2°;
or 16.0±0.2°, 17.3±0.2°, 20.0+0.2°, 18.5±0.2°, 20.5±0.2°, and 14.4±0.2°;
or 22.4±0.2°, 17.3±0.2°, 20.0+0.2°, 18.5+0.2°, 20.5±0.2°, and 14.4±0.2°.

The X-ray powder diffraction pattern of the crystal form F of the sulfate optionally further comprises one or more diffraction peaks at 20 of 24.9±0.2°, 24.4+0.2°, 17.5±0.2°, 26.2±0.2°, 18.0±0.2°, 27.5±0.2°, and 21.5±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form F of the sulfate has diffraction peaks at 20 of:

6.0±0.2°, 16.0±0.2°, 22.4±0.2°, 17.3±0.2°, 20.0±0.2°, 18.5±0.2°, 24.9±0.2°, and 24.4±0.2°;
or 6.0±0.2°, 16.0±0.2°, 22.4±0.2°, 17.3±0.2°, 20.0±0.2°, 18.5±0.2°, 24.9±0.2°, 24.4±0.2°, and 17.5±0.2°;
or 6.0±0.2°, 16.0±0.2°, 22.4±0.2°, 17.3±0.2°, 20.0+0.2°, 18.5+0.2°, 24.9±0.2°, 24.4±0.2°, 17.5±0.2°, and 26.2 ±0.2°.

The X-ray powder diffraction pattern of the crystal form F of the sulfate comprises one or more diffraction peaks at 20 of 6.0±0.2°, 16.0±0.2°, 22.4±0.2°, 17.3±0.2°, 20.0+0.2°, 18.5±0.2°, 20.5±0.2°, 14.4±0.2°, 24.9±0.2°, 24.4±0.2°, 17.5±0.2°, 26.24±0.2°, 18.0±0.2°, 27.5±0.2°, and 21.5±0.2°; and preferably comprises diffraction peaks optionally at **4,** 6, 8, or 10 positions selected therefrom.

For example, the X-ray powder diffraction pattern of the crystal form F of the sulfate has diffraction peaks at 20 of:

6.0±0.2°, 16.0±0.2°, 22.4±0.2°, 17.3±0.2°, 20.0±0.2°, 18.5±0.2°, 20.5±0.2°, and 14.4±0.2°;
or 16.0+0.2°, 22.4±0.2°, 17.3±0.2°, 20.0+0.2°, 18.5+0.2°, 20.5±0.2°, 14.4±0.2°, and 24.9±0.2°;
or 6.0±0.2°, 16.0±0.2°, 22.4±0.2°, 17.3±0.2°, 20.0±0.2°, 18.5+0.2°, 20.5±0.2°, 14.4±0.2°, 24.9±0.2°, and 24.4 ±0.2°;

or 16.0±0.2°, 22.4±0.2°, 17.3±0.2°, 20.0±0.2°, 18.5+0.2°, 20.5±0.2°, 14.4±0.2°, 24.9±0.2°, 24.4±0.2°, and 17.5 ±0.2°.

The X-ray powder diffraction pattern of the crystal form F of the sulfate comprises one or more diffraction peaks at 2θ of 6.0±0.2°, 16.0±0.2°, 22.4±0.2°, 17.3±0.2°, 20.0+0.2°, 18.5±0.2°, 20.5±0.2°, 14.4±0.2°, 24.9±0.2°, 24.4±0.2°, 17.5±0.2°, 26.2±0.2°, 18.0+0.2°, 27.5±0.2°, 21.5±0.2°, 12.8±0.2°, 23.2+0.2°, 20.9+0.2°, 30.2+0.2°, and 28.8±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form F of the sulfate has diffraction peaks at 2θ of:

6.0±0.2°, 16.0±0.2°, 22.4±0.2°, 17.3±0.2°, 20.0±0.2°, 18.5±0.2°, 20.5±0.2°, and 14.4±0.2°;
or 6.0±0.2°, 16.0±0.2°, 17.3±0.2°, 20.0±0.2°, 18.5+0.2°, 20.5±0.2°, 14.4±0.2°, and 24.9±0.2°;
or 16.0+0.2°, 22.4±0.2°, 17.3±0.2°, 20.0+0.2°, 18.5±0.2°, 20.5±0.2°, 14.4±0.2°, and 24.9±0.2°;
or 6.0±0.2°, 16.0±0.2°, 22.4±0.2°, 17.3±0.2°, 20.0±0.2°, 18.5+0.2°, 20.5±0.2°, 14.4±0.2°, 24.9±0.2°, and 24.4 ±0.2°;
or 6.0±0.2°, 16.0±0.2°, 17.3±0.2°, 20.0±0.2°, 18.5+0.2°, 20.5±0.2°, 14.4±0.2°, 17.5±0.2°, 26.2±0.2°, and 18.0 ±0.2°;
or 16.0±0.2°, 22.4±0.2°, 17.3±0.2°, 20.0±0.2°, 18.5+0.2°, 20.5±0.2°, 26.2±0.2°, 18.0±0.2°, 27.5±0.2°, and 21.5 ±0.2°;
or 16.0±0.2°, 22.4±0.2°, 17.3±0.2°, 18.5±0.2°, 20.5±0.2°, 24.9±0.2°, 24.4±0.2°, 27.5±0.2°, 21.5±0.2°, and 12.8 ±0.2°.

Most preferably, its X-ray characteristic diffraction peaks represented by a 2θ angle and an interplanar spacing d using Cu-Kα radiation are as shown in Table 10.

Table 10

| Serial No. | XRPD data of crystal form F of sulfate | | | |
|---|---|---|---|---|
| | 2θ (±0.2°) | d value | Peak height | Ratio (I%) |
| 1 | 6.027 | 14.6519 | 648 | 100 |
| 2 | 12.801 | 6.9097 | 76 | 11.7 |
| 3 | 14.398 | 6.1469 | 143 | 22.1 |
| 4 | 15.964 | 5.547 | 317 | 48.9 |
| 5 | 17.263 | 5.1324 | 171 | 26.4 |
| 6 | 17.541 | 5.0517 | 132 | 20.4 |
| 7 | 18.006 | 4.9224 | 93 | 14.4 |
| 8 | 18.517 | 4.7875 | 162 | 25 |
| 9 | 20.015 | 4.4326 | 168 | 25.9 |
| 10 | 20.545 | 4.3194 | 149 | 23 |
| 11 | 20.925 | 4.2418 | 66 | 10.2 |
| 12 | 21.518 | 4.1262 | 82 | 12.7 |
| 13 | 22.364 | 3.972 | 259 | 40 |
| 14 | 22.816 | 3.8944 | 51 | 7.9 |
| 15 | 23.198 | 3.831 | 70 | 10.8 |
| 16 | 24.413 | 3.6431 | 137 | 21.1 |
| 17 | 24.882 | 3.5755 | 143 | 22.1 |
| 18 | 26.242 | 3.3932 | 105 | 16.2 |
| 19 | 27.538 | 3.2363 | 83 | 12.8 |
| 20 | 28.760 | 3.1016 | 57 | 8.8 |
| 21 | 30.191 | 2.9577 | 59 | 9.1 |

(continued)

| Serial No. | XRPD data of crystal form F of sulfate | | | |
|---|---|---|---|---|
| | 2θ (±0.2°) | d value | Peak height | Ratio (I%) |
| 22 | 31.625 | 2.8268 | 56 | 8.6 |

Further preferably, the X-ray powder diffraction pattern of the crystal form F of the sulfate is substantially as shown in FIG. 32; its DSC pattern is substantially as shown in FIG. 33; and its TGA pattern is substantially as shown in FIG. 34.

A further preferred embodiment of the present invention provides a crystal form G of a sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 5.9±0.2°, 16.7±0.2°, and 17.6±0.2°, preferably comprises 2 diffraction peaks selected therefrom, more preferably comprises 3 diffraction peaks selected therefrom; optionally, further may comprise at least one diffraction peak at 2θ of 5.6±0.2°, 16.9±0.2°, 22.2±0.2°, 29.5±0.2°, and 27.7±0.2°, and preferably comprises 2, 3, 4, or 5 diffraction peaks selected therefrom.

For example, the X-ray powder diffraction pattern of the crystal form G of the sulfate has diffraction peaks at 2θ of:

5.9±0.2°, 5.6+0.2°, and 16.9±0.2°;
or 5.9±0.2°, 5.6±0.2°, 16.9±0.2°, and 27.7±0.2°;
or 16.7±0.2°, 16.9±0.2°, 22.2±0.2°, and 29.5+0.2°;
or 5.9±0.2°, 5.6±0.2°, 16.9±0.2°, 22.2±0.2°, 29.5±0.2°, and 27.7±0.2°.

The X-ray powder diffraction pattern of the crystal form G of the sulfate optionally further comprises one or more diffraction peaks at 20 of 25.1+0.2°, 10.2±0.2°, 24.4+0.2°, 8.4±0.2°, 12.1±0.2°, 24.2±0.2°, and 15.4±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form G of the sulfate has diffraction peaks at 2θ of:

5.9±0.2°, 16.7±0.2°, 17.6±0.2°, 5.6±0.2°, 16.9±0.2°, 22.2±0.2°, 25.1±0.2°, and 10.2±0.2°;
or 5.9±0.2°, 16.7±0.2°, 17.6±0.2°, 5.6±0.2°, 16.9±0.2°, 22.2±0.2°, 25.1±0.2°, 10.2±0.2°, and 24.4±0.2°;
or 5.9±0.2°, 16.7±0.2°, 17.6±0.2°, 5.6±0.2°, 16.9±0.2°, 22.2±0.2°, 25.1±0.2°, 10.2±0.2°, 24.4±0.2°, and 8.4 ±0.2°.

The X-ray powder diffraction pattern of the crystal form G of the sulfate comprises one or more diffraction peaks at 20 of 5.9±0.2°, 16.7±0.2°, 17.6±0.2°, 5.6±0.2°, 16.9±0.2°, 22.2±0.2°, 29.5±0.2°, 27.7±0.2°, 25.1±0.2°, 10.2±0.2°, 24.4±0.2°, 8.4±0.2°, 12.1±0.2°, 24.2±0.2°, and 15.4±0.2°; and preferably comprises diffraction peaks optionally at 4, 6, 8, or 10 positions selected therefrom.

For example, the X-ray powder diffraction pattern of the crystal form G of the sulfate has diffraction peaks at 2θ of:

5.9±0.2°, 16.7±0.2°, 17.6±0.2°, 5.6±0.2°, 16.9±0.2°, 22.2±0.2°, 29.5±0.2°, and 27.7±0.2°;
or 16.7±0.2°, 17.6±0.2°, 5.6+0.2°, 16.9±0.2°, 22.2±0.2°, 29.5±0.2°, 27.7±0.2°, and 25.1±0.2°;
or 5.9±0.2°, 16.7±0.2°, 17.6±0.2°, 5.6±0.2°, 16.9±0.2°, 22.2±0.2°, 29.5±0.2°, 27.7±0.2°, 25.1±0.2°, and 10.2 ±0.2°;
or 16.7±0.2°, 17.6±0.2°, 5.6±0.2°, 16.9±0.2°, 22.2±0.2°, 29.5±0.2°, 27.7±0.2°, 25.1±0.2°, 10.2±0.2°, and 24.4 ±0.2°.

The X-ray powder diffraction pattern of the crystal form G of the sulfate comprises one or more diffraction peaks at 20 of 5.9±0.2°, 16.7±0.2°, 17.6±0.2°, 5.6±0.2°, 16.9±0.2°, 22.2±0.2°, 29.5±0.2°, 27.7±0.2°, 25.1±0.2°, 10.2±0.2°, 24.4±0.2°, 8.4±0.2°, 12.1±0.2°, 24.2±0.2°, 15.4±0.2°, 19.6±0.2°, 18.8±0.2°, 23.5±0.2°, 13.1±0.2°, and 20.3±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form G of the sulfate has diffraction peaks at 2θ of:

5.9±0.2°, 16.7±0.2°, 17.6±0.2°, 5.6±0.2°, 16.9±0.2°, 22.2±0.2°, 29.5±0.2°, and 27.7±0.2°;
or 5.9±0.2°, 16.7±0.2°, 5.6±0.2°, 16.9±0.2°, 22.2±0.2°, 29.5±0.2°, 27.7±0.2°, and 25.1±0.2°;
or 16.7±0.2°, 17.6±0.2°, 5.6+0.2°, 16.9±0.2°, 22.2±0.2°, 29.5±0.2°, 27.7±0.2°, and 25.1±0.2°;
or 17.6±0.2°, 5.6±0.2°, 16.9±0.2°, 22.2±0.2°, 29.5±0.2°, 27.7±0.2°, 25.1±0.2°, 10.2±0.2°, 24.4±0.2°, and 8.4±0.2°;
or 17.6±0.2°, 22.2±0.2°, 29.5±0.2°, 27.7±0.2°, 24.4±0.2°, 8.4±0.2°, 19.6+0.2°, 18.8±0.2°, 23.5±0.2°, and 13.1 ±0.2°.

Most preferably, its X-ray characteristic diffraction peaks represented by a 2θ angle and an interplanar spacing d using Cu-Kα radiation are as shown in Table 11.

Table 11

| Serial No. | XRPD data of crystal form G of sulfate | | | |
|---|---|---|---|---|
| | 2θ (±0.2°) | d value | Peak height | Ratio (I%) |
| 1 | 5.561 | 15.8778 | 611 | 22.7 |
| 2 | 5.865 | 15.0571 | 2696 | 100 |
| 3 | 8.357 | 10.5711 | 150 | 5.6 |
| 4 | 10.180 | 8.6818 | 168 | 6.2 |
| 5 | 12.090 | 7.3145 | 151 | 5.6 |
| 6 | 13.086 | 6.7599 | 129 | 4.8 |
| 7 | 15.416 | 5.743 | 146 | 5.4 |
| 8 | 16.651 | 5.3197 | 970 | 36 |
| 9 | 16.897 | 5.243 | 542 | 20.1 |
| 10 | 17.565 | 5.045 | 674 | 25 |
| 11 | 18.840 | 4.7064 | 144 | 5.3 |
| 12 | 19.634 | 4.5176 | 145 | 5.4 |
| 13 | 20.322 | 4.3662 | 127 | 4.7 |
| 14 | 22.166 | 4.0071 | 389 | 14.4 |
| 15 | 23.503 | 3.782 | 135 | 5 |
| 16 | 24.174 | 3.6785 | 148 | 5.5 |
| 17 | 24.375 | 3.6487 | 165 | 6.1 |
| 18 | 25.059 | 3.5506 | 183 | 6.8 |
| 19 | 26.542 | 3.3555 | 121 | 4.5 |
| 20 | 27.722 | 3.2153 | 192 | 7.1 |
| 21 | 29.483 | 3.0272 | 193 | 7.2 |
| 22 | 33.680 | 2.6589 | 112 | 4.2 |

Further preferably, the X-ray powder diffraction pattern of the crystal form G of the sulfate is substantially as shown in FIG. 35; its DSC pattern is substantially as shown in FIG. 36; and its TGA pattern is substantially as shown in FIG. 37.

A further preferred embodiment of the present invention provides a crystal form A of a hydrochloride of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 2θ of 22.4±0.2°, 14.0±0.2°, and 17.1±0.2°, preferably comprises 2 diffraction peaks selected therefrom, more preferably comprises 3 diffraction peaks selected therefrom; optionally, further may comprise at least one diffraction peak at 2θ of 6.2±0.2°, 19.4±0.2°, 25.2±0.2°, 17.5±0.2°, and 21.6±0.2°, and preferably comprises 2, 3, 4, or 5 diffraction peaks selected therefrom.

For example, the X-ray powder diffraction pattern of the crystal form A of the hydrochloride has diffraction peaks at 2θ of:

22.4±0.2° and 6.2±0.2°;
or 22.4±0.2°, 6.2±0.2°, and 19.4±0.2°;

or 22.4±0.2°, 6.2±0.2°, 19.4±0.2°, and 25.2±0.2°;
or 14.0±0.2°, 19.4±0.2°, 25.2±0.2°, and 17.5±0.2°;
or 22.4±0.2°, 6.2±0.2°, 19.4±0.2°, 25.2±0.2°, 17.5±0.2°, and 21.6±0.2°;
or 17.1±0.2°, 6.24±0.2°, 19.4±0.2°, 25.2±0.2°, 17.5±0.2°, and 21.6±0.2°.

The X-ray powder diffraction pattern of the crystal form A of the hydrochloride optionally further comprises one or more diffraction peaks at 2θ of 19.8±0.2°, 23.4+0.2°, 10.6±0.2°, 30.5+0.2°, 12.4±0.2°, 9.8±0.2°, and 11.1±0.2°; preferably comprises at least any 2-3, or 4-5, or 6-7 diffraction peaks selected therefrom, and further preferably comprises any 2, 3, 4, 6, or 7 diffraction peaks selected therefrom;
for example, the X-ray powder diffraction pattern of the crystal form A of the hydrochloride has diffraction peaks at 2θ of:

22.4±0.2°, 14.0±0.2°, 17.1±0.2°, 6.2±0.2°, 19.4±0.2°, 25.2±0.2°, 19.8±0.2°, and 23.4±0.2°;
or 22.4±0.2°, 14.0±0.2°, 17.1±0.2°, 6.2±0.2°, 19.4±0.2°, 25.2±0.2°, 19.8±0.2°, 23.4±0.2°, 10.6±0.2°, and 30.5 ±0.2°.

The X-ray powder diffraction pattern of the crystal form A of the hydrochloride comprises one or more diffraction peaks at 2θ of 22.4±0.2°, 14.0±0.2°, 17.1±0.2°, 6.2±0.2°, 19.4±0.2°, 25.2±0.2°, 17.5±0.2°, 21.6±0.2°, 19.8±0.2°, 23.4±0.2°, 10.6±0.2°, 30.5±0.2°, 12.4±0.2°, 9.8±0.2°, and 11.1±0.2°; and preferably comprises diffraction peaks optionally at 4, 6, 8, or 10 positions selected therefrom.

For example, the X-ray powder diffraction pattern of the crystal form A of the hydrochloride has diffraction peaks at 2θ of:

22.4±0.2°, 14.0±0.2°, 17.1±0.2°, 6.2±0.2°, 19.4±0.2°, 25.2±0.2°, 17.5±0.2°, and 21.6±0.2°;
or 14.0±0.2°, 17.1±0.2°, 6.2±0.2°, 19.4±0.2°, 25.2±0.2°, 17.5±0.2°, 21.6±0.2°, and 19.8±0.2°;
or 22.4±0.2°, 14.0±0.2°, 17.1±0.2°, 6.2±0.2°, 19.4±0.2°, 25.2±0.2°, 17.5±0.2°, 21.6±0.2°, 19.8±0.2°, and 23.4 ±0.2°.

The X-ray powder diffraction pattern of the crystal form A of the hydrochloride comprises one or more diffraction peaks at 2θ of 22.4±0.2°,14.0±0.2°,17.1±0.2°,6.2±0.2°, 19.4±0.2°, 25.2±0.2°, 17.5±0.2°, 21.6±0.2°, 19.8±0.2°, 23.4 ±0.2°, 10.6±0.2°, 30.5±0.2°, 12.4±0.2°, 9.8±0.2°, 11.1±0.2°, 24.5±0.2°, 26.6±0.2°, 26.9±0.2°, 31.4+0.2°, and 35.2 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the hydrochloride has diffraction peaks at 2θ of:

22.4±0.2°, 14.0±0.2°, 17.1±0.2°, 6.2±0.2°, 19.4±0.2°, 25.2±0.2°, 17.5±0.2°, and 21.6±0.2°;
or 22.4±0.2°, 14.0+0.2°, 6.2+0.2°, 19.4±0.2°, 25.2±0.2°, 17.5±0.2°, 21.6±0.2°, and 19.8±0.2°;
or 22.4±0.2°, 14.0±0.2°, 17.1±0.2°, 6.2±0.2°, 19.4±0.2°, 25.2±0.2°, 26.6±0.2°, 26.9±0.2°, 31.4±0.2°, and 35.2 ±0.2°.

Most preferably, its X-ray characteristic diffraction peaks represented by a 2θ angle and an interplanar spacing d using Cu-Kα radiation are as shown in Table 12.

Table 12

| Serial No. | XRPD data of crystal form A of hydrochloride | | | |
|---|---|---|---|---|
| | 2θ (±0.2°) | d value | Peak height | Ratio (I%) |
| 1 | 6.208 | 14.225 | 302 | 52.3 |
| 2 | 9.815 | 9.0039 | 79 | 13.7 |
| 3 | 10.589 | 8.3475 | 127 | 22 |
| 4 | 11.113 | 7.955 | 77 | 13.3 |
| 5 | 12.414 | 7.1242 | 88 | 15.3 |
| 6 | 14.035 | 6.3049 | 541 | 93.8 |
| 7 | 17.096 | 5.1822 | 393 | 68.1 |

(continued)

| Serial No. | XRPD data of crystal form A of hydrochloride | | | |
|---|---|---|---|---|
| | 2θ (±0.2°) | d value | Peak height | Ratio (I%) |
| 8 | 17.460 | 5.0751 | 180 | 31.2 |
| 9 | 18.781 | 4.721 | 51 | 8.8 |
| 10 | 19.449 | 4.5602 | 214 | 37.1 |
| 11 | 19.751 | 4.4913 | 149 | 25.8 |
| 12 | 21.597 | 4.1114 | 159 | 27.6 |
| 13 | 22.388 | 3.9678 | 577 | 100 |
| 14 | 23.427 | 3.7941 | 133 | 23.1 |
| 15 | 24.545 | 3.6239 | 72 | 12.5 |
| 16 | 25.205 | 3.5304 | 182 | 31.5 |
| 17 | 26.605 | 3.3478 | 69 | 12 |
| 18 | 26.885 | 3.3135 | 57 | 9.9 |
| 19 | 28.373 | 3.143 | 47 | 8.1 |
| 20 | 30.536 | 2.9251 | 111 | 19.2 |
| 21 | 31.365 | 2.8497 | 52 | 9 |
| 22 | 35.195 | 2.5478 | 52 | 9 |

Further preferably, the X-ray powder diffraction pattern of the crystal form A of the hydrochloride is substantially as shown in FIG. 12; its DSC pattern is substantially as shown in FIG. 13; and its TGA pattern is substantially as shown in FIG. 14.

A further preferred embodiment of the present invention provides a crystal form B of a hydrochloride of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 6.7±0.2°, 27.0±0.2°, and 23.4±0.2°, preferably comprises 2 diffraction peaks selected therefrom, more preferably comprises 3 diffraction peaks selected therefrom; optionally, further may comprise at least one diffraction peak at 2θ of 13.4±0.2°, 11.0±0.2°, 24.1±0.2°, 15.6±0.2°, and 4.5 ±0.2°, and preferably comprises 2, 3, 4, or 5 diffraction peaks selected therefrom.

For example, the X-ray powder diffraction pattern of the crystal form B of the hydrochloride has diffraction peaks at 20 of:

6.7±0.2° and 13.4±0.2°;
or 6.7±0.2°, 13.4±0.2°, and 1.0±0.2°;
or 6.7±0.2°, 13.4±0.2°, 11.0±0.2°, and 24.1±0.2°;
or 6.7±0.2°, 13.4±0.2°, 11.0±0.2°, 24.1±0.2°, 15.6±0.2°, and 4.5±0.2°;
or 27.0+0.2°, 13.4±0.2°, 11.0+0.2°, 24.1±0.2°, 15.6±0.2°, and 4.5±0.2°.

The X-ray powder diffraction pattern of the crystal form B of the hydrochloride optionally further comprises one or more diffraction peaks at 2θ of 20.0±0.2°, 10.2±0.2°, 14.3±0.2°, 10.0±0.2°, 20.5±0.2°, 23.0±0.2°, and 31.0±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the hydrochloride has diffraction peaks at 20 of:

6.7±0.2°, 27.0+0.2°, 23.4±0.2°, 13.4±0.2°, 11.0±0.2°, 24.1±0.2°, 20.0±0.2°, and 10.2±0.2°;
or 6.7±0.2°, 27.0±0.2°, 23.4±0.2°, 13.4±0.2°, 11.0±0.2°, 24.1±0.2°, 20.0±0.2°, 10.2±0.2°, 14.3±0.2°, and 10.0 ±0.2°.

The X-ray powder diffraction pattern of the crystal form B of the hydrochloride comprises one or more diffraction

peaks at 2θ of 6.7±0.2°, 27.0+0.2°, 23.4±0.2°, 13.4±0.2°, 11.0±0.2°, 24.1±0.2°, 15.6±0.2°, 4.5±0.2°, 20.0±0.2°, 10.2 ±0.2°, 14.3±0.2°, 10.0+0.2°, 20.5±0.2°, 23.0+0.2°, and 31.0±0.2°; and preferably comprises diffraction peaks optionally at 4, 6, 8, or 10 positions selected therefrom.

For example, the X-ray powder diffraction pattern of the crystal form B of the hydrochloride has diffraction peaks at 20 of:

6.7±0.2°, 27.0+0.2°, 23.4±0.2°, 13.4±0.2°, 11.0±0.2°, 24.1±0.2°, 15.6±0.2°, and 4.5±0.2°;
or 27.0+0.2°, 23.4±0.2°, 13.4±0.2°, 11.0±0.2°, 24.1±0.2°, 15.6±0.2°, 4.5±0.2°, and 20.0±0.2°;
or 6.7±0.2°, 27.0±0.2°, 23.4±0.2°, 13.4±0.2°, 11.0±0.2°, 24.1±0.2°, 15.6±0.2°, 4.5±0.2°, 20.0±0.2°, and 10.2 ±0.2°.

The X-ray powder diffraction pattern of the crystal form B of the hydrochloride comprises one or more diffraction peaks at 2θ of 6.7±0.2°, 27.0+0.2°, 23.4±0.2°, 13.4±0.2°, 11.0±0.2°, 24.1±0.2°, 15.6±0.2°, 4.5±0.2°, 20.0±0.2°, 10.2 ±0.2°, 14.3±0.2°, 10.0+0.2°, 20.5±0.2°, 23.0+0.2°, 31.0+0.2°, 25.4+0.2°, 30.2±0.2°, 22.3±0.2°, 18.6±0.2°, and 17.4 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom .

For example, the X-ray powder diffraction pattern of the crystal form B of the hydrochloride has diffraction peaks at 20 of:

6.7±0.2°, 27.0+0.2°, 23.4±0.2°, 13.4±0.2°, 11.0±0.2°, 24.1±0.2°, 15.6±0.2°, and 4.5±0.2°;
or 6.7±0.2°, 27.0±0.2°, 13.4±0.2°, 11.0±0.2°, 24.1±0.2°, 15.6±0.2°, 4.5±0.2°, and 20.0±0.2°;
or 6.7±0.2°, 27.0±0.2°, 23.4±0.2°, 13.4±0.2°, 11.0±0.2°, 24.1±0.2°, 15.6±0.2°, 4.5±0.2°, 20.0±0.2°, and 10.2 ±0.2°.

Most preferably, its X-ray characteristic diffraction peaks represented by a 20 angle and an interplanar spacing d using Cu-Kα radiation are as shown in Table 13.

Table 13

| Serial No. | XRPD data of crystal form B of hydrochloride | | | |
|---|---|---|---|---|
| | 20 (±0.2°) | d value | Peak height | Ratio (I%) |
| 1 | 4.485 | 19.6876 | 107 | 22 |
| 2 | 6.734 | 13.1148 | 487 | 100 |
| 3 | 10.015 | 8.8245 | 84 | 17.2 |
| 4 | 10.244 | 8.628 | 98 | 20.1 |
| 5 | 10.952 | 8.0718 | 141 | 29 |
| 6 | 13.409 | 6.5978 | 159 | 32.6 |
| 7 | 14.303 | 6.1873 | 97 | 19.9 |
| 8 | 15.602 | 5.6751 | 108 | 22.2 |
| 9 | 17.449 | 5.0782 | 52 | 10.7 |
| 10 | 18.598 | 4.767 | 55 | 11.3 |
| 11 | 20.015 | 4.4326 | 101 | 20.7 |
| 12 | 20.521 | 4.3245 | 81 | 16.6 |
| 13 | 22.270 | 3.9886 | 62 | 12.7 |
| 14 | 22.976 | 3.8676 | 81 | 16.6 |
| 15 | 23.402 | 3.7982 | 160 | 32.9 |
| 16 | 24.116 | 3.6873 | 116 | 23.8 |
| 17 | 25.407 | 3.5027 | 65 | 13.3 |
| 18 | 27.011 | 3.2983 | 237 | 48.7 |

(continued)

| Serial No. | XRPD data of crystal form B of hydrochloride | | | |
|---|---|---|---|---|
| | 2θ (±0.2°) | d value | Peak height | Ratio (I%) |
| 19 | 28.456 | 3.134 | 49 | 10.1 |
| 20 | 30.156 | 2.9611 | 63 | 12.9 |
| 21 | 30.963 | 2.8857 | 77 | 15.8 |
| 22 | 37.670 | 2.3859 | 48 | 9.9 |

Further preferably, the X-ray powder diffraction pattern of the crystal form B of the hydrochloride is substantially as shown in FIG. 15.

A further preferred embodiment of the present invention provides a crystal form C of a hydrochloride of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 2θ of 16.5±0.2°, 20.4±0.2°, and 22.2±40.2°, preferably comprises 2 diffraction peaks selected therefrom, more preferably comprises 3 diffraction peaks selected therefrom; optionally, further may comprise at least one diffraction peak at 20 of 9.7±0.2°, 17.8±0.2°, 5.3±0.2°, 17.5±0.2°, and 6.0 ±0.2°, and preferably comprises 2, 3, 4, or 5 diffraction peaks selected therefrom.

For example, the X-ray powder diffraction pattern of the crystal form C of the hydrochloride has diffraction peaks at 20 of:

16.5±0.2° and 9.7±0.2°;
or 16.5±0.2°, 9.7±0.2°, and 17.8±0.2°;
or 16.5±0.2°, 17.8±0.2°, 5.3±0.2°, and 6.0±0.2°;
or 16.5±0.2°, 9.7±0.2°, 17.8±0.2°, 5.3±0.2°, 17.5±0.2°, and 6.0±0.2°;
or 20.4±0.2°, 9.7±0.2°, 17.8±0.2°, 5.3±0.2°, 17.5±0.2°, and 6.0±0.2°;
or 22.210.2°, 9.710.2°, 17.8±0.2°, 5.3±0.2°, 17.5±0.2°, and 6.0±0.2°.

The X-ray powder diffraction pattern of the crystal form C of the hydrochloride optionally further comprises one or more diffraction peaks at 2θ of 14.3±0.2°, 21.7±0.2°, 24.6±0.2°, 10.9±0.2°, 27.2±0.2°, 20.8±0.2°, and 19.7±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form C of the hydrochloride has diffraction peaks at 20 of:

16.5±0.2°, 20.4±0.2°, 22.2±0.2°, 9.7±0.2°, 17.8±0.2°, 5.3±0.2°, 14.3±0.2°, and 21.7±0.2°;
or 16.5±0.2°, 20.4±0.2°, 22.2±0.2°, 9.7±0.2°, 17.8±0.2°, 5.3±0.2°, 14.3±0.2°, 21.7±0.2°, 24.6±0.2°, and 10.9 ±0.2°.

The X-ray powder diffraction pattern of the crystal form C of the hydrochloride comprises one or more diffraction peaks at 20 of 16.5±0.2°, 20.4±0.2°, 22.2±0.2°, 9.7±0.2°, 17.8±0.2°, 5.3±0.2°, 17.5±0.2°, 6.0±0.2°, 14.3±0.2°, 21.7 ±0.2°, 24.6±0.2°, 10.9±0.2°, 27.2±0.2°, 20.8±0.2°, and 19.7±0.2°; and preferably comprises diffraction peaks optionally at 4, 6, 8, or 10 positions selected therefrom.

For example, the X-ray powder diffraction pattern of the crystal form C of the hydrochloride has diffraction peaks at 20 of:

16.5±0.2°, 20.4±0.2°, 22.2±0.2°,9.7±0.2°, 17.8±0.2°, 5.3±0.2°, 17.5±0.2°, and 6.0±0.2°;
or 20.4±0.2°, 22.2±0.2°, 9.7±0.2°, 17.8±0.2°, 5.3±0.2°, 17.5±0.2°, 6.0±0.2°, and 14.3±0.2°;
or 16.510.2°, 20.410.2°, 22.2±0.2°, 9.710.2°, 17.8±0.2°, 5.3±0.2°, 17.5±0.2°, 6.0+0.2°, 14.3±0.2°, and 21.7±0.2°.

The X-ray powder diffraction pattern of the crystal form C of the hydrochloride comprises one or more diffraction peaks at 20 of 16.5±0.2°, 20.4±0.2°, 22.2±0.2°, 9.7±0.2°, 17.8±0.2°, 5.3±0.2°, 17.5±0.2°, 6.0±0.2°, 14.3±0.2°, 21.7 ±0.2°, 24.6±0.2°, 10.9±0.2°, 27.2±0.2°, 20.8+0.2°, 19.7±0.2°, 15.6±0.2°, 26.0±0.2°, 18.8±0.2°, 30.7±0.2°, and 10.5 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form C of the hydrochloride has diffraction peaks at 20 of:

16.5±0.2°, 20.4±0.2°, 22.2±0.2°,9.7±0.2°, 17.8±0.2°, 5.3±0.2°, 17.5±0.2°, and 6.0±0.2°;
or 16.5±0.2°, 20.4±0.2°, 9.7+0.2°, 17.8±0.2°, 5.3±0.2°, 17.5±0.2°, 6.0±0.2°, and 14.3±0.2°;
or 20.4±0.2°, 22.2±0.2°, 9.7±0.2°, 17.8±0.2°, 5.3±0.2°, 17.5±0.2°, 6.0±0.2°, and 14.3±0.2°;
or 16.510.2°, 20.410.2°, 22.2±0.2°, 9.710.2°, 17.8±0.2°, 5.3±0.2°, 17.5±0.2°, 6.0+0.2°, 14.3±0.2°, and 21.7±0.2°.

Most preferably, its X-ray characteristic diffraction peaks represented by a 20 angle and an interplanar spacing d using Cu-Kα radiation are as shown in Table 14.

Table 14

| Serial No. | XRPD data of crystal form C of hydrochloride | | | |
|---|---|---|---|---|
| | 2θ (±0.2°) | d value | Peak height | Ratio (I%) |
| 1 | 5.278 | 16.7298 | 198 | 39.9 |
| 2 | 6.048 | 14.6018 | 187 | 37.7 |
| 3 | 9.660 | 9.1485 | 220 | 44.4 |
| 4 | 10.522 | 8.4008 | 70 | 14.1 |
| 5 | 10.916 | 8.0986 | 98 | 19.8 |
| 6 | 11.944 | 7.4038 | 67 | 13.5 |
| 7 | 12.347 | 7.1627 | 70 | 14.1 |
| 8 | 14.340 | 6.1714 | 130 | 26.2 |
| 9 | 15.637 | 5.6624 | 88 | 17.7 |
| 10 | 16.486 | 5.3725 | 496 | 100 |
| 11 | 17.519 | 5.058 | 189 | 38.1 |
| 12 | 17.803 | 4.978 | 216 | 43.5 |
| 13 | 18.822 | 4.7107 | 84 | 16.9 |
| 14 | 19.746 | 4.4923 | 89 | 17.9 |
| 15 | 20.381 | 4.3538 | 386 | 77.8 |
| 16 | 20.769 | 4.2733 | 95 | 19.2 |
| 17 | 21.675 | 4.0967 | 123 | 24.8 |
| 18 | 22.227 | 3.9963 | 279 | 56.3 |
| 19 | 24.598 | 3.6161 | 119 | 24 |
| 20 | 26.016 | 3.4221 | 86 | 17.3 |
| 21 | 27.236 | 3.2716 | 98 | 19.8 |
| 22 | 30.698 | 2.9101 | 76 | 15.3 |

Further preferably, the X-ray powder diffraction pattern of the crystal form C of the hydrochloride is substantially as shown in FIG. 16.

A further preferred embodiment of the present invention provides a crystal form A of a p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide, wherein the number of acids is 1, and its X-ray powder diffraction pattern comprises at least one or more diffraction peaks at 20 of 16.8±0.2°, 19.9±0.2°, and 5.7±0.2°, preferably comprises 2 diffraction peaks selected therefrom, more preferably comprises 3 diffraction peaks selected therefrom; optionally, further may comprise at least one diffraction peak at 20 of 22.5±0.2°, 21.8±0.2°, 24.9±0.2°, 22.3±0.2°, and 20.8±0.2°, and preferably comprises 2, 3, 4, or 5 diffraction peaks selected therefrom.

For example, the X-ray powder diffraction pattern of the crystal form A of the p-tosylate has diffraction peaks at 20 of:

16.8±0.2° and 22.5±0.2°;
or 16.8±0.2°, 22.5±0.2°, and 21.8±0.2°;
or 16.8±0.2°, 22.5±0.2°, 21.8±0.2°, and 24.9±0.2°;
or 19.9±0.2°, 24.9±0.2°, 22.3±0.2°, and 20.8±0.2°;
or 16.8±0.2°, 22.5±0.2°, 21.8±0.2°, 24.9±0.2°, 22.3±0.2°, and 20.8±0.2°;
or 19.9±0.2°, 22.5±0.2°, 21.8±0.2°, 24.9±0.2°, 22.3±0.2°, and 20.8±0.2°;
or 5.7±0.2°, 22.5±0.2°, 21.8±0.2°, 24.9±0.2°, 22.3±0.2°, and 20.8±0.2°.

The X-ray powder diffraction pattern of the crystal form A of the p-tosylate optionally further comprises one or more diffraction peaks at 2θ of 26.6±0.2°, 12.4±0.2°, 15.1±0.2°, 13.8±0.2°, 21.3±0.2°, 27.7±0.2°, and 20.5±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the p-tosylate has diffraction peaks at 20 of:

16.8±0.2°, 19.9±0.2°, 5.7±0.2°,22.5±0.2°, 21.8±0.2°, 24.9±0.2°, 26.6±0.2°, and 12.4±0.2°;
or 16.8±0.2°, 19.9±0.2°, 5.7±0.2°, 22.5±0.2°, 21.8±0.2°, 24.9±0.2°, 26.6±0.2°, 12.4±0.2°, 15.1±0.2°, and 13.8 ±0.2°.

The X-ray powder diffraction pattern of the crystal form A of the p-tosylate comprises one or more diffraction peaks at 20 of 16.8±0.2°, 19.9±0.2°, 5.7±0.2°, 22.5±0.2°, 21.8±0.2°, 24.9±0.2°, 22.3±0.2°, 20.8±0.2°, 26.6±0.2°, 12.4±0.2°, 15.1±0.2°, 13.8±0.2°, 21.3±0.2°, 27.7±0.2°, and 20.5±0.2°; and preferably comprises diffraction peaks optionally at 4, 6, 8, or 10 positions selected therefrom.

For example, the X-ray powder diffraction pattern of the crystal form A of the p-tosylate has diffraction peaks at 20 of:

16.8±0.2°, 19.9±0.2°, 5.7±0.2°, 22.5±0.2°, 21.8±0.2°, 24.9±0.2°, 22.3±0.2°, and 20.8±0.2°;
or 19.9±0.2°, 5.7±0.2°, 22.5+0.2°, 21.8±0.2°, 24.9±0.2°, 22.3±0.2°, 20.8±0.2°, and 26.6±0.2°;
or 16.8±0.2°, 19.9±0.2°, 5.7±0.2°, 22.5±0.2°, 21.8±0.2°, 24.9±0.2°, 22.3±0.2°, 20.8±0.2°, 26.6±0.2°, and 12.4 ±0.2°;
or 19.9±0.2°, 5.7±0.2°, 22.5±0.2°, 21.8±0.2°, 24.9±0.2°, 22.3±0.2°, 20.8±0.2°, 26.6±0.2°, 12.4±0.2°, and 15.1 ±0.2°.

The X-ray powder diffraction pattern of the crystal form A of the p-tosylate comprises one or more diffraction peaks at 20 of 16.8±0.2°, 19.9±0.2°, 5.7±0.2°, 22.5±0.2°, 21.8±0.2°, 24.9±0.2°, 22.3±0.2°, 20.8±0.2°, 26.6±0.2°, 12.4±0.2°, 15.1±0.2°, 13.8±0.2°, 21.3±0.2°, 27.7±0.2°, 20.5±0.2°, 24.7+0.2°, 31.2±0.2°, 17.3±0.2°, 32.1±0.2°, and 25.8±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the p-tosylate has diffraction peaks at 20 of:

16.8±0.2°, 19.9±0.2°, 5.7±0.2°, 22.5±0.2°, 21.8±0.2°, 24.9±0.2°, 22.3±0.2°, and 20.8±0.2°;
16.8±0.2°, 19.9±0.2°, 5.7±0.2°, 12.4±0.2°, 13.8+0.2°, 22.5±0.2°, 21.8±0.2°, and 20.8±0.2°;
or 16.8±0.2°, 19.9±0.2°, 22.5±0.2°, 21.8±0.2°, 24.9+0.2°, 22.3±0.2°, 20.8±0.2°, and 26.6±0.2°;
or 19.9±0.2°, 5.7±0.2°, 22.5+0.2°, 21.8±0.2°, 24.9±0.2°, 22.3±0.2°, 20.8±0.2°, and 26.6±0.2°;
or 19.9±0.2°, 21.8±0.2°, 24.9±0.2°, 22.3±0.2°, 20.8±0.2°, 26.6±0.2°, 32.1±0.2°, and 25.8±0.2°;
or 16.8±0.2°, 19.9±0.2°, 5.7±0.2°, 22.5±0.2°, 21.8±0.2°, 24.9±0.2°, 22.3±0.2°, 20.8±0.2°, 26.6±0.2°, and 12.4 ±0.2°;
or 16.8±0.2°, 19.9±0.2°, 22.5±0.2°, 21.8±0.2°, 24.9±0.2°, 22.3±0.2°, 20.8±0.2°, 26.6±0.2°, 12.4±0.2°, and 15.1 ±0.2°;
or 16.8±0.2°, 19.9±0.2°, 24.9±0.2°, 22.3±0.2°, 20.8±0.2°, 26.6±0.2°, 31.2±0.2°, 17.3±0.2°, 32.1±0.2°, and 25.8 ±0.2°.

Most preferably, its X-ray characteristic diffraction peaks represented by a 20 angle and an interplanar spacing d using Cu-Kα radiation are as shown in Table 15.

Table 15

| Serial No. | XRPD data of crystal form A of p-tosylate | | | |
|---|---|---|---|---|
| | 20 (±0.2°) | d value | Peak height | Ratio (I%) |
| 1 | 5.678 | 15.5517 | 823 | 55.8 |
| 2 | 11.155 | 7.9255 | 141 | 9.6 |
| 3 | 12.398 | 7.1335 | 271 | 18.4 |
| 4 | 13.345 | 6.6291 | 139 | 9.4 |
| 5 | 13.773 | 6.4243 | 245 | 16.6 |
| 6 | 15.147 | 5.8443 | 257 | 17.4 |
| 7 | 16.832 | 5.263 | 1474 | 100 |
| 8 | 17.285 | 5.1261 | 185 | 12.6 |
| 9 | 19.895 | 4.459 | 878 | 59.6 |
| 10 | 20.459 | 4.3374 | 225 | 15.3 |
| 11 | 20.845 | 4.258 | 326 | 22.1 |
| 12 | 21.272 | 4.1734 | 244 | 16.6 |
| 13 | 21.842 | 4.0658 | 658 | 44.6 |
| 14 | 22.267 | 3.989 | 355 | 24.1 |
| 15 | 22.510 | 3.9466 | 666 | 45.2 |
| 16 | 24.657 | 3.6075 | 222 | 15.1 |
| 17 | 24.942 | 3.567 | 374 | 25.4 |
| 18 | 25.772 | 3.454 | 149 | 10.1 |
| 19 | 26.604 | 3.3479 | 282 | 19.1 |
| 20 | 27.659 | 3.2225 | 244 | 16.6 |
| 21 | 31.209 | 2.8636 | 218 | 14.8 |
| 22 | 32.122 | 2.7842 | 150 | 10.2 |

Further preferably, the X-ray powder diffraction pattern of the crystal form A of the p-tosylate of the compound is substantially as shown in FIG. 38; its DSC pattern is substantially as shown in FIG. 39; and its TGA pattern is substantially as shown in FIG. 40.

A further preferred embodiment of the present invention provides a crystal form B of a p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 20 of 5.5±0.2°, 19.9±0.2°, and 13.2±0.2°, preferably comprises 2 diffraction peaks selected therefrom, more preferably comprises 3 diffraction peaks selected therefrom; optionally, further may comprise at least one diffraction peak at 2θ of 21.9±0.2°, 28.1±0.2°, 14.1±0.2°, 10.9±0.2°, and 17.6±0.2°, and preferably comprises 2, 3, 4, or 5 diffraction peaks selected therefrom.

For example, the X-ray powder diffraction pattern of the crystal form B of the p-tosylate has diffraction peaks at 20 of:

5.5±0.2° and 21.9±0.2°;
or 5.5±0.2°, 21.9±0.2°, and 28.1±0.2°;
or 5.5±0.2°, 21.9±0.2°, 28.1±0.2°, and 14.1±0.2°;
or 5.5±0.2°, 21.9±0.2°, 28.1±0.2°, 14.1±0.2°, 10.9±0.2°, and 17.6±0.2°.

The X-ray powder diffraction pattern of the crystal form B of the p-tosylate optionally further comprises one or more diffraction peaks at 20 of 9.5±0.2°, 20.4+0.2°, 17.8±0.2°, 22.1±0.2°, 21.5±0.2°, 16.3±0.2°, and 26.5±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the p-tosylate has diffraction peaks at 20 of:

5.5±0.2°, 19.9±0.2°, 13.2±0.2°,21.9±0.2°, 28.1±0.2°, 14.1±0.2°, 9.5±0.2°, and 20.4±0.2°;
or 5.5±0.2°, 19.9±0.2°, 13.2±0.2°, 21.9±0.2°, 28.1±0.2°, 14.1±0.2°, 9.5±0.2°, 20.4±0.2°, 17.8±0.2°, and 22.1 ±0.2°.

The X-ray powder diffraction pattern of the crystal form B of the p-tosylate comprises one or more diffraction peaks at 2θ of 5.5±0.2°, 19.9±0.2°, 13.2±0.2°, 21.9±0.2°, 28.1±0.2°, 14.1±0.2°, 10.9±0.2°, 17.6±0.2°, 9.5±0.2°, 20.4 ±0.2°, 17.8±0.2°, 22.1±0.2°, 21.5±0.2°, 16.3±0.2°, and 26.5±0.2°; and preferably comprises diffraction peaks optionally at 4, 6, 8, or 10 positions selected therefrom.

For example, the X-ray powder diffraction pattern of the crystal form B of the p-tosylate has diffraction peaks at 20 of:

5.5±0.2°, 19.9±0.2°, 13.2±0.2°, 21.9±0.2°, 28.1±0.2°, 14.1±0.2°, 10.9±0.2°, and 17.6±0.2°;
or 5.5±0.2°, 19.9±0.2°, 13.2±0.2°, 21.9±0.2°, 28.1±0.2°, 14.1±0.2°, 10.9±0.2°, 17.6±0.2°, 9.5±0.2°, and 20.4 ±0.2°.

The X-ray powder diffraction pattern of the crystal form B of the p-tosylate comprises one or more diffraction peaks at 2θ of 5.5±0.2°, 19.9±0.2°, 13.2±0.2°, 21.9±0.2°, 28.1±0.2°, 14.1±0.2°, 10.9±0.2°, 17.6±0.2°, 9.5±0.2°, 20.4 ±0.2°, 17.8±0.2°, 22.1±0.2°, 21.5±0.2°, 16.3±0.2°, 26.5±0.2°, 27.4±0.2°, 10.1±0.2°, 12.9±0.2°, 29.2±0.2°, and 22.9 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form B of the p-tosylate has diffraction peaks at 20 of:

5.5±0.2°, 19.9±0.2°, 13.210.2°, 21.9±0.2°, 28.1±0.2°, and 14.1±0.2°;
or 19.9±0.2°, 21.9±0.2°, 28.1±0.2°, 14.1±0.2°, and 10.9±0.2°;
or 5.5±0.2°, 19.9±0.2°, 13.2±0.2°, 21.9±0.2°, 28.1±0.2°, 14.1±0.2°, 10.9±0.2°, and 17.6±0.2°;
or 19.9±0.2°, 21.9±0.2°, 28.1±0.2°, 14.1±0.2°, 10.9±0.2°, 17.6±0.2°, and 9.5±0.2°.

Most preferably, its X-ray characteristic diffraction peaks represented by a 20 angle and an interplanar spacing d using Cu-Kα radiation are as shown in Table 16.

Table 16

| Serial No. | XRPD data of crystal form B of p-tosylate | | | |
|---|---|---|---|---|
| | 20 (±0.2°) | d value | Peak height | Ratio (I%) |
| 1 | 5.477 | 16.1226 | 1755 | 100 |
| 2 | 9.516 | 9.2866 | 276 | 15.7 |
| 3 | 10.121 | 8.7322 | 128 | 7.3 |
| 4 | 10.913 | 8.1009 | 373 | 21.3 |
| 5 | 12.936 | 6.838 | 94 | 5.4 |
| 6 | 13.198 | 6.7027 | 536 | 30.5 |
| 7 | 14.055 | 6.2958 | 378 | 21.5 |
| 8 | 16.348 | 5.4176 | 156 | 8.9 |
| 9 | 17.584 | 5.0397 | 301 | 17.2 |
| 10 | 17.825 | 4.972 | 201 | 11.5 |
| 11 | 19.077 | 4.6483 | 82 | 4.7 |
| 12 | 19.914 | 4.4549 | 710 | 40.5 |
| 13 | 20.399 | 4.3499 | 259 | 14.8 |

(continued)

| Serial No. | XRPD data of crystal form B of p-tosylate | | | |
|---|---|---|---|---|
| | 2θ (±0.2°) | d value | Peak height | Ratio (I%) |
| 14 | 21.496 | 4.1303 | 159 | 9.1 |
| 15 | 21.882 | 4.0585 | 438 | 25 |
| 16 | 22.127 | 4.014 | 185 | 10.5 |
| 17 | 22.854 | 3.8879 | 86 | 4.9 |
| 18 | 25.567 | 3.4812 | 80 | 4.6 |
| 19 | 26.508 | 3.3597 | 149 | 8.5 |
| 20 | 27.420 | 3.25 | 149 | 8.5 |
| 21 | 28.125 | 3.1701 | 387 | 22.1 |
| 22 | 29.234 | 3.0523 | 90 | 5.1 |

Further preferably, the X-ray powder diffraction pattern of the crystal form B of the p-tosylate is substantially as shown in FIG. 41; its DSC pattern is substantially as shown in FIG. 42; and its TGA pattern is substantially as shown in FIG. 43.

A further preferred embodiment of the present invention provides a crystal form C of a p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 5.8±0.2°, 17.3±0.2°, and 16.7±0.2°, preferably comprises 2 diffraction peaks selected therefrom, more preferably comprises 3 diffraction peaks selected therefrom; optionally, further may comprise at least one diffraction peak at 2θ of 22.0±0.2°, 19.6±0.2°, 23.1±0.2°, 22.4±0.2°, and 20.1±0.2°, and preferably comprises 2, 3, 4, or 5 diffraction peaks selected therefrom.

For example, the X-ray powder diffraction pattern of the crystal form C of the p-tosylate has diffraction peaks at 2θ of:

5.8±0.2° and 22.0±0.2°;
or 5.8±0.2°, 22.0±0.2°, and 19.6±0.2°;
or 5.8±0.2°, 22.0±0.2°, 19.6±0.2°, and 23.1±0.2°;
or 5.8±0.2°, 22.0±0.2°, 19.6±0.2°, 23.1±0.2°, 22.4±0.2°, and 20.1±0.2°.

The X-ray powder diffraction pattern of the crystal form C of the p-tosylate optionally further comprises one or more diffraction peaks at 2θ of 29.0±0.2°, 12.8±0.2°, 21.6±0.2°, 11.5±0.2°, 13.8±0.2°, 27.4±0.2°, and 20.9±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form C of the p-tosylate has diffraction peaks at 2θ of:

5.8±0.2°, 17.3±0.2°, 16.7±0.2°,22.0±0.2°, 19.6±0.2°, 23.1±0.2°, 29.0±0.2°, and 12.8±0.2°;
or 5.8±0.2°, 17.3±0.2°, 16.7±0.2°, 22.0±0.2°, 19.6±0.2°, 23.1±0.2°, 29.0±0.2°, 12.8±0.2°, 21.6±0.2°, and 11.5 ±0.2°.

The X-ray powder diffraction pattern of the crystal form C of the p-tosylate comprises one or more diffraction peaks at 2θ of 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, 12.8±0.2°, 21.6±0.2°, 11.5±0.2°, 13.8±0.2°, 27.4±0.2°, and 20.9±0.2°; and preferably comprises diffraction peaks optionally at 4, 6, 8, or 10 positions selected therefrom.

For example, the X-ray powder diffraction pattern of the crystal form C of the p-tosylate has diffraction peaks at 2θ of:

5.8±0.2°, 17.3±0.2°, 16.7±0.2°, 22.0±0.2°, 19.6±0.2°, 23.1±0.2°, 22.4±0.2°, and 20.1±0.2°;
or 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, and 12.8 ±0.2°.

The X-ray powder diffraction pattern of the crystal form C of the p-tosylate comprises one or more diffraction peaks at 2θ of 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, 12.8±0.2°, 21.6±0.2°, 11.5±0.2°, 13.8±0.2°, 27.4+0.2°, 20.9±0.2°, 33.2±0.2°, 25.7+0.2°, 9.8±0.2°, 25.2±0.2°, and 32.8±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form C of the p-tosylate has diffraction peaks at 2θ of:

5.8±0.2°, 17.3±0.2°, 16.7±0.2°, 22.0±0.2°, 19.6±0.2°, 23.1±0.2°, 22.4±0.2°, and 20.1±0.2°;
or 5.8±0.2°, 17.3±0.2°, 23.1±0.2°, 22.4±0.2°, 20.1±0.2°, 29.0±0.2°, 12.8±0.2°, and 21.6±0.2°;
or 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, and 12.8 ±0.2°.

Most preferably, its X-ray characteristic diffraction peaks represented by a 2θ angle and an interplanar spacing d using Cu-Kα radiation are as shown in Table 17.

Table 17

| Serial No. | XRPD data of crystal form C of p-tosylate | | | |
|---|---|---|---|---|
| | 2θ (±0.2°) | d value | Peak height | Ratio (I%) |
| 1 | 5.802 | 15.2193 | 1763 | 100 |
| 2 | 9.759 | 9.0552 | 65 | 3.7 |
| 3 | 11.519 | 7.676 | 146 | 8.3 |
| 4 | 12.776 | 6.923 | 192 | 10.9 |
| 5 | 13.750 | 6.4347 | 138 | 7.8 |
| 6 | 14.401 | 6.1456 | 44 | 2.5 |
| 7 | 16.692 | 5.3069 | 661 | 37.5 |
| 8 | 17.260 | 5.1335 | 806 | 45.7 |
| 9 | 19.587 | 4.5285 | 406 | 23 |
| 10 | 20.098 | 4.4145 | 235 | 13.3 |
| 11 | 20.889 | 4.2491 | 101 | 5.7 |
| 12 | 21.616 | 4.1078 | 178 | 10.1 |
| 13 | 22.024 | 4.0325 | 504 | 28.6 |
| 14 | 22.387 | 3.968 | 284 | 16.1 |
| 15 | 23.115 | 3.8446 | 295 | 16.7 |
| 16 | 24.099 | 3.6899 | 59 | 3.3 |
| 17 | 25.187 | 3.5328 | 66 | 3.7 |
| 18 | 25.653 | 3.4697 | 90 | 5.1 |
| 19 | 26.846 | 3.3181 | 55 | 3.1 |
| 20 | 27.414 | 3.2508 | 103 | 5.8 |
| 21 | 28.978 | 3.0787 | 232 | 13.2 |
| 22 | 30.747 | 2.9055 | 40 | 2.3 |
| 23 | 32.849 | 2.7242 | 66 | 3.7 |
| 24 | 33.170 | 2.6986 | 91 | 5.2 |
| 25 | 34.878 | 2.5703 | 46 | 2.6 |

Further preferably, the X-ray powder diffraction pattern of the crystal form C of the p-tosylate is substantially as shown in FIG. 44; its DSC pattern is substantially as shown in FIG. 45; and its TGA pattern is substantially as shown in FIG. 46.

A further preferred embodiment of the present invention provides a crystal form D of a p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 4.910.2°, 5.7+0.2°, and 17.2±0.2°, preferably comprises 2 diffraction peaks selected therefrom, more preferably comprises 3 diffraction peaks selected therefrom; optionally, further may comprise at least one diffraction peak at 2θ of 22.0±0.2°, 19.5±0.2°, 28.9±0.2°, 25.5±0.2°, and 12.7±0.2°, and preferably comprises 2, 3, 4, or 5 diffraction peaks selected therefrom.

For example, the X-ray powder diffraction pattern of the crystal form D of the p-tosylate has diffraction peaks at 2θ of:

4.9±0.2° and 22.0±0.2°;
or 4.9±0.2°, 22.0±0.2°, and 19.5±0.2°;
or 4.9±0.2°, 22.0±0.2°, 19.5±0.2°, and 28.9±0.2°;
or 4.9±0.2°, 22.0±0.2°, 19.5±0.2°, 28.9±0.2°, 25.5±0.2°, and 12.7±0.2°.

The X-ray powder diffraction pattern of the crystal form D of the p-tosylate optionally further comprises one or more diffraction peaks at 2θ of 14.8±0.2°, 23.0±0.2°, 20.6±0.2°, 27.3±0.2°, 30.4±0.2°, 24.8±0.2°, and 27.7±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form D of the p-tosylate has diffraction peaks at 2θ of:

4.9±0.2°, 5.7±0.2°, 17.2±0.2°,22.0±0.2°, 19.5±0.2°, 28.9+0.2°, 14.8±0.2°, and 23.0±0.2°;
or 4.9±0.2°, 5.7±0.2°, 17.2±0.2°, 22.0±0.2°, 19.5±0.2°, 28.9±0.2°, 14.8±0.2°, 23.0±0.2°, 20.6±0.2°, and 27.3 ±0.2°.

The X-ray powder diffraction pattern of the crystal form D of the p-tosylate comprises one or more diffraction peaks at 2θ of 4.9±0.2°, 5.7±0.2°, 17.24±0.2°, 22.0±0.2°, 19.5±0.2°, 28.9±0.2°, 25.5±0.2°, 12.7±0.2°, 14.8±0.2°, 23.0±0.2°, 20.6±0.2°, 27.3±0.2°, 30.4±0.2°, 24.8±0.2°, and 27.7±0.2°; and preferably comprises diffraction peaks optionally at 4, 6, 8, or 10 positions selected therefrom.

For example, the X-ray powder diffraction pattern of the crystal form D of the p-tosylate has diffraction peaks at 2θ of:

4.910.2°, 5.7±0.2°, 17.2±0.2°, 22.0±0.2°, 19.5±0.2°, 28.9±0.2°, 25.5±0.2°, and 12.7±0.2°;
or 4.9±0.2°, 5.7±0.2°, 17.2±0.2°, 22.0±0.2°, 19.5±0.2°, 28.9±0.2°, 25.5±0.2°, 12.7±0.2°, 14.8±0.2°, and 23.0 ±0.2°.

The X-ray powder diffraction pattern of the crystal form D of the p-tosylate comprises one or more diffraction peaks at 2θ of 4.9±0.2°, 5.7±0.2°, 17.24±0.2°, 22.0±0.2°, 19.5±0.2°, 28.9±0.2°, 25.5±0.2°, 12.7±0.2°, 14.8±0.2°, 23.0±0.2°, 20.6±0.2°, 27.3±0.2°, 30.4±0.2°, 24.8±0.2°, 27.7±0.2°, 11.5±0.2°, 34.9±0.2°, 9.9±0.2°, and 35.0±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form D of the p-tosylate has diffraction peaks at 2θ of:

4.9±0.2°, 5.7±0.2°, 17.2±0.2°, 22.0±0.2°, 19.5±0.2°, 28.9±0.2°, 25.5±0.2°, and 12.7±0.2°;
or 4.9±0.2°, 5.7±0.2°, 19.5±0.2°, 28.9±0.2°, 25.5±0.2°, 12.7±0.2°, 14.8±0.2°, and 23.0±0.2°;
or 4.9±0.2°, 5.7±0.2°, 17.2±0.2°, 22.0±0.2°, 19.5±0.2°, 28.9±0.2°, 25.5±0.2°, 12.7±0.2°, 14.8±0.2°, and 23.0 ±0.2°.

Most preferably, its X-ray characteristic diffraction peaks represented by a 2θ angle and an interplanar spacing d using Cu-Kα radiation are as shown in Table 18.

Table 18

| Serial No. | XRPD data of crystal form D of p-tosylate | | | |
|---|---|---|---|---|
| | 2θ (±0.2°) | d value | Peak height | Ratio (I%) |
| 1 | 4.935 | 17.893 | 10460 | 100 |
| 2 | 5.723 | 15.4307 | 956 | 9.1 |
| 3 | 9.856 | 8.9669 | 33 | 0.3 |
| 4 | 11.501 | 7.6876 | 50 | 0.5 |
| 5 | 12.659 | 6.9866 | 122 | 1.2 |
| 6 | 14.801 | 5.9802 | 127 | 1.2 |
| 7 | 17.218 | 5.1459 | 543 | 5.2 |
| 8 | 19.509 | 4.5463 | 234 | 2.2 |
| 9 | 20.643 | 4.2991 | 104 | 1 |
| 10 | 22.041 | 4.0296 | 346 | 3.3 |
| 11 | 23.039 | 3.8572 | 122 | 1.2 |
| 12 | 24.755 | 3.5935 | 58 | 0.6 |
| 13 | 25.473 | 3.4938 | 157 | 1.5 |
| 14 | 27.253 | 3.2695 | 78 | 0.7 |
| 15 | 27.721 | 3.2155 | 64 | 0.6 |
| 16 | 28.917 | 3.0851 | 184 | 1.8 |
| 17 | 30.432 | 2.9348 | 75 | 0.7 |
| 18 | 34.876 | 2.5704 | 39 | 0.4 |
| 19 | 34.957 | 2.5646 | 34 | 0.3 |

Further preferably, the X-ray powder diffraction pattern of the crystal form D of the p-tosylate is substantially as shown in FIG. 47; its DSC pattern is substantially as shown in FIG. 48; and its TGA pattern is substantially as shown in FIG. 49.

A further preferred embodiment of the present invention provides a crystal form E of a p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 5.4±0.2°, 16.1±0.2°, and 9.9±0.2°, preferably comprises 2 diffraction peaks selected therefrom, more preferably comprises 3 diffraction peaks selected therefrom; optionally, further may comprise at least one diffraction peak at 2θ of 16.7±0.2°, 8.4±0.2°, 23.1±0.2°, 26.9±0.2°, and 25.7±0.2°, and preferably comprises 2, 3, 4, or 5 diffraction peaks selected therefrom.

For example, the X-ray powder diffraction pattern of the crystal form E of the p-tosylate has diffraction peaks at 2θ of:

5.44±0.2° and 16.7±0.2°;
or 5.4±0.2°, 16.7±0.2°, and 8.4±0.2°;
or 5.4±0.2°, 16.7±0.2°, 8.4±0.2°, and 23.1±0.2°;
or 16.1±0.2°, 23.1±0.2°, 26.9±0.2°, and 25.7±0.2°;
or 9.9±0.2°, 23.14±0.2°, 26.9±0.2°, and 25.7±0.2°;
or 5.4±0.2°, 16.7±0.2°, 8.4±0.2°, 23.1±0.2°, 26.9±0.2°, and 25.7±0.2°.

The X-ray powder diffraction pattern of the crystal form E of the p-tosylate optionally further comprises one or more diffraction peaks at 2θ of 25.2±0.2°, 28.2±0.2°, 18.5±0.2°, 16.9±0.2°, 32.4±0.2°, 11.6±0.2°, and 15.1±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form E of the p-tosylate has diffraction peaks at 2θ of:

5.4±0.2°, 16.1±0.2°, 9.9±0.2°,16.7±0.2°, 8.4±0.2°, 23.1±0.2°, 25.2±0.2°, and 28.2±0.2°;
or 5.4±0.2°, 16.1±0.2°, 9.9±0.2°, 16.7±0.2°, 8.4±0.2°, 23.1±0.2°, 25.2±0.2°, 28.2±0.2°, 18.5±0.2°, and 16.9 ±0.2°.

The X-ray powder diffraction pattern of the crystal form E of the p-tosylate comprises one or more diffraction peaks at 2θ of 5.4±0.2°, 16.1±0.2°, 9.9±0.2°, 16.7±0.2°, 8.4±0.2°, 23.1±0.2°, 26.9±0.2°, 25.7±0.2°, 25.2±0.2°, 28.2 ±40.2°, 18.5±0.2°, 16.9±0.2°, 32.4±0.2°, 11.6±0.2°, and 15.1±0.2°; and preferably comprises diffraction peaks optionally at 4, 6, 8, or 10 positions selected therefrom.

For example, the X-ray powder diffraction pattern of the crystal form E of the p-tosylate has diffraction peaks at 2θ of:

16.1±0.2°, 9.9±0.2°, 16.7±0.2°, 8.4±0.2°, 23.1±0.2°, 26.9±0.2°, 25.7±0.2°, and 25.2±0.2°;
or 16.1±0.2°, 9.9±0.2°, 16.7±0.2°, 8.4±0.2°, 23.1±0.2°, 26.9±0.2°, 25.7±0.2°, 25.2±0.2°, 28.2±0.2°, and 18.5 ±0.2°.

The X-ray powder diffraction pattern of the crystal form E of the p-tosylate comprises one or more diffraction peaks at 2θ of 5.4±0.2°, 16.1±0.2°, 9.9±0.2°, 16.7±0.2°, 8.4±0.2°, 23.1±0.2°, 26.9±0.2°, 25.7±0.2°, 25.2±0.2°, 28.2 ±0.2°, 18.5±0.2°, 16.9±0.2°, 32.4±0.2°, 11.6±0.2°, 15.1±0.2°, 21.5±0.2°, 23.5±0.2°, 20.0±0.2°, 13.8±0.2°, and 17.5 ±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form E of the p-tosylate has diffraction peaks at 2θ of:

5.4±0.2°, 16.1±0.2°, 9.9±0.2°, 16.7±0.2°, 8.4±0.2°, 23.1±0.2°, 26.9±0.2°, and 25.7±0.2°;
or 5.4±0.2°, 16.1±0.2°, 8.4±0.2°, 23.1±0.2°, 26.9±0.2°, 25.7±0.2°, 25.2±0.2°, and 28.2±0.2°;
or 5.4±0.2°, 16.1±0.2°, 9.9±0.2°, 16.7±0.2°, 8.4±0.2°, 23.1±0.2°, 26.9±0.2°, 25.7±0.2°, 25.2±0.2°, and 28.2 ±0.2°.

Most preferably, its X-ray characteristic diffraction peaks represented by a 2θ angle and an interplanar spacing d using Cu-Kα radiation are as shown in Table 19.

Table 19

| Serial No. | XRPD data of crystal form E of p-tosylate | | | |
|---|---|---|---|---|
| | 2θ (±0.2°) | d value | Peak height | Ratio (I%) |
| 1 | 5.380 | 16.4132 | 3432 | 100 |
| 2 | 8.352 | 10.5781 | 499 | 14.5 |
| 3 | 9.917 | 8.9117 | 585 | 17 |
| 4 | 11.598 | 7.6236 | 75 | 2.2 |
| 5 | 13.809 | 6.4074 | 62 | 1.8 |
| 6 | 15.063 | 5.8769 | 72 | 2.1 |
| 7 | 16.086 | 5.5052 | 968 | 28.2 |
| 8 | 16.688 | 5.3081 | 536 | 15.6 |
| 9 | 16.894 | 5.2438 | 115 | 3.4 |
| 10 | 17.525 | 5.0565 | 60 | 1.7 |
| 11 | 18.536 | 4.7829 | 150 | 4.4 |
| 12 | 20.014 | 4.4328 | 65 | 1.9 |
| 13 | 20.869 | 4.2532 | 54 | 1.6 |
| 14 | 21.491 | 4.1313 | 72 | 2.1 |
| 15 | 23.062 | 3.8533 | 301 | 8.8 |
| 16 | 23.547 | 3.7751 | 68 | 2 |

(continued)

| Serial No. | XRPD data of crystal form E of p-tosylate | | | |
|---|---|---|---|---|
| | 2θ (±0.2°) | d value | Peak height | Ratio (I%) |
| 17 | 25.226 | 3.5275 | 194 | 5.7 |
| 18 | 25.667 | 3.4678 | 210 | 6.1 |
| 19 | 26.932 | 3.3078 | 246 | 7.2 |
| 20 | 28.228 | 3.1588 | 165 | 4.8 |
| 21 | 32.442 | 2.7574 | 80 | 2.3 |
| 22 | 32.442 | 2.7574 | 80 | 2.3 |

Further preferably, the X-ray powder diffraction pattern of the crystal form E of the p-tosylate is substantially as shown in FIG. 50; its DSC pattern is substantially as shown in FIG. 51; and its TGA pattern is substantially as shown in FIG. 52.

A further preferred embodiment of the present invention provides a crystal form A of a besylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 20 of 5.7±0.2°, 17.2±0.2°, and 21.8±0.2°, preferably comprises 2 diffraction peaks selected therefrom, more preferably comprises 3 diffraction peaks selected therefrom; optionally, further may comprise at least one diffraction peak at 2θ of 16.6±0.2°, 23.0±0.2°, 17.6±0.2°, and 20.3±0.2°, and preferably comprises 2, 3, 4, or 5 diffraction peaks selected therefrom.

For example, the X-ray powder diffraction pattern of the crystal form A of the besylate has diffraction peaks at 20 of:

5.7±0.2° and 5.5±0.2°;
or 5.7±0.2°, 5.5±0.2°, and 16.6±0.2°;
or 5.74±0.2°, 5.5±0.2°, 16.6±0.2°, and 23.0±0.2°;
or 5.7±0.2°, 5.5±0.2°, 16.6±0.2°, 23.0±0.2°, 17.6±0.2°, and 20.3±0.2°.

The X-ray powder diffraction pattern of the crystal form A of the besylate optionally further comprises one or more diffraction peaks at 2θ of 27.3±0.2°, 28.8±0.2°, 11.5±0.2°, 13.8±0.2°, 25.5±0.2°, 19.9±0.2°, and 21.3±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the besylate has diffraction peaks at 20 of:

5.7±0.2°, 17.2±0.2°, 21.8±0.2°,5.5±0.2°, 16.6±0.2°, 23.0±0.2°, 27.3±0.2°, and 28.8±0.2°;
or 5.7±0.2°, 17.2±0.2°, 21.8±0.2°, 5.5±0.2°, 16.6±0.2°, 23.0±0.2°, 27.3±0.2°, 28.8±0.2°, 11.5±0.2°, and 13.8 ±0.2°.

The X-ray powder diffraction pattern of the crystal form A of the besylate comprises one or more diffraction peaks at 20 of 55.7±0.2°, 17.2±0.2°, 21.8±0.2°, 5.5±0.2°, 16.6±0.2°, 23.0±0.2°, 17.6±0.2°, 20.3±0.2°, 27.3±0.2°, 28.8 ±0.2°, 11.5±0.2°, 13.8±0.2°, 25.5±0.2°, 19.9±0.2°, and 21.3±0.2°; and preferably comprises diffraction peaks optionally at 4, 6, 8, or 10 positions selected therefrom.

For example, the X-ray powder diffraction pattern of the crystal form A of the besylate has diffraction peaks at 20 of:

55.7±0.2°, 17.2±0.2°, 21.8±0.2°, 5.5±0.2°, 16.6±0.2°, 23.0+0.2°, 17.6±0.2°, and 20.3±0.2°;
or 55.7±0.2°, 17.2±0.2°, 21.8+0.2°, 16.6±0.2°, 23.0±0.2°, 17.6±0.2°, 20.3±0.2°, 27.3±0.2°, and 28.8±0.2°.

The X-ray powder diffraction pattern of the crystal form A of the besylate comprises one or more diffraction peaks at 2θ of 5.7±0.2°, 17.2+0.2°, 21.8±0.2°, 16.6±0.2°, 23.0+0.2°, 17.6±0.2°, 20.3±0.2°, 27.3+0.2°, 28.8±0.2°, 11.5±0.2°, 13.8±0.2°, 25.5±0.2°, 19.9±0.2°, 21.3±0.2°, 24.3±0.2°, 11.9±0.2°, 23.8±0.2°, 26.5±0.2°, and 25.3±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the besylate has diffraction peaks at 2θ of:

5.7±0.2°, 17.2±0.2°, 21.8±0.2°, 5.5±0.2°, 16.6±0.2°, 23.0+0.2°, 17.6±0.2°, and 20.3±0.2°;
or 5.7±0.2°, 17.2±0.2°, 16.6±0.2°, 23.0±0.2°, 17.6±0.2°, 20.3±0.2°, 27.3±0.2°, and 13.8±0.2°;
or 5.7±0.2°, 17.2±0.2°, 21.8±0.2°, 5.5±0.2°, 16.6±0.2°, 23.0±0.2°, 17.6±0.2°, 20.3±0.2°, 27.3±0.2°, and 28.8 ±0.2°.

Most preferably, its X-ray characteristic diffraction peaks represented by a 2θ angle and an interplanar spacing d using Cu-Kα radiation are as shown in Table 20.

Table 20

| Serial No. | XRPD data of crystal form A of besylate | | | |
|---|---|---|---|---|
| | 2θ (±0.2°) | d value | Peak height | Ratio (1%) |
| 1 | 5.457 | 16.1823 | 214 | 30.7 |
| 2 | 5.722 | 15.4311 | 696 | 100 |
| 3 | 9.944 | 8.8879 | 37 | 5.3 |
| 4 | 11.476 | 7.7043 | 78 | 11.2 |
| 5 | 11.924 | 7.4159 | 45 | 6.5 |
| 6 | 12.650 | 6.9919 | 37 | 5.3 |
| 7 | 13.757 | 6.4318 | 73 | 10.5 |
| 8 | 16.597 | 5.3368 | 207 | 29.7 |
| 9 | 17.197 | 5.152 | 234 | 33.6 |
| 10 | 17.586 | 5.039 | 112 | 16.1 |
| 11 | 19.896 | 4.4589 | 49 | 7 |
| 12 | 20.323 | 4.3661 | 110 | 15.8 |
| 13 | 21.295 | 4.1689 | 47 | 6.8 |
| 14 | 21.761 | 4.0807 | 215 | 30.9 |
| 15 | 22.995 | 3.8644 | 171 | 24.6 |
| 16 | 23.812 | 3.7337 | 42 | 6 |
| 17 | 24.254 | 3.6666 | 46 | 6.6 |
| 18 | 25.256 | 3.5233 | 38 | 5.5 |
| 19 | 25.526 | 3.4867 | 51 | 7.3 |
| 20 | 25.984 | 3.4263 | 37 | 5.3 |
| 21 | 26.527 | 3.3573 | 42 | 6 |
| 22 | 27.253 | 3.2696 | 84 | 12.1 |

Further preferably, the X-ray powder diffraction pattern of the crystal form A of the besylate is substantially as shown in FIG. 53.

A further preferred embodiment of the present invention provides a crystal form B of a besylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 19.7±0.2°, 17.4±0.2°, and 13.6±0.2°, preferably comprises 2 diffraction peaks selected therefrom, more preferably comprises 3 diffraction peaks selected therefrom; optionally, further may comprise at least one diffraction peak at 2θ of 22.6±0.2°, 9.7±0.2°, 5.7±0.2°, 14.2±0.2°, and 29.1 ±0.2°, and preferably comprises 2, 3, 4, or 5 diffraction peaks selected therefrom.

For example, the X-ray powder diffraction pattern of the crystal form B of the besylate has diffraction peaks at 2θ of:

19.7±0.2° and 22.6±0.2°;
or 19.7±0.2°, 22.6±0.2°, and 9.7±0.2°;
or 19.7±0.2°, 22.6±0.2°, 9.7±0.2°, and 5.7±0.2°;
or 19.7±0.2°, 22.6±0.2°, 9.7±0.2°, 5.7±0.2°, 14.2±0.2°, and 29.1+0.2°.

The X-ray powder diffraction pattern of the crystal form B of the besylate optionally further comprises one or more diffraction peaks at 2θ of 12.8±0.2°, 23.7±0.2°, 26.4±0.2°, 27.3±0.2°, 24.0±0.2°, 20.7±0.2°, and 21.6±0.2°; preferably comprises at least any 2-3, or 4-5, or 6-7 diffraction peaks selected therefrom, and further preferably comprises any 2, 3, 4, 6, or 7 diffraction peaks selected therefrom.
For example, the X-ray powder diffraction pattern of the crystal form B of the besylate has diffraction peaks at 20 of:

19.7±0.2°, 17.4±0.2°, 13.6±0.2°,22.6±0.2°, 9.7+0.2°, 5.7±0.2°, 12.8±0.2°, and 23.7±0.2°;
or 19.7±0.2°, 17.4±0.2°, 13.6±0.2°, 22.6±0.2°, 9.7±0.2°, 5.7±0.2°, 12.8±0.2°, 23.7±0.2°, 26.4±0.2°, and 27.3 ±0.2°.

The X-ray powder diffraction pattern of the crystal form B of the besylate comprises one or more diffraction peaks at 2θ of 19.7±0.2°, 17.4±0.2°, 13.6±0.2°, 22.6±0.2°, 9.7±0.2°, 5.7±0.2°, 14.2±0.2°, 29.1±0.2°, 12.8±0.2°, 23.7±0.2°, 26.4±0.2°, 27.3±0.2°, 24.0±0.2°, 20.7±0.2°, and 21.6±0.2°; and preferably comprises diffraction peaks optionally at 4, 6, 8, or 10 positions selected therefrom.
For example, the X-ray powder diffraction pattern of the crystal form B of the besylate has diffraction peaks at 20 of:

19.7±0.2°, 17.4±0.2°, 13.6±0.2°, 22.6±0.2°, 9.7+0.2°, 5.7±0.2°, 14.2±0.2°, and 29.1±0.2°;
or 19.7±0.2°, 17.4±0.2°, 13.6±0.2°, 22.6±0.2°, 9.7±0.2°, 5.7±0.2°, 14.24±0.2°, 29.1±0.2°, 12.8±0.2°, and 23.7 ±0.2°.

The X-ray powder diffraction pattern of the crystal form B of the besylate comprises one or more diffraction peaks at 2θ of 19.7±0.2°, 17.4±0.2°, 13.6±0.2°, 22.6±0.2°, 9.7±0.2°, 5.7±0.2°, 14.2±0.2°, 29.1±0.2°, 12.8±0.2°, 23.7±0.2°, 26.4±0.2°, 27.3±0.2°, 24.0+0.2°, 20.7±0.2°, 21.6±0.2°, 16.2±0.2°, 22.0±0.2°, 22.9±0.2°, 18.7±0.2°, and 19.3±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.
For example, the X-ray powder diffraction pattern of the crystal form B of the besylate has diffraction peaks at 2θ of:

19.7±0.2°, 17.4±0.2°, 13.610.2°, 22.6±0.2°, 9.7+0.2°, 5.7±0.2°, 14.2±0.2°, and 29.1±0.2°;
or 19.7±0.2°, 17.4±0.2°, 9.7+0.2°, 5.7±0.2°, 14.2±0.2°, 29.1±0.2°, 12.8±0.2°, and 23.7±0.2°;
or 19.7±0.2°, 17.4±0.2°, 13.6±0.2°, 22.6±0.2°, 9.7±0.2°, 5.7±0.2°, 14.24±0.2°, 29.1±0.2°, 12.8±0.2°, and 23.7 ±0.2°.

Most preferably, its X-ray characteristic diffraction peaks represented by a 20 angle and an interplanar spacing d using Cu-Kα radiation are as shown in Table 21.

Table 21

| Serial No. | XRPD data of crystal form B of besylate | | | |
|---|---|---|---|---|
| | 20 (±0.2°) | d value | Peak height | Ratio (I%) |
| 1 | 5.659 | 15.6041 | 339 | 19.5 |
| 2 | 9.651 | 9.1568 | 431 | 24.7 |
| 3 | 12.759 | 6.9325 | 208 | 11.9 |
| 4 | 13.564 | 6.5227 | 759 | 43.6 |
| 5 | 14.209 | 6.228 | 267 | 15.3 |
| 6 | 16.189 | 5.4704 | 126 | 7.2 |
| 7 | 17.403 | 5.0914 | 849 | 48.7 |
| 8 | 18.745 | 4.7298 | 113 | 6.5 |

(continued)

| Serial No. | XRPD data of crystal form B of besylate | | | |
|---|---|---|---|---|
| | 2θ (±0.2°) | d value | Peak height | Ratio (I%) |
| 9 | 19.322 | 4.5899 | 105 | 6 |
| 10 | 19.733 | 4.4952 | 1742 | 100 |
| 11 | 20.660 | 4.2957 | 134 | 7.7 |
| 12 | 21.574 | 4.1156 | 128 | 7.3 |
| 13 | 22.022 | 4.0329 | 120 | 6.9 |
| 14 | 22.628 | 3.9263 | 486 | 27.9 |
| 15 | 22.898 | 3.8807 | 118 | 6.8 |
| 16 | 23.684 | 3.7536 | 189 | 10.8 |
| 17 | 24.033 | 3.6998 | 153 | 8.8 |
| 18 | 24.541 | 3.6244 | 99 | 5.7 |
| 19 | 26.423 | 3.3703 | 178 | 10.2 |
| 20 | 27.251 | 3.2698 | 164 | 9.4 |
| 21 | 29.138 | 3.0622 | 253 | 14.5 |
| 22 | 32.627 | 2.7423 | 105 | 6 |

Further preferably, the X-ray powder diffraction pattern of the crystal form B of the besylate is substantially as shown in FIG. 54; its DSC pattern is substantially as shown in FIG. 55; and its TGA pattern is substantially as shown in FIG. 56.

A further preferred embodiment of the present invention provides a crystal form C of a besylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 5.4±0.2°, 16.6±0.2°, and 16.9±0.2°, preferably comprises 2 diffraction peaks selected therefrom, more preferably comprises 3 diffraction peaks selected therefrom; optionally, further may comprise at least one diffraction peak at 2θ of 15.0±0.2°, 12.7±0.2°, 19.4±0.2°, 8.3±0.2°, and 20.9±0.2°, and preferably comprises 2, 3, 4, or 5 diffraction peaks selected therefrom.

For example, the X-ray powder diffraction pattern of the crystal form C of the besylate has diffraction peaks at 2θ of:

5.4±0.2° and 15.0±0.2°;
or 5.4±0.2°, 15.0±0.2°, and 12.7±0.2°;
or 5.4±0.2°, 15.0±0.2°, 12.7±0.2°, and 19.4±0.2°;
or 5.4±0.2°, 15.0±0.2°, 12.7±0.2°, 19.4±0.2°, 8.3±0.2°, and 20.9±0.2°.

The X-ray powder diffraction pattern of the crystal form C of the besylate optionally further comprises one or more diffraction peaks at 2θ of 13.8±0.2°, 9.9±0.2°, 25.6±0.2°, 25.2±0.2°, 16.2±0.2°, 18.5±0.2°, and 19.9±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form C of the besylate has diffraction peaks at 2θ of:

5.4±0.2°, 16.6±0.2°, 16.9±0.2°,15.0±0.2°, 12.7±0.2°, 19.4±0.2°, 13.8±0.2°, and 9.9±0.2°;
or 5.4±0.2°, 16.6±0.2°, 16.9±0.2°, 15.0±0.2°, 12.7±0.2°, 19.4±0.2°, 13.8±0.2°, 9.9±0.2°, 25.6±0.2°, and 25.2 ±40.2°.

The X-ray powder diffraction pattern of the crystal form C of the besylate comprises one or more diffraction peaks at 2θ of 5.4±0.2°, 16.6±0.2°, 16.9±0.2°, 15.0±0.2°, 12.7±0.2°, 19.4±0.2°, 8.3±0.2°, 20.9±0.2°, 13.8±0.2°, 9.9±0.2°, 25.6±0.2°, 25.2±0.2°, 16.2±0.2°, 18.5±0.2°, and 19.9±0.2°; and preferably comprises diffraction peaks optionally at 4, 6, 8, or 10 positions selected therefrom.

For example, the X-ray powder diffraction pattern of the crystal form C of the besylate has diffraction peaks at 20 of:

5.4±0.2°, 16.6±0.2°, 16.9±0.2°, 15.0±0.2°, 12.7±0.2°, 19.4±0.2°, 8.3±0.2°, and 20.9±0.2°;
or 5.4±0.2°, 16.6±0.2°, 16.9±0.2°, 15.0±0.2°, 12.7±0.2°, 19.4±0.2°, 8.3±0.2°, 20.9±0.2°, 13.8±0.2°, and 9.9 ±0.2°.

The X-ray powder diffraction pattern of the crystal form C of the besylate comprises one or more diffraction peaks at 2θ of 5.4±0.2°, 16.6±0.2°, 16.9±0.2°, 15.0±0.2°, 12.7±0.2°, 19.4±0.2°, 8.3±0.2°, 20.9±0.2°, 13.8±0.2°, 9.9±0.2°, 25.6±0.2°, 25.2+0.2°, 16.2±0.2°, 18.5±0.2°, 19.9±0.2°, 22.1±0.2°, 30.2±0.2°, 26.1±0.2°, 27.0±0.2°, and 12.9±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form C of the besylate has diffraction peaks at 20 of:

5.4±0.2°, 16.6±0.2°, 16.9±0.2°, 15.0±0.2°, 12.7±0.2°, 19.4±0.2°, 8.3±0.2°, and 20.9±0.2°;
or 5.4±0.2°, 16.6±0.2°, 19.4±0.2°, 8.3±0.2°, 20.9±0.2°, 13.8+0.2°, 9.9±0.2°, and 25.6±0.2°;
or 5.4±0.2°, 16.6±0.2°, 16.9±0.2°, 15.0±0.2°, 12.7±0.2°, 19.4±0.2°, 8.3±0.2°, 20.9±0.2°, 13.8±0.2°, and 9.9 ±0.2°.

Most preferably, its X-ray characteristic diffraction peaks represented by a 20 angle and an interplanar spacing d using Cu-Kα radiation are as shown in Table 22.

Table 22

| Serial No. | XRPD data of crystal form C of besylate | | | |
|---|---|---|---|---|
| | 2θ (±0.2°) | d value | Peak height | Ratio (I%) |
| 1 | 5.418 | 16.2982 | 1940 | 100 |
| 2 | 8.322 | 10.6154 | 366 | 18.9 |
| 3 | 9.919 | 8.9101 | 257 | 13.2 |
| 4 | 12.675 | 6.9781 | 422 | 21.8 |
| 5 | 12.948 | 6.8318 | 99 | 5.1 |
| 6 | 13.771 | 6.4252 | 273 | 14.1 |
| 7 | 15.048 | 5.8828 | 440 | 22.7 |
| 8 | 16.162 | 5.4797 | 178 | 9.2 |
| 9 | 16.631 | 5.326 | 598 | 30.8 |
| 10 | 16.856 | 5.2555 | 534 | 27.5 |
| 11 | 18.496 | 4.793 | 147 | 7.6 |
| 12 | 19.387 | 4.5747 | 383 | 19.7 |
| 13 | 19.938 | 4.4495 | 139 | 7.2 |
| 14 | 20.867 | 4.2534 | 358 | 18.5 |
| 15 | 22.122 | 4.0149 | 133 | 6.9 |
| 16 | 23.762 | 3.7414 | 97 | 5 |
| 17 | 25.224 | 3.5278 | 202 | 10.4 |
| 18 | 25.630 | 3.4728 | 248 | 12.8 |
| 19 | 26.057 | 3.4168 | 113 | 5.8 |
| 20 | 26.973 | 3.3029 | 104 | 5.4 |
| 21 | 27.412 | 3.251 | 85 | 4.4 |
| 22 | 30.215 | 2.9555 | 129 | 6.6 |

Further preferably, the X-ray powder diffraction pattern of the crystal form C of the besylate is substantially as shown in FIG. 57; its DSC pattern is substantially as shown in FIG. 58; and its TGA pattern is substantially as shown in FIG. 59.

A further preferred embodiment of the present invention provides a crystal form A of an isethionate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 20 of 5.4±0.2°, 16.1±0.2°, and 20.9±0.2°, preferably comprises 2 diffraction peaks selected therefrom, more preferably comprises 3 diffraction peaks selected therefrom; optionally, further may comprise at least one diffraction peak at 2θ of 20.0+0.2°, 25.2±0.2°, 15.1±0.2°, 16.7±0.2°, and 25.7±0.2°, and preferably comprises 2, 3, 4, or 5 diffraction peaks selected therefrom.

For example, the X-ray powder diffraction pattern of the crystal form A of the isethionate has diffraction peaks at 20 of:

5.4±0.2° and 20.0±0.2°;
or 5.4±0.2°, 20.0±0.2°, and 25.2±0.2°;
or 5.4±0.2°, 20.0±0.2°, 25.2±0.2°, and 15.1±0.2°;
or 5.4±0.2°, 20.0±0.2°, 25.2±0.2°, 15.1±0.2°, 16.7±0.2°, and 25.7±0.2°.

The X-ray powder diffraction pattern of the crystal form A of the isethionate optionally further comprises one or more diffraction peaks at 2θ of 12.7±0.2°, 19.5±0.2°, 22.1±0.2°, 8.4±0.2°, 23.7±0.2°, 28.3±0.2°, and 9.9±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the isethionate has diffraction peaks at 20 of:

5.4±0.2°, 16.1±0.2°, 20.9±0.2°,20.0±0.2°, 25.2±0.2°, 15.1±0.2°, 12.7±0.2°, and 19.5±0.2°;
or 5.4±0.2°, 16.1±0.2°, 20.9±0.2°, 20.0±0.2°, 25.2±0.2°, 15.1±0.2°, 12.7±0.2°, 19.5±0.2°, 22.1±0.2°, and 8.4 ±0.2°.

The X-ray powder diffraction pattern of the crystal form A of the isethionate comprises one or more diffraction peaks at 20 of 5.4±0.2°, 16.1±0.2°, 20.9±0.2°, 20.0+0.2°, 25.2±0.2°, 15.1±0.2°, 16.7±0.2°, 25.7±0.2°, 12.7±0.2°, 19.5±0.2°, 22.1±0.2°, 8.4±0.2°, 23.7±0.2°, 28.3±0.2°, and 9.9±0.2°; and preferably comprises diffraction peaks optionally at 4, 6, 8, or 10 positions selected therefrom.

For example, the X-ray powder diffraction pattern of the crystal form A of the isethionate has diffraction peaks at 20 of:

5.4±0.2°, 16.1±0.2°, 20.9±0.2°, 20.0±0.2°, 25.2±0.2°, 15.1±0.2°, 16.7±0.2°, and 25.7±0.2°;
or 5.4±0.2°, 16.1±0.2°, 20.9±0.2°, 20.0±0.2°, 25.2±0.2°, 15.1±0.2°, 16.7±0.2°, 25.7±0.2°, 12.7±0.2°, and 19.5 ±0.2°.

The X-ray powder diffraction pattern of the crystal form A of the isethionate comprises one or more diffraction peaks at 20 of 5.4±0.2°, 16.1±0.2°, 20.9±0.2°, 20.0+0.2°, 25.2±0.2°, 15.1±0.2°, 16.7±0.2°, 25.7±0.2°, 12.7±0.2°, 19.5±0.2°, 22.1±0.2°, 8.4±0.2°, 23.7±0.2°, 28.3±0.2°, 9.9±0.2°, 20.7±0.2°, 16.9±0.2°, 27.0±0.2°, 26.1±0.2°, and 13.6±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the isethionate has diffraction peaks at 20 of:

5.4±0.2°, 16.1±0.2°, 20.9±0.2°, 20.0±0.2°, 25.2±0.2°, 15.1±0.2°, 16.7±0.2°, and 25.7±0.2°;
or 5.4±0.2°, 20.9±0.2°, 20.0±0.2°, 16.7±0.2°, 25.7±0.2°, 12.7±0.2°, 19.5±0.2°, and 22.1±0.2°;
or 5.4±0.2°, 16.1±0.2°, 20.9±0.2°, 20.0±0.2°, 25.2±0.2°, 15.1±0.2°, 16.7±0.2°, 25.7±0.2°, 12.7±0.2°, and 19.5 ±.2°.

Most preferably, its X-ray characteristic diffraction peaks represented by a 20 angle and an interplanar spacing d using Cu-Kα radiation are as shown in Table 23.

Table 23

| Serial No. | XRPD data of crystal form A of isethionate | | | |
|---|---|---|---|---|
| | 2θ (±0.2°) | d value | Peak height | Ratio (I%) |
| 1 | 5.397 | 16.3614 | 623 | 100 |
| 2 | 8.360 | 10.5673 | 49 | 7.9 |
| 3 | 9.944 | 8.8877 | 43 | 6.9 |
| 4 | 12.734 | 6.9457 | 60 | 9.6 |
| 5 | 13.616 | 6.4978 | 33 | 5.3 |
| 6 | 15.057 | 5.8792 | 68 | 10.9 |
| 7 | 16.144 | 5.4855 | 178 | 28.6 |
| 8 | 16.707 | 5.302 | 66 | 10.6 |
| 9 | 16.904 | 5.2407 | 42 | 6.7 |
| 10 | 19.495 | 4.5497 | 58 | 9.3 |
| 11 | 20.036 | 4.428 | 71 | 11.4 |
| 12 | 20.651 | 4.2976 | 43 | 6.9 |
| 13 | 20.891 | 4.2486 | 115 | 18.5 |
| 14 | 22.100 | 4.0189 | 57 | 9.1 |
| 15 | 23.680 | 3.7541 | 48 | 7.7 |
| 16 | 25.185 | 3.5331 | 71 | 11.4 |
| 17 | 25.734 | 3.459 | 61 | 9.8 |
| 18 | 26.117 | 3.4092 | 36 | 5.8 |
| 19 | 26.972 | 3.303 | 40 | 6.4 |
| 20 | 28.262 | 3.1551 | 44 | 7.1 |
| 21 | 32.499 | 2.7528 | 31 | 5 |

Further preferably, the X-ray powder diffraction pattern of the crystal form A of the isethionate is substantially as shown in FIG. 60.

A further preferred embodiment of the present invention provides a crystal form B of an isethionate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide, wherein the number of acids is 1, and its X-ray powder diffraction pattern comprises at least one or more diffraction peaks at 2θ of 5.9±0.2°, 16.7±0.2°, and 21.2±0.2°, preferably comprises 2 diffraction peaks selected therefrom, more preferably comprises 3 diffraction peaks selected therefrom; optionally, further may comprise at least one diffraction peak at 2θ of 19.5±0.2°, 22.5±0.2°, 10.0±0.2°, 13.0±0.2°, and 24.3±0.2°, and preferably comprises 2, 3, 4, or 5 diffraction peaks selected therefrom.

For example, the X-ray powder diffraction pattern of the crystal form B of the isethionate has diffraction peaks at 2θ of:

5.9±90.2° and 19.5±0.2°;
or 5.9±0.2°, 19.5±0.2°, and 22.5±0.2°;
or 5.9±0.2°, 19.5±0.2°, 22.5±0.2°, and 10.0±0.2°;
or 5.9±0.2°, 19.5±0.2°, 22.5±0.2°, 10.0±0.2°, 13.0±0.2°, and 24.3±0.2°.

The X-ray powder diffraction pattern of the crystal form B of the isethionate optionally further comprises one or more diffraction peaks at 2θ of 15.5±0.2°, 17.5±0.2°, 20.1±0.2°, 17.7±0.2°, 26.2±0.2°, 16.9±0.2°, and 27.6±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form B of the isethionate has diffraction peaks at 2θ of:

5.9±0.2°, 16.7±0.2°, 21.24±0.2°,19.54±0.2°, 22.5+0.2°, 10.0+0.2°, 15.5±0.2°, and 17.5±0.2°;
or 5.9±0.2°, 16.7±0.2°, 21.2±0.2°, 19.5±0.2°, 22.5±0.2°, 10.0±0.2°, 15.5±0.2°, 17.5±0.2°, 20.1±0.2°, and 17.7 ±0.2°.

The X-ray powder diffraction pattern of the crystal form B of the isethionate comprises one or more diffraction peaks at 2θ of 5.9±0.2°, 16.7+0.2°, 21.2±0.2°, 19.5±0.2°, 22.5±0.2°, 10.0±0.2°, 13.0±0.2°, 24.3±0.2°, 15.5±0.2°, 17.5±0.2°, 20.1±0.2°, 17.7±0.2°, 26.2±0.2°, 16.9±0.2°, and 27.6±0.2°; and preferably comprises diffraction peaks optionally at 4, 6, 8, or 10 positions selected therefrom.

For example, the X-ray powder diffraction pattern of the crystal form B of the isethionate has diffraction peaks at 2θ of:

5.9±0.2°, 16.7±0.2°, 21.2±0.2°, 19.5±0.2°, 22.5+0.2°, 10.0+0.2°, 13.0+0.2°, and 24.3±0.2°;
or 5.9±0.2°, 16.7±0.2°, 21.2±0.2°, 19.5±0.2°, 22.5±0.2°, 10.0±0.2°, 13.0±0.2°, 24.3±0.2°, 15.5±0.2°, and 17.5 ±0.2°.

The X-ray powder diffraction pattern of the crystal form B of the isethionate comprises one or more diffraction peaks at 2θ of 5.9±0.2°, 16.7+0.2°, 21.2±0.2°, 19.5±0.2°, 22.5±0.2°, 10.0±0.2°, 13.0±0.2°, 24.3±0.2°, 15.5±0.2°, 17.5±0.2°, 20.1±0.2°, 17.7±0.2°, 26.2±0.2°, 16.9±0.2°, 27.6±0.2°, 18.9±0.2°, 25.8±0.2°, 30.4±0.2°, 25.1±0.2°, and 22.1±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form B of the isethionate has diffraction peaks at 2θ of:

5.9±0.2°, 16.7±0.2°, 21.2±0.2°, 19.5±0.2°, 22.5+0.2°, 10.0+0.2°, 13.0+0.2°, and 24.3±0.2°;
or 5.9±0.2°, 16.7±0.2°, 22.5+0.2°, 10.0+0.2°, 13.0+0.2°, 24.3±0.2°, 15.5±0.2°, and 17.5±0.2°;
or 5.9±0.2°, 16.7±0.2°, 21.2±0.2°, 19.5±0.2°, 22.5±0.2°, 10.0±0.2°, 13.0±0.2°, 24.3±0.2°, 15.5±0.2°, and 17.5 ±0.2°.

Most preferably, its X-ray characteristic diffraction peaks represented by a 2θ angle and an interplanar spacing d using Cu-Kα radiation are as shown in Table 24.

Table 24

| Serial No. | XRPD data of crystal form B of isethionate | | | |
|---|---|---|---|---|
| | 2θ (±0.2°) | d value | Peak height | Ratio (I%) |
| 1 | 5.864 | 15.0599 | 597 | 100 |
| 2 | 10.040 | 8.803 | 234 | 39.2 |
| 3 | 13.040 | 6.7835 | 215 | 36 |
| 4 | 15.454 | 5.7291 | 167 | 28 |
| 5 | 16.667 | 5.3146 | 502 | 84.1 |
| 6 | 16.914 | 5.2377 | 102 | 17.1 |
| 7 | 17.462 | 5.0746 | 165 | 27.6 |
| 8 | 17.723 | 5.0004 | 116 | 19.4 |
| 9 | 18.860 | 4.7015 | 85 | 14.2 |
| 10 | 19.469 | 4.5556 | 267 | 44.7 |
| 11 | 20.135 | 4.4064 | 126 | 21.1 |
| 12 | 21.189 | 4.1895 | 315 | 52.8 |
| 13 | 22.066 | 4.025 | 56 | 9.4 |
| 14 | 22.513 | 3.946 | 241 | 40.4 |
| 15 | 24.252 | 3.6669 | 193 | 32.3 |
| 16 | 25.081 | 3.5476 | 58 | 9.7 |

(continued)

| Serial No. | XRPD data of crystal form B of isethionate | | | |
|---|---|---|---|---|
| | 2θ (±0.2°) | d value | Peak height | Ratio (I%) |
| 17 | 25.751 | 3.4567 | 80 | 13.4 |
| 18 | 26.222 | 3.3958 | 109 | 18.3 |
| 19 | 27.558 | 3.2341 | 101 | 16.9 |
| 20 | 28.163 | 3.1659 | 43 | 7.2 |
| 21 | 29.506 | 3.0248 | 44 | 7.4 |
| 22 | 30.360 | 2.9417 | 69 | 11.6 |

Further preferably, the crystal form B of the isethionate is substantially as shown in FIG. 61; its DSC pattern is substantially as shown in FIG. 62; and its TGA pattern is substantially as shown in FIG. 63.

A further preferred embodiment of the present invention provides a crystal form A of a 1,5-napadisylate of (3R)-N-(4-(chlorodifluoromethoxy)phenyl)-2-(difluoromethyl)-3-methyl-3,4,5a, 6-tetrahydro-5-oxa-1,2a,6,8-tetraazabenzo[5,7]cyclooctyl[1,2,3-cd]indene-11-carboxamide, wherein the number of acids is 1, and its X-ray powder diffraction pattern comprises at least one or more diffraction peaks at 2θ of 21.3±0.2°, 10.2±0.2°, and 9.5±0.2°, preferably comprises 2 diffraction peaks selected therefrom, more preferably comprises 3 diffraction peaks selected therefrom; optionally, further may comprise at least one diffraction peak at 2θ of 17.1±0.2°, 9.9±0.2°, 16.7±0.2°, 25.8±0.2°, and 4.5 ±0.2°, and preferably comprises 2, 3, 4, or 5 diffraction peaks selected therefrom.

For example, the X-ray powder diffraction pattern of the crystal form A of the 1,5-napadisylate has diffraction peaks at 2θ of:

21.3±0.2° and 17.1±0.2°;
or 21.3±0.2°, 17.1±0.2°, and 9.9±0.2°;
or 21.3±0.2°, 17.1±0.2°, 9.9±0.2°, and 16.7±0.2°;
or 21.3±0.2°, 17.1±0.2°, 9.9±0.2°, 16.7±0.2°, 25.8±0.2°, and 5.7±0.2°.

The X-ray powder diffraction pattern of the crystal form A of the 1,5-napadisylate optionally further comprises one or more diffraction peaks at 2θ of 8.0±0.2°, 23.7±0.2°, 23.0±0.2°, 18.2±0.2°, 19.9±0.2°, 12.2±0.2°, and 13.7±0.2°; preferably comprises at least any 2-3, or 4-5, or 6-7 diffraction peaks selected therefrom, and further preferably comprises any 2, 3, 4, 6, or 7 diffraction peaks selected therefrom.

For example, the X-ray powder diffraction pattern of the crystal form A of the 1,5-napadisylate has diffraction peaks at 2θ of:

21.3±0.2°, 10.2±0.2°, 9.5±0.2°, 17.1±0.2°, 9.94±0.2°, 16.7±0.2°, 8.0±0.2°, and 23.7±0.2°;
or 21.3±0.2°, 10.210.2°, 9.510.2°, 17.1±0.2°, 9.9±0.2°, 16.7±0.2°, 8.0+0.2°, 23.7+0.2°, 23.0+0.2°, and 18.2±0.2°.

The X-ray powder diffraction pattern of the crystal form A of the 1,5-napadisylate comprises one or more diffraction peaks at 2θ of 21.3±0.2°, 10.2±0.2°, 9.5±0.2°, 17.1±0.2°, 9.9±0.2°, 16.7±0.2°, 25.8±0.2°, 5.7±0.2°, 8.0 ±0.2°, 23.7±0.2°, 23.0±0.2°, 18.2±0.2°, 19.9±0.2°, 12.2±0.2°, and 13.7±0.2°; and preferably comprises diffraction peaks optionally at 4, 6, 8, or 10 positions selected therefrom.

For example, the X-ray powder diffraction pattern of the crystal form A of the 1,5-napadisylate has diffraction peaks at 2θ of:

21.3±0.2°, 10.2±0.2°, 9.5±0.2°, 17.1±0.2°, 9.94±0.2°, 16.7±0.2°, 25.8±0.2°, and 5.7±0.2°;
or 21.3±0.2°, 10.2±0.2°, 9.5±0.2°, 17.1±0.2°, 9.9±0.2°, 16.7±0.2°, 25.8±0.2°, 5.7±0.2°, 8.0±0.2°, and 23.7 ±0.2°.

The X-ray powder diffraction pattern of the crystal form A of the 1,5-napadisylate comprises one or more diffraction peaks at 2θ of 21.3±0.2°, 10.2±0.2°, 9.5±0.2°, 17.1±0.2°, 9.9±0.2°, 16.7±0.2°, 25.8±0.2°, 5.7±0.2°, 8.0 ±0.2°, 23.7±0.2°, 23.0±0.2°, 18.2±0.2°, 19.9+0.2°, 12.2±0.2°, 13.7±0.2°, 22.3±0.2°, 14.4±0.2°, 13.4±0.2°, 24.3 ±0.2°, and 29.1±0.2°; preferably comprises at least diffraction peaks at any 2-3, or 4-5, or 7-8, or 10-12, or 15-18 positions selected therefrom; and further preferably comprises diffraction peaks at any 2, 3, 4, 6, 8, 10, 12, 16, or 18 positions selected therefrom.

For example, the X-ray powder diffraction pattern of the crystal form A of the 1,5-napadisylate has diffraction peaks at 2θ of:

21.3±0.2°, 10.2±0.2°, 9.5±0.2°,17.1±0.2°, 9.9±0.2°, 16.7±0.2°, 25.8±0.2°, and 5.7±0.2°;
or 21.3±0.2°, 17.1±0.2°, 9.94±0.2°, 16.7±0.2°, 23.0±0.2°, 18.2±0.2°, 19.9±0.2°, and 12.2±0.2°;
or 21.3±0.2°, 10.2±0.2°, 9.5±0.2°, 17.1±0.2°, 9.9±0.2°, 16.7±0.2°, 25.8±0.2°, 5.7±0.2°, 8.0±0.2°, and 23.7 ±0.2°.

Most preferably, its X-ray characteristic diffraction peaks represented by a 20 angle and an interplanar spacing d using Cu-Kα radiation are as shown in Table 25.

Table 25

| Serial No. | XRPD data of crystal form A of 1,5-napadisylate | | | |
|---|---|---|---|---|
| | 2θ (±0.2°) | d value | Peak height | Ratio (I%) |
| 1 | 5.665 | 15.5875 | 97 | 51.9 |
| 2 | 7.176 | 12.3084 | 45 | 24.1 |
| 3 | 7.973 | 11.0801 | 86 | 46 |
| 4 | 9.474 | 9.327 | 180 | 96.3 |
| 5 | 9.918 | 8.9106 | 113 | 60.4 |
| 6 | 10.242 | 8.6297 | 184 | 98.4 |
| 7 | 11.298 | 7.8252 | 44 | 23.5 |
| 8 | 12.174 | 7.2642 | 73 | 39 |
| 9 | 13.382 | 6.6111 | 62 | 33.2 |
| 10 | 13.650 | 6.4819 | 71 | 38 |
| 11 | 14.422 | 6.1365 | 64 | 34.2 |
| 12 | 16.689 | 5.3076 | 108 | 57.8 |
| 13 | 17.097 | 5.1819 | 141 | 75.4 |
| 14 | 18.191 | 4.8728 | 76 | 40.6 |
| 15 | 19.447 | 4.5606 | 38 | 20.3 |
| 16 | 19.875 | 4.4634 | 76 | 40.6 |
| 17 | 20.622 | 4.3034 | 44 | 23.5 |
| 18 | 21.334 | 4.1614 | 187 | 100 |
| 19 | 22.326 | 3.9786 | 68 | 36.4 |
| 20 | 22.992 | 3.865 | 80 | 42.8 |
| 21 | 23.725 | 3.7472 | 83 | 44.4 |
| 22 | 24.337 | 3.6543 | 58 | 31 |
| 23 | 25.046 | 3.5524 | 36 | 19.3 |
| 24 | 25.789 | 3.4518 | 107 | 57.2 |
| 25 | 29.054 | 3.0708 | 48 | 25.7 |
| 26 | 31.528 | 2.8353 | 36 | 19.3 |

Further preferably, the X-ray powder diffraction pattern of the crystal form A of the 1,5-napadisylate is substantially as shown in FIG. 64; its DSC pattern is substantially as shown in FIG. 65; and its TGA pattern is substantially as shown in FIG. 66.

In a further preferred embodiment of the present invention, 20 errors between diffraction peaks with top ten relative peak intensities in the X-ray powder diffraction patterns of the crystal form A of the ethylsulfonate, the crystal form B

of the ethylsulfonate, the crystal form A of the tosylate, the crystal form B of the tosylate, the crystal form A of the hydrochloride, the crystal form B of the hydrochloride, the crystal form C of the hydrochloride, the crystal form A of the sulfate, the crystal form B of the sulfate, the crystal form C of the sulfate, the crystal form D of the sulfate, the crystal form E of the sulfate, the crystal form F of the sulfate, the crystal form G of the sulfate, the crystal form A of the p-tosylate, the crystal form B of p-tosylate, the crystal form C of the p-tosylate, the crystal form D of the p-tosylate, the crystal form E of the p-tosylate, the crystal form A of the besylate, the crystal form B of the besylate, the crystal form C of the besylate, the crystal form A of the isethionate, the crystal form B of the isethionate, and the crystal form A of the 1,5-napadisylate of the compound represented by formula (I) and diffraction peaks at corresponding positions in FIGS. 1, 4, 7, 10, 12, 15, 16, 17, 20, 23, 26, 29, 32, 35, 38, 41, 44, 47, 50, 53, 54, 57, 60, 61, and 64 are $\pm0.2°$-$\pm0.5°$ respectively, preferably $\pm0.2°$-$\pm0.3°$, and most preferably $\pm0.2°$.

In a preferable embodiment of the present invention, the crystal form of the acid salt of any one of the compounds of formula (I) is a hydrate or an anhydrate; when the crystal form of the acid salt is a hydrate, the number of water is 0.2-3, preferably 0.2, 0.5, 1, 1.5, 2, 2.5, or 3, more preferably 0.5, 1, 2, or 3; and further, water in the hydrate is pipe water or crystal water or a combination thereof.

In a preferred embodiment of the present invention, the crystal form of the acid salt of any one of the compounds of formula (I) in is an anhydrate.

As is well known to those with ordinary skills in the art, the XRPD may produce certain shifts and intensity deviations due to the detection methods, conditions, and instruments. As a specific example of a crystal form of the present invention, its XRPD is as shown in pattern X. However, those with ordinary skills in the art will understand that when 2θ shift of key characteristic peaks deviates within $\pm0.5$, particularly around $\pm0.2$, they can be identified as a same crystal form.

The present invention further provides a method for preparing the crystal form of the acid salt of the compound represented by formula (I), comprising steps of:

1) weighing an appropriate amount of a free base and dissolving the free base in a good solvent;
2) weighing an appropriate amount of an acid, and optionally dissolving the acid in an organic solvent; preferably the amount of the acid is 1.0-1.5 equivalents;
3) combining the above two solutions and stirring for precipitation, or adding a poor solvent dropwise and then stirring for precipitation; and
4) rapidly centrifuging the resulting system or allowing the system to stand still, and drying the system to obtain a target product;

wherein:

the good solvent is selected from acetone, toluene, acetonitrile, methanol, isopropanol, dichloromethane, tetrahydrofuran, ethyl formate, isopropyl acetate, toluene, ethyl acetate, 2-methyltetrahydrofuran, 2-butanone, n-butanol, 1,4-dioxane, isobutanol, N,N-dimethylformamide, N,N-dimethylacetamide, n-propanol, or tert-butanol; preferably toluene, ethyl acetate, acetone, methanol, or acetonitrile;

the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyltetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and the organic solution need, when used, to be mutually soluble; and

the poor solvent is selected from heptane, water, methyl tert-butyl ether, cyclohexane, toluene, isopropyl ether, ethyl acetate, acetone, or acetonitrile; preferably water, methyl tert-butyl ether, or isopropyl ether;

or specifically comprising steps of:

1) weighing an appropriate amount of a free base and suspending the free base in a poor solvent;
2) weighing an appropriate amount of an acid, and dissolving the acid in an organic solvent; preferably the amount of the acid is 1.0-1.5 equivalents;
3) adding the solution from the above step 2) to the suspension from the above step 1) while stirring; and
4) rapidly centrifuging the resulting system or allowing the system to stand still, and drying the system to obtain a target product;

wherein:

the poor solvent is selected from ethanol, acetone, ethyl acetate, ethyl formate, isopropanol, isopropyl acetate, methyl tert-butyl ether, methanol, 1,4-dioxane, 2-butanone, 2-methyltetrahydrofuran, anisole, acetonitrile, chlorobenzene, benzene, toluene, n-butanol, isobutanol, or 3-pentanone; preferably ethanol, 2-methyltetrahydrofuran, acetonitrile, methanol, or ethyl acetate;

the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyltetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and the organic solution need, when used, to be mutually soluble; and

the acid is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, decyclic acid, hexanoic acid, octanoic acid, cinnamic acid, citric acid, cyclamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecylsulfuric acid, dibenzoyltartaric acid, ethane-1,2-disulfonic acid, ethylsulfonic acid, formic acid, fumaric acid, galactonic acid, gentisic acid, glutaric acid, 2-ketoglutaric acid, glycolic acid, benzoylglycine, isethionic acid, lactobionic acid, ascorbic acid, aspartic acid, lauric acid, camphoric acid, maleic acid, malonic acid, methylsulfonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, pamoic acid, formic acid, undecylenic acid, trifluoroacetic acid, benzenesulfonic acid, p-toluenesulfonic acid, or L-malic acid; preferably hydrobromic acid, hydrochloric acid, sulfuric acid, p-toluenesulfonic acid, methylsulfonic acid, benzenesulfonic acid, oxalic acid, acetic acid, ethylsulfonic acid, maleic acid, phosphoric acid, fumaric acid, succinic acid, malonic acid, adipic acid, malic acid, tartaric acid, 1,5-naphthalenedisulfonic acid, isethionic acid, citric acid, hippuric acid, lactic acid, benzoic acid, palmitic acid, or salicylic acid; more preferably hydrobromic acid, ethylsulfonic acid, methylsulfonic acid, sulfuric acid, hydrochloric acid, p-toluenesulfonic acid, benzenesulfonic acid, isethionic acid, 1,5-naphthalenedisulfonic acid, maleic acid, fumaric acid, succinic acid, malic acid, or tartaric acid; and further preferably ethylsulfonic acid, methanesulfonic acid, sulfuric acid, hydrochloric acid, p-toluenesulfonic acid, benzenesulfonic acid, isethionic acid, or 1,5-naphthalenedisulfonic acid.

The present invention further provides a method for preparing the crystal form of the acid salt of the compound represented by formula (I), comprising steps of:

1) weighing an appropriate amount of a salt of a compound and suspending the salt in a poor solvent; and
2) shaking the suspension from the step 1);
3) rapidly centrifuging the suspension from the step 2), removing the supernatant, and drying residual solid to a constant weight to obtain a target product;

wherein:

the poor solvent is selected from methanol, ethanol, dichloromethane, 1,4-dioxane, acetonitrile, dichloromethane, chlorobenzene, chloroform, benzene, toluene, acetone, ethyl acetate, water, 88% acetone, isopropyl acetate, 3-pentanone, ethyl formate, tetrahydrofuran, 2-methyltetrahydrofuran, isopropanol, n-butanol, isobutanol, n-propanol, methyl tert-butyl ether, n-heptane, tert-butanol, or 2-butanone.

An objective of the present invention is to further provide a pharmaceutical composition, comprising a therapeutically effective amount of the crystal form of the compound represented by the general formula (I), and one or more pharmaceutically acceptable carriers or excipients.

The present invention further provides use of the acid salt of the compound represented by formula (I) or the crystal form thereof, and the pharmaceutical composition in manufacturing a tyrosine kinase active drug that inhibits a protein selected from an Abelson protein (ABL1), an Abelson-related protein (ABL2), and a chimeric protein BCR-ABL1.

The present invention further provides use of the acid salt of the compound represented by formula (I) or the crystal form thereof and the pharmaceutical composition in manufacturing a drug for the treatment of a leukemia-related disease, wherein preferably, the leukemia is chronic myeloid leukemia (CML), acute myeloid leukemia (AML), or acute lymphocytic leukemia (ALL); more preferably, the CML is resistant to the treatment with one or more of standard treatments of care such as imatinib, nilotinib, and dasatinib, and the AML is secondary AML that develops after myelodysplastic syndrome (MDS) or myeloproliferative neoplasm (MPN).

The present invention further provides use of the acid salt of the compound represented by formula (I) or the crystal form thereof and the pharmaceutical composition in manufacturing a drug for the treatment of a cancer-related disease, wherein preferably, the cancer is selected from melanoma, hereditary leiomyomatosis, renal cell carcinoma (HLRCC), brain cancer, glioblastoma, or other solid tumors.

The present invention further provides use of the acid salt of the compound represented by formula (I) or the

crystal form thereof and the pharmaceutical composition in manufacturing a drug for the treatment of a central nervous system disease-related disease, wherein preferably, the central nervous system disease-related disease is stroke, traumatic brain or spinal cord injury, Alzheimer's disease, Parkinson's disease, Huntington's disease, or motor neuron disease.

## DETAILED DESCRIPTION OF THE INVENTION

Unless stated to the contrary, the terms used in the specification and claims have the following meanings.

The term "alkyl" refers to a saturated aliphatic hydrocarbon group, which is a linear or branched group comprising 1 to 20 carbon atoms, preferably alkyl group comprising 1 to 8 carbon atoms, more preferably an alkyl group of 1 to 6 carbon atoms, and most preferably an alkyl group of 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferred are lower alkyl containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, and may be, when substituted, substituted with a substituent at any usable attachment point, wherein the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate, and is preferably methyl, ethyl, isopropyl, tert-butyl, haloalkyl, deuteroalkyl, alkoxy-substituted alkyl, and hydroxy-substituted alkyl in the present invention.

The term "aryl" refers to a 6-14-membered all carbon monocyclic or fused polycyclic (i.e., rings sharing an adjacent carbon atom pair) group having a conjugated $\pi$ electron system, and is preferably 6-10-membered, e.g., phenyl and naphthyl. More preferred, it is phenyl. The aryl ring can be fused onto a heteroaryl, heterocyclyl or cycloalkyl ring, including benzo-3-8-membered cycloalkyl, benzo-3-8-membered heteroalkyl, preferably benzo-3-6-membered cycloalkyl, benzo-3-6-membered heteroalkyl, wherein the heterocyclyl is a heterocyclic group comprising 1-3 nitrogen atoms, oxygen atoms, and sulfur atoms; or it also comprises a three-membered nitrogen-containing fused ring containing a benzene ring, wherein the ring connected to the parent structure is an aryl ring.

The aryl may be substituted or unsubstituted, and may be, when substituted, substituted with a substituent that is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

The term "heteroaryl" refers to a heteroaromatic system comprising 1 to 4 heteroatoms and 5 to 14 ring atoms, wherein the heteroatom is selected from oxygen, sulfur and nitrogen. The heteroaryl is preferably 5-10-membered, more preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, or pyrazinyl, preferably triazolyl, thienyl, imidazolyl, pyrazolyl, oxazolyl, pyrimidinyl, or thiazolyl; and more preferably pyrazolyl and oxazolyl. The heteroaryl ring may be fused to an aryl, heterocyclyl or cycloalkyl ring, wherein a ring attached to the parent structure is a heteroaryl ring.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentyloxy, and cyclohexyloxy. The alkoxy may be optionally substituted or unsubstituted, and may be, when substituted, substituted with a substituent which is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate.

The "haloalkyl" refers to alkyl substituted with one or more halogens, wherein the alkyl is as defined above.

The "haloalkoxy" refers to alkoxy substituted with one or more halogens, wherein the alkoxy is as defined above.

The "hydroxyl" refers to a -OH group.

The "carboxyl" refers to -C(O)OH.

Different wordings, such as "X is selected from A, B, or C," "X is selected from A, B, and C," "X is A, B, or C," and "X is A, B, and C," express a same meaning, i.e., X may be any one or more of A, B, and C.

The hydrogen atoms described in the present invention can be replaced by its isotope deuterium, and any hydrogen atom in the example compounds involved in the present invention can also be replaced by a deuterium atom.

"Optional" or "optionally" means that the event or circumstance described subsequently may occur but not necessary to occur, and the description includes the situations in which the event or circumstance occurs or does not occur. For example, "heterocyclic groups optionally substituted with alkyl" means that alkyl may but do not need to be present, and the description includes cases in which heterocyclic groups are substituted with alkyl and cases in which heterocyclic groups are not substituted with alkyl.

"Substituted" means that one or more hydrogen atoms in the group, preferably up to 5, more preferably 1 to 3 hydrogen atoms, are independently substituted with a corresponding number of substituents. It goes without saying that the substituents are only in their possible chemical positions, and those skilled in the art can determine (either experimentally or theoretically) possible or impossible substitutions without undue effort. For example, amino or hydroxyl with a free hydrogen may be unstable when bound to a carbon atom with an unsaturated (such as olefinic) bond. Optional substituents include one or more of deuterium, halogen, amino, hydroxyl, cyano, oxo, thio, alkyl, alkenyl, alkynyl, deuterated alkyl, haloalkyl, hydroxyalkyl, alkoxy, alkylthio, haloalkoxy, cycloalkyl, heterocyclyl, aryl and heteroaryl, preferably deuterium, halogen, amino, hydroxyl, cyano, oxo, thio, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3-12-membered heterocyclyl, $C_{6-14}$ aryl and 5-14-membered heteroaryl.

"Pharmaceutical composition" means a mixture of one or more of the compounds described herein or physiologically/pharmaceutically acceptable salts or prodrugs thereof with other chemical components, and other components such as physiologically/pharmaceutically acceptable carriers and excipients. The purpose of pharmaceutical compositions is to facilitate administration to living organisms and facilitate the absorption of active ingredients to exert biological activity.

Identical samples of a same crystal form usually have identical primary XRPD characteristic peaks, but may have certain operational errors. When those with ordinary skills in the art use same instruments and detection methods to detect samples of a same crystal form obtained using a corresponding method, characteristic peak errors are usually within ±0.2°. (However, different skilled persons using different instruments may occasionally have a few characteristic peaks with errors beyond this range. For example, an error within ±0.5° or ±0.3° should be considered as XRPD characteristic peaks of a same crystal form.) Therefore, peaks with characteristic peak errors within ±0.5°, ±0.3°, or ±0.2° can all be construed as being encompassed within the scope of protection of the present invention.

## BRIEF DESCRIPTION OF THE DRAWINGS

FIGS. 1-3 show XRPD, DSC, and TGA patterns of a crystal form A of an ethylsulfonate.
FIGS. 4-6 show XRPD, DSC, and TGA patterns of a crystal form B of an ethylsulfonate.
FIGS. 7-9 show XRPD, DSC, and TGA patterns of a crystal form A of a mesylate.
FIGS. 10-11 show XRPD and DSC patterns of a crystal form B of a mesylate.
FIGS. 12-14 show XRPD, DSC, and TGA patterns of a crystal form A of a hydrochloride.
FIG. 15 shows an XRPD pattern of a crystal form B of a hydrochloride.
FIG. 16 shows an XRPD pattern of a crystal form C of a hydrochloride.
FIGS. 17-19 show XRPD, DSC, and TGA patterns of a crystal form A of a sulfate.
FIGS. 20-22 show XRPD, DSC, and TGA patterns of a crystal form B of a sulfate.
FIGS. 23-25 show XRPD, DSC, and TGA patterns of a crystal form C of a sulfate.
FIGS. 26-28 show XRPD, DSC, and TGA patterns of a crystal form D of a sulfate.
FIGS. 29-31 show XRPD, DSC, and TGA patterns of a crystal form E of a sulfate.
FIGS. 32-34 show XRPD, DSC, and TGA patterns of a crystal form F of a sulfate.
FIGS. 35-37 show XRPD, DSC, and TGA patterns of a crystal form G of a sulfate.
FIGS. 38-40 show XRPD, DSC, and TGA patterns of a crystal form A of a p-tosylate.
FIGS. 41-43 show XRPD, DSC, and TGA patterns of a crystal form B of a p-tosylate.
FIGS. 44-46 show XRPD, DSC, and TGA patterns of a crystal form C of a p-tosylate.
FIGS. 47-49 show XRPD, DSC, and TGA patterns of a crystal form D of a p-tosylate.
FIGS. 50-52 show XRPD, DSC, and TGA patterns of a crystal form E of a p-tosylate.
FIG. 53 shows an XRPD pattern of a crystal form A of a besylate.
FIGS. 54-56 show XRPD, DSC, and TGA patterns of a crystal form B of a besylate.
FIGS. 57-59 show XRPD, DSC, and TGA patterns of a crystal form C of a besylate.
FIG. 60 shows an XRPD pattern of a crystal form A of an isethionate.
FIGS. 61-63 show XRPD, DSC, and TGA patterns of a crystal form B of an isethionate.

FIGS. 64-66 show XRPD, DSC, and TGA patterns of a crystal form A of a 1,5-napadisylate.

## DETAILED DESCRIPTION

### I. Preparation of compounds

The present invention will be further described below with reference to the examples, but these examples are not intended to limit the scope of the present invention.

Example 1 p-tosylate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-ed]indene-11-carboxamide

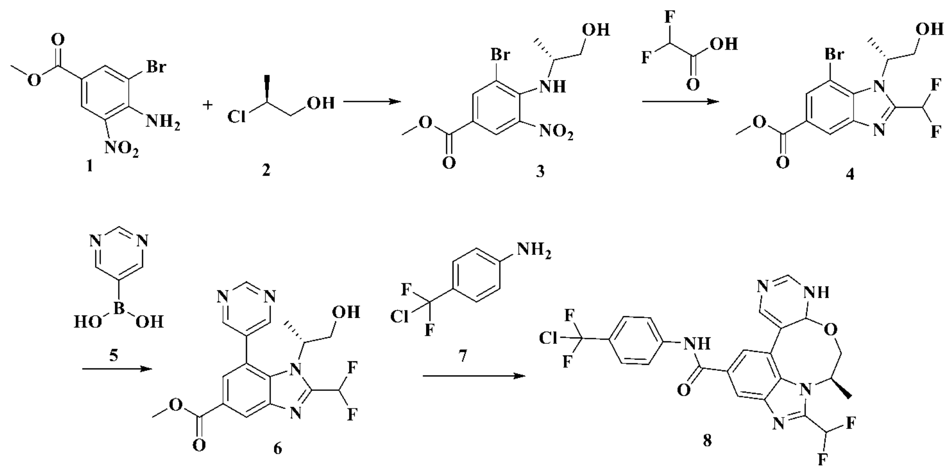


To a 500 mL reaction flask, 20 g of methyl 4-amino-3-bromo-5-nitrobenzoate (compound 1), 5.30 g of cetyltrimethylammonium bromide, 20.07 g of potassium carbonate, and 200 mL of acetonitrile were added. A solution of (*S*)-2-chloro-1-propanol in acetonitrile solution (10.3 g/50 mL) was added dropwise while stirring at room temperature. After the dropwise addition was completed, the mixture was heated to reflux to react for 8 h. After the reaction was completed, the reaction mixture was concentrated to dryness, ethyl acetate and water were added to the residue, and the mixture was extracted. The organic phase was washed with saturated sodium chloride and dried over anhydrous sodium sulfate. The system was filtered, and the filtrate was purified by column chromatography to obtain 13.32 g of compound 3, with a yield of 55%.

To a 100 mL reaction flask, 10 g of methyl (*R*)-3-bromo-4-[(1-hydroxyisopropan-2-yl)amino]-5-nitrobenzoate (compound 3) and 38 mL of difluoroacetic acid were added, 16.76 g of iron powder was added at room temperature, and the mixture was heated to 40-50°C to react for 3 h. The reaction system was cooled, and ethyl acetate was added thereto. The reaction system was filtered. The filtrate was washed once with water and saturated sodium chloride respectively, dried over anhydrous sodium sulfate, and filtered. The filtrate was concentrated and directly put into the next reaction step.

To a 250 mL three-necked flask, 15 g of methyl (*R*)-7-bromo-2-(difluoromethyl)-1-[1-hydroxyprop-2-yl]-1H-benzo[d]imidazol-5-carboxylate (compound 4), 16.2 g of potassium acetate, 7.9 g of potassium fluoride, 105 mL of 1,4-dioxane, and 15 mL of water were added, and the system was subjected to nitrogen replacement for 3 times. 2.4 g of $Pd(PPh_3)_4$ was added, nitrogen replacement was carried out for 3 times, the system was heated to 80°C, and a solution of 5-pyrimidineboronic acid in tetrahydrofuran (6.1g/15mL) was added dropwise. After the dropwise addition was completed, the reaction was further stirred for 5 h, and after TLC monitoring showed that the raw materials reacted completely, the system was cooled to room temperature. 250 mL of water, 120 mL of ethyl acetate, and 3 g of N-acetyl cysteine were added to the reaction system, the system was stirred for 0.5 h, and 3 L of a saturated sodium bicarbonate solution was added. The system was separated into layers, and the aqueous phase was extracted with 120 mL of ethyl acetate. The organic phases were combined, washed with 5 L of a saturated sodium chloride solution, concentrated to dryness, and purified by column chromatography to obtain 10.3 g of compound 6 with a yield of 69%.

To a 250 mL reaction flask, 10 g of compound 6, 5.8 g of 4-(chlorodifluoromethoxy)aniline and 100 mL of tetrahydrofuran were added, and 44 mL of lithium bistrimethylsilylamide (1.0 M in THF) was added dropwise at 5-10°C. After the dropwise addition was completed, the system was stirred to react at room temperature for 15 h. 100 mL of a saturated aqueous ammonium chloride solution was added dropwise to the reaction mixture to quench the reaction, 200

mL of ethyl acetate was added for extraction, and the organic phase was concentrated to dryness. 100 mL of ethyl acetate was added to the concentrate, the concentrate was heated to 50°C, stirred for 0.5 h, and 50 mL of n-heptane was slowly added dropwise. The system was further stirred for 0.5 h, then cooled to 20-30°C, stirred for 3 h, filtered, and dried under vacuum to obtain 9.1g of an off-white solid, with a yield of 82%.

$^1$HNMR (400Hz, Acetone-$d_6$) δ10.13 (s, 1H), 9.51 (s, 1H), 9.37 (s, 1H), 9.09-9.10 (d, *J*=2.4 Hz, 1H), 7.90-7.92 (m, 3H), 7.7.81 (s, 1H), 7.74 (t, *J*=52.4 Hz, 1H), 7.27-7.29 (d, *J*=8.4 Hz, 2H), 6.04 (s, 1H), 4.66-4.67 (m, 1H), 3.98-4.04 (m, 1H), 3.73-3.77 (m, 1H), 1.56-1.58 (d, *J*=6.8 Hz, 3H).

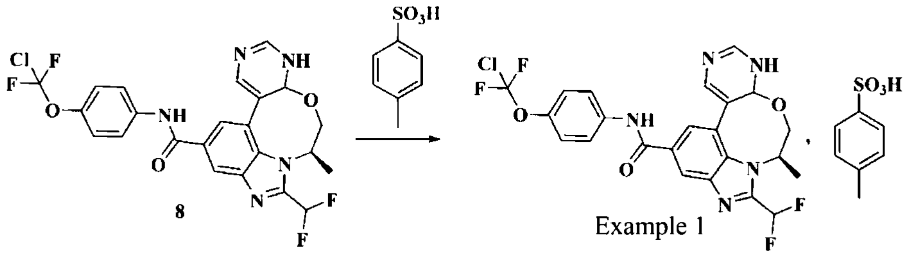

8

Example 1

9 g of compound 8 was weighed, and 90 mL of acetonitrile was added. The system was heated to 50°C, and stirred at this temperature for 30 min. A solution of p-toluenesulfonic acid in ethanol (4 g/20 mL) was added. After the addition was completed, the system was kept at this temperature for reaction for 22-24 h. The system was filtered, and the filter cake was dried under vacuum at 50°C for 24 h to obtain the product (10.1 g, yield: 84%).

Other compounds and salts were prepared with reference to Example 1.

## Biological test and evaluation

The present invention is further described and interpreted below with reference to the test examples, but these examples are not intended to limit the scope of the present invention.

### (I) Study on inhibitory activity of compounds of the present invention against ABL1 WT kinase

1. Test objective

The test objective was to evaluate the in vitro inhibitory activity of the compounds of the present invention against ABL1 WT kinase.

2. Test methods

Capillary electrophoresis was used to detect the phosphorylation conversion rate of the substrate peptide to determine the $IC_{50}$ value of the test compound for inhibiting the kinase (ABL1-WT). The maximum concentration of the compound tested in the test was 1000 nM, with 3-fold dilution, and a total of 12 concentrations (1000 - 0.0056 nM). First, an enzyme reaction system (the concentration of enzyme ABL1-WT was 1.3 nM, the concentration of substrate FLPeptide2 was 1.5 μM, and the reaction factor was 10 mM $MgCl_2$) was prepared. After incubation at room temperature for 30 min, 5 μL of 4×ATP solution was added to start the enzyme reaction. After reacting at room temperature for 90 min, the reaction was stopped by adding a stop buffer (containing 0.5 M EDTA). The sample was analyzed using an EZ reader (analysis conditions: pressure -1.5PSI, upper voltage current -2250V, lower voltage current -500V, separation time 40 sec, system delay 100.0 sec).

3. Data processing

From the conversion rate read by EZ Reader, the remaining activity was calculated according to the following formula.

$$\text{Remaining Activity (\%)} = 100\% \times \frac{CR_{Sample} - CR_{LC}}{CR_{HC} - CR_{LC}}$$

$IC_{50}$ was calculated using XLfit, and $IC_{50}$ was calculated by Formula 201 as the fitting formula.

4. Test results

**Table 1. In vitro enzymatic inhibitory activity of the compounds of the present invention against ABL1 WT kinase**

| Compound | Maximum concentration (μM) | ABL1-WT $IC_{50}$ (nM) |
|---|---|---|
| Example 1 | 1 | 0.43 |

In vitro enzymatic assay shows that the compounds of the present invention show favorable inhibitory activity against the ABL1 WT kinase.

**(II) Inhibitory activity against proliferation of BaF3 cell strains overexpressing different BCR-ABL1 fusion mutations**

1. Test objective

The test objective was to evaluate the inhibitory activity of the compounds of the present invention against proliferation of the Ba/F3 BCR-ABL1-T315I, Ba/F3 BCR-ABL1-E255K, Ba/F3 BCR-ABL1-E255V, and Ba/F3 BCR-ABL1-G250E mutation models of mouse primary B cells cultured in vitro.

2. Cell strains

| Cell line | Cell type |
|---|---|
| Ba/F3 BCR-ABL1-T315I | Mouse primary B cells<br>Overexpressing BCR-ABL1 fusion gene, and amino acid at position 315 on ABL1 being mutated to I |
| Ba/F3 BCR-ABL1-E255V | Mouse primary B cells<br>Overexpressing BCR-ABL1 fusion gene, and amino acid at position 255 on ABL1 being mutated to V |
| Ba/F3 BCR-ABL1-E255K | Mouse primary B cells<br>Overexpressing BCR-ABL1 fusion gene, and amino acid at position 255 on ABL1 being mutated to K |
| Ba/F3 BCR-ABL1-G250E | Mouse primary B cells<br>Overexpressing BCR-ABL1 fusion gene, and amino acid at position 250 on ABL1 being mutated to E |

3. Test methods

CellTiter-Glo® Luminescent Cell Viability Assay was used to detect the inhibitory effect of drugs on tumor cell proliferation and growth. The logarithmic growth phase cells cultured overnight were directly taken, pipetted up and down, mixed and counted, the cell density was adjusted according to different cell density requirements, and the cells were prepared into a cell suspension and plated on a 96-well plate. Drugs at different concentrations were added, 3 replicate wells were set for each concentration, and corresponding vehicle controls were set. The maximum test concentration in Ba/F3 BCR-ABL1-T315I and Ba/F3 BCR-ABL1-E255K cells was 10000 nM, with 3.16-fold gradient dilution and a total of 9 concentrations (10000-1.0058 nM). The maximum concentration of the compounds detected in other cells was 500 nM, with 3.16-fold gradient dilution, and a total of 9 concentrations (500-0.0503 nM). The cells with the compound being added were cultured for another 72 h at 37°C and 5% $CO_2$. The culture plate and its contents were equilibrated to room temperature, the CellTiter-Glo® reagent was added, the contents were mixed on a shaker for 5 min to induce cell lysis, the culture plate was further incubated at room temperature in the dark for 20 min, and the luminescence was read with a microplate reader.

4. Test results

**Table 2. The inhibitory activity of the compounds of the present invention against proliferation of BCR-ABL1 mutant cells**

| Compound | $IC_{50}$ (nM) | | | |
|---|---|---|---|---|
| | Ba/F3 BCR-ABL1-T315I | Ba/F3 BCR-ABL1-E255V | Ba/F3 BCR-ABL1-E255K | Ba/F3 BCR-ABL1-G250E |
| Example 1 | 46.76 | 7.15 | 26.27 | 5.36 |

The compounds of the present invention can significantly inhibit the proliferation of Ba/F3 BCR-ABL1-T315I, E255K, E255V, and G250E mutant cells.

**(III). Study on inhibitory activity against proliferation of tumor cell strains carrying BCR-ABL1 fusion mutations**

1. Test objective

The test objective was to evaluate the inhibitory activity of the compounds of the present invention against proliferation of human erythroleukemia cells K562, human peripheral blood basophilic leukemia cells Ku812, and human chronic granulocytic leukemia cells KCL22-s and KCL22-r cultured in vitro.

2. Cell strains

| Cell line | Cell type |
|---|---|
| K562 | Human erythroleukemia cells<br>BCR-ABL1 gene fusion mutation |
| Ku812 | Human peripheral blood basophilic leukemia cells<br>BCR-ABL1 gene fusion mutation |
| KCL22-s | Human chronic granulocytic leukemia cells, BCR-ABL1 gene fusion mutation, imatinib-sensitive strain |
| KCL22-r | Human chronic granulocytic leukemia cells, BCR-ABL1 gene fusion mutation, imatinib-resistant strain |

3. Test methods

CellTiter-Glo® Luminescent Cell Viability Assay was used to detect the inhibitory effect of drugs on tumor cell proliferation and growth. The logarithmic growth phase cells cultured overnight were directly taken, pipetted up and down, mixed and counted, the cell density was adjusted according to different cell density requirements, and the cells were prepared into a cell suspension and plated on a 96-well plate. Drugs at different concentrations were added, three replicate wells were set for each concentration, and corresponding solvent controls were set. The maximum concentration of compound detected was 500 nM, with 3-fold gradient dilution, and a total of 9 concentrations (500-0.076 nM). The cells with the compound being added were cultured for another 72 h at 37°C and 5% $CO_2$. The culture plate and its contents were equilibrated to room temperature, the CellTiter-Glo® reagent was added, the contents was mixed on a shaker for 3 min to induce cell lysis, the culture plate was further incubated at room temperature in the dark for 10 min, and the chemiluminescence signal value was determined by a microplate reader (BioTek SynergyH1).

4. Test results

**Table 3 The inhibitory activity of the compounds of the present invention against proliferation of BCR-ABL1 gene fusion mutant cells**

| Compound | $IC_{50}$ (nM) | | | |
|---|---|---|---|---|
| | K562 | Ku812 | KCL22-s | KCL22-r |
| Example 1 | 13.00 | 4.13 | 2.99 | 14.64 |

The test results show that the compounds of the present invention can significantly inhibit the proliferation of tumor cells carrying BCR-ABL1 fusion mutations.

**(IV). Study on efficacy on a xenograft tumor model of human chronic myeloid leukemia cell strains KCL22-**s

1. Experimental objective:

The experimental objective was to evaluate the efficacy of the test compound on the BALB/c nude mouse subcutaneous xenograft tumor model of the human chronic myeloid leukemia cell strain KCL22-s.

2 Experimental operations and data processing:

2.1 Animals

BALB/c nude mice, 8-10 weeks.

2.2 Preparation for Cell culture and cell suspension

a. A strain of KCL22-s cells was taken out from the cell bank, and the cells were resuscitated with RPMI-1640 medium (RPMI-1640+10% FBS+1% P/S). The resuscitated cells were placed in a cell culture flask (the cell type, date, name of the person for culture and so on were marked on the flask wall) and then placed in a $CO_2$ incubator (the incubator temperature was 37°C and the $CO_2$ concentration was 5%) for incubation.
b. The cells were passaged. After passage, the cells continued to be cultured in the $CO_2$ incubator. This process was repeated until the cell number met the in vivo drug efficacy requirements.
c. The cultured cells were collected and counted with a fully automatic cell counter. The cells were resuspended in PBS according to the counting results to make a cell suspension (at a density of $5\times10^7$/mL), which was placed in an ice box for later use.

2.3 Cell inoculation

a. The nude mice were labeled with disposable ear tags for mice and rats before inoculation.
b. When inoculated, the cell suspension was mixed evenly, 0.1-1 mL of the cell suspension was drawn using a 1 mL syringe, air bubbles were removed, and then the syringe was placed on an ice pack for later use.
c. The nude mouse was secured with the left hand, the right side of the nude mouse's back near the right shoulder (inoculation site) was disinfected with 75% alcohol, and inoculation was started after 30 seconds.
d. The experimental nude mice were inoculated in sequence (inoculation of 0.1 mL of cell suspension for each mouse).

2.4 Tumor measurement, grouping and dosing for tumor-bearing mice

a. According to the tumor growth, the tumor was measured on the Day 15 after inoculation and the tumor size was calculated.

$$\text{Tumor volume calculation: Tumor volume (mm}^3) = \text{length (mm)} \times \text{width (mm)} \times \text{width (mm)} /2$$

b. According to the body weight and tumor size of tumor-bearing mice, random grouping method was used to group the mice.
c. According to the grouping results, it started to administer the test drug (administration route: oral administration; dose: 1.5, 3, and 7.5 mg/kg; dosing volume: 10 mL/kg; dosing frequency: 1-2 times/day; dosing cycle: 15 days; and solvent: 0.5% HPMC K4M).
d. After starting to administer the test drug, the tumor was measured and weighed twice a week.
e. The animals were euthanized after the experiment.
f. The data were processed using Excel and other software. Calculation of tumor inhibition rate TGI (%) of compounds : In the case of absence of tumor regression, TGI (%) = [1-(average tumor volume at the end of administration in a treatment group - average tumor volume at the beginning of administration in the treatment group)/(average tumor volume at the end of treatment in the solvent control group - average tumor volume at the beginning of treatment in the solvent control group)] × 100%. In the case of presence of tumor regression, TGI (%) = [1-(average tumor volume at the end of administration in a certain treatment group - average tumor volume at the beginning of administration in the treatment group)/average tumor volume at the beginning of administration in the treatment group] × 100%.

3 Experimental results:

**Table 4. Evaluation of the anti-tumor efficacy of the compounds of the present invention for the KCL22-s xenograft tumor model**

| Grouping | Tumor volume ($mm^3$) | T/C (%) | TGI (%) |
|---|---|---|---|
| Vehicle bid | 2,034±235 | - | - |
| Imatinib 75 mg/kg qd | 954±220 | 46.92 | 56.03 |
| Example 1 3 mg/kg qd | 81±19 | 3.98 | 127.26 |
| Example 1 1.5 mg/kg bid | 143±35 | 7.05 | 98.20 |
| Example 1 3 mg/kg bid | 14±5 | 0.69 | 187.08 |
| Example 1 7.5 mg/kg bid | 11±7 | 0.52 | 190.62 |

The compounds of the present invention have significant anti-tumor effects on the xenograft tumor model of the human chronic granulocytic leukemia cell strains KCL22-s.

**II. Study on salts and crystal forms of compounds**

**1. Experimental instruments**

1.1 Some parameters of physicochemical detection instruments

| | | |
|---|---|---|
| X-ray powder diffraction (XRPD) | Instrument model | BRUKER D8 ADVANCE |
| | Diffracted ray | CuK (40 kV, 25 mA) |
| | Scan rate | 0.02°/S (2θ value) |
| | Scan range | 4°-40° (20 value) |
| Differential scanning calorimetry (DSC) | Instrument model | NETZSCH DSC 214 polyma |
| | Purge gas | Nitrogen |
| | Purge speed | 40 mL/min |
| | Heating rate | *10* °C /min |
| | Temperature range | 25-250 °C |
| | Disk type | Aluminum disk |

(continued)

| | | |
|---|---|---|
| Thermogravimetric analysis (TGA) | Instrument model | NETZSCH TG 209 Tarsus |
| | Purge gas | Nitrogen |
| | Purge speed | 40 mL/min |
| | Heating rate | 10 °C /min |
| | Temperature range | 35-350 °C |
| | Disk type | $Al_2O_3$ |
| High performance liquid chromatography (HPLC) | Instrument model | Thermo U 3000 |
| | Pump | LPG-3400SDN |
| | Sample injector | WPS-3000TSL |
| | Column oven | TCC-3000SD |
| | Detector | DAD-3000 |
| Dynamic vapor sorption (DVS) | Instrument model | SMS Intrinsic |
| | Experimental temperature | 25 °C |
| | Drying time | 0%RH 120 min |
| | Balance dm/dt | 0.02%/min (min 10 min, max 180 min) |
| | RH (%) measurement step size | 10% |
| | Measurement gradient | 0-95-0% |
| | Number of cycles | 2 |

**2. Study on crystal forms of salts of compounds**

**2.1 Study on crystal forms of salts of compounds**

2.1.1 Experimental objective:

The experimental objective as to screen salts of compounds in crystal forms.

2.1.2 Experimental steps:

1) Instruments and devices

| Name | Model | Source |
|---|---|---|
| Analytical balance | BSA224S-CW | Sartorius |
| Ultrasonic cleaner | SK5200LHC | Shanghai Kudos Ultrasounic Instrument |
| Pipette | Eppendorf (50 mL,1000 μL) | Eppendorf |

2) Study on crystal forms of salts

① Solventing out or suspending to form salt crystals

10 mg of the free base from Example 1 was weighed, and added to 100 μL of different reaction solvents. Different counterionic acid solutions (base:acid=1:1.2 molar reaction ratio) were added respectively at 50°C for reaction, with the results as follows:

| Serial No. | Solvent | Hydrochloride | | Sulfate | | P-tosylate | |
|---|---|---|---|---|---|---|---|
| | | Phenomenon | Result | Phenomenon | Result | Phenomenon | Result |
| 1 | Chloroform | Fully dissolved, and precipitated after addition of MTBE | Crystal form | Fully dissolved, and precipitated after addition of MTBE | Crystal form | Fully dissolved, and precipitated after addition of MTBE | Crystal form |
| 2 | 2-methyltetrahydrofuran | Fully dissolved, and precipitated after addition of MTBE | Crystal form | Fully dissolved, and precipitated after addition of MTBE | Crystal form | Solventing out | Crystal form |
| 3 | Ethyl formate | Fully dissolved, and precipitated after addition of MTBE | Amorphous | Fully dissolved, and precipitated after addition of MTBE | Crystal form | Solventing out | Crystal form |
| 4 | Ethyl acetate | Fully dissolved, and precipitated after addition of MTBE | Crystal form | Fully dissolved, and precipitated after addition of MTBE | Crystal form | Solventing out | Crystal form |
| 5 | Acetone | Fully dissolved, and precipitated after addition of MTBE | Amorphous | Fully dissolved, and precipitated after addition of MTBE | Crystal form | Fully dissolved, and precipitated after addition of MTBE | Crystal form |
| 6 | Dichloromethane | Fully dissolved, and precipitated after addition of MTBE | Crystal form | Fully dissolved, and precipitated after addition of MTBE | Crystal form | Fully dissolved, and precipitated after addition of MTBE | Crystal form |
| 7 | 2-butanone | Fully dissolved, and precipitated after addition of MTBE | Amorphous | Fully dissolved, and precipitated after addition of MTBE | Crystal form | Fully dissolved, and precipitated after addition of MTBE | Crystal form |

| Serial No. | Solvent | Ethylsulfonate | | Methanesulfonate | |
|---|---|---|---|---|---|
| | | Phenomenon | Result | Phenomenon | Result |
| 1 | Acetone | Solventing out | Crystal form | Solventing out | Crystal form |
| 2 | Methanol | Solventing out | Crystal form | Solventing out | Crystal form |
| 3 | Isopropanol | Suspension | Crystal form | Suspension | Crystal form |
| 4 | 2-butanone | Solventing out | Crystal form | Solventing out | Crystal form |
| 5 | Ethyl acetate | Solventing out | Crystal form | Solventing out | Crystal form |
| 6 | Dichloromethane | Solventing out | Crystal form | Solventing out | Crystal form |
| 7 | Isopropyl acetate | Solventing out | Crystal form | Solventing out | Crystal form |
| 8 | Acetonitrile | Solventing out | Crystal form | Solventing out | Crystal form |
| 9 | Toluene | Solventing out | Crystal form | Solventing out | Crystal form |

(continued)

| Serial No. | Solvent | Ethylsulfonate | | Methanesulfonate | |
|---|---|---|---|---|---|
| | | Phenomenon | Result | Phenomenon | Result |
| 10 | Tetrahydrofuran | Solventing out | Crystal form | Solventing out | Crystal form |
| 11 | 2-methyltetrahydrofuran | Suspension | Crystal form | Suspension | Oil, not tested |
| 12 | Chloroform | Suspension | Crystal form | Suspension | Crystal form |
| 13 | 3-pentanone | Solventing out | Crystal form | Solventing out | Crystal form |
| 14 | Ethyl formate | Suspension | Crystal form | Suspension | Crystal form |

② Slurrying

10 mg of the crystal form A of the p-tosylate was weighed, 100 μL (100 μL pipette) of different slurrying solvents were added, and the system was stirred at room temperature for 2 weeks, to obtain a solid, which was centrifuged, dried, and characterized by XRD, specifically as follows:

| **Solvent** | **Crystal form results** |
|---|---|
| -(Initial) | Crystal form |
| Methanol | Crystal form |
| Ethanol | Crystal form |
| Ethyl acetate | Crystal form |
| Isopropanol | Crystal form |
| Dichloromethane | Crystal form |
| Acetonitrile | Crystal form |
| 2-butanone | Crystal form |
| Toluene | Crystal form |
| Isopropyl acetate | Crystal form |
| n-heptane | Crystal form |
| Acetone | Crystal form |
| Tetrahydrofuran | Crystal form |
| 2-methyltetrahydrofuran | Crystal form |
| Chloroform | Crystal form |
| 3-pentanone | Crystal form |
| Ethyl formate | Crystal form |

③ Naturally drying

An appropriate amount of the crystal form A of the p-tosylate was weighed, and fully dissolved in different solvents by ultrasonication or heating. The system was filtered, and naturally dried in open air at room temperature, to obtain a solid, which was centrifuged, dried, and then characterized by XRD, specifically as follows:

| **Solvent** | **Result** |
|---|---|
| Methanol | Crystal form |
| Ethanol | Crystal form |
| 2-butanone | Crystal form |

(continued)

| Solvent | Result |
|---|---|
| Tetrahydrofuran | Crystal form |

④ Antisolvent Method

An appropriate amount of the p-tosylate A was weighed, and fully dissolved in different good solvents by heating. The systems were filtered, and an antisolvent was added, to observe whether there was precipitation. A system with precipitation was centrifuged, dried, and then characterized, specifically as follows:

| Good Solvent | Antisolvent | Phenomenon | Crystal form |
|---|---|---|---|
| Methanol | MTBE | Precipitation | Crystal form |
| | | | |
| **Good Solvent** | **Antisolvent** | **Phenomenon** | **Phenomenon** |
| Ethanol | Isopropyl ether | Precipitation | Crystal form |
| | n-heptane | Precipitation | Crystal form |
| | n-hexane | Precipitation | Crystal form |
| | Cyclohexane | Precipitation | Crystal form |

2.1.3 Experimental results

The salt crystal form screening experiment shows that the sulfate, p-tosylate, ethylsulfonate, and methylsulfonate are most advantageous based on the salt form crystallinity and difficulty in the crystallization process.

**2.2 Crystal form screening of compounds**

2.2.1 Experimental objective:

The experimental objective was to screen salt crystal forms of compounds.

2.2.2 Experimental steps:

(1) Instruments and devices

| Name | Model | Source |
|---|---|---|
| Analytical balance | BSA224S-CW | Sartorius |
| Ultrasonic cleaner | SK5200LHC | Shanghai Kudos Ultrasounic Instrument |
| Pipette | Eppendorf (50 mL,1000 μL) | Eppendorf |

(2) Operation procedure

1) Preparation of crystal form A of ethylsulfonate

140 mg of the free base from Example 1 was weighed, and fully dissolved in 3.5 mL of acetone by heating at 50°C. The system was filtered for use as a stock solution. 250 μL of the stock solution was taken, 22.9 μL of a solution of 1.0 M ethylsulfonic acid in methanol was slowly added to the system, and the system was stirred at 45°C to precipitate a large amount of solid, which was immediately centrifuged, and dried under vacuum to obtain the crystal form A of the ethylsulfonate. Detection and analysis show that it has the XRPD pattern as shown in FIG. 1, the DSC pattern as shown

in FIG. 2, and the TGA pattern as shown in FIG. 3.

2) Preparation of crystal form B of ethylsulfonate

100 mg of the free base from Example 1 was weighed, 1 mL of acetonitrile was added, the system was heated while stirring at 50°C, and 229 μL of a solution of 1.0 M ethylsulfonic acid in methanol was slowly added to the system. After full dissolution, a large amount of solid was precipitated. After centrifugation and then vacuum drying, the crystal form B of the ethylsulfonate was obtained. Detection and analysis show that it has the XRPD pattern as shown in FIG. 4, the DSC pattern as shown in FIG. 5, and the TGA pattern as shown in FIG. 6.

3) Preparation of crystal form A of mesylate

10 mg of the free base from Example 1 was weighed, 100 μL of ethyl acetate was added, the system was heated while stirring at 50°C, and 22.9 μL of a solution of 1.0 M methylsulfonic acid in methanol was slowly added to the system. After full dissolution, a large amount of solid was precipitated. After centrifugation and then vacuum drying, the crystal form A of the mesylate was obtained. Detection and analysis show that it has the XRPD pattern as shown in FIG. 7, the DSC pattern as shown in FIG. 8, and the TGA pattern as shown in FIG. 9.

4) Preparation of crystal form B of mesylate

10 mg of the free base from Example 1 was weighed, 100 μL of methanol was added, the system was heated while stirring at 50°C, and 22.9 μL of a solution of 1.0 M methylsulfonic acid in ethanol was slowly added to the system. After full dissolution, a large amount of solid was precipitated. After centrifugation and then vacuum drying, the crystal form B of the mesylate was obtained. Detection and analysis show that it has the XRPD pattern as shown in FIG. 10 and the DSC pattern as shown in FIG. 11.

5) Preparation of crystal form A of hydrochloride

10 mg of the free base from Example 1 was weighed, 100 μL of acetonitrile was added, the system was heated while stirring at 50°C, and 22.9 μL of a solution of 1 M hydrochloric acid in methanol was slowly added to the system. After full dissolution, a large amount of solid was precipitated. After centrifugation and then vacuum drying, the crystal form A of the hydrochloride was obtained. Detection and analysis show that it has the XRPD pattern as shown in FIG. 12, the DSC pattern as shown in FIG. 13, and the TGA pattern as shown in FIG. 14.

6) Preparation of crystal form B of hydrochloride

10 mg of the free base from Example 1 was weighed, 100 μL of methanol was added, the system was heated while stirring at 50°C, and 22.9 μL of a solution of 1 M hydrochloric acid in methanol was slowly added to the system. After full dissolution, a large amount of solid was precipitated. After centrifugation and then vacuum drying, the crystal form B of the hydrochloride was obtained. Detection and analysis show that it has the XRPD pattern as shown in FIG. 15.

7) Preparation of crystal form C of hydrochloride

10 mg of the free base from Example 1 was weighed, 100 μL of dichloromethane was added, the system was stirred at room temperature, and 22.9 μL of a solution of 1 M hydrochloric acid in methanol was slowly added to the system for full dissolution. After addition of MTBE, a large amount of solid was precipitated. After centrifugation and then vacuum drying, the crystal form C of the hydrochloride was obtained. Detection and analysis show that it has the XRPD pattern as shown in FIG. 16.

8) Preparation of crystal form A of sulfate

10 mg of the free base from Example 1 was weighed, 100 μL of acetonitrile was added, the system was stirred at room temperature, and 22.9 μL of a solution of 1 M sulfuric acid in methanol was slowly added to the system. After full dissolution, a large amount of solid was precipitated. After centrifugation and then vacuum drying, the crystal form A of the sulfate was obtained. Detection and analysis show that it has the XRPD pattern as shown in FIG. 17, the DSC pattern as shown in FIG. 18, and the TGA pattern as shown in FIG. 19.

9) Preparation of crystal form B of sulfate

10 mg of the free base from Example 1 was weighed, 100 μL of methanol was added, the system was heated while stirring at 50°C, and 22.9 μL of a solution of 1 M sulfuric acid in methanol was slowly added to the system. After full dissolution, a large amount of solid was precipitated. After centrifugation and then vacuum drying, the crystal form B of the sulfate was obtained. Detection and analysis show that it has the XRPD pattern as shown in FIG. 20, the DSC pattern as shown in FIG. 21, and the TGA pattern as shown in FIG. 22.

10) Preparation of crystal form C of sulfate

10 mg of the free base from Example 1 was weighed, 100 μL of chloroform was added, the system was heated while stirring at 50°C, and 22.9 μL of a solution of 1 M sulfuric acid in methanol was slowly added to the system for full dissolution. After addition of MTBE, a large amount of solid was precipitated. After centrifugation and then vacuum drying, the crystal form C of the sulfate was obtained. Detection and analysis show that it has the XRPD pattern as shown in FIG. 23, the DSC pattern as shown in FIG. 24, and the TGA pattern as shown in FIG. 25.

11) Preparation of crystal form D of sulfate

10 mg of the free base from Example 1 was weighed, 100 μL of acetone was added, the system was heated while stirring at 50°C, and 22.9 μL of a solution of 1 M sulfuric acid in methanol was slowly added to the system for full dissolution. After addition of MTBE, a large amount of solid was precipitated. After centrifugation and then vacuum drying, the crystal form D of the sulfate was obtained. Detection and analysis show that it has the XRPD pattern as shown in FIG. 26, the DSC pattern as shown in FIG. 27, and the TGA pattern as shown in FIG. 28.

12) Preparation of crystal form E of sulfate

10 mg of the free base from Example 1 was weighed, 100 μL of dichloromethane was added, the system was heated while stirring at 50°C, and 22.9 μL of a solution of 1 M sulfuric acid in methanol was slowly added to the system for full dissolution. After addition of MTBE, a large amount of solid was precipitated. After centrifugation and then vacuum drying, the crystal form E of the sulfate was obtained. Detection and analysis show that it has the XRPD pattern as shown in FIG. 29, the DSC pattern as shown in FIG. 30, and the TGA pattern as shown in FIG. 31.

13) Preparation of crystal form F of sulfate

10 mg of the free base from Example 1 was weighed, 100 μL of 2-butanone was added, the system was heated while stirring at 50°C, and 22.9 μL of a solution of 1 M sulfuric acid in methanol was slowly added to the system for full dissolution. After addition of MTBE, a large amount of solid was precipitated. After centrifugation and then vacuum drying, the crystal form F of the sulfate was obtained. Detection and analysis show that it has the XRPD pattern as shown in FIG. 32, the DSC pattern as shown in FIG. 33, and the TGA pattern as shown in FIG. 34.

14) Preparation of crystal form G of sulfate

100 mg of the free base from Example 1 was weighed, 1 mL was added, the system was heated while stirring at 50°C, and 229 μL of a solution of 1 M sulfuric acid in methanol was slowly added to the system for full dissolution. After addition of MTBE, a large amount of solid was precipitated. The system was stirred for 4 h, and then centrifuged and dried under vacuum, to obtain the crystal form D of the sulfate. Detection and analysis show that it has the XRPD pattern as shown in FIG. 35, the DSC pattern as shown in FIG. 36, and the TGA pattern as shown in FIG. 37.

15) Preparation of crystal form A of p-tosylate

10 mg of the free base from Example 1 was weighed, 100 μL of acetonitrile was added, the system was heated while stirring at 50°C, and 22.9 μL of a solution of 1 M p-toluenesulfonic acid in methanol was slowly added to the system. After full dissolution, a large amount of solid was precipitated. After centrifugation and then vacuum drying, the crystal form A of the p-tosylate was obtained. Detection and analysis show that it has the XRPD pattern as shown in FIG. 38, the DSC pattern as shown in FIG. 39, and the TGA pattern as shown in FIG. 40.

16) Preparation of crystal form B of p-tosylate

10 mg of the free base from Example 1 was weighed, 100 μL of methanol was added, the system was heated while stirring at 50°C, and 22.9 μL of a solution of 1 M p-toluenesulfonic acid in methanol was slowly added to the system. After full dissolution, a large amount of solid was precipitated. After centrifugation and then vacuum drying, the crystal form B of the p-tosylate was obtained. Detection and analysis show that it has the XRPD pattern as shown in FIG. 41, the DSC pattern as shown in FIG. 42, and the TGA pattern as shown in FIG. 43.

17) Preparation of crystal form C of p-tosylate

10 mg of the free base from Example 1 was weighed, 100 μL of acetonitrile was added, the system was heated while stirring at 50°C, and 22.9 μL of a solution of 1 M p-toluenesulfonic acid in methanol was slowly added to the system. After full dissolution, a large amount of solid was precipitated. After immediate centrifugation and vacuum drying, the crystal form C of the p-tosylate was obtained. Detection and analysis show that it has the XRPD pattern as shown in FIG. 44, the DSC pattern as shown in FIG. 45, and the TGA pattern as shown in FIG. 46.

18) Preparation of crystal form D of p-tosylate

50 mg of the crystal form A of the p-tosylate was weighed, 15 mL of 2-butanone was added, and the system was heated or ultrasonicated for full dissolution. The system was filtered for use as a stock solution. 4 mL of the stock solution was taken, an antisolvent isopropyl ether was slowly added to the system, and the system was stirred for precipitation. After centrifugation and vacuum drying, the crystal form D of the p-tosylate was obtained. Detection and analysis show that it has the XRPD pattern as shown in FIG. 47, the DSC pattern as shown in FIG. 48, and the TGA pattern as shown in FIG. 49.

19) Preparation of crystal form E of p-tosylate

12 mg of the crystal form A of the p-tosylate was weighed, 0.5 mL of methanol was added, and the system was heated or ultrasonicated for full dissolution. The system was filtered, and naturally dried in open air at room temperature, to obtain the crystal form E of the p-tosylate. Detection and analysis show that it has the XRPD pattern as shown in FIG. 50, the DSC pattern as shown in FIG. 51, and the TGA pattern as shown in FIG. 52.

20) Preparation of crystal form A of besylate

240 mg of the free base from Example 1 was weighed, 6 mL of tetrahydrofuran was added, and the system was heated or ultrasonicated for full dissolution. The system was filtered for use as a stock solution. 250 μL of the stock solution was taken, 22.9 μL of a solution of 1 M benzenesulfonic acid in methanol was slowly added to the system, and the system was naturally dried in open air at room temperature to obtain the crystal form A of the besylate. Detection and analysis show that it has the XRPD pattern as shown in FIG. 53.

21) Preparation of crystal form B of besylate

10 mg of the free base from Example 1 was weighed, 100 μL of acetonitrile was added, the system was heated while stirring at 50°C, and 22.9 μL of a solution of 1 M benzenesulfonic acid in methanol was slowly added to the system. After addition of MTBE, a large amount of solid was precipitated. After centrifugation and vacuum drying, the crystal form B of the besylate was obtained. Detection and analysis show that it has the XRPD pattern as shown in FIG. 54, the DSC pattern as shown in FIG. 55, and the TGA pattern as shown in FIG. 56.

22) Preparation of crystal form C of besylate

10 mg of the free base from Example 1 was weighed, 100 μL of methanol was added, the system was heated while stirring at 50°C, and 22.9 μL of a solution of 1 M benzenesulfonic acid in methanol was slowly added to the system. After addition of MTBE, a large amount of solid was precipitated. After centrifugation and vacuum drying, the crystal form C of the besylate was obtained. Detection and analysis show that it has the XRPD pattern as shown in FIG. 57, the DSC pattern as shown in FIG. 58, and the TGA pattern as shown in FIG. 59.

23) Preparation of crystal form A of isethionate

240 mg of the free base from Example 1 was weighed, 6 mL of tetrahydrofuran was added, and the system was heated or ultrasonicated for full dissolution. The system was filtered for use as a stock solution. 250 μL of the stock solution was taken, 22.9 μL of a solution of 1 M isethionic acid in methanol was slowly added to the system, and the system was naturally dried in open air at room temperature to obtain the crystal form A of the isethionate. Detection and analysis show that it has the XRPD pattern as shown in FIG. 60.

24) Preparation of crystal form B of isethionate

10 mg of the free base from Example 1 was weighed, 100 μL of methanol was added, the system was heated while stirring at 50°C, and 22.9 μL of a solution of 1 M isethionic acid in methanol was slowly added to the system. After addition of MTBE, a large amount of solid was precipitated. After centrifugation and vacuum drying, the crystal form B of the isethionate was obtained. Detection and analysis show that it has the XRPD pattern as shown in FIG. 61, the DSC pattern as shown in FIG. 62, and the TGA pattern as shown in FIG. 63.

25) Preparation of crystal form A of 1,5-napadisylate

10 mg of the free base from Example 1 was weighed, 100 μL of methanol was added, the system was heated while stirring at 50°C, and 183 μL of a solution of 0.125 M 1,5-naphthalenedisulfonic acid in ethanol was slowly added to the system. After addition of MTBE, a large amount of solid was precipitated. After centrifugation and vacuum drying, the crystal form A of the 1,5-napadisylate was obtained. Detection and analysis show that it has the XRPD pattern as shown in FIG. 64, the DSC pattern as shown in FIG. 65, and the TGA pattern as shown in FIG. 66.

## 3. Quantitative study on salts of compounds

### 3.1 Quantitative study on sulfate of compounds

3.1.1 Experimental objective:

The experimental objective was to quantify the number of conjugated acids in the sulfate of each of the compounds by HPLC-ELSD.

3.1.2 Experimental steps:

An appropriate amount of ammonium sulfate was weighed, and prepared into a series of linear solutions at different concentrations using a diluent acetonitrile-water (50:50). An appropriate amount of sulfate in different batches was weighed, and prepared into a solution of sulfate at 2 mg/mL respectively using a diluent acetonitrile-water (50:50).

The above solutions were taken. The linear solutions and sulfate samples were filtered, and then subjected to HPLC-ELSD. Specific HPLC-ELSD analysis method:

| | |
|---|---|
| Mobile phase | A: 75 mM ammonium acetate solution (pH 4.8); B: acetonitrile |
| Chromatographic column | HILIC ZIC (150 mm*4.6 mm, 3.5 um) |
| Column temperature | 35°C |
| Injection volume | 5 uL |
| ELSD drift tube temperature | 80°C |
| Nitrogen pressure | 3.5 b |
| Proportion | 50%B (v/v) |

3.1.3 Experimental results:

Table 1 Quantitative study results of sulfate

| Content/batch | Batch 1 | Batch 2 |
|---|---|---|
| Determination of percentage content of $SO_4^{2-}$ in sulfuric acid | 13.70% | 14.63% |
| Theoretical value of percentage content of $SO_4^{2-}$ with 0.5 conjugated sulfuric acid | 9.35% | |
| Theoretical value of percentage content of $SO_4^{2-}$ when 1 conjugated sulfuric acid | 15.76% | |

3.1.4 Experimental conclusions:

As verified from calculation of the percentage content of $SO_4^{2-}$ in sulfate of different batches, the number of conjugated sulfuric acids in the crystal form D of the sulfate of the compound is 1.

**3.2 Quantification of p-tosylate of compounds**

3.2.1 Experimental objective:

The experimental objective was to quantify the number of conjugated acids in the p-tosylate of the compounds by HPLC-ELSD.

3.2.2 Experimental steps:

An appropriate amount of toluene-4-sulfonic acid (monohydrate) was weighed, and prepared into a series of linear solutions at different concentrations using a diluent acetonitrile-water (50:50). An appropriate amount of p-tosylate in different batches was weighed, and prepared into a solution of p-tosylate at 2 mg/mL respectively using a diluent acetonitrile-water (50:50).

The above solutions were taken. The linear solutions and p-tosylate samples were filtered, and then subjected to HPLC-ELSD. Specific HPLC-ELSD analysis method:

| | |
|---|---|
| Mobile phase | A: 75 mM ammonium acetate solution (pH 4.8); B: acetonitrile |
| Chromatographic column | HILIC ZIC (150 mm*4.6 mm, 3.5 um) |
| Column temperature | 35°C |
| Injection volume | 5 uL |
| ELSD drift tube temperature | 80°C |
| Nitrogen pressure | 3.5 b |
| Proportion | 80%B (v/v) |

3.2.3 Experimental results:

Table 2 Quantitative study results of p-tosylate

| Content/batch | Batch 1 | Batch 2 |
|---|---|---|
| Determination of percentage content of p-toluenesulfonic acid in p-tosylate | 23.96% | 23.52% |
| Theoretical value of percentage content of p-toluenesulfonic acid with 0.5 conjugated p-tosylates | 16.44% | |
| Theoretical value of percentage content of p-toluenesulfonic acid with 1 conjugated p-tosylate | 24.74% | |

3.2.4 Experimental conclusions:

As verified from calculation of the percentage content of p-toluenesulfonic acid in the p-tosylate of different

batches, the number of conjugated p-toluenesulfonic acids in the crystal form A of the p-tosylate of the compound is 1.

**4. Solid stability experiment**

4.1 Experimental objective:

The experimental objective was to investigate the physicochemical stability of the crystal forms of the compounds under conditions of illuminance under 5000 lx, high temperature at 60°C, high humidity at 92.5% RH, and high temperature & humidity at 50°C & 75% RH, to provide a basis for storage of the compounds.

4.2 Instruments and liquid chromatography conditions

4.2.1 Instruments and devices:

| Instrument Name | Model |
|---|---|
| Analytical balance | Sartorius BSA224S-CW |
| Water purifier | Milli-Q Plus, Millipore |
| High performance liquid chromatograph | Thermo U 3000 |
| Pump | LPG-3400SDN |
| Sample injector | WPS-3000TSL |
| Column oven | TCC-3000SD |
| Detector | DAD-3000 |

4.2.2 Chromatographic conditions:

| | | | |
|---|---|---|---|
| Chromatographic column | Waters Xbridge C18 (4.6*150 mm,3.5 um) | | |
| Mobile phase | A: 25 mM ammonium dihydrogen phosphate solution (Ph 2.0); B: acetonitrile | | |
| Flow rate | 1.0 mL/min | | |
| Column temperature | 35°C | | |
| Detection wavelength | 230 nm | | |
| Injection volume | 5.0 μL | | |
| Gradient | T (min) | A (%) | B (%) |
| | 0.00 | 40.0 | 60.0 |
| | 10.00 | 15.0 | 85.0 |
| | 10.10 | 40.0 | 60.0 |
| | 15.00 | 40.0 | 60.0 |

4.3 Experimental solutions:

About 1 mg of each of different salt crystal forms of the compounds was taken, and investigated under conditions of illuminance under 5000 lx, high temperature at 60°C, high humidity at 92.5% RH, and high temperature & humidity at 50°C & 75% RH for 7 days and 14 days, to determine the content by HPLC and an external standard method, and calculate changes of the relevant substances by chromatographic peak area normalization.

4.4 Experimental results:

Table 3 Stability results

| Salt form \ Conditions | Stability result (impurity increase%) | | | | | | | |
|---|---|---|---|---|---|---|---|---|
| | Illuminance | | High temperature 60°C | | High humidity 92.5% RH | | High temperature & humidity 50°C 75%RH | |
| | 7 days | 14 days | 7 days | 14 days | 7 days | 14 days | 7 days | 14 days |
| Crystal form B of ethylsulfonate | 0.04 | - | <0.02 | - | 0.17 | - | 0.30 | - |
| Crystal form A of mesylate | 0.16 | - | 0.07 | - | 0.10 | - | - | - |
| Crystal form D of sulfate | 0.10 | 0.33 | 0.20 | 0.20 | <0.02 | <0.02 | 0.17 | 0.41 |
| Crystal form A of p-tosylate | 0.12 | 0.07 | 0.05 | <0.02 | <0.02 | <0.02 | 0.02 | <0.02 |

4.5 Experimental conclusions:

The above data suggest that the salt crystal forms of the compounds are stable under each condition of lighting, high temperature, high humidity, and high temperature & humidity, without significant increase of impurities.

**5. Dynamic vapor sorption experiment**

5.1 Experimental objective:

The experimental objective was to investigate vapor sorption of the salt crystal forms of the compounds under different relative humidity conditions, providing a basis for the screening and storage of the salt crystal forms of the compounds.

5.2 Experimental solutions:

The crystal form A of the p-tosylate of the compound was placed in saturated water vapor with different relative humidities to reach a dynamic equilibrium between the compound and the water vapor, and the percentage of hygroscopic weight gain of the compound after reaching the equilibrium was calculated.

5.3 Experimental results:

The crystal form A of the p-tosylate of the compound had hygroscopic weight gain of 0.3545% at RH 80%. After 2 hygroscopic-dehygroscopic cycles at relative humidity of 0-95%, the XRPD pattern of the crystal form A of the p-tosylate remained changed, i.e., the crystal form remained unchanged.

5.4 Experimental conclusions:

The crystal form A of the p-tosylate is stable in a humid environment.

**6. Thermodynamical stability experiment**

6.1 Experimental objective:

The experimental objective was to obtain a thermodynamically stable crystal form through polymorph screening and crystal form competitive experiments.

6.2 Experimental solutions:

An organic solvent with certain solubility was selected, and the crystal form A of the p-tosylate of the compound was suspended in the solvent system. The resulting system was slurried by stirring at room temperature for 2 weeks, and centrifuged. The supernatant was discarded. After the solid was dried under vacuum at 50°C (-0.1 MPa) for 16 h, the XRPD pattern of the solid was measured and compared with the XRPD of the salt of the starting compound.

6.3 Experimental results:

Table 5 Results of polymorph screening experiment

| Solvent | Crystal form results |
|---|---|
| -(Initial) | Form A |
| Methanol | Form A |
| Ethanol | Form A |
| Ethyl acetate | Form A |
| Isopropanol | Form A |
| Dichloromethane | Form A |
| Acetonitrile | Form A |
| 2-butanone | Form A |
| Toluene | Form A |
| Isopropyl acetate | Form A |
| n-heptane | Form A |
| Acetone | Form A |
| Tetrahydrofuran | Form A |
| 2-methyltetrahydrofuran | Form A |
| Chloroform | Form A |
| 3-pentanone | Form A |
| Ethyl formate | Form A |

6.4 Experimental conclusions:

The crystallization solvent and the crystallization method were changed by slurrying, to obtain total 5 crystal forms of the p-tosylate, namely the crystal form A, the crystal form B, the crystal form C, the crystal form D, and the crystal form E of the p-tosylate. As can be determined from comparison of DSC patterns of different crystal forms, the crystal form A of the p-tosylate is the most thermodynamically stable crystal form among these crystal forms, and is an anhydrate.

**7. Pharmacokinetic study in rats**

7.1. Experimental objective:

The experimental objective was to investigate the pharmacokinetic parameters of the crystal form A of the p-tosylate of the compound in rats through animal PK studies.

7.2 Experimental instruments and reagents:

| Instruments | Reagents |
|---|---|
| Analytical balance | Acetonitrile |
| Centrifuge | Methyl Alcohol |
| Ultrasonic cleaner | Formic Acid |
| Triple quadrupole mass spectrometer | Dexamethasone |
| High performance liquid chromatograph | HPMC K4M |
| Ultrasonic cell crusher | |
| 4°C refrigerator | |
| -20°C refrigerator | |
| Pipette | |

7.3 Experimental animals:

| Animal species | Strain | Age | Gender |
|---|---|---|---|
| Rat | SD | 7 weeks old with body weight of 200 g | Male |

7.4 Test compound:

Crystal form A of p-tosylate of the compound.

7.5 Experimental solutions:

The crystal form A of the p-tosylate of the compound was fully suspended in an aqueous solution of 0.5% HPMC (hydroxypropylmethyl cellulose) K4M, and then administered to rats by gavage at a dosage of 10 mg/kg and 30 mg/kg, with three rats in each parallel experiment. The amount of the compound was fully converted into the amount of an equivalent free base.

7.6 Experimental results:

Table 6 Results of pharmacokinetic experiment in rats

| Rat PO dose: | 10 mg/kg | 30 mg/kg |
|---|---|---|
| Parameters | Crystal form A of p-tosylate | Crystal form A of p-tosylate |
| $T_{1/2}$(hr) | 3.55 | 2.71 |
| $T_{max}$ (hr) | 1.00 | 1.00 |
| $C_{max}$ (ng/mL) | 881.00 | 4513.33 |
| $AUC_{0-\infty}$(ng·hr/mL) | 2597.74 | 15570.48 |
| MRT (hr) | 2.86 | 2.97 |

7.7 Experimental conclusions:

As can be seen from the results of the pharmacokinetic experiment in rats in the table, the crystal form A of the p-tosylate of the compound in the present invention shows good metabolic properties at the dose of 30 mg/kg.

**8. Pharmacokinetic study in dogs**

8.1. Experimental objective:

The experimental objective was to investigate the pharmacokinetic parameters of the crystal form A of the p-tosylate in dogs.

8.2 Experimental instruments and reagents:

| Instruments | Reagents |
|---|---|
| LC-MS/MS | Formic acid |
| Chromatographic column | Dimethyl sulfoxide |
| Multi-tube vortex oscillator | Beagle dog plasma (EDTA-$K_2$ anticoagulant) |
| | Hydroxypropylmethyl cellulose (HPMC K4M) |
| | Sterile water for injection |

8.3 Experimental animals:

| Animal species | Grade | Age | Gender |
|---|---|---|---|
| Beagle dog | Ordinary grade | 8-15 months old, 8.38-11.34 kg | Male |

8.4 Test compound:

Crystal form A of p-tosylate of the compound.

8.5 Experimental solutions:

The crystal form A of the p-tosylate of the compound was fully suspended in an aqueous solution of 0.5% HPMC (hydroxypropylmethyl cellulose) K4M, and then administered to beagle dogs by gavage at a dosage of 20 mg/kg and a dose volume of 5 mL/kg, with three dogs in each parallel experiment. The amount of the compound was fully converted into the amount of an equivalent free base.

8.6 Experimental results:

Table 7 Results of pharmacokinetic experiment in dogs

| Rat PO dose: | 20 mg/kg |
|---|---|
| Parameters | Crystal form A of p-tosylate |
| $T_{1/2}$(hr) | 5.58 |
| $T_{max}$ (hr) | 1.33 |
| $C_{max}$ (ng/mL) | 5721.52 |
| $AUC_{0-\infty}$ (ng·hr/mL) | 40387.92 |
| MRT (hr) | 5.47 |
| F (%) | 68.6% |

8.7 Experimental conclusions:

As can be seen from the results of the pharmacokinetic experiment in dogs in the table, the crystal form A of the p-tosylate shows good metabolic properties, exposure dosage, and significantly improved bioavailability.

Moreover, comparison of the results of the pharmacokinetic experiment in rats shows that the bioavailability of the compound in dogs is significantly higher than that in rats, suggesting significant differences between species, but the exposure dosage of the crystal form A of the p-tosylate in rats and dogs has consistent relative trends.

## Claims

**1.** A crystal form of an acid salt of a compound represented by formula (I),

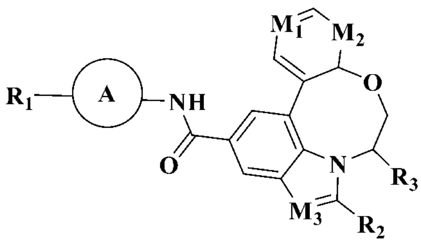

(I)

wherein:

ring A is $C_{6-10}$ aryl or 5-6-membered heteroaryl;
$R_1$ is selected from $C_{1-3}$ alkyl, $C_{1-3}$ deuteroalkyl, $C_{1-3}$ haloalkyl, $C_{1-3}$ hydroxyalkyl, $C_{1-3}$ alkoxy, $C_{13}$ alkylthio, or $C_{1-3}$ haloalkoxy;
$M_1$ is selected from N or CH;
$M_2$ is selected from NH or $CH_2$;
$M_3$ is selected from N or CH;
$R_2$ or $R_3$ is each independently selected from $C_{1-3}$ alkyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, or $C_{1-3}$ haloalkoxy; and
the acid salt is selected from an ethylsulfonate, a mesylate, a sulfate, a hydrochloride, a p-tosylate, a besylate, an isethionate, or a 1,5-napadisylate.

**2.** A crystal form of an acid salt of a compound represented by formula (I-a):

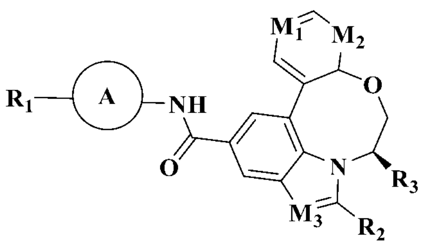

(I-a)

wherein

ring A is phenyl or pyridyl;
$R_1$ is selected from $C_{1-3}$ alkyl, $C_{1-3}$ haloalkyl, $C_{1-3}$ alkoxy, or $C_{1-3}$ haloalkoxy;
$M_1$ is N;
$M_2$ is NH;
$M_3$ is N;
$R_2$ or $R_3$ is each independently selected from hydrogen, $C_{1-3}$ alkyl, or $C_{1-3}$ haloalkyl; and
the acid salt is selected from an ethylsulfonate, a mesylate, a sulfate, a hydrochloride, a p-tosylate, a besylate, an

isethionate, or a 1,5-napadisylate.

3. The crystal form of the acid salt according to claim 1 or 2, wherein a specific structure of the compound represented by formula (I) is as follows:

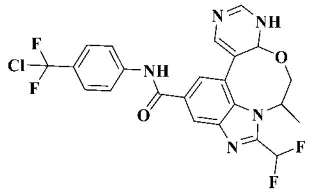 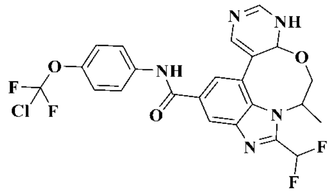 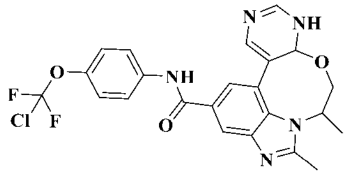

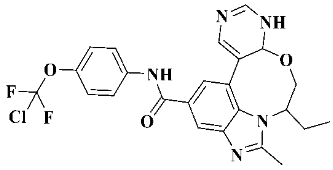 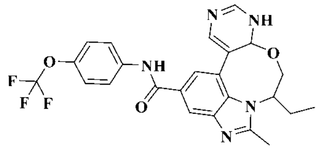 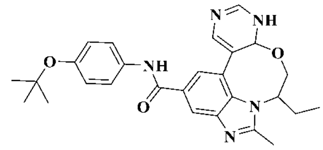

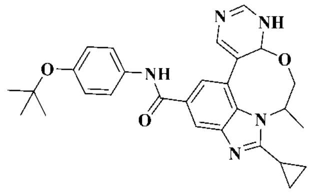 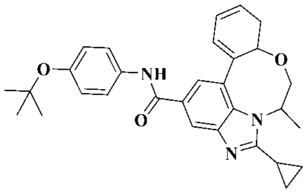 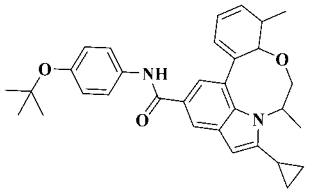

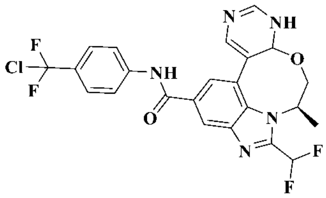 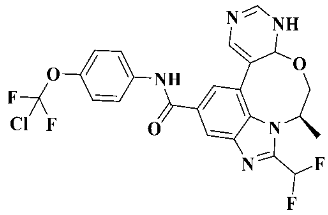 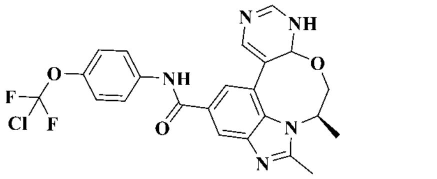

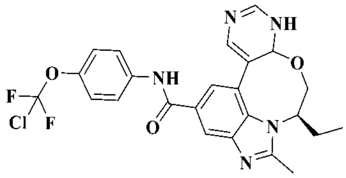 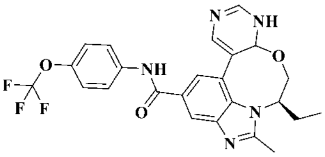 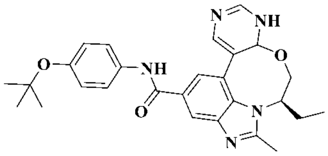

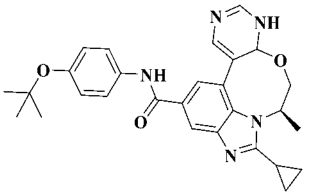 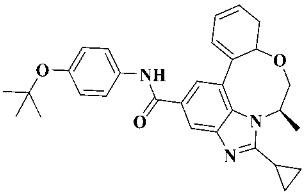 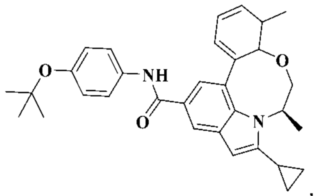.

4. The crystal form of the acid salt according to any one of claims 1 to 3, wherein the crystal form is one of crystal forms A-B of an ethylsulfonate of (3R)-N-(4-(chlorodifluoromethoxy) phenyl)-2-(difluoromethyl)-3-methyl-3,4,5a,6-tetrahydro-5-oxa-1,2a,6,8-tetraazabenzo[4,5] cyclooctyl[1,2,3-cd]indene-11-carboxamide;

wherein an X-ray powder diffraction pattern of the crystal form A of the ethylsulfonate has a diffraction peak at 5.9 ±0.2°; or a diffraction peak at 17.7±0.2°; or a diffraction peak at 22.3±0.2°; or a diffraction peak at 16.7±0.2°; or a diffraction peak at 21.0±0.2°; or a diffraction peak at 18.0±0.2°; or a diffraction peak at 5.6±0.2°; or a diffraction peak at 29.7±0.2°; or a diffraction peak at 23.8±0.2°; or a diffraction peak at 12.2±0.2°; preferably comprises any

2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the ethylsulfonate has a diffraction peak at 5.6±0.2°; or a diffraction peak at 16.5±0.2°; or a diffraction peak at 8.4±0.2°; or a diffraction peak at 10.0±0.2°; or a diffraction peak at 17.6±0.2°; or a diffraction peak at 23.7±0.2°; or a diffraction peak at 27.7±0.2°; or a diffraction peak at 15.2±0.2°; or a diffraction peak at 28.9±0.2°; or a diffraction peak at 12.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-B of the mesylate of (3R)-N-(4-(chlorodifluoromethoxy)phenyl)-2-(difluoromethyl)-3-methyl-3,4,5a,6-tetrahydro-5-oxa-1,2a,6,8 -tetraazabenzo[4,5]cyclooctyl[1,2,3-cd]indene-11-carboxamide;

wherein an X-ray powder diffraction pattern of the crystal form A of the mesylate has a diffraction peak at 6.0±0.2°; or a diffraction peak at 17.8±0.2°; or a diffraction peak at 21.4±0.2°; or a diffraction peak at 16.5±0.2°; or a diffraction peak at 22.4±0.2°; or a diffraction peak at 12.2±0.2°; or a diffraction peak at 24.3±0.2°; or a diffraction peak at 23.5±0.2°; or a diffraction peak at 29.8±0.2°; or a diffraction peak at 19.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the mesylate has a diffraction peak at 6.0±0.2°; or a diffraction peak at 18.0±0.2°; or a diffraction peak at 22.6±0.2°; or a diffraction peak at 30.1±0.2°; or a diffraction peak at 6.6±0.2°; or a diffraction peak at 12.2±0.2°; or a diffraction peak at 13.2±0.2°; or a diffraction peak at 15.5 ±0.2°; or a diffraction peak at 21.4±0.2°; or a diffraction peak at 24.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-G of the sulfate of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide;

wherein an X-ray powder diffraction pattern of the crystal form A of the sulfate has a diffraction peak at 5.8±0.2°; or a diffraction peak at 21.6±0.2°; or a diffraction peak at 17.6±0.2°; or a diffraction peak at 19.7±0.2°; or a diffraction peak at 16.5±0.2°; or a diffraction peak at 12.0±0.2°; or a diffraction peak at 12.3±0.2°; or a diffraction peak at 17.2±0.2°; or a diffraction peak at 13.6±0.2°; or a diffraction peak at 25.9±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the sulfate has a diffraction peak at 5.7±0.2°; or a diffraction peak at 16.9±0.2°; or a diffraction peak at 17.4±0.2°; or a diffraction peak at 22.5±0.2°; or a diffraction peak at 19.3±0.2°; or a diffraction peak at 9.9±0.2°; or a diffraction peak at 20.1±0.2°; or a diffraction peak at 13.8 ±0.2°; or a diffraction peak at 11.1±0.2°; or a diffraction peak at 18.6±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the sulfate has a diffraction peak at 5.6±0.2°; or a diffraction peak at 16.7±0.2°; or a diffraction peak at 8.3±0.2°; or a diffraction peak at 12.7±0.2°; or a diffraction peak at 15.3±0.2°; or a diffraction peak at 17.6±0.2°; or a diffraction peak at 10.0±0.2°; or a diffraction peak at 15.5±0.2°; or a diffraction peak at 13.1±0.2°; or a diffraction peak at 21.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form D of the sulfate has a diffraction peak at 17.3±0.2°; or a diffraction peak at 24.3±0.2°; or a diffraction peak at 20.6±0.2°; or a diffraction peak at 26.2±0.2°; or a diffraction peak at 22.1±0.2°; or a diffraction peak at 18.6±0.2°; or a diffraction peak at 15.1±0.2°; or a diffraction peak at 12.9±0.2°; or a diffraction peak at 25.9±0.2°; or a diffraction peak at 18.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form E of the sulfate has a diffraction peak at 5.8±0.2°; or a diffraction peak at 17.24±0.2°; or a diffraction peak at 9.8±0.2°; or a diffraction peak at 13.8±0.2°; or a diffraction peak at 20.0±0.2°; or a diffraction peak at 22.6±0.2°; or a diffraction peak at 19.2±0.2°; or a diffraction peak at 22.2±0.2°; or a diffraction peak at 11.1±0.2°; or a diffraction peak at 26.2+0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form F of the sulfate has a diffraction peak at 6.0±0.2°; or a diffraction peak at 16.0±0.2°; or a diffraction peak at 22.4±0.2°; or a diffraction peak at 17.3±0.2°; or a diffraction

peak at 20.0±0.2°; or a diffraction peak at 18.5±0.2°; or a diffraction peak at 20.5±.2°; or a diffraction peak at 14.4 ±0.2°; or a diffraction peak at 24.9±0.2°; or a diffraction peak at 24.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form G of the sulfate has a diffraction peak at 5.9±0.2°; or a diffraction peak at 16.7±0.2°; or a diffraction peak at 17.6±0.2°; or a diffraction peak at 5.6±0.2°; or a diffraction peak at 16.9±0.2°; or a diffraction peak at 22.2±0.2°; or a diffraction peak at 29.5±0.2°; or a diffraction peak at 27.7±0.2°; or a diffraction peak at 25.1±0.2°; or a diffraction peak at 10.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-C of the hydrochloride of (3R)-N-(4-(chlorodifluoromethoxy) phenyl)-2-(difluoromethyl)-3-methyl-3,4,5a,6-tetrahydro-5-oxa-1,2a,6,8 - tetraazabenzo[4,5]cyclooctyl[1,2,3-ed]indene-11-carboxamide;

wherein an X-ray powder diffraction pattern of the crystal form A of the hydrochloride has a diffraction peak at 22.4 ±0.2°; or a diffraction peak at 14.0±0.2°; or a diffraction peak at 17.1±0.2°; or a diffraction peak at 6.24±0.2°; or a diffraction peak at 19.4±0.2°; or a diffraction peak at 25.2±0.2°; or a diffraction peak at 17.5±0.2°; or a diffraction peak at 21.6±0.2°; or a diffraction peak at 19.8±0.2°; or a diffraction peak at 23.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the hydrochloride has a diffraction peak at 6.7±0.2°; or a diffraction peak at 27.0±0.2°; or a diffraction peak at 23.4±0.2°; or a diffraction peak at 13.4±0.2°; or a diffraction peak at 11.0±0.2°; or a diffraction peak at 24.1±0.2°; or a diffraction peak at 15.6±0.2°; or a diffraction peak at 4.5±0.2°; or a diffraction peak at 20.0±0.2°; or a diffraction peak at 10.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the hydrochloride has a diffraction peak at 16.5±0.2°; or a diffraction peak at 20.4±0.2°; or a diffraction peak at 22.2±0.2°; or a diffraction peak at 9.7±0.2°; or a diffraction peak at 17.8±0.2°; or a diffraction peak at 5.3±0.2°; or a diffraction peak at 17.5±0.2°; or a diffraction peak at 6.0 ±0.2°; or a diffraction peak at 14.3±0.2°; or a diffraction peak at 21.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-E of the p-tosylate of (3R)-N-(4-(chlorodifluoromethoxy)phenyl)-2-(difluoromethyl)-3-methyl-3,4,5a,6-tetrahydro-5-oxa-1,2a,6,8 -tetraazabenzo[4,5]cyclooctyl[1,2,3-cd]indene-11-carboxamide;

wherein an X-ray powder diffraction pattern of the crystal form A of the p-tosylate has a diffraction peak at 16.8 ±0.2°; or a diffraction peak at 19.9±0.2°; or a diffraction peak at 5.7±0.2°; or a diffraction peak at 22.5±0.2°; or a diffraction peak at 21.8±0.2°; or a diffraction peak at 24.9±0.2°; or a diffraction peak at 22.3±0.2°; or a diffraction peak at 20.8±0.2°; or a diffraction peak at 26.6±0.2°; or a diffraction peak at 12.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the p-tosylate has a diffraction peak at 5.5±0.2°; or a diffraction peak at 19.9±0.2°; or a diffraction peak at 13.2±0.2°; or a diffraction peak at 21.9±0.2°; or a diffraction peak at 28.1±0.2°; or a diffraction peak at 14.1±0.2°; or a diffraction peak at 10.9±0.2°; or a diffraction peak at 17.6±0.2°; or a diffraction peak at 9.5±0.2°; or a diffraction peak at 20.4±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the p-tosylate has a diffraction peak at 5.8±0.2°; or a diffraction peak at 17.3±0.2°; or a diffraction peak at 16.7±0.2°; or a diffraction peak at 22.0±0.2°; or a diffraction peak at 19.6±0.2°; or a diffraction peak at 23.1±0.2°; or a diffraction peak at 22.4±0.2°; or a diffraction peak at 20.1±0.2°; or a diffraction peak at 29.0±0.2°; or a diffraction peak at 12.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form D of the p-tosylate has a diffraction peak at 4.9±0.2°; or a diffraction peak at 5.74±0.2°; or a diffraction peak at 17.24±0.2°; or a diffraction peak at 22.0±0.2°; or a diffraction peak at 19.5±0.2°; or a diffraction peak at 28.9±0.2°; or a diffraction peak at 25.5±0.2°; or a diffraction peak at 12.7±0.2°; or a diffraction peak at 14.8±0.2°; or a diffraction peak at 23.0±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form E of the p-tosylate has a diffraction peak at 5.4±0.2°; or a diffraction peak at 16.1±0.2°; or a diffraction peak at 9.9±0.2°; or a diffraction peak at 16.7±0.2°; or a diffraction peak at 8.4±0.2°; or a diffraction peak at 23.1±0.2°; or a diffraction peak at 26.9±0.2°; or a diffraction peak at 25.7 ±0.2°; or a diffraction peak at 25.2±0.2°; or a diffraction peak at 28.2±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-C of the besylate of (3R)-N-(4-(chlorodifluoromethoxy)phenyl)-2-(difluoromethyl)-3-methyl-3,4,5a,6-tetrahydro-5-oxa-1,2a,6,8 -tetraazabenzo[4,5]cyclooctyl[1,2,3-cd]indene-11-carboxamide;

wherein an X-ray powder diffraction pattern of the crystal form A of the besylate has a diffraction peak at 5.7±0.2°; or a diffraction peak at 17.2±0.2°; or a diffraction peak at 21.8±0.2°; or a diffraction peak at 5.5±0.2°; or a diffraction peak at 16.6±0.2°; or a diffraction peak at 23.0±0.2°; or a diffraction peak at 17.6±0.2°; or a diffraction peak at 20.3±0.2°; or a diffraction peak at 27.3±0.2°; or a diffraction peak at 28.8±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the besylate has a diffraction peak at 19.7±0.2°; or a diffraction peak at 17.4±0.2°; or a diffraction peak at 13.6±0.2°; or a diffraction peak at 22.6±0.2°; or a diffraction peak at 9.7±0.2°; or a diffraction peak at 5.74±0.2°; or a diffraction peak at 14.2±0.2°; or a diffraction peak at 29.1 ±0.2°; or a diffraction peak at 12.8±0.2°; or a diffraction peak at 23.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form C of the besylate has a diffraction peak at 5.4±0.2°; or a diffraction peak at 16.6±0.2°; or a diffraction peak at 16.9±0.2°; or a diffraction peak at 15.0±0.2°; or a diffraction peak at 12.7±0.2°; or a diffraction peak at 19.4±0.2°; or a diffraction peak at 8.3±0.2°; or a diffraction peak at 20.9 ±0.2°; or a diffraction peak at 13.8±0.2°; or a diffraction peak at 9.9±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is one of crystal forms A-B of the isethionate of (3R)-N-(4-(chlorodifluoromethoxy)phenyl)-2-(difluoromethyl)-3-methyl-3,4,5a,6-tetrahydro-5-oxa-1,2a,6,8 -tetraazabenzo[4,5]cyclooctyl[1,2,3-cd]indene-11-carboxamide;

wherein an X-ray powder diffraction pattern of the crystal form A of the isethionate has a diffraction peak at 5.4 ±0.2°; or a diffraction peak at 16.1±0.2°; or a diffraction peak at 20.9±0.2°; or a diffraction peak at 20.0±0.2°; or a diffraction peak at 25.2±0.2°; or a diffraction peak at 15.1±0.2°; or a diffraction peak at 16.7±0.2°; or a diffraction peak at 25.7±0.2°; or a diffraction peak at 12.7±0.2°; or a diffraction peak at 19.5±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

an X-ray powder diffraction pattern of the crystal form B of the isethionate has a diffraction peak at 5.9±0.2°; or a diffraction peak at 16.7±0.2°; or a diffraction peak at 21.2±0.2°; or a diffraction peak at 19.5±0.2°; or a diffraction peak at 22.5±0.2°; or a diffraction peak at 10.0±0.2°; or a diffraction peak at 13.0±0.2°; or a diffraction peak at 24.3±0.2°; or a diffraction peak at 15.5±0.2°; or a diffraction peak at 17.5±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom;

or, the crystal form is a crystal form A of the 1,5-napadisylate of (3R)-N-(4-(chlorodifluoromethoxy)phenyl)-2-(difluoromethyl)-3-methyl-3,4,5a,6-tetrahydro-5-oxa-1,2a,6,8 -tetraazabenzo[4,5]cyclooctyl[1,2,3-cd]indene-11-carboxamide, wherein an X-ray powder diffraction pattern of a crystal form A of the 1,5-napadisylate has a diffraction peak at 21.3±0.2°; or a diffraction peak at 10.2±0.2°; or a diffraction peak at 9.5±0.2°; or a diffraction peak at 17.1±0.2°; or a diffraction peak at 9.9±0.2°; or a diffraction peak at 16.7±0.2°; or a diffraction peak at 25.8 ±0.2°; or a diffraction peak at 5.74±0.2°; or a diffraction peak at 8.0±0.2°; or a diffraction peak at 23.7±0.2°; preferably comprises any 2-5, or 3-5, or 3-6, or 3-8, or 5-8, or 6-8 of the above diffraction peaks, and more preferably comprises any 6, 7, or 8 diffraction peaks selected therefrom.

**5.** The crystal form of the acid salt according to claim 4, wherein the X-ray powder diffraction pattern of the crystal form A of the ethylsulfonate comprises at least one or more diffraction peaks at 20 of 5.9±0.2°, 17.7±0.2°, and 22.3±0.2°, preferably comprises 2 diffraction peaks selected therefrom, more preferably comprises 3 diffraction peaks selected therefrom; optionally may further comprise at least one diffraction peak at 2θ of 16.7±0.2°, 21.0±0.2°, 18.0±0.2°, 5.6 ±0.2°, and 29.7±0.2°, and preferably comprises 2, 3, 4, or 5 diffraction peaks selected therefrom,

for example, the X-ray powder diffraction pattern of the crystal form A of the ethylsulfonate has diffraction peaks at 20 of:

5.9±0.2°, 16.7±0.2°, and 21.0±0.2°,
or 5.9±0.2°, 16.7±0.2°, 5.6+0.2°, and 29.7±0.2°,
or 5.9±0.2°, 17.7±0.2°, 16.7+0.2°, 21.0+0.2°, 5.6+0.2°, and 29.7±0.2°,
or 17.7±0.2°, 16.7±0.2°, 21.0+0.2°, 18.0+0.2°, 5.6+0.2°, and 29.7±0.2°;

the X-ray powder diffraction pattern of the crystal form B of the ethylsulfonate comprises at least one or more diffraction peaks at 20 of 5.6±0.2°, 16.5±0.2°, and 8.4±0.2°, preferably comprises 2 diffraction peaks selected therefrom, more preferably comprises 3 diffraction peaks selected therefrom; optionally may further comprise at least one diffraction peak at 2θ of 10.0±0.2°, 17.6±0.2°, 23.7+0.2°, 27.7±0.2°, and 15.2±0.2°, and preferably comprises 2, 3, 4, or 5 diffraction peaks selected therefrom,

for example, the X-ray powder diffraction pattern of the crystal form B of the ethylsulfonate has diffraction peaks at 20 of:
5.6±0.2°, 10.0+0.2°, and 17.6±0.2°,
or 5.6±0.2°, 10.0±0.2°, 17.6±0.2°, and 23.7±0.2°,
or 5.6±0.2°, 10.0+0.2°, 17.6+0.2°, 23.7±0.2°, 27.7+0.2°, and 15.2±0.2°;

the X-ray powder diffraction pattern of the crystal form A of the mesylate comprises at least one or more diffraction peaks at 20 of 6.0±0.2°, 17.8±0.2°, and 21.4±0.2°, preferably comprises 2 diffraction peaks selected therefrom, more preferably comprises 3 diffraction peaks selected therefrom; optionally may further comprise at least one diffraction peak at 2θ of 16.5+0.2°, 22.4±0.2°, 12.2+0.2°, 24.3±0.2°, and 23.5±0.2°, and preferably comprises 2, 3, 4, or 5 diffraction peaks selected therefrom,

for example, the X-ray powder diffraction pattern of the crystal form A of the mesylate has diffraction peaks at 20 of:
6.0+0.2°, 17.8±0.2°, and 16.5±0.2°,
or 6.0±0.2°, 17.8±0.2°, 16.5±0.2°, and 22.4±0.2°,
or 6.0±0.2°, 16.5±0.2°, 22.4+0.2°, 12.2±0.2°24.3±0.2°, and 23.5±0.2°,
or 21.4±0.2°, 16.5±0.2°, 22.4±0.2°, 12.2±0.2°, 24.3±0.2°, and 23.5±0.2°;

the X-ray powder diffraction pattern of the crystal form B of the mesylate comprises at least one or more diffraction peaks at 20 of 6.0±0.2°, 18.0±0.2°, and 22.6±0.2°, preferably comprises 2 diffraction peaks selected therefrom, more preferably comprises 3 diffraction peaks selected therefrom; optionally may further comprise at least one diffraction peak at 20 of 30.1±0.2°, 6.6±0.2°, 12.2±0.2°, 13.2±0.2°, and 15.5±0.2°, and preferably comprises 2, 3, 4, or 5 diffraction peaks selected therefrom,

for example, the X-ray powder diffraction pattern of the crystal form B of the mesylate has diffraction peaks at 20 of:
6.0±0.2°, 30.1±0.2°, and 6.6±0.2°,
or 6.0±0.2°, 30.1±0.2°, 12.2+0.2°, and 13.2±0.2°,
or 18.0±0.2°, 30.1±0.2°, 6.6+0.2°, and 12.2±0.2°,
or 22.6±0.2°, 12.2±0.2°, 13.2±0.2°, and 15.5±0.2°,
or 6.0±0.2°, 30.1±0.2°, 6.6+0.2°, 12.2±0.2°, 13.2±0.2°, and 15.5±0.2°,
or 22.6±0.2°, 30.1±0.2°, 6.6+0.2°, 12.2±0.2°, 13.2±0.2°, and 15.5±0.2°;

the X-ray powder diffraction pattern of the crystal form A of the sulfate comprises at least one or more diffraction peaks at 2θ of 5.8±0.2°, 21.6±0.2°, and 17.6±0.2°, preferably comprises 2 diffraction peaks selected therefrom, more preferably comprises 3 diffraction peaks selected therefrom; optionally may further comprise at least one diffraction peak at 2θ of 19.7±0.2°, 16.5±0.2°, 12.0±0.2°, 12.3±0.2°, and 17.2±0.2°, and preferably comprises 2, 3, 4, or 5 diffraction peaks selected therefrom,

for example, the X-ray powder diffraction pattern of the crystal form A of the sulfate has diffraction peaks at 20 of:
5.810.2°, 19.710.2°, and 16.5±0.2°,
or 21.6±0.2°, 16.5±0.2°, and 12.0±0.2°,
or 5.8±0.2°, 19.7±0.2°, 16.5+0.2°, and 12.0±0.2°,
or 5.8±0.2°, 19.7±0.2°, 16.5+0.2°, 12.0+0.2°, 12.3+0.2°, and 17.2±0.2°,
or 17.6±0.2°, 19.7±0.2°, 16.5±0.2°, 12.0±0.2°, 12.3±0.2°, and 17.24±0.2°;

the X-ray powder diffraction pattern of the crystal form B of the sulfate comprises at least one or more diffraction peaks at 2θ of 5.7±0.2°, 16.9±0.2°, and 17.4±0.2°, preferably comprises 2 diffraction peaks selected therefrom, more preferably comprises 3 diffraction peaks selected therefrom; optionally may further comprise at least one diffraction peak at 20 of 22.5±0.2°, 19.3±0.2°, 9.9±0.2°, 20.1±0.2°, and 13.8±0.2°, and preferably comprises 2, 3, 4, or 5 diffraction peaks selected therefrom,

for example, the X-ray powder diffraction pattern of the crystal form B of the sulfate has diffraction peaks at 20 of:
5.7±0.2°, 22.5±0.2°, and 19.3±0.2°,
or 5.7±0.2°, 22.5±0.2°, 19.3±0.2°, and 20.1±0.2°,
or 5.7±0.2°, 22.5±0.2°, 19.3+0.2°, 9.9±0.2°, 20.1+0.2°, and 13.8±0.2°,
or 17.4±0.2°, 22.5±0.2°, 19.3±0.2°, 9.9±0.2°, 20.1+0.2°, and 13.8±0.2°;

the X-ray powder diffraction pattern of the crystal form C of the sulfate comprises at least one or more diffraction

peaks at 2θ of 5.6±0.2°, 16.7±0.2°, and 8.3±0.2°, preferably comprises 2 diffraction peaks selected therefrom, more preferably comprises 3 diffraction peaks selected therefrom; optionally may further comprise at least one diffraction peak at 2θ of 12.7±0.2°, 15.3±0.2°, 17.6±0.2°, 10.0±0.2°, and 15.5±0.2°, and preferably comprises 2, 3, 4, or 5 diffraction peaks selected therefrom,

for example, the X-ray powder diffraction pattern of the crystal form C of the sulfate has diffraction peaks at 20 of:
5.6±0.2°, 12.7±0.2°, and 15.3±0.2°,
or 5.6±0.2°, 15.3±0.2°, 17.6+0.2°, and 15.5±0.2°,
or 5.6±0.2°, 12.7±0.2°, 15.3+0.2°, 17.6±0.2°, 10.0+0.2°, and 15.5±0.2°,
or 8.3±0.2°, 12.7±0.2°, 15.3+0.2°, 17.6±0.2°, 10.0+0.2°, and 15.5±0.2°;

the X-ray powder diffraction pattern of the crystal form D of the sulfate comprises at least one or more diffraction peaks at 20 of 17.3±0.2°, 24.3±0.2°, and 20.6±0.2°, preferably comprises 2 diffraction peaks selected therefrom, more preferably comprises 3 diffraction peaks selected therefrom; optionally may further comprise at least one diffraction peak at 2θ of 26.2±0.2°, 22.1±0.2°, 18.6±0.2°, 15.1±0.2°, and 12.9±0.2°, and preferably comprises 2, 3, 4, or 5 diffraction peaks selected therefrom,

for example, the X-ray powder diffraction pattern of the crystal form D of the sulfate has diffraction peaks at 20 of:
17.3±0.2°, 26.2±0.2°, and 12.9±0.2°,
or 24.3±0.2°, 26.2±0.2°, 22.1±0.2°, and 18.6±0.2°,
or 17.3±0.2°, 26.2±0.2°, 22.1±0.2°, 18.6±0.2°, 15.1+0.2°, and 12.9±0.2°,
or 20.6±0.2°, 26.2±0.2°, 22.1±0.2°, 18.6±0.2°, 15.1±0.2°, and 12.9±0.2°;

the X-ray powder diffraction pattern of the crystal form E of the sulfate comprises at least one or more diffraction peaks at 2θ of 5.8±0.2°, 17.2±0.2°, and 9.8±0.2°, preferably comprises 2 diffraction peaks selected therefrom, more preferably comprises 3 diffraction peaks selected therefrom; optionally may further comprise at least one diffraction peak at 2θ of 13.8±0.2°, 20.0±0.2°, 22.6±0.2°, 19.2±0.2°, and 22.2±0.2°, and preferably comprises 2, 3, 4, or 5 diffraction peaks selected therefrom,

for example, the X-ray powder diffraction pattern of the crystal form E of the sulfate has diffraction peaks at 2θ of:
5.8±0.2°, 13.8±0.2°, and 22.6±0.2°,
or 5.8±0.2°, 13.8±0.2°, 20.0+0.2°, and 22.6±0.2°,
or 5.8±0.2°, 13.8±0.2°, 20.0+0.2°, 22.6±0.2°, 19.2±0.2°, and 22.2±0.2°,
or 9.8±0.2°, 13.8±0.2°, 20.0+0.2°, 22.6±0.2°, 19.2±0.2°, and 22.2±0.2°;

the X-ray powder diffraction pattern of the crystal form F of the sulfate comprises at least one or more diffraction peaks at 2θ of 6.0±0.2°, 16.0±0.2°, and 22.4±0.2°, preferably comprises 2 diffraction peaks selected therefrom, more preferably comprises 3 diffraction peaks selected therefrom; optionally may further comprise at least one diffraction peak at 2θ of 17.3±0.2°, 20.0±0.2°, 18.5±0.2°, 20.5±0.2°, and 14.4±0.2°, and preferably comprises 2, 3, 4, or 5 diffraction peaks selected therefrom,

for example, the X-ray powder diffraction pattern of the crystal form F of the sulfate has diffraction peaks at 20 of:
6.0+0.2°, 17.3±0.2°, and 14.4±0.2°,
or 6.0+0.2°, 17.3±0.2°, 20.0+0.2°, and 18.5±0.2°,
or 6.0+0.2°, 17.3±0.2°, 20.0+0.2°, 18.5±0.2°, 20.5+0.2°, and 14.4±0.2°,
or 22.4±0.2°, 17.3±0.2°, 20.0+0.2°, 18.5±0.2°, 20.5±0.2°, and 14.4±0.2°;

the X-ray powder diffraction pattern of the crystal form G of the sulfate comprises at least one or more diffraction peaks at 2θ of 5.9±0.2°, 16.7±0.2°, and 17.6±0.2°, preferably comprises 2 diffraction peaks selected therefrom, more preferably comprises 3 diffraction peaks selected therefrom; optionally may further comprise at least one diffraction peak at 20 of 5.6±0.2°, 16.9±0.2°, 22.2±0.2°, 29.5±0.2°, and 27.7±0.2°, and preferably comprises 2, 3, 4, or 5 diffraction peaks selected therefrom,

for example, the X-ray powder diffraction pattern of the crystal form G of the sulfate has diffraction peaks at 20 of:
5.9±0.2°, 5.6+0.2°, and 16.9±0.2°,
or 5.9±0.2°, 5.6±0.2°, 16.9+0.2°, and 27.7±0.2°,
or 5.9±0.2°, 5.6±0.2°, 16.9+0.2°, 22.2±0.2°, 29.5±0.2°, and 27.7±0.2°,
or 17.6±0.2°, 5.6±0.2°, 16.9+0.2°, 22.2±0.2°, 29.5+0.2°, and 27.7±0.2°;

the X-ray powder diffraction pattern of the crystal form A of the hydrochloride comprises at least one or more diffraction peaks at 20 of 22.4±0.2°, 14.0±0.2°, and 17.1±0.2°, preferably comprises 2 diffraction peaks selected therefrom, more preferably comprises 3 diffraction peaks selected therefrom; optionally may further comprise at least one diffraction peak at 20 of 6.2±0.2°, 19.4+0.2°, 25.2±0.2°, 17.5±0.2°, and 21.6±0.2°, and preferably comprises 2, 3, 4, or 5 diffraction peaks selected therefrom,

for example, the X-ray powder diffraction pattern of the crystal form A of the hydrochloride has diffraction peaks at 20 of:
22.4±0.2°, 6.2±0.2°, and 19.4±0.2°,
or 22.4±0.2°, 6.2±0.2°, 19.4±0.2°, and 25.2±0.2°,

or 22.4±0.2°, 6.2±0.2°, 19.4+0.2°, 25.2±0.2°, 17.5±0.2°, and 21.6±0.2°,
or 17.1±0.2°, 6.2±0.2°, 19.4+0.2°, 25.2±0.2°, 17.5±0.2°, and 21.6±0.2°;
the X-ray powder diffraction pattern of the crystal form B of the hydrochloride comprises at least one or more diffraction peaks at 20 of 6.7±0.2°, 27.0±0.2°, and 23.4±0.2°, preferably comprises 2 diffraction peaks selected therefrom, more preferably comprises 3 diffraction peaks selected therefrom; optionally may further comprise at least one diffraction peak at 20 of 13.4±0.2°, 11.0±0.2°, 24.1±0.2°, 15.6±0.2°, and 4.5±0.2°, and preferably comprises 2, 3, 4, or 5 diffraction peaks selected therefrom,
for example, the X-ray powder diffraction pattern of the crystal form B of the hydrochloride has diffraction peaks at 20 of:
6.7±0.2°, 13.4±0.2°, and 1.0±0.2°,
or 6.7±0.2°, 13.4±0.2°, 11.0±0.2°, and 24.1±0.2°,
or 6.7±0.2°, 13.4±0.2°, 11.0+0.2°, 24.1±0.2°, 15.6±0.2°, and 4.5±0.2°,
or 23.4±0.2°, 13.4±0.2°, 11.0±0.2°, 24.1±0.2°, 15.6±0.2°, and 4.5±0.2°;
the X-ray powder diffraction pattern of the crystal form C of the hydrochloride comprises at least one or more diffraction peaks at 20 of 16.5±0.2°, 20.4±0.2°, and 22.2±0.2°, preferably comprises 2 diffraction peaks selected therefrom, more preferably comprises 3 diffraction peaks selected therefrom; optionally may further comprise at least one diffraction peak at 2θ of 9.7±0.2°, 17.8±0.2°, 5.3±0.2°, 17.5±0.2°, and 6.0±0.2°, and preferably comprises 2, 3, 4, or 5 diffraction peaks selected therefrom,
for example, the X-ray powder diffraction pattern of the crystal form C of the hydrochloride has diffraction peaks at 20 of:
16.5±0.2°, 9.7±0.2°, and 17.8±0.2°,
or 16.5±0.2°, 17.8±0.2°, 5.3+0.2°, and 6.0±0.2°,
or 16.5±0.2°, 9.7±0.2°, 17.8+0.2°, 5.3±0.2°, 17.5±0.2°, and 6.0±0.2°,
or 22.2±0.2°, 9.710.2°, 17.8+0.2°, 5.3±0.2°, 17.5±0.2°, and 6.0±0.2°;
the X-ray powder diffraction pattern of the crystal form A of the p-tosylate comprises at least one or more diffraction peaks at 20 of 16.8±0.2°, 19.9±0.2°, and 5.7±0.2°, preferably comprises 2 diffraction peaks selected therefrom, more preferably comprises 3 diffraction peaks selected therefrom; optionally may further comprise at least one diffraction peak at 2θ of 22.5+0.2°, 21.8±0.2°, 24.9±0.2°, 22.3±0.2°, and 20.8±0.2°, and preferably comprises 2, 3, 4, or 5 diffraction peaks selected therefrom,
for example, the X-ray powder diffraction pattern of the crystal form A of the p-tosylate has diffraction peaks at 20 of:
16.8±0.2°, 19.9±0.2°, and 5.7±0.2°,
or 16.8±0.2°, 22.5±0.2°, 21.8±0.2°, and 24.9±0.2°,
or 16.8±0.2°, 22.5±0.2°, 21.8±0.2°, 24.9±0.2°, 22.3±0.2°, and 20.8±0.2°,
or 5.7±0.2°, 22.5±0.2°, 21.8±0.2°, 24.9±0.2°, 22.3±0.2°, and 20.8±0.2°;
the X-ray powder diffraction pattern of the crystal form B of the p-tosylate comprises at least one or more diffraction peaks at 20 of 5.5±0.2°, 19.9±0.2°, and 13.2±0.2°, preferably comprises 2 diffraction peaks selected therefrom, more preferably comprises 3 diffraction peaks selected therefrom; optionally may further comprise at least one diffraction peak at 2θ of 21.9±0.2°, 28.1±0.2°, 14.1±0.2°, 10.9±0.2°, and 17.6±0.2°, and preferably comprises 2, 3, 4, or 5 diffraction peaks selected therefrom,
for example, the X-ray powder diffraction pattern of the crystal form B of the p-tosylate has diffraction peaks at 20 of:
5.5±0.2°, 21.9±0.2°, and 28.1±0.2°,
or 5.5±0.2°, 21.9±0.2°, 28.1±0.2°, and 14.1±0.2°,
or 5.5±0.2°, 21.9±0.2°, 28.1+0.2°, 14.1±0.2°, 10.9+0.2°, and 17.6±0.2°,
or 13.2±0.2°, 21.9±0.2°, 28.1±0.2°, 14.1±0.2°, 10.9±0.2°, and 17.6±0.2°;
the X-ray powder diffraction pattern of the crystal form C of the p-tosylate comprises at least one or more diffraction peaks at 20 of 5.8±0.2°, 17.3±0.2°, and 16.7±0.2°, preferably comprises 2 diffraction peaks selected therefrom, more preferably comprises 3 diffraction peaks selected therefrom; optionally may further comprise at least one diffraction peak at 2θ of 22.0±0.2°, 19.6±0.2°, 23.1+0.2°, 22.4±0.2°, and 20.1±0.2°, and preferably comprises 2, 3, 4, or 5 diffraction peaks selected therefrom,
for example, the X-ray powder diffraction pattern of the crystal form C of the p-tosylate has diffraction peaks at 20 of:
5.8±0.2°, 22.0+0.2°, and 19.6±0.2°,
or 5.8±0.2°, 22.0±0.2°, 19.6±0.2°, and 23.1±0.2°,
or 17.3±0.2°, 23.1±0.2°, 22.4±0.2°, and 20.1±0.2°,
or 5.8±0.2°, 22.0±0.2°, 19.6+0.2°, 23.1±0.2°, 22.4+0.2°, and 20.1±0.2°,
or 16.7±0.2°, 22.0+0.2°, 19.6±0.2°, 23.1±0.2°, 22.4±0.2°, and 20.1±0.2°;

the X-ray powder diffraction pattern of the crystal form D of the p-tosylate comprises at least one or more diffraction peaks at 20 of 4.9±0.2°, 5.7±0.2°, and 17.2±0.2°, preferably comprises 2 diffraction peaks selected therefrom, more preferably comprises 3 diffraction peaks selected therefrom; optionally may further comprise at least one diffraction peak at 2θ of 22.0+0.2°, 19.5±0.2°, 28.9±0.2°, 25.5±0.2°, and 12.7±0.2°, and preferably comprises 2, 3, 4, or 5 diffraction peaks selected therefrom,

for example, the X-ray powder diffraction pattern of the crystal form D of the p-tosylate has diffraction peaks at 20 of:

4.9±0.2°, 22.0+0.2°, and 19.5±0.2°,

or 4.9±0.2°, 22.0±0.2°, 19.5±0.2°, and 28.9±0.2°,

or 4.9±0.2°, 22.0±0.2°, 19.5+0.2°, 28.9±0.2°, 25.5+0.2°, and 12.7±0.2°,

or 17.2±0.2°, 22.0+0.2°, 19.5±0.2°, 28.9±0.2°, 25.5±0.2°, and 12.7±0.2°;

the X-ray powder diffraction pattern of the crystal form E of the p-tosylate comprises at least one or more diffraction peaks at 20 of 5.4±0.2°, 16.1±0.2°, and 9.9±0.2°, preferably comprises 2 diffraction peaks selected therefrom, more preferably comprises 3 diffraction peaks selected therefrom; optionally may further comprise at least one diffraction peak at 20 of 16.7±0.2°, 8.4+0.2°, 23.1±0.2°, 26.9+0.2°, and 25.7±0.2°, and preferably comprises 2, 3, 4, or 5 diffraction peaks selected therefrom,

for example, the X-ray powder diffraction pattern of the crystal form E of the p-tosylate has diffraction peaks at 20 of:

5.4±0.2°, 16.7±0.2°, and 8.4±0.2°,

or 5.4±0.2°, 16.7±0.2°, 8.4+0.2°, and 23.1±0.2°,

or 9.9±0.2°, 23.1±0.2°, 26.9±0.2°, and 25.7±0.2°,

or 5.4±0.2°, 16.7±0.2°, 8.4+0.2°, 23.1±0.2°, 26.9±0.2°, and 25.7±0.2°,

or 9.9±0.2°, 16.7±0.2°, 8.4+0.2°, 23.1±0.2°, 26.9±0.2°, and 25.7±0.2°;

the X-ray powder diffraction pattern of the crystal form A of the besylate comprises at least one or more diffraction peaks at 20 of 5.7±0.2°, 17.2±0.2°, and 21.8±0.2°, preferably comprises 2 diffraction peaks selected therefrom, more preferably comprises 3 diffraction peaks selected therefrom; optionally may further comprise at least one diffraction peak at 20 of 5.5±0.2°, 16.6+0.2°, 23.0+0.2°, 17.6±0.2°, and 20.3±0.2°, and preferably comprises 2, 3, 4, or 5 diffraction peaks selected therefrom,

for example, the X-ray powder diffraction pattern of the crystal form A of the besylate has diffraction peaks at 20 of:

5.710.2°, 5.5+0.2°, and 16.6±0.2°,

or 5.7±0.2°, 5.5±0.2°, 16.6+0.2°, and 23.0±0.2°,

or 5.7±0.2°, 5.5±0.2°, 16.6+0.2°, 23.0+0.2°, 17.6±0.2°, and 20.3±0.2°,

or 21.8±0.2°, 5.5±0.2°, 16.6+0.2°, 23.0+0.2°, 17.6±0.2°, and 20.3±0.2°;

the X-ray powder diffraction pattern of the crystal form B of the besylate comprises at least one or more diffraction peaks at 20 of 19.7±0.2°, 17.4±0.2°, and 13.6±0.2°, preferably comprises 2 diffraction peaks selected therefrom, more preferably comprises 3 diffraction peaks selected therefrom; optionally may further comprise at least one diffraction peak at 20 of 22.6+0.2°, 9.7±0.2°, 5.7+0.2°, 14.2±0.2°, and 29.1±0.2°, and preferably comprises 2, 3, 4, or 5 diffraction peaks selected therefrom,

for example, the X-ray powder diffraction pattern of the crystal form B of the besylate has diffraction peaks at 2θ of:

19.7±0.2°, 22.6±0.2°, and 9.7±0.2°,

or 19.7±0.2°, 22.6±0.2°, 9.7+0.2°, and 5.7±0.2°,

or 19.7±0.2°, 22.6±0.2°, 9.7+0.2°, 5.7±0.2°, 14.2±0.2°, and 29.1±0.2°,

or 13.6±0.2°, 22.6±0.2°, 9.7+0.2°, 5.7±0.2°, 14.2±0.2°, and 29.1±0.2°;

the X-ray powder diffraction pattern of the crystal form C of the besylate comprises at least one or more diffraction peaks at 2θ of 5.4±0.2°, 16.6±0.2°, and 16.9±0.2°, preferably comprises 2 diffraction peaks selected therefrom, more preferably comprises 3 diffraction peaks selected therefrom; optionally may further comprise at least one diffraction peak at 20 of 15.0±0.2°, 12.7±0.2°, 19.4±0.2°, 8.3±0.2°, and 20.9±0.2°, and preferably comprises 2, 3, 4, or 5 diffraction peaks selected therefrom,

for example, the X-ray powder diffraction pattern of the crystal form C of the besylate has diffraction peaks at 2θ of:

5.4±0.2°, 15.0+0.2°, and 12.7±0.2°,

or 5.4±0.2°, 15.0+0.2°, 12.7+0.2°, and 19.4±0.2°,

or 5.4±0.2°, 15.0±0.2°, 12.7±0.2°, 19.4±0.2°, 8.3+0.2°, and 20.9±0.2°,

or 16.9±0.2°, 15.0±0.2°, 12.7±0.2°, 19.4±0.2°, 8.3+0.2°, and 20.9±0.2°;

the X-ray powder diffraction pattern of the crystal form A of the isethionate comprises at least one or more diffraction peaks at 20 of 5.4±0.2°, 16.1±0.2°, and 20.9±0.2°, preferably comprises 2 diffraction peaks selected therefrom, more preferably comprises 3 diffraction peaks selected therefrom; optionally may further comprise at least one diffraction peak at 2θ of 20.0±0.2°, 25.2±0.2°, 15.1±0.2°, 16.7±0.2°, and 25.7±0.2°, and preferably comprises 2, 3, 4, or 5 diffraction peaks selected therefrom,

for example, the X-ray powder diffraction pattern of the crystal form A of the isethionate has diffraction peaks at 2θ of:

5.4±0.2°, 20.0+0.2°, and 25.2±0.2°,
or 5.4±0.2°, 20.0±0.2°, 25.2±0.2°, and 15.1±0.2°,
or 5.4±0.2°, 20.0±0.2°, 25.2+0.2°, 15.1±0.2°, 16.7+0.2°, and 25.7±0.2°,
or 20.9±0.2°, 20.0±0.2°, 25.2±0.2°, 15.1±0.2°, 16.7±0.2°, and 25.7±0.2°;

the X-ray powder diffraction pattern of the crystal form B of the isethionate comprises at least one or more diffraction peaks at 2θ of 5.9±0.2°, 16.7±0.2°, and 21.2±0.2°, preferably comprises 2 diffraction peaks selected therefrom, more preferably comprises 3 diffraction peaks selected therefrom; optionally may further comprise at least one diffraction peak at 2θ of 19.5+0.2°, 22.5±0.2°, 10.0±0.2°, 13.0±0.2°, and 24.3±0.2°, and preferably comprises 2, 3, 4, or 5 diffraction peaks selected therefrom,

for example, the X-ray powder diffraction pattern of the crystal form B of the isethionate has diffraction peaks at 2θ of:

5.9±0.2°, 19.5±0.2°, and 22.5±0.2°,
or 5.9±0.2°, 19.5±0.2°, 22.5±0.2°, and 10.0±0.2°,
or 5.9±0.2°, 19.5±0.2°, 22.5+0.2°, 10.0±0.2°, 13.0±0.2°, and 24.3±0.2°,
or 21.2±0.2°, 19.5±0.2°, 22.5±0.2°, 10.0+0.2°, 13.0±0.2°, and 24.3±0.2°; and

the X-ray powder diffraction pattern of the crystal form A of the 1,5-napadisylate comprises at least one or more diffraction peaks at 2θ of 21.3±0.2°, 10.2±0.2°, and 9.5±0.2°, preferably comprises 2 diffraction peaks selected therefrom, more preferably comprises 3 diffraction peaks selected therefrom; optionally may further comprise at least one diffraction peak at 2θ of 17.1+0.2°, 9.9±0.2°, 16.7+0.2°, 25.8±0.2°, and 5.7±0.2°, and preferably comprises 2, 3, 4, or 5 diffraction peaks selected therefrom,

for example, the X-ray powder diffraction pattern of the crystal form A of the 1,5-napadisylate has diffraction peaks at 2θ of:

21.3±0.2°, 17.1±0.2°, and 9.9±0.2°,
or 21.3±0.2°, 17.1±0.2°, 9.9+0.2°, and 16.7±0.2°,
or 21.3±0.2°, 17.1±0.2°, 9.9+0.2°, 16.7±0.2°, 25.8+0.2°, and 5.7±0.2°,
or 9.5±0.2°, 17.1±0.2°, 9.9+0.2°, 16.7±0.2°, 25.8±0.2°, and 5.7±0.2°.

**6.** The crystal form of the acid salt according to claim 4, wherein the X-ray powder diffraction pattern of the crystal form A of the ethylsulfonate comprises one or more diffraction peaks at 2θ of 5.9±0.2°, 17.7±0.2°, 22.3±0.2°, 16.7±0.2°, 21.0±0.2°, 18.0±0.2°, 5.6±0.2°, 29.7±0.2°, 23.8±0.2°, 12.2±0.2°, 18.4±0.2°, 28.0±0.2°, 24.9±0.2°, 10.0±0.2°, and 11.8±0.2°; and preferably comprises diffraction peaks optionally at 4, 6, 8, or 10 positions selected therefrom, for example, the X-ray powder diffraction pattern of the crystal form A of the ethylsulfonate has diffraction peaks at 2θ of:

5.9±0.2°, 17.7±0.2°, 22.3±0.2°, and 16.7±0.2°,
or 5.9±0.2°, 17.7±0.2°, 22.3+0.2°, 16.7±0.2°, 21.0+0.2°, and 18.0±0.2°,
or 5.9±0.2°, 17.7±0.2°, 22.3+0.2°, 16.7±0.2°, 21.0+0.2°, 18.0+0.2°, 5.6+0.2°, and 29.7±0.2°,
or 5.9±0.2°, 22.3±0.2°, 21.0±0.2°, 18.0+0.2°, 5.6+0.2°, 29.7+0.2°, 23.8+0.2°, and 12.2±0.2°,
or 5.9±0.2°, 17.7±0.2°, 22.3±0.2°, 16.7±0.2°, 21.0±0.2°, 18.0±0.2°, 5.6±0.2°, 29.7±0.2°, 23.8±0.2°, and 12.2±0.2°,
or 17.7±0.2°, 16.7±0.2°, 5.6±0.2°, 29.7±0.2°, 23.8±0.2°, 12.2±0.2°, 18.4±0.2°, 28.0±0.2°, 24.9±0.2°, and 10.0±0.2°;

the X-ray powder diffraction pattern of the crystal form B of the ethylsulfonate comprises one or more diffraction peaks at 2θ of 5.6±0.2°, 16.5±0.2°, 8.4±0.2°, 10.0±0.2°, 17.6±0.2°, 23.7+0.2°, 27.7±0.2°, 15.2±0.2°, 28.9+0.2°, 12.8±0.2°, 13.8±0.2°, 21.1±0.2°, 11.8±0.2°, 18.6±0.2°, and 13.1±0.2°; and preferably comprises diffraction peaks optionally at 4, 6, 8, or 10 positions selected therefrom,

for example, the X-ray powder diffraction pattern of the crystal form B of the ethylsulfonate has diffraction peaks at 2θ of:

5.6±0.2°, 16.5±0.2°, 8.4±0.2°, and 10.0±0.2°,
or 5.6±0.2°, 16.5±0.2°, 8.4+0.2°, 10.0±0.2°, 17.6±0.2°, and 23.7±0.2°,
or 5.6±0.2°, 16.5±0.2°, 8.4+0.2°, 10.0±0.2°, 17.6±0.2°, 23.7±0.2°, 27.7±0.2°, and 15.2±0.2°,
or 5.6±0.2°, 8.4±0.2°, 10.0+0.2°, 17.6±0.2°, 23.7±0.2°, 27.7±0.2°, 15.2±0.2°, and 12.8±0.2°,
or 5.6±0.2°, 16.5±0.2°, 8.4±0.2°, 10.0±0.2°, 17.6±0.2°, 23.7±0.2°, 27.7±0.2°, 15.2±0.2°, 28.9±0.2°, and 12.8±0.2°,
or 16.5±0.2°, 8.4±0.2°, 10.0±0.2°, 17.6±0.2°, 23.7±0.2°, 27.7±0.2°, 15.2±0.2°, 28.9±0.2°, 12.8±0.2°, and 13.8±0.2°;

the X-ray powder diffraction pattern of the crystal form A of the mesylate comprises one or more diffraction peaks at 2θ of 6.0±0.2°, 17.8+0.2°, 21.4±0.2°, 16.5±0.2°, 22.4±0.2°, 12.2+0.2°, 24.3±0.2°, 23.5+0.2°, 29.8±0.2°, 19.8±0.2°, 16.7±0.2°, 25.8±0.2°, 14.8±0.2°, 28.0±0.2°, and 33.9±0.2°; and preferably comprises diffraction peaks optionally at 4, 6, 8, or 10 positions selected therefrom,

for example, the X-ray powder diffraction pattern of the crystal form A of the mesylate has diffraction peaks at 2θ of: 6.0±0.2°, 17.8±0.2°, 21.4±0.2°, and 16.5±0.2°,

or 6.0+0.2°, 17.8±0.2°, 21.4+0.2°, 16.5±0.2°, 22.4+0.2°, and 12.2±0.2°,

or 6.0±0.2°, 17.8±0.2°, 21.4+0.2°, 16.5±0.2°, 22.4+0.2°, 12.2±0.2°, 24.3+0.2°, and 23.5±0.2°,

or 17.8±0.2°, 21.4±0.2°, 16.5±0.2°, 22.4±0.2°, 12.2±0.2°, 24.3±0.2°, 23.5±0.2°, and 29.8±0.2°,

or 6.0±0.2°, 17.8±0.2°, 21.4±0.2°, 16.5±0.2°, 22.4±0.2°, 12.2±0.2°, 24.3±0.2°, 23.5±0.2°, 29.8±0.2°, and 19.8±0.2°,

or 17.8±0.2°, 21.4±0.2°, 16.5±0.2°, 22.4±0.2°, 12.2±0.2°, 24.3±0.2°, 23.5±0.2°, 29.8±0.2°, 19.8±0.2°, and 16.7±0.2°;

the X-ray powder diffraction pattern of the crystal form B of the mesylate comprises one or more diffraction peaks at 2θ of 6.0±0.2°, 18.0±0.2°, 22.6±0.2°, 30.1±0.2°, 6.6±0.2°, 12.2±0.2°, 13.2±0.2°, 15.5±0.2°, 21.4±0.2°, 24.0±0.2°, 12.0±0.2°, 10.1±0.2°, 24.6±0.2°, 16.6±0.2°, and 19.8±0.2°; and preferably comprises diffraction peaks optionally at 4, 6, 8, or 10 positions selected therefrom,

for example, the X-ray powder diffraction pattern of the crystal form B of the mesylate has diffraction peaks at 2θ of: 6.0+0.2°, 18.0+0.2°, 22.6±0.2°, and 30.1±0.2°,

or 6.0±0.2°, 18.0+0.2°, 22.6+0.2°, 30.1±0.2°, 6.6+0.2°, and 12.2±0.2°,

or 6.0±0.2°, 18.0+0.2°, 22.6+0.2°, 30.1±0.2°, 6.6+0.2°, 12.2±0.2°, 13.2±0.2°, and 15.5±0.2°,

or 18.0±0.2°, 22.6±0.2°, 30.1±0.2°, 6.6±0.2°, 12.2±0.2°, 13.2±0.2°, 15.5±0.2°, and 21.4±0.2°,

or 6.0±0.2°, 18.0±0.2°, 22.6±0.2°, 30.1±0.2°, 6.6±0.2°, 12.2±0.2°, 13.2±0.2°, 15.5±0.2°, 21.4±0.2°, and 24.0±0.2°;

the X-ray powder diffraction pattern of the crystal form A of the sulfate comprises one or more diffraction peaks at 2θ of 5.8±0.2°, 21.6±0.2°,17.6±0.2°, 19.7±0.2°, 16.5±0.2°, 12.0±0.2°, 12.3±0.2°, 17.2±0.2°, 13.6±0.2°, 25.9±0.2°, 23.5±0.2°, 21.8±0.2°, 14.4±0.2°, 10.4±0.2°, and 24.7±0.2°; and preferably comprises diffraction peaks optionally at 4, 6, 8, or 10 positions selected therefrom,

for example, the X-ray powder diffraction pattern of the crystal form A of the sulfate has diffraction peaks at 2θ of: 5.8±0.2°, 21.6±0.2°,17.6±0.2°, and 19.7±0.2°,

or 5.8±0.2°, 21.6±0.2°,17.6+0.2°, 19.7±0.2°, 16.5±0.2°, and 12.0±0.2°,

or 5.8±0.2°, 21.6±0.2°,17.6+0.2°, 19.7±0.2°, 16.5+0.2°, 12.0±0.2°, 12.3±0.2°, and 17.2±0.2°,

or 5.8±0.2°, 21.6±0.2°,17.6±0.2°, 19.7±0.2°, 16.5±0.2°, 12.0±0.2°, 12.3±0.2°, 17.2±0.2°, 13.6±0.2°, and 25.9±0.2°,

or 21.6±0.2°, 17.6±0.2°, 19.7±0.2°, 16.5±0.2°, 12.0±0.2°, 12.3±0.2°, 17.2±0.2°, 13.6±0.2°, 25.9±0.2°, and 23.5±0.2°;

the X-ray powder diffraction pattern of the crystal form B of the sulfate comprises one or more diffraction peaks at 2θ of 5.7±0.2°, 16.9±0.2°, 17.4±0.2°, 22.5±0.2°, 19.3±0.2°, 9.9±0.2°, 20.1+0.2°, 13.8±0.2°, 11.1+0.2°, 18.6 ±0.2°, 27.2±0.2°, 26.8±0.2°, 24.2±0.2°, 25.4±0.2°, and 23.7±0.2°; and preferably comprises diffraction peaks optionally at 4, 6, 8, or 10 positions selected therefrom,

for example, the X-ray powder diffraction pattern of the crystal form B of the sulfate has diffraction peaks at 2θ of: 5.7±0.2°, 16.9±0.2°, 17.4±0.2°, and 22.5±0.2°,

or 16.9±0.2°, 17.4±0.2°, 22.5±0.2°, 19.3±0.2°, 9.9+0.2°, and 20.1±0.2°,

or 5.7±0.2°, 16.9±0.2°, 17.4±0.2°, 22.5±0.2°, 19.3+0.2°, 9.9+0.2°, 20.1+0.2°, and 13.8±0.2°,

or 5.7±0.2°, 16.9±0.2°, 17.4±0.2°, 22.5±0.2°, 19.3±0.2°, 9.9±0.2°, 20.1±0.2°, 13.8±0.2°, 11.1±0.2°, and 18.6±0.2°;

the X-ray powder diffraction pattern of the crystal form C of the sulfate comprises one or more diffraction peaks at 2θ of 5.6±0.2°, 16.7±0.2°, 8.3+0.2°, 12.7±0.2°, 15.3±0.2°, 17.6±0.2°, 10.0±0.2°, 15.5±0.2°, 13.1+0.2°, 21.0+0.2°, 25.7±0.2°, 25.3±0.2°, 19.5±0.2°, 20.1+0.2°, and 18.6±0.2°; and preferably comprises diffraction peaks optionally at 4, 6, 8, or 10 positions selected therefrom,

for example, the X-ray powder diffraction pattern of the crystal form C of the sulfate has diffraction peaks at 2θ of: 5.6±0.2°, 16.7±0.2°, 8.3+0.2°, and 12.7±0.2°,

or 5.6±0.2°, 16.7±0.2°, 8.3+0.2°, 12.7±0.2°, 15.3±0.2°, and 17.6±0.2°,

or 5.6±0.2°, 16.7±0.2°, 8.3+0.2°, 12.7±0.2°, 15.3±0.2°, 17.6±0.2°, 10.0±0.2°, and 15.5±0.2°,

or 5.6±0.2°, 16.7±0.2°, 8.3±0.2°, 12.7±0.2°, 15.3±0.2°, 17.6±0.2°, 10.0±0.2°, 15.5±0.2°, 13.1±0.2°, and 21.0±0.2°;

the X-ray powder diffraction pattern of the crystal form D of the sulfate comprises one or more diffraction peaks at 2θ of 17.3±0.2°, 24.3±0.2°, 20.6±0.2°, 26.2±0.2°, 22.1±0.2°, 18.6±0.2°, 15.1±0.2°, 12.9±0.2°, 25.9±0.2°,

18.0+0.2°, 25.8±0.2°, 22.9±0.2°, 29.110.2°, 6.8±0.2°, and 11.4±0.2°; and preferably comprises diffraction peaks optionally at 4, 6, 8, or 10 positions selected therefrom,

for example, the X-ray powder diffraction pattern of the crystal form D of the sulfate has diffraction peaks at 20 of: 17.3±0.2°, 24.3±0.2°, 20.6±0.2°, and 26.2±0.2°,

or 17.3±0.2°, 24.3±0.2°, 20.6±0.2°, 26.2±0.2°, 22.1+0.2°, and 18.6±0.2°,

or 17.3±0.2°, 24.3±0.2°, 20.6±0.2°, 26.2±0.2°, 22.1±0.2°, 18.6±0.2°, 15.1+0.2°, and 12.9±0.2°,

or 17.3±0.2°, 24.3±0.2°, 20.6±0.2°, 26.2±0.2°, 22.1±0.2°, 18.6±0.2°, 15.1±0.2°, 12.9±0.2°, 25.9±0.2°, and 18.0±0.2°;

the X-ray powder diffraction pattern of the crystal form E of the sulfate comprises one or more diffraction peaks at 20 of 5.8±0.2°, 17.2±0.2°, 9.8±0.2°, 13.8±0.2°, 20.0+0.2°, 22.6±0.2°, 19.2+0.2°, 22.2±0.2°, 11.1±0.2°, 26.2 ±0.2°, 24.3±0.2°, 20.6±0.2°, 18.6±0.2°, 19.7±0.2°, and 15.1±0.2°; and preferably comprises diffraction peaks optionally at 4, 6, 8, or 10 positions selected therefrom,

for example, the X-ray powder diffraction pattern of the crystal form E of the sulfate has diffraction peaks at 2θ of: 5.8±0.2°, 17.2±0.2°, 9.8±0.2°, and 13.8±0.2°,

or 5.8±0.2°, 17.2±0.2°, 9.8+0.2°, 13.8±0.2°, 20.0+0.2°, and 22.6±0.2°,

or 5.8±0.2°, 17.2±0.2°, 9.8+0.2°, 13.8±0.2°, 20.0+0.2°, 22.6±0.2°, 19.2±0.2°, and 22.2±0.2°,

or 5.8±0.2°, 17.2±0.2°, 9.8±0.2°, 13.8±0.2°, 20.0±0.2°, 22.6±0.2°, 19.2±0.2°, 22.2±0.2°, 11.1±0.2°, and 26.2±0.2°;

the X-ray powder diffraction pattern of the crystal form F of the sulfate comprises one or more diffraction peaks at 20 of 6.0±0.2°, 16.0+0.2°, 22.4±0.2°, 17.3+0.2°, 20.0+0.2°, 18.5±0.2°, 20.5±0.2°, 14.4+0.2°, 24.9±0.2°, 24.4 ±0.2°, 17.5±0.2°, 26.2±0.2°, 18.0±0.2°, 27.5±0.2°, and 21.5±0.2°; and preferably comprises diffraction peaks optionally at 4, 6, 8, or 10 positions selected therefrom,

for example, the X-ray powder diffraction pattern of the crystal form F of the sulfate has diffraction peaks at 20 of: 6.0+0.2°, 16.0+0.2°, 22.4±0.2°, and 17.3±0.2°,

or 6.0+0.2°, 16.0+0.2°, 22.4+0.2°, 17.3±0.2°, 20.0+0.2°, and 18.5±0.2°,

or 16.0+0.2°, 22.4±0.2°, 17.3±0.2°, 20.0+0.2°, 18.5±0.2°, and 20.5±0.2°,

or 16.0+0.2°, 22.4±0.2°, 17.3±0.2°, 20.0+0.2°, 18.5+0.2°, 20.5±0.2°, 14.4±0.2°, and 24.9±0.2°,

or 6.0±0.2°, 16.0±0.2°, 22.4±0.2°, 17.3±0.2°, 20.0±0.2°, 18.5±0.2°, 20.5±0.2°, 14.4±0.2°, 24.9±0.2°, and 24.4±0.2°;

the X-ray powder diffraction pattern of the crystal form G of the sulfate comprises one or more diffraction peaks at 20 of 5.9±0.2°, 16.7±0.2°, 17.6±0.2°, 5.6±0.2°, 16.9±0.2°, 22.2±0.2°, 29.5±0.2°, 27.7±0.2°, 25.1±0.2°, 10.2 ±0.2°, 24.4±0.2°, 8.4±0.2°, 12.1±0.2°, 24.2±0.2°, and 15.4±0.2°; and preferably comprises diffraction peaks optionally at 4, 6, 8, or 10 positions selected therefrom,

for example, the X-ray powder diffraction pattern of the crystal form G of the sulfate has diffraction peaks at 20 of: 5.9±0.2°, 16.7±0.2°, 17.6±0.2°, and 5.6±0.2°,

or 5.9±0.2°, 16.7±0.2°, 17.6+0.2°, 5.6±0.2°, 16.9±0.2°, and 22.2±0.2°,

or 5.9±0.2°, 16.7±0.2°, 17.6±0.2°, 5.6±0.2°, 16.9+0.2°, 22.2±0.2°, 29.5+0.2°, and 27.7±0.2°,

or 5.9±0.2°, 16.7±0.2°, 17.6±0.2°, 5.6±0.2°, 16.9±0.2°, 22.2±0.2°, 29.5±0.2°, 27.7±0.2°, 25.1±0.2°, and 10.2±0.2°;

the X-ray powder diffraction pattern of the crystal form A of the hydrochloride comprises one or more diffraction peaks at 20 of 22.4±0.2°, 14.0±0.2°, 17.1±0.2°, 6.2±0.2°, 19.4±0.2°, 25.2±0.2°, 17.5±0.2°, 21.6+0.2°, 19.8 ±0.2°, 23.4±0.2°, 10.6±0.2°, 30.5±0.2°, 12.4±0.2°, 9.8±0.2°, and 11.1±0.2°; and preferably comprises diffraction peaks optionally at 4, 6, 8, or 10 positions selected therefrom,

for example, the X-ray powder diffraction pattern of the crystal form A of the hydrochloride has diffraction peaks at 20 of:

22.4±0.2°, 14.0+0.2°, 17.1±0.2°, and 6.2±0.2°,

or 22.4±0.2°, 14.0+0.2°, 17.1±0.2°, 6.2±0.2°, 19.4+0.2°, and 25.2±0.2°,

or 22.4±0.2°, 14.0±0.2°, 17.1±0.2°, 6.2±0.2°, 19.4+0.2°, 25.2±0.2°, 17.5±0.2°, and 21.6±0.2°,

or 22.4±0.2°, 14.0±0.2°, 17.1±0.2°, 6.2±0.2°, 19.4±0.2°, 25.2±0.2°, 17.5±0.2°, 21.6+0.2°, 19.8±0.2°, and 23.4±0.2°,

the X-ray powder diffraction pattern of the crystal form B of the hydrochloride comprises one or more diffraction peaks at 20 of 6.7±0.2°, 27.0+0.2°, 23.410.2°, 13.4±0.2°, 11.0+0.2°, 24.1±0.2°, 15.6+0.2°, 4.5±0.2°, 20.0+0.2°, 10.2±0.2°, 14.3±0.2°, 10.0+0.2°, 20.5±0.2°, 23.0+0.2°, and 31.0+0.2°; and preferably comprises diffraction peaks optionally at 4, 6, 8, or 10 positions selected therefrom,

for example, the X-ray powder diffraction pattern of the crystal form B of the hydrochloride has diffraction peaks at 20 of:

6.7±0.2°, 27.0+0.2°, 23.4±0.2°, and 13.4±0.2°,

or 6.7±0.2°, 27.0±0.2°, 23.4±0.2°, 13.4±0.2°, 11.0±0.2°, and 24.1±0.2°,

or 6.7±0.2°, 27.0±0.2°, 23.4±0.2°, 13.4±0.2°, 11.0+0.2°, 24.1±0.2°, 15.6±0.2°, and 4.5±0.2°,
or 6.7±0.2°, 27.0±0.2°, 23.4±0.2°, 13.4±0.2°, 11.0±0.2°, 24.1±0.2°, 15.6±0.2°, 4.5±0.2°, 20.0±0.2°, and 10.2±0.2°;
the X-ray powder diffraction pattern of the crystal form C of the hydrochloride comprises one or more diffraction peaks at 2θ of 16.5±0.2°, 20.4±0.2°, 22.2±0.2°, 9.7±0.2°, 17.8±0.2°, 5.3±0.2°, 17.5±0.2°, 6.0±0.2°, 14.3 ±0.2°, 21.7±0.2°, 24.6±0.2°, 10.9±0.2°, 27.2±0.2°, 20.8±0.2°, and 19.7±0.2°; and preferably comprises diffraction peaks optionally at 4, 6, 8, or 10 positions selected therefrom,
for example, the X-ray powder diffraction pattern of the crystal form C of the hydrochloride has diffraction peaks at 2θ of:
16.5±0.2°, 20.4±0.2°, 22.2±0.2°, and 9.7±0.2°,
or 16.5±0.2°, 20.4±0.2°, 22.2±0.2°, 9.7±0.2°, 17.8+0.2°, and 5.3±0.2°,
or 16.5±0.2°, 20.4±0.2°, 22.2±0.2°, 9.7±0.2°, 17.8+0.2°, 5.3±0.2°, 17.5±0.2°, and 6.0±0.2°,
or 16.5±0.2°, 20.410.2°, 22.2±0.2°, 9.7±0.2°, 17.8+0.2°, 5.3±0.2°, 17.5±0.2°, 6.0+0.2°, 14.3±0.2°, and 21.7 ±0.2°;
the X-ray powder diffraction pattern of the crystal form A of the p-tosylate comprises one or more diffraction peaks at 2θ of 16.8±0.2°, 19.9±0.2°, 5.7±0.2°, 22.5±0.2°, 21.8±0.2°, 24.9±0.2°, 22.3±0.2°, 20.8±0.2°, 26.6±0.2°, 12.4±0.2°, 15.1±0.2°, 13.8±0.2°, 21.3±0.2°, 27.7±0.2°, and 20.5±0.2°; and preferably comprises diffraction peaks optionally at 4, 6, 8, or 10 positions selected therefrom,
for example, the X-ray powder diffraction pattern of the crystal form A of the p-tosylate has diffraction peaks at 2θ of:
16.8±0.2°, 19.9±0.2°, 5.7±0.2°, and 22.5±0.2°,
or 16.8±0.2°, 19.9±0.2°, 5.7+0.2°, 22.5±0.2°, 21.8+0.2°, and 24.9±0.2°,
or 16.8±0.2°, 19.9±0.2°, 5.7+0.2°, 12.4±0.2°, 13.8+0.2°, 22.5±0.2°, 21.8+0.2°, and 20.8±0.2°,
or 19.9±0.2°, 5.7±0.2°, 22.5±0.2°, 21.8±0.2°, 24.9±0.2°, 22.3±0.2°, 20.8±0.2°, 26.6±0.2°, 12.4±0.2°, and 15.1±0.2°;
the X-ray powder diffraction pattern of the crystal form B of the p-tosylate comprises one or more diffraction peaks at 2θ of 5.5±0.2°, 19.9±0.2°, 13.2±0.2°, 21.9±0.2°, 28.1+0.2°, 14.1±0.2°, 10.9±0.2°, 17.6±0.2°, 9.5±0.2°, 20.4±0.2°, 17.8±0.2°, 22.1+0.2°, 21.5±0.2°, 16.3±0.2°, and 26.5±0.2°; and preferably comprises diffraction peaks optionally at 4, 6, 8, or 10 positions selected therefrom,
for example, the X-ray powder diffraction pattern of the crystal form B of the p-tosylate has diffraction peaks at 2θ of:
5.5±0.2°, 19.9±0.2°, 13.2±0.2°, and 21.9±0.2°,
or 5.5±0.2°, 19.9±0.2°, 13.2+0.2°, 21.9±0.2°, 28.1±0.2°, and 14.1±0.2°,
or 5.5±0.2°, 19.9±0.2°, 13.2+0.2°, 21.9±0.2°, 28.1+0.2°, 14.1±0.2°, 10.9+0.2°, and 17.6±0.2°,
or 19.9±0.2°, 13.2±0.2°, 21.9±0.2°, 28.1±0.2°, 14.1±0.2°, 10.9±0.2°, 17.6±0.2°, 9.5±0.2°, 20.4±0.2°, and 17.8±0.2°;
the X-ray powder diffraction pattern of the crystal form C of the p-tosylate comprises one or more diffraction peaks at 2θ of 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, 12.8±0.2°, 21.6±0.2°, 11.5±0.2°, 13.8±0.2°, 27.4±0.2°, and 20.9±0.2°; and preferably comprises diffraction peaks optionally at 4, 6, 8, or 10 positions selected therefrom,
for example, the X-ray powder diffraction pattern of the crystal form C of the p-tosylate has diffraction peaks at 2θ of:
5.8±0.2°, 17.3±0.2°, 16.7±0.2°, and 22.0±0.2°,
or 5.8±0.2°, 17.3±0.2°, 16.7±0.2°, 22.0+0.2°, 19.6±0.2°, and 23.1±0.2°,
or 5.8±0.2°, 17.3±0.2°, 16.7±0.2°, 22.0±0.2°, 19.6±0.2°, 23.14±0.2°, 22.4+0.2°, and 20.1±0.2°,
or 5.8±0.2°, 17.3±0.2°, 16.7±0.2°, 22.0±0.2°, 19.6±0.2°, 23.1±0.2°, 22.4±0.2°, 20.1±0.2°, 29.0±0.2°, and 12.8±0.2°;
the X-ray powder diffraction pattern of the crystal form D of the p-tosylate comprises one or more diffraction peaks at 2θ of 4.9±0.2°, 5.7±0.2°, 17.2±0.2°, 22.0+0.2°, 19.5±0.2°, 28.9+0.2°, 25.5±0.2°, 12.7±0.2°, 14.8±0.2°, 23.0±0.2°, 20.6±0.2°, 27.3±0.2°, 30.4±0.2°, 24.8±0.2°, and 27.7±0.2°; and preferably comprises diffraction peaks optionally at 4, 6, 8, or 10 positions selected therefrom,
for example, the X-ray powder diffraction pattern of the crystal form D of the p-tosylate has diffraction peaks at 2θ of:
4.9±0.2°, 5.7±0.2°, 17.2±0.2°, and 22.0±0.2°,
or 4.9±0.2°, 5.7±0.2°, 17.2+0.2°, 22.0+0.2°, 19.5±0.2°, and 28.9±0.2°,
or 4.9±0.2°, 5.7±0.2°, 17.2+0.2°, 22.0+0.2°, 19.5±0.2°, 28.9±0.2°, 25.5±0.2°, and 12.7±0.2°,
or 4.9±0.2°, 5.7±0.2°, 17.2±0.2°, 22.0±0.2°, 19.5±0.2°, 28.9±0.2°, 25.5±0.2°, 12.7±0.2°, 14.8±0.2°, and 23.0±0.2°;

the X-ray powder diffraction pattern of the crystal form E of the p-tosylate comprises one or more diffraction peaks at 2θ of 5.4±0.2°, 16.1±0.2°, 9.94±0.2°, 16.7±0.2°, 8.44±0.2°, 23.1±0.2°, 26.9±0.2°, 25.7±0.2°, 25.2±0.2°, 28.2±0.2°, 18.5±0.2°, 16.9±0.2°, 32.4±0.2°, 11.6±0.2°, and 15.1±0.2°; and preferably comprises diffraction peaks optionally at 4, 6, 8, or 10 positions selected therefrom,

for example, the X-ray powder diffraction pattern of the crystal form E of the p-tosylate has diffraction peaks at 2θ of:

5.4±0.2°, 16.1±0.2°, 9.9±0.2°, and 16.7±0.2°,

or 5.4±0.2°, 16.14±0.2°, 9.9±0.2°, 16.7±0.2°, 8.4±0.2°, and 23.1±0.2°,

or 5.4±0.2°, 16.14±0.2°, 9.9±0.2°, 16.7±0.2°, 8.4±0.2°, 23.1±0.2°, 26.9±0.2°, and 25.7±0.2°,

or 5.4±0.2°, 16.110.2°, 9.9±0.2°, 16.7+0.2°, 8.4±0.2°, 23.1±0.2°, 26.9±0.2°, 25.7±0.2°, 25.2±0.2°, and 28.2 ±0.2°;

the X-ray powder diffraction pattern of the crystal form A of the besylate comprises one or more diffraction peaks at 2θ of 55.7±0.2°, 17.2±0.2°, 21.8±0.2°, 5.5+0.2°, 16.6+0.2°, 23.0+0.2°, 17.6±0.2°, 20.3+0.2°, 27.3±0.2°, 28.8 ±0.2°, 11.5±0.2°, 13.8±0.2°, 25.5±0.2°, 19.9±0.2°, and 21.3±0.2°; and preferably comprises diffraction peaks optionally at 4, 6, 8, or 10 positions selected therefrom,

for example, the X-ray powder diffraction pattern of the crystal form A of the besylate has diffraction peaks at 2θ of:

55.7±0.2°, 17.2±0.2°, 21.8±0.2°, and 5.54±0.2°,

or 55.7±0.2°, 17.2±0.2°, 21.8±0.2°, 5.5±0.2°, 16.6±0.2°, and 23.0±0.2°,

or 55.7±0.2°, 17.2±0.2°, 21.8±0.2°, 5.510.2°, 16.6+0.2°, 23.0+0.2°, 17.6±0.2°, and 20.3±0.2°,

or 55.7±0.2°, 17.2±0.2°, 21.8+0.2°, 5.5±0.2°, 16.6+0.2°, 23.0+0.2°, 17.6±0.2°, 20.3+0.2°, 27.3±0.2°, and 28.8 ±0.2°;

the X-ray powder diffraction pattern of the crystal form B of the besylate comprises one or more diffraction peaks at 2θ of 19.7+0.2°, 17.4±0.2°, 13.6±0.2°, 22.6±0.2°, 9.7±0.2°, 5.7+0.2°, 14.2+0.2°, 29.1+0.2°, 12.8+0.2°, 23.7 ±0.2°, 26.4±0.2°, 27.3±0.2°, 24.0+0.2°, 20.7±0.2°, and 21.6±0.2°; and preferably comprises diffraction peaks optionally at 4, 6, 8, or 10 positions selected therefrom,

for example, the X-ray powder diffraction pattern of the crystal form B of the besylate has diffraction peaks at 2θ of:

19.7±0.2°, 17.4±0.2°, 13.6±0.2°, and 22.6±0.2°,

or 19.7±0.2°, 17.4±0.2°, 13.6±0.2°, 22.6±0.2°, 9.7+0.2°, and 5.7±0.2°,

or 19.7±0.2°, 17.4±0.2°, 13.6±0.2°, 22.6±0.2°, 9.7+0.2°, 5.7±0.2°, 14.2±0.2°, and 29.1±0.2°,

or 19.7±0.2°, 17.4±0.2°, 13.6±0.2°, 22.6±0.2°, 9.7±0.2°, 5.7±0.2°, 14.24±0.2°, 29.1±0.2°, 12.8±0.2°, and 23.7±0.2°;

the X-ray powder diffraction pattern of the crystal form C of the besylate comprises one or more diffraction peaks at 2θ of 5.4±0.2°, 16.6±0.2°, 16.94±0.2°, 15.0±0.2°, 12.74±0.2°, 19.4±0.2°, 8.3±0.2°, 20.9±0.2°, 13.8±0.2°, 9.9+0.2°, 25.6±0.2°, 25.2±0.2°, 16.2±0.2°, 18.5±0.2°, and 19.9±0.2°; and preferably comprises diffraction peaks optionally at 4, 6, 8, or 10 positions selected therefrom,

for example, the X-ray powder diffraction pattern of the crystal form C of the besylate has diffraction peaks at 2θ of:

5.4±0.2°, 16.6±0.2°, 16.9±0.2°, and 15.0±0.2°,

or 5.4±0.2°, 16.6±0.2°, 16.9+0.2°, 15.0+0.2°, 12.7+0.2°, and 19.4±0.2°,

or 5.4±0.2°, 16.6±0.2°, 16.9±0.2°, 15.0+0.2°, 12.7±0.2°, 19.4±0.2°, 8.3+0.2°, and 20.9±0.2°,

or 5.4±0.2°, 16.6±0.2°, 16.9±0.2°, 15.0+0.2°, 12.7±0.2°, 19.4+0.2°, 8.3±0.2°, 20.94±0.2°, 13.8±0.2°, and 9.9 ±0.2°;

the X-ray powder diffraction pattern of the crystal form A of the isethionate comprises one or more diffraction peaks at 2θ of 5.4±0.2°, 16.1+0.2°, 20.9±0.2°, 20.0+0.2°, 25.2±0.2°, 15.1±0.2°, 16.7±0.2°, 25.7+0.2°, 12.7 ±0.2°, 19.5±0.2°, 22.1±0.2°, 8.4±0.2°, 23.7±0.2°, 28.3±0.2°, and 9.9±0.2°; and preferably comprises diffraction peaks optionally at 4, 6, 8, or 10 positions selected therefrom,

for example, the X-ray powder diffraction pattern of the crystal form A of the isethionate has diffraction peaks at 2θ of:

5.4±0.2°, 16.1±0.2°, 20.9±0.2°, and 20.0±0.2°,

or 5.4±0.2°, 16.1±0.2°, 20.9+0.2°, 20.0+0.2°, 25.2+0.2°, and 15.14±0.2°,

or 5.4±0.2°, 16.110.2°, 20.9+0.2°, 20.0+0.2°, 25.2+0.2°, 15.14±0.2°, 16.7±0.2°, and 25.7±0.2°,

or 5.4±0.2°, 16.1±0.2°, 20.9±0.2°, 20.0±0.2°, 25.2±0.2°, 15.1±0.2°, 16.7±0.2°, 25.7±0.2°, 12.7±0.2°, and 19.5±0.2°;

the X-ray powder diffraction pattern of the crystal form B of the isethionate comprises one or more diffraction peaks at 2θ of 5.9±0.2°, 16.7+0.2°, 21.2±0.2°, 19.5±0.2°, 22.5±0.2°, 10.0±0.2°, 13.0+0.2°, 24.3+0.2°, 15.5 ±0.2°, 17.5±0.2°, 20.1±0.2°, 17.7±0.2°, 26.2±0.2°, 16.9±0.2°, and 27.6±0.2°; and preferably comprises diffraction peaks optionally at 4, 6, 8, or 10 positions selected therefrom,

for example, the X-ray powder diffraction pattern of the crystal form B of the isethionate has diffraction peaks at 2θ of:

5.9±0.2°, 16.7±0.2°, 21.2±0.2°, and 19.54±0.2°,
or 5.9±0.2°, 16.710.2°, 21.2+0.2°, 19.5±0.2°, 22.5±0.2°, and 10.0±0.2°,
or 5.9±0.2°, 16.7±0.2°, 21.2+0.2°, 19.5±0.2°, 22.5+0.2°, 10.0±0.2°, 13.0±0.2°, and 24.3±0.2°,
or 5.9±0.2°, 16.7±0.2°, 21.2±0.2°, 19.5±0.2°, 22.5±0.2°, 10.0±0.2°, 13.0±0.2°, 24.3±0.2°, 15.5±0.2°, and 17.5±0.2°; and
the X-ray powder diffraction pattern of the crystal form A of the 1,5-napadisylate comprises one or more diffraction peaks at 20 of 21.3±0.2°, 10.24±0.2°, 9.5±0.2°, 17.1±0.2°, 9.9±0.2°, 16.7±0.2°, 25.8±0.2°, 5.7±0.2°, 8.0 ±0.2°, 23.7±0.2°, 23.0±0.2°, 18.2±0.2°, 19.9±0.2°, 12.2±0.2°, and 13.7±0.2°; and preferably comprises diffraction peaks optionally at 4, 6, 8, or 10 positions selected therefrom,
for example, the X-ray powder diffraction pattern of the crystal form A of the 1,5-napadisylate has diffraction peaks at 2θ of:
21.3±0.2°, 10.2±0.2°, 9.5±0.2°, and 17.1±0.2°,
or 21.3±0.2°, 10.2±0.2°, 9.5+0.2°, 17.1±0.2°, 9.9+0.2°, and 16.7±0.2°,
or 21.3±0.2°, 10.2±0.2°, 9.5+0.2°, 17.1±0.2°, 9.9+0.2°, 16.7+0.2°, 25.8+0.2°, and 5.7±0.2°,
or 21.3±0.2°, 10.2±0.2°, 9.5±0.2°, 17.1±0.2°, 9.9±0.2°, 16.7+0.2°, 25.8+0.2°, 5.7±0.2°, 8.0±0.2°, and 23.7 ±0.2°.

**7.** The crystal form of the acid salt according to claim 4, wherein

the crystal form A of the ethylsulfonate has an X-ray powder diffraction pattern as shown in FIG. 1, or has a DSC pattern as shown in FIG. 2, or has a TGA pattern as shown in FIG. 3;
the crystal form B of the ethylsulfonate has an X-ray powder diffraction pattern as shown in FIG. 4, or has a DSC pattern as shown in FIG. 5, or has a TGA pattern as shown in FIG. 6;
the crystal form A of the mesylate has an X-ray powder diffraction pattern as shown in FIG. 7, or has a DSC pattern as shown in FIG. 8, or has a TGA pattern as shown in FIG. 9;
the crystal form B of the mesylate has an X-ray powder diffraction pattern as shown in FIG. 10 or has a DSC pattern as shown in FIG. 11;
the crystal form A of the sulfate has an X-ray powder diffraction pattern as shown in FIG. 17, or has a DSC pattern as shown in FIG. 18, or has a TGA pattern as shown in FIG. 19;
the crystal form B of the sulfate has an X-ray powder diffraction pattern as shown in FIG. 20, or has a DSC pattern as shown in FIG. 21, or has a TGA pattern as shown in FIG. 22;
the crystal form C of the sulfate has an X-ray powder diffraction pattern as shown in FIG. 23, has a DSC pattern as shown in FIG. 24, or has a TGA pattern as shown in FIG. 25;
the crystal form D of the sulfate has an X-ray powder diffraction pattern as shown in FIG. 26, has a DSC pattern as shown in FIG. 27, or has a TGA pattern as shown in FIG. 28;
the crystal form E of the sulfate has an X-ray powder diffraction pattern as shown in FIG. 29, has a DSC pattern as shown in FIG. 30, or has a TGA pattern as shown in FIG. 31;
the crystal form F of the sulfate has an X-ray powder diffraction pattern as shown in FIG. 32, has a DSC pattern as shown in FIG. 33, or has a TGA pattern as shown in FIG. 34;
the crystal form G of the sulfate has an X-ray powder diffraction pattern as shown in FIG. 35, has a DSC pattern as shown in FIG. 36, or has a TGA pattern as shown in FIG. 37;
the crystal form A of the hydrochloride has an X-ray powder diffraction pattern as shown in FIG. 12, or has a DSC pattern as shown in FIG. 13, or has a TGA pattern as shown in FIG. 14;
the crystal form B of the hydrochloride has an X-ray powder diffraction pattern as shown in FIG. 15;
the crystal form C of the hydrochloride has an X-ray powder diffraction pattern as shown in FIG. 16;
The crystal form A of the p-tosylate has an X-ray powder diffraction pattern as shown in FIG. 38, or has a DSC pattern as shown in FIG. 39, or has a TGA pattern as shown in FIG. 40;
the crystal form B of the p-tosylate has an X-ray powder diffraction pattern as shown in FIG. 41, or has a DSC pattern as shown in FIG. 42, or has a TGA pattern as shown in FIG. 43;
the crystal form C of the p-tosylate has an X-ray powder diffraction pattern as shown in FIG. 44, or has a DSC pattern as shown in FIG. 45, or has a TGA pattern as shown in FIG. 46;
the crystal form D of the p-tosylate has an X-ray powder diffraction pattern as shown in FIG. 47, or has a DSC pattern as shown in FIG. 48, or has a TGA pattern as shown in FIG. 49;
the crystal form E of the p-tosylate has an X-ray powder diffraction pattern as shown in FIG. 50, or has a DSC pattern as shown in FIG. 51, or has a TGA pattern as shown in FIG. 52;
the crystal form A of the besylate has an X-ray powder diffraction pattern as shown in FIG. 53;
the crystal form B of the besylate has an X-ray powder diffraction pattern as shown in FIG. 54, has a DSC pattern as shown in FIG. 55, or has a TGA pattern as shown in FIG. 56;

the crystal form C of the besylate has an X-ray powder diffraction pattern as shown in FIG. 57, has a DSC pattern as shown in FIG. 58, or has a TGA pattern as shown in FIG. 59;
the crystal form A of the isethionate has an X-ray powder diffraction pattern as shown in FIG. 60;
the crystal form B of the isethionate has an X-ray powder diffraction pattern as shown in FIG. 61, or has a DSC pattern as shown in FIG. 62, or has a TGA pattern as shown in FIG. 63; and
the crystal form A of the 1,5-napadisylate has an X-ray powder diffraction pattern as shown in FIG. 64; or has a DSC pattern as shown in FIG. 65; or has a TGA pattern as shown in FIG. 66.

**8.** The crystal form of the acid salt of the compound according to any one of claims 1-7, wherein the number of acids is 0.2-3; preferably 0.2, 0.5, 1, 1.5, 2, 2.5, or 3; more preferably 0.5, 1, 2, or 3; and further more preferably 1.

**9.** The crystal form of the acid salt of the compound according to any one of claims 1-8, wherein the crystal form of the acid salt is a hydrate or an anhydrate; when the crystal form of the acid salt is a hydrate, the number of water is 0.2-3, preferably 0.2, 0.5, 1, 1.5, 2, 2.5, or 3, more preferably 0.5, 1, 2, or 3; and further, water in the hydrate is pipe water or crystal water or a combination thereof.

**10.** A method for preparing the crystal form of the acid salt of the compound according to any one of claims 1 to 9, specifically comprising steps of:

1) weighing an appropriate amount of a free base and dissolving the free base in a good solvent;
2) weighing an appropriate amount of an acid, and optionally dissolving the acid in an organic solvent; preferably the amount of the acid is 1.0-1.5 equivalents;
3) combining the above two solutions and stirring for precipitation, or adding a poor solvent dropwise and then stirring for precipitation; and
4) rapidly centrifuging the resulting system or allowing the system to stand still, and drying the system to obtain a target product;

wherein:

the good solvent is selected from acetone, toluene, acetonitrile, methanol, isopropanol, dichloromethane, tetrahydrofuran, ethyl formate, isopropyl acetate, toluene, ethyl acetate, 2-methyltetrahydrofuran, 2-butanone, n-butanol, 1,4-dioxane, isobutanol, N,N-dimethylformamide, N,N-dimethylacetamide, n-propanol, or tert-butanol; preferably toluene, ethyl acetate, acetone, methanol, or acetonitrile;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyltetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and the organic solution need, when used, to be mutually soluble; and
the poor solvent is selected from heptane, water, methyl tert-butyl ether, cyclohexane, toluene, isopropyl ether, ethyl acetate, acetone, or acetonitrile; preferably water, methyl tert-butyl ether, or isopropyl ether;
or specifically comprising steps of:

1) weighing an appropriate amount of a free base and suspending the free base in a poor solvent;
2) weighing an appropriate amount of an acid, and dissolving the acid in an organic solvent; preferably the amount of the acid is 1.0-1.5 equivalents;
3) adding the solution from the above step 2) to the suspension from the above step 1) while stirring; and
4) rapidly centrifuging the resulting system or allowing the system to stand still, and drying the system to obtain a target product;

wherein:

the poor solvent is selected from ethanol, acetone, ethyl acetate, ethyl formate, isopropanol, isopropyl acetate, methyl tert-butyl ether, methanol, 1,4-dioxane, 2-butanone, 2-methyltetrahydrofuran, anisole, acetonitrile, chlorobenzene, benzene, toluene, n-butanol, isobutanol, or 3-pentanone; preferably ethanol, 2-methyltetrahydrofuran, acetonitrile, methanol, or ethyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyltetrahydrofuran, 2-butanone, 3-pentanone, heptane, methyl tert-butyl ether, isopropyl

ether, 1,4-dioxane, tert-butanol, or N,N-dimethylformamide; preferably methanol, ethanol, or acetonitrile; the above good solvent and the organic solution need, when used, to be mutually soluble; and
the acid is selected from ethylsulfonic acid, methylsulfonic acid, sulfuric acid, hydrochloric acid, p-toluenesulfonic acid, benzenesulfonic acid, isethionic acid, or 1,5-naphthalenedisulfonic acid;
or specifically comprising steps of:

1) weighing an appropriate amount of a salt of a compound and suspending the salt in a poor solvent; and
2) rapidly centrifuging the suspension from the step 1), removing the supernatant, and drying residual solid to a constant weight to obtain a target product;

wherein:
the poor solvent is selected from methanol, ethanol, dichloromethane, 1,4-dioxane, acetonitrile, dichloromethane, chlorobenzene, chloroform, benzene, toluene, acetone, ethyl acetate, water, 88% acetone, isopropyl acetate, 3-pentanone, ethyl formate, tetrahydrofuran, 2-methyltetrahydrofuran, isopropanol, n-butanol, isobutanol, n-propanol, methyl tert-butyl ether, n-heptane, tert-butanol, or 2-butanone.

**11.** A pharmaceutical composition, comprising a therapeutically effective amount of the crystal form of the acid salt of the compound according to any one of claims 1-9, and one or more pharmaceutically acceptable carriers or excipients.

**12.** Use of the crystal form of the acid salt of the compound according to any one of claims 1-9, and the pharmaceutical composition according to claim 11 in manufacturing a tyrosine kinase active drug that inhibits a protein selected from an Abelson protein, an Abelson-related protein, and a chimeric protein BCR-ABL1.

**13.** Use of the crystal form of the acid salt of the compound according to any one of claims 1-9, and the pharmaceutical composition according to claim 11 in manufacturing a drug for the treatment of a leukemia-related disease, wherein preferably, the leukemia is chronic myeloid leukemia, acute myeloid leukemia, or acute lymphocytic leukemia; more preferably, the chronic myeloid leukemia is resistant to the treatment with one or more of standard treatments of care such as imatinib, nilotinib, and dasatinib, and the acute myeloid leukemia is secondary acute lymphocytic leukemia that develops after myelodysplastic syndrome or myeloproliferative neoplasm.

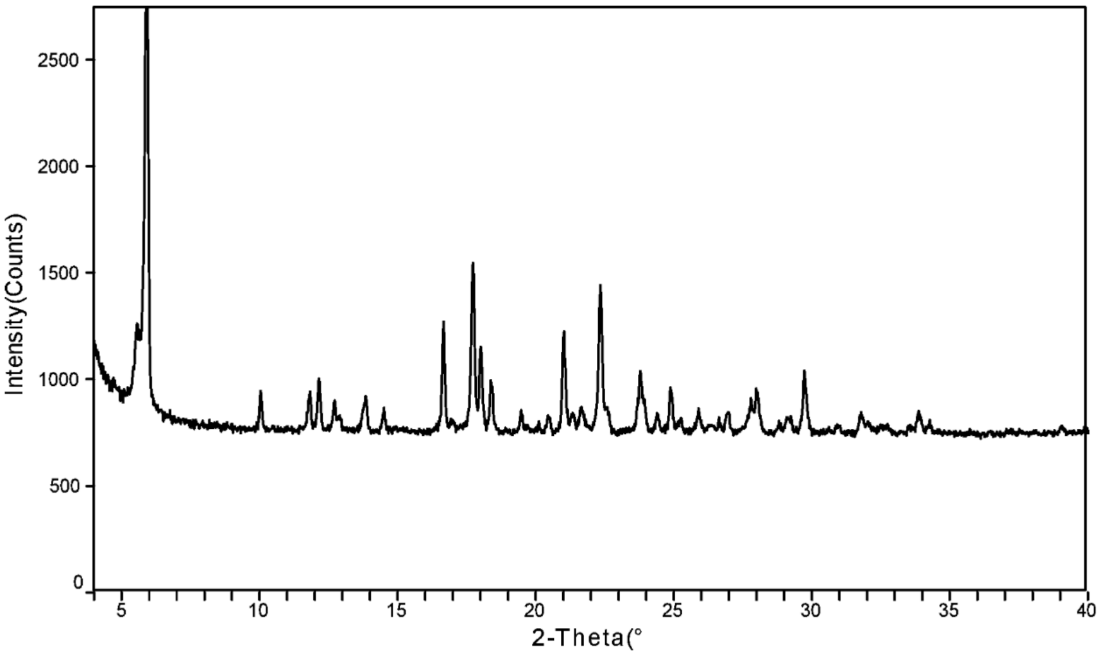
Intensity(Counts)
2500
2000
1500
1000
500
0
5
10
15
20
25
30
35
40
2-Theta(°

FIG. 1

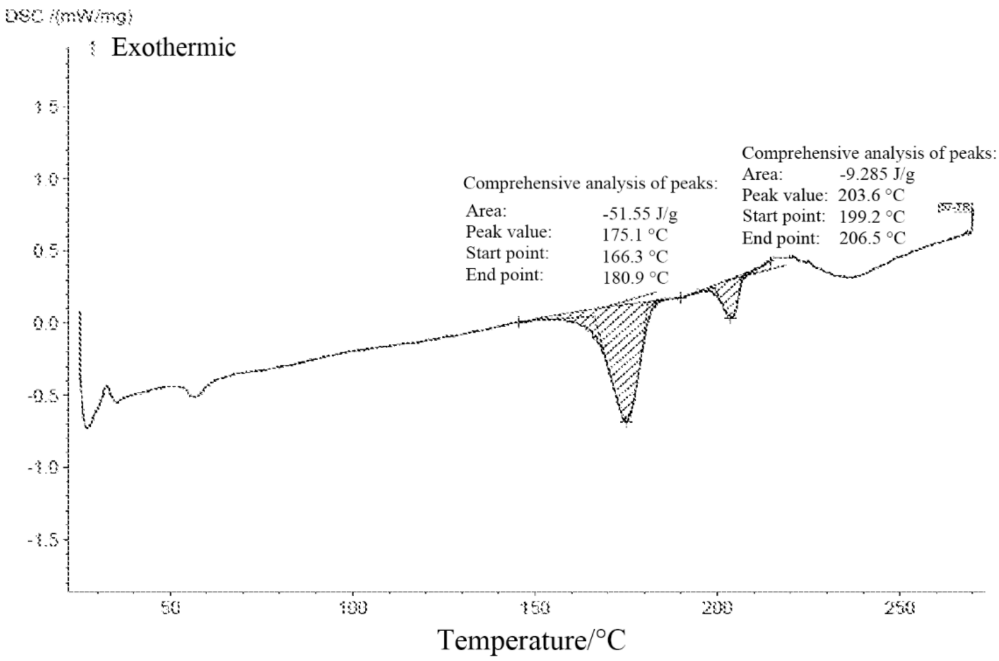
DSC /(mW/mg)
↑ Exothermic
1.5
1.0
0.5
0.0
-0.5
-1.0
-1.5
Comprehensive analysis of peaks:
Area: -51.55 J/g
Peak value: 175.1 °C
Start point: 166.3 °C
End point: 180.9 °C
Comprehensive analysis of peaks:
Area: -9.285 J/g
Peak value: 203.6 °C
Start point: 199.2 °C
End point: 206.5 °C
50
100
150
200
250
Temperature/°C

FIG. 2

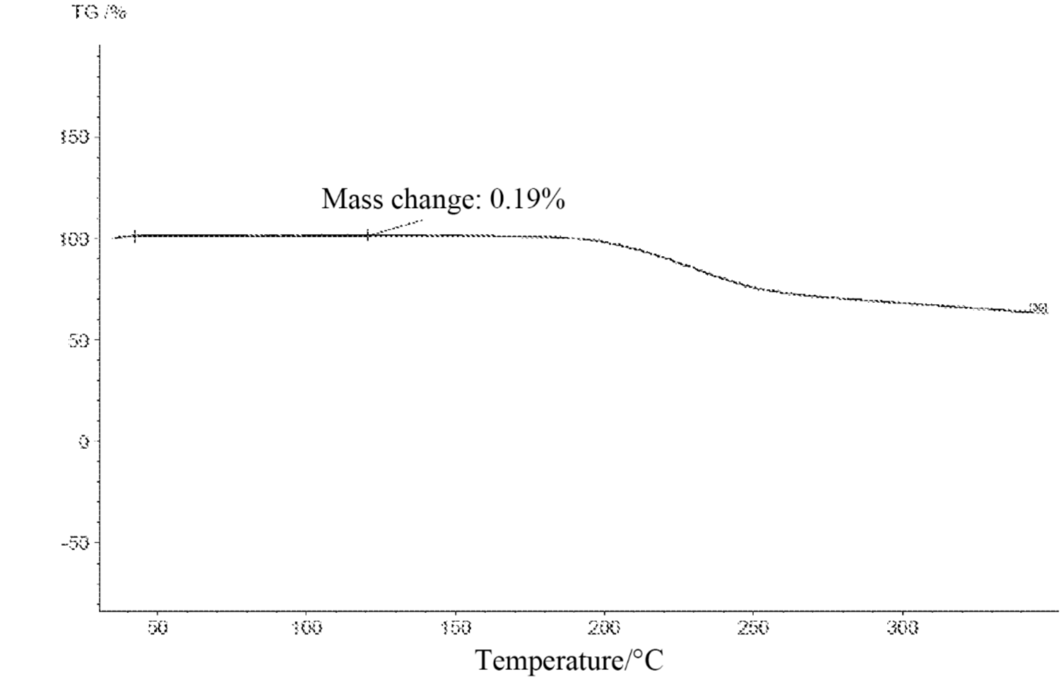
TG /%
150
100
50
0
-50
Mass change: 0.19%
50
100
150
200
250
300
Temperature/°C

FIG. 3

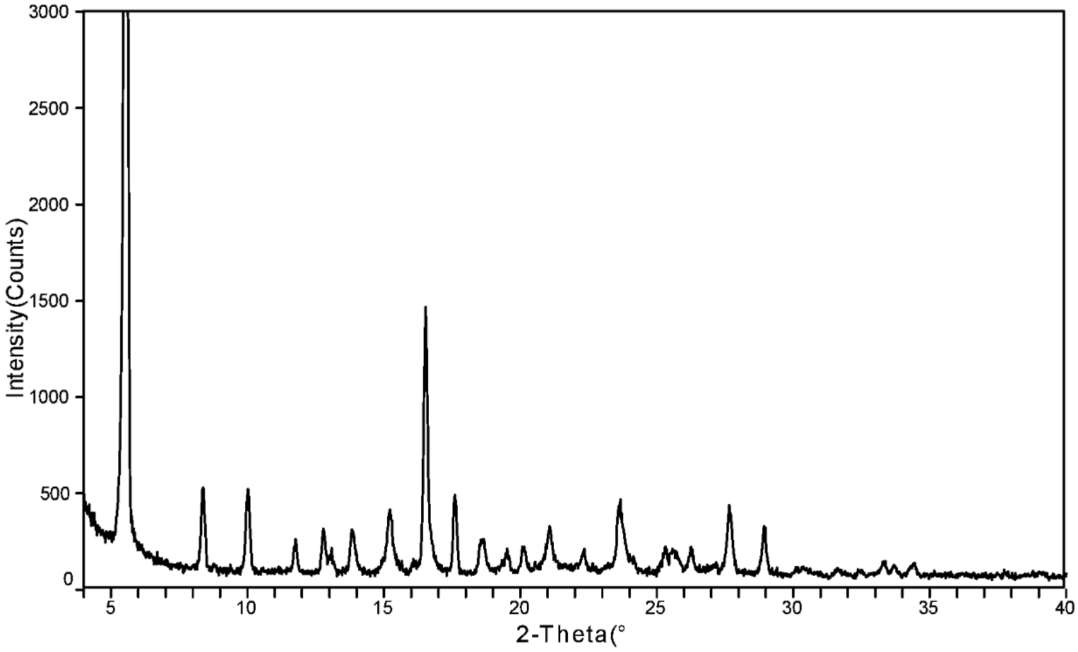
3000
2500
2000
1500
1000
500
0
Intensity(Counts)
5
10
15
20
25
30
35
40
2-Theta(°

FIG. 4

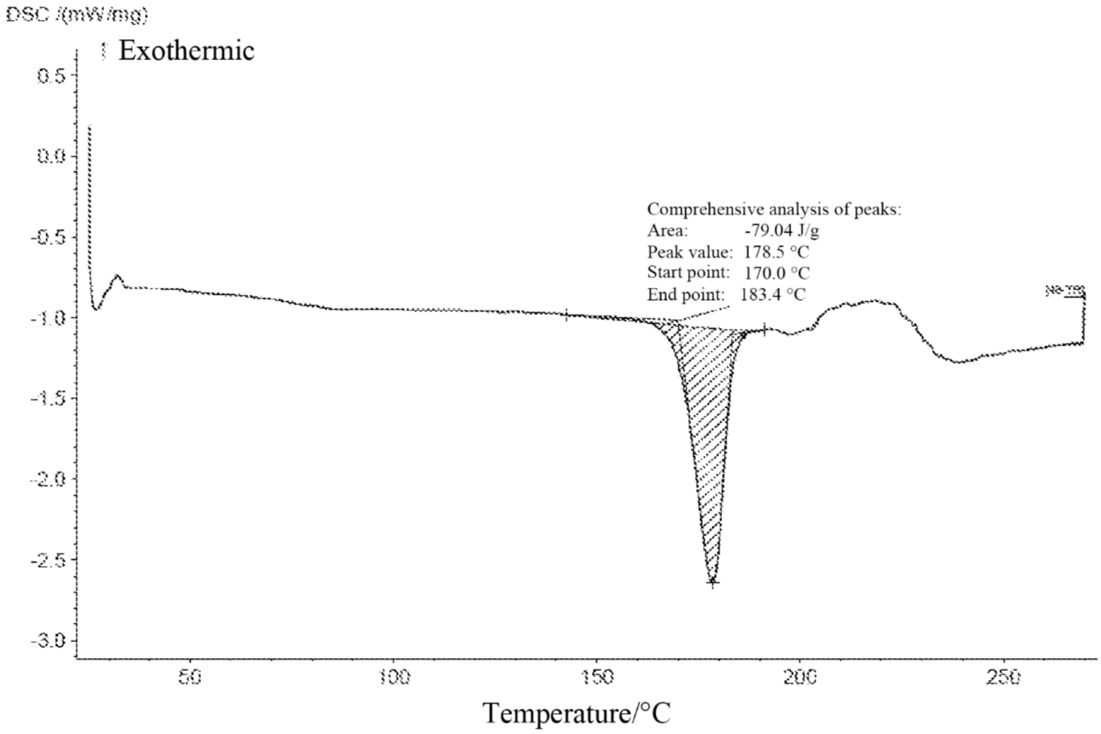
DSC /(mW/mg)
Exothermic
0.5
0.0
-0.5
-1.0
-1.5
-2.0
-2.5
-3.0
Comprehensive analysis of peaks:
Area: -79.04 J/g
Peak value: 178.5 °C
Start point: 170.0 °C
End point: 183.4 °C
50
100
150
200
250
Temperature/°C

FIG. 5

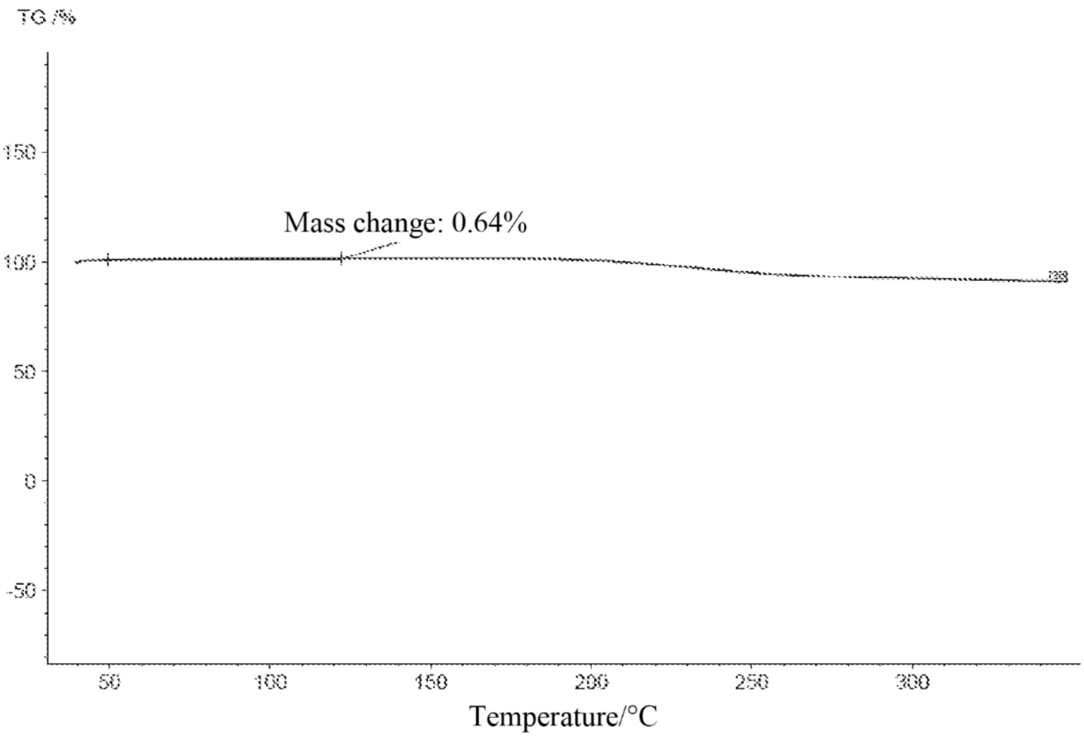
TG /%
150
100
50
0
-50
Mass change: 0.64%
50
100
150
200
250
300
Temperature/°C

FIG. 6

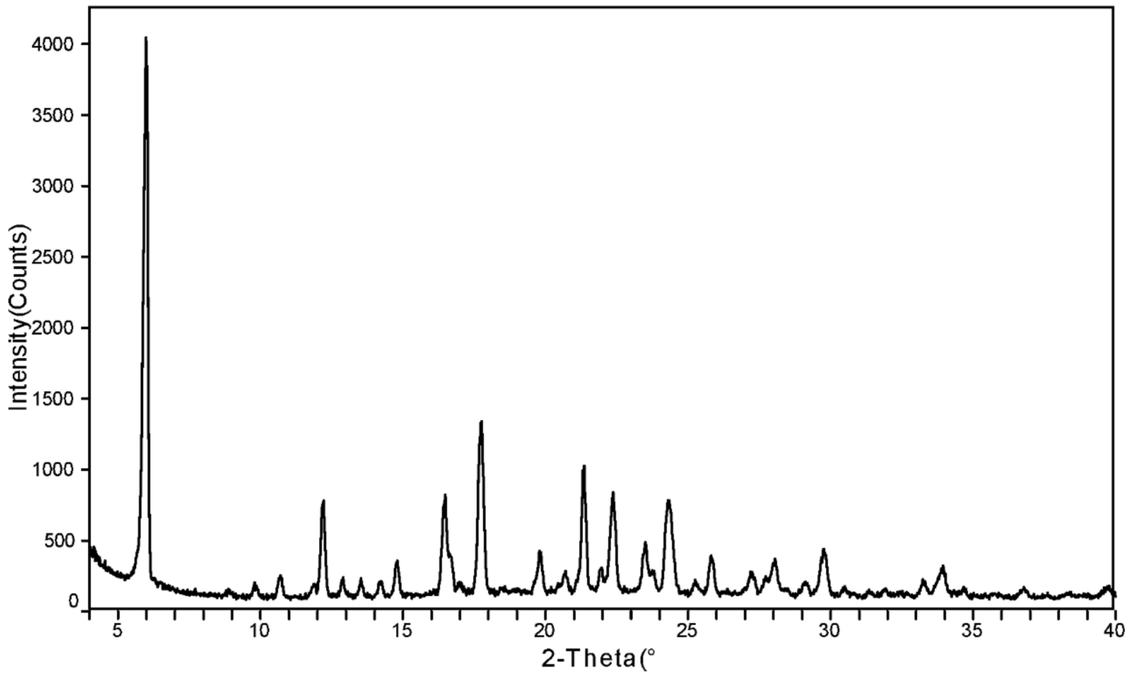
Intensity(Counts)
4000
3500
3000
2500
2000
1500
1000
500
0
5
10
15
20
25
30
35
40
2-Theta(°

FIG. 7

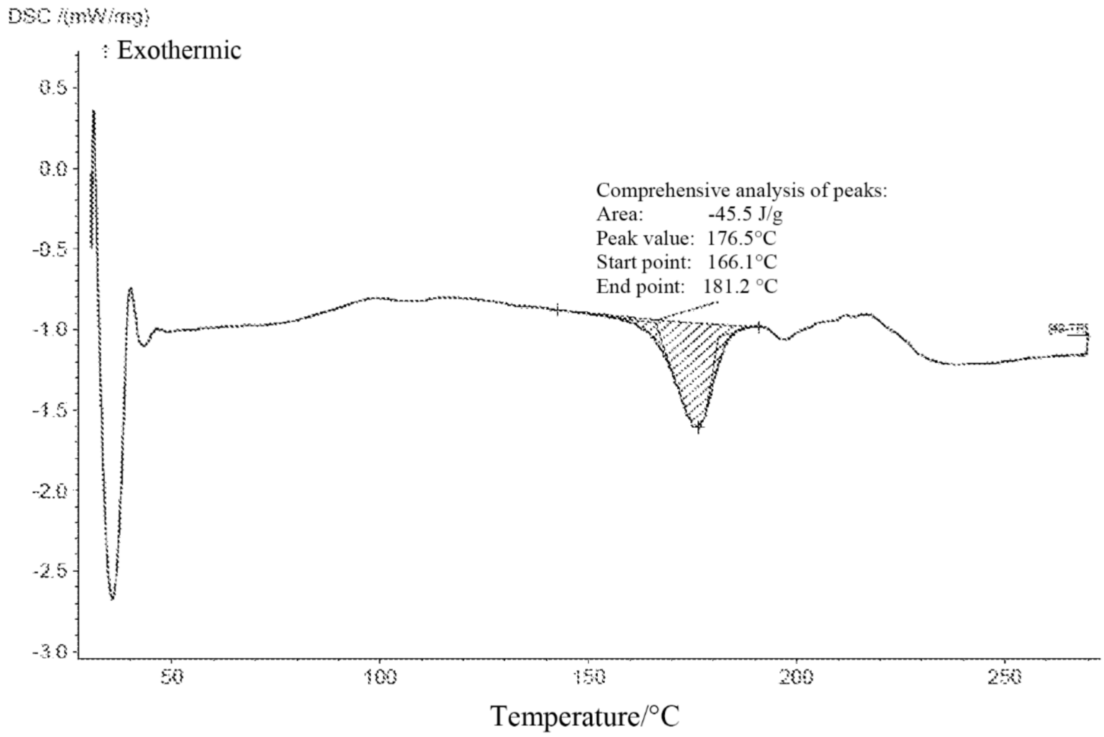
DSC /(mW/mg)
↑ Exothermic
0.5
0.0
-0.5
-1.0
-1.5
-2.0
-2.5
-3.0
50
100
150
200
250
Comprehensive analysis of peaks:
Area: -45.5 J/g
Peak value: 176.5°C
Start point: 166.1°C
End point: 181.2 °C
Temperature/°C

FIG. 8

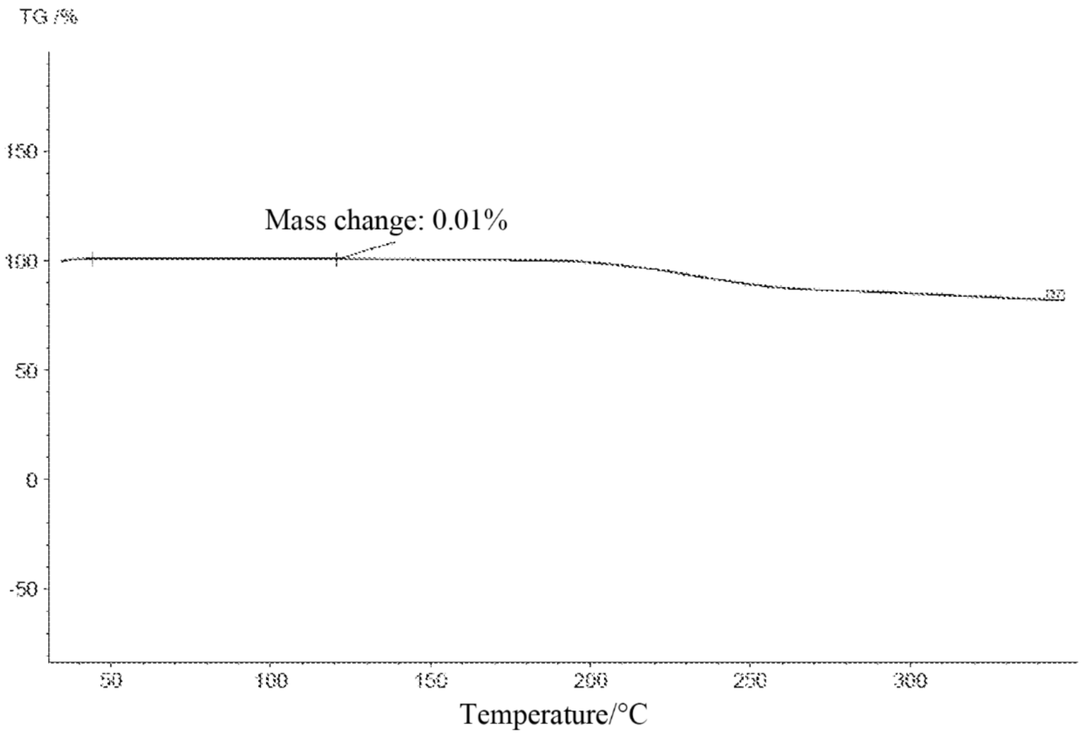
TG /%
150
100
50
0
-50
Mass change: 0.01%
50
100
150
200
250
300
Temperature/°C

FIG. 9

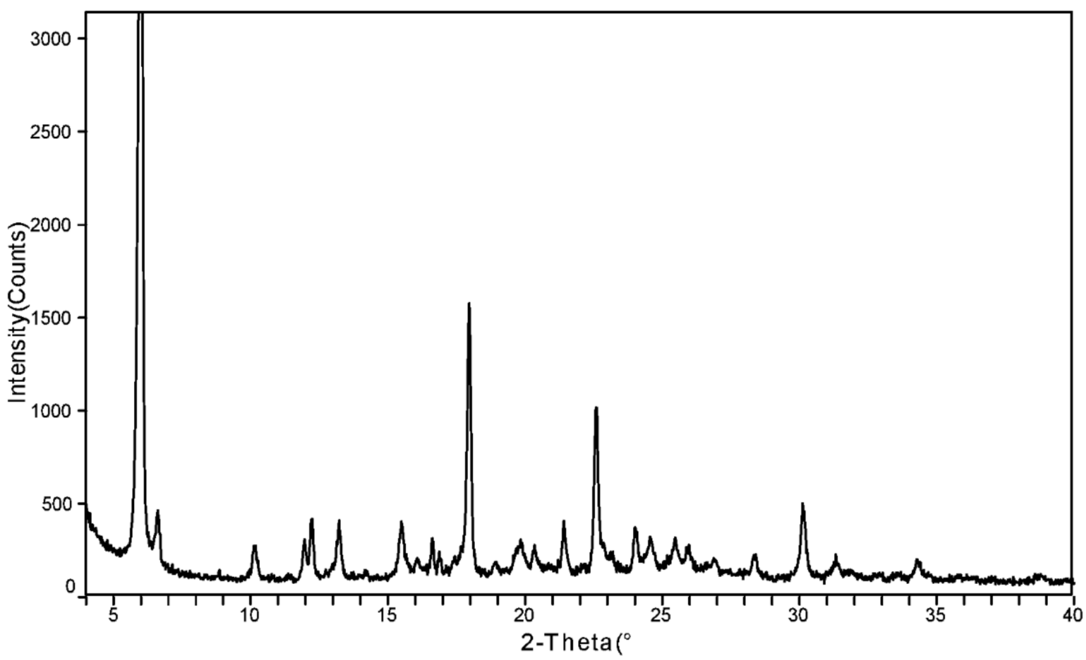
3000
2500
2000
1500
1000
500
0
Intensity(Counts)
5
10
15
20
25
30
35
40
2-Theta(°

FIG. 10

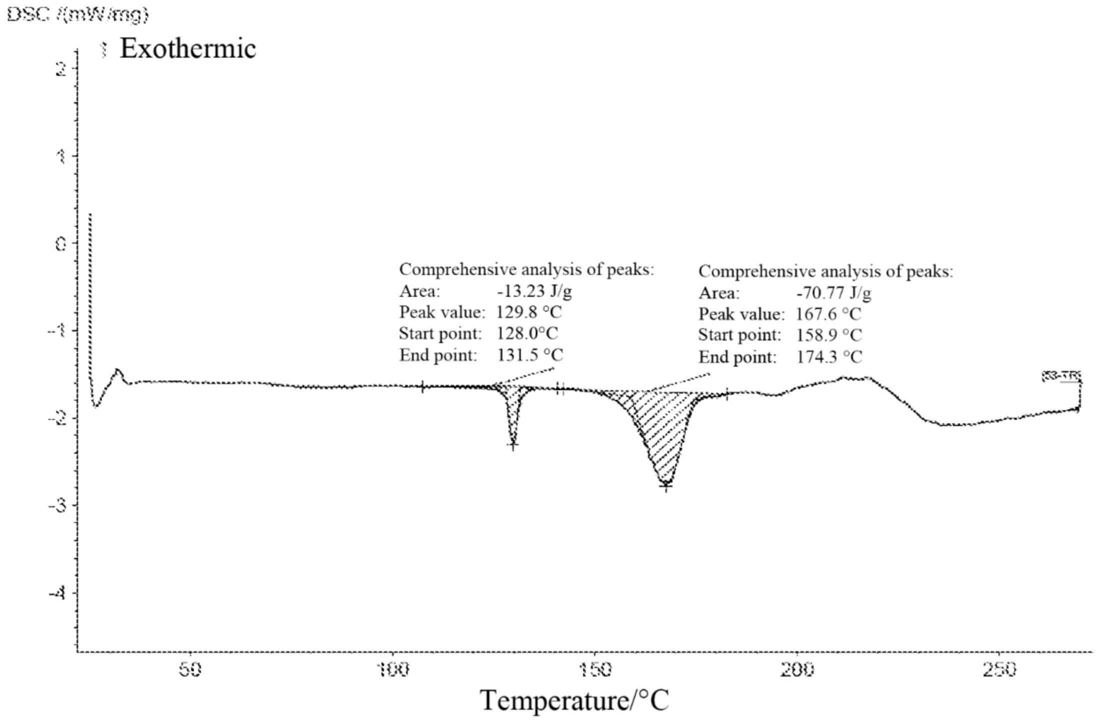
DSC /(mW/mg)
↑ Exothermic
Comprehensive analysis of peaks:
Area: -13.23 J/g
Peak value: 129.8 °C
Start point: 128.0°C
End point: 131.5 °C
Comprehensive analysis of peaks:
Area: -70.77 J/g
Peak value: 167.6 °C
Start point: 158.9 °C
End point: 174.3 °C
Temperature/°C

FIG. 11

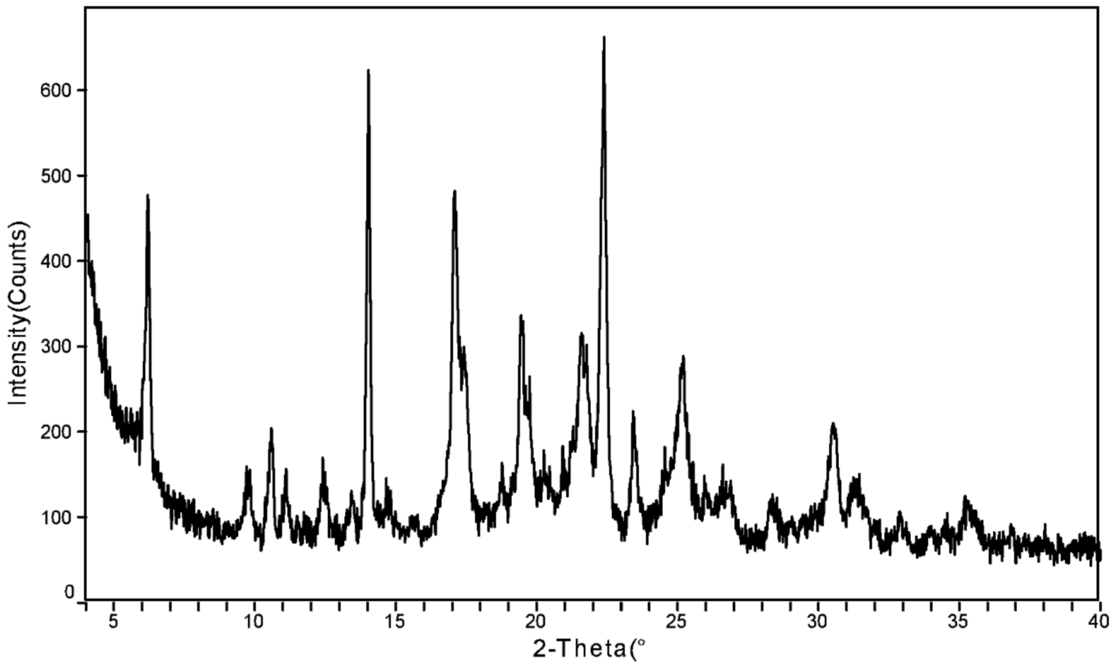
Intensity(Counts)
2-Theta(°

FIG. 12

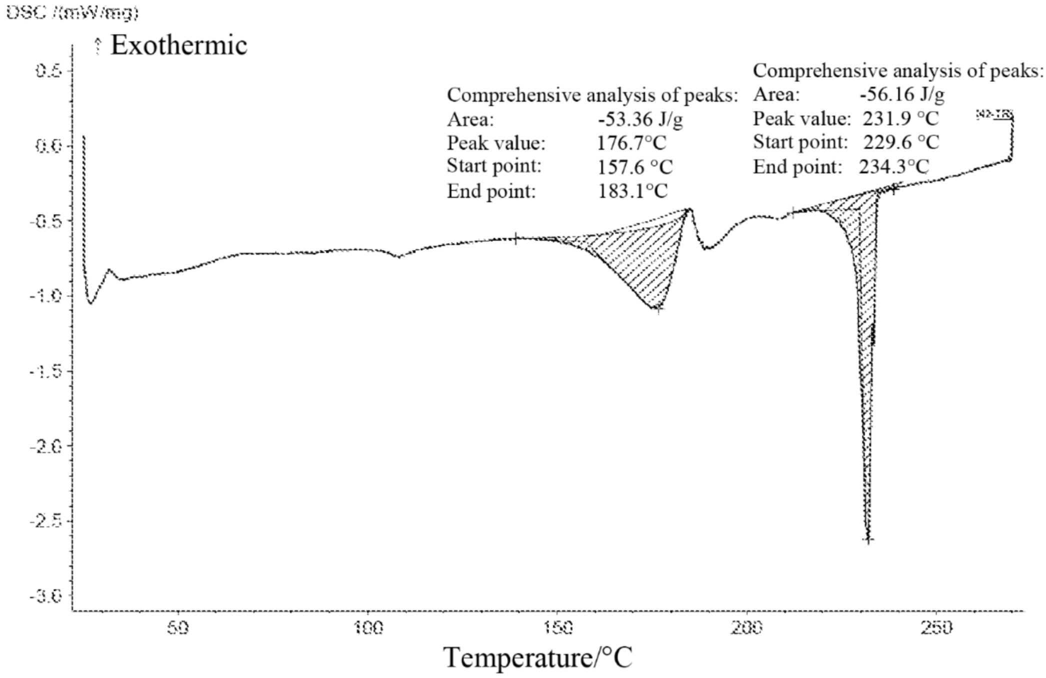
DSC /(mW/mg)
↑ Exothermic
Comprehensive analysis of peaks:
Area: -53.36 J/g
Peak value: 176.7°C
Start point: 157.6 °C
End point: 183.1°C
Comprehensive analysis of peaks:
Area: -56.16 J/g
Peak value: 231.9 °C
Start point: 229.6 °C
End point: 234.3°C
0.5
0.0
-0.5
-1.0
-1.5
-2.0
-2.5
-3.0
50
100
150
200
250
Temperature/°C

FIG. 13

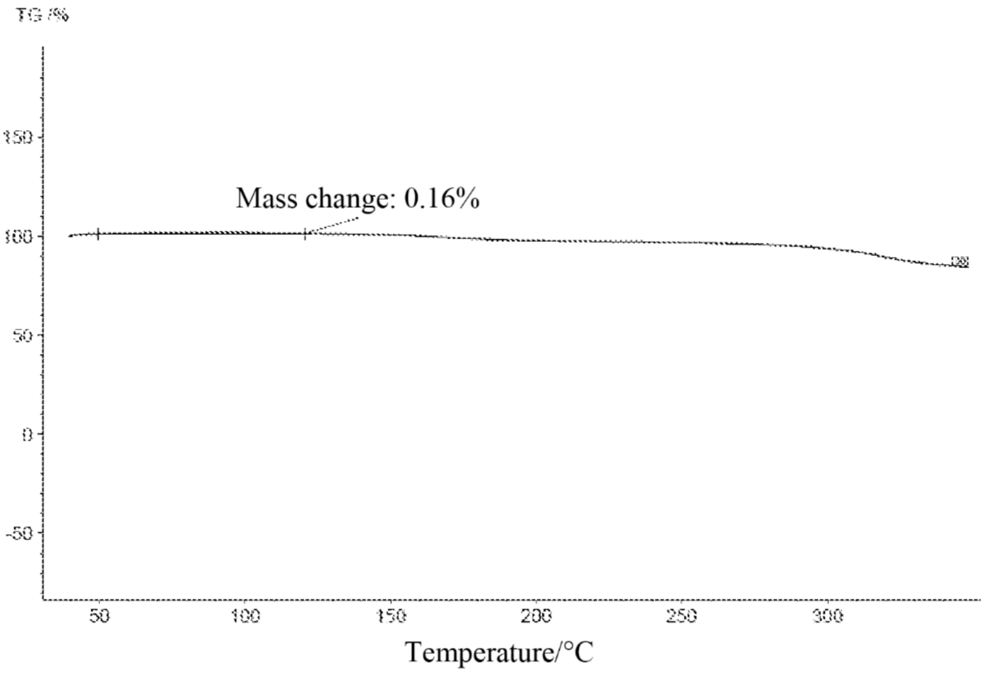
TG /%
Mass change: 0.16%
150
100
50
0
-50
50
100
150
200
250
300
Temperature/°C

FIG. 14

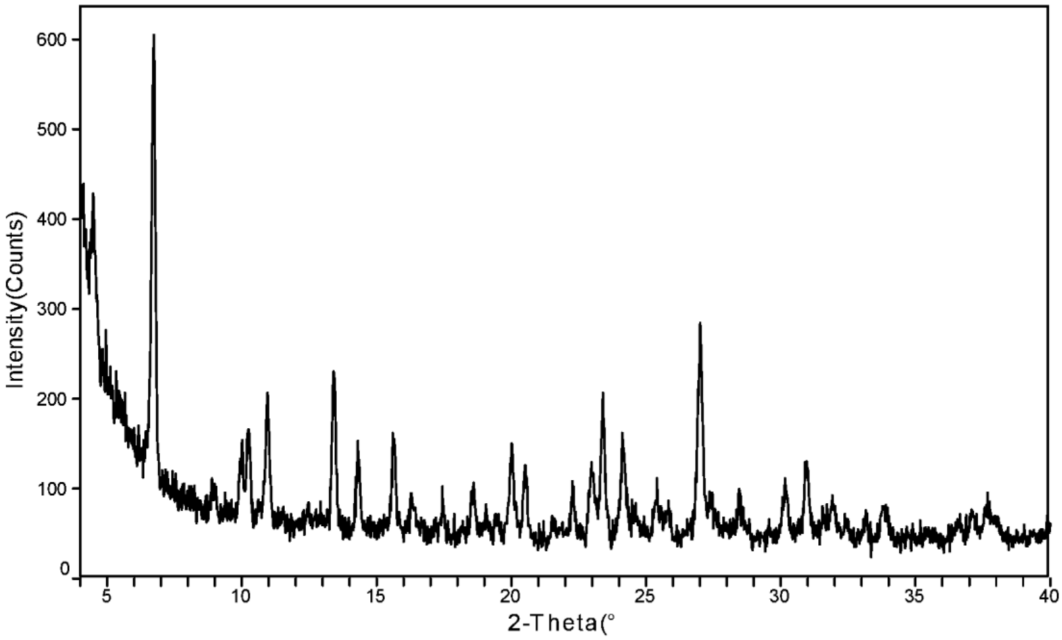
Intensity(Counts)
2-Theta(°
0
100
200
300
400
500
600
5
10
15
20
25
30
35
40

FIG. 15

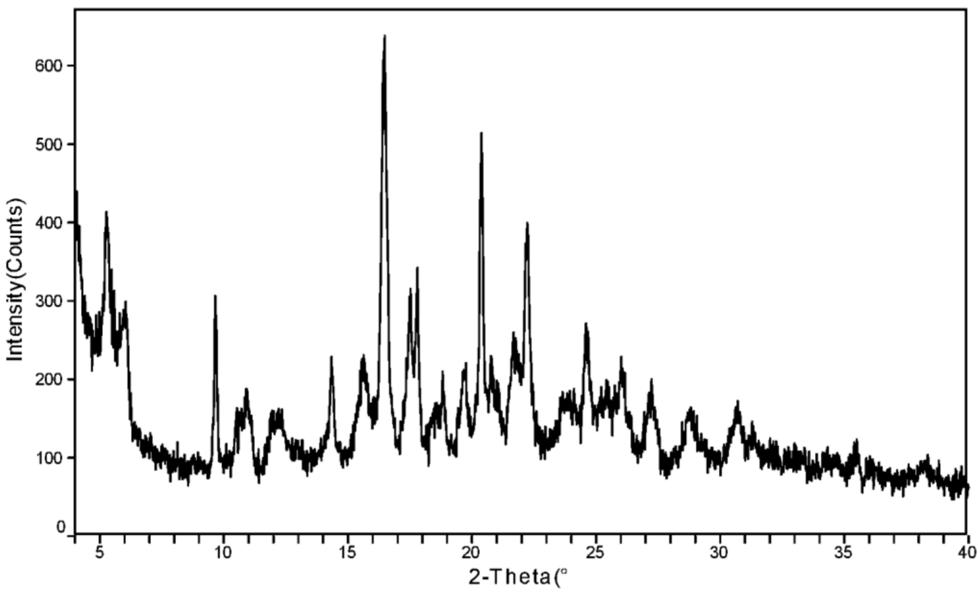
Intensity(Counts)
2-Theta(°
0
100
200
300
400
500
600
5
10
15
20
25
30
35
40

FIG. 16

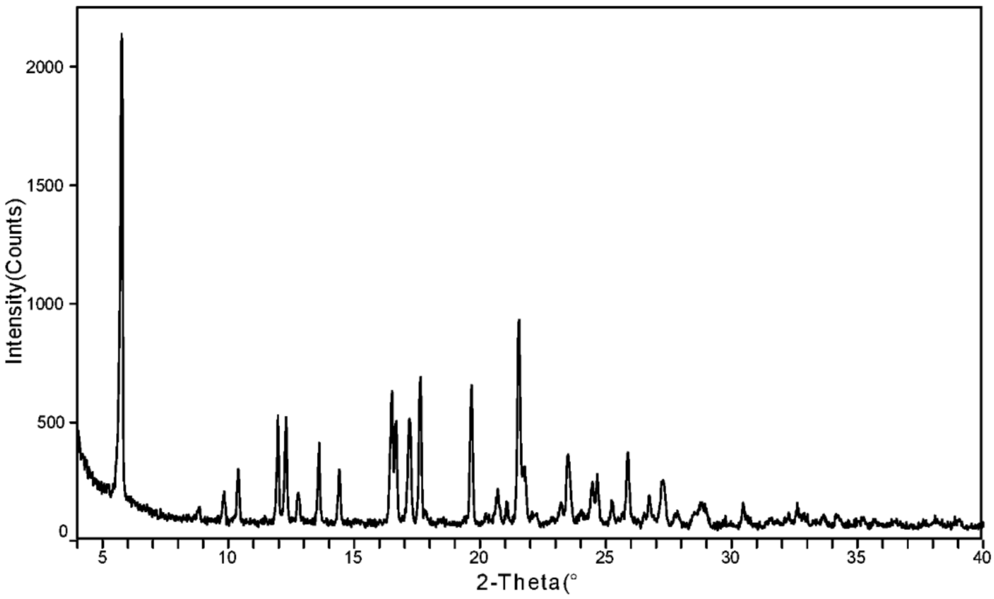
Intensity(Counts)
2-Theta(°

FIG. 17

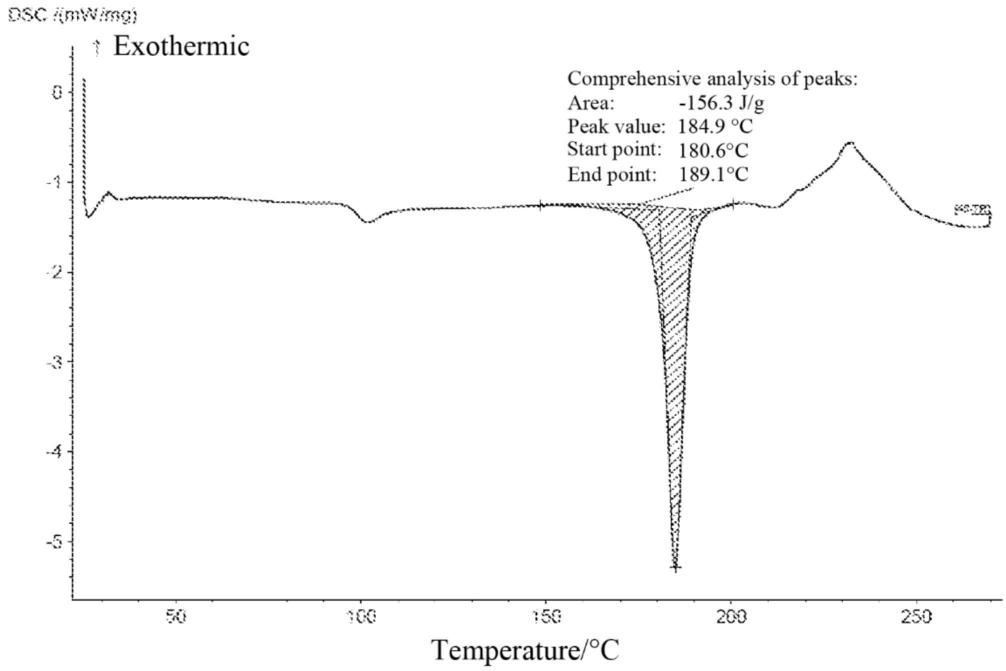
DSC /(mW/mg)
↑ Exothermic
Comprehensive analysis of peaks:
Area: -156.3 J/g
Peak value: 184.9 °C
Start point: 180.6°C
End point: 189.1°C
Temperature/°C

FIG. 18

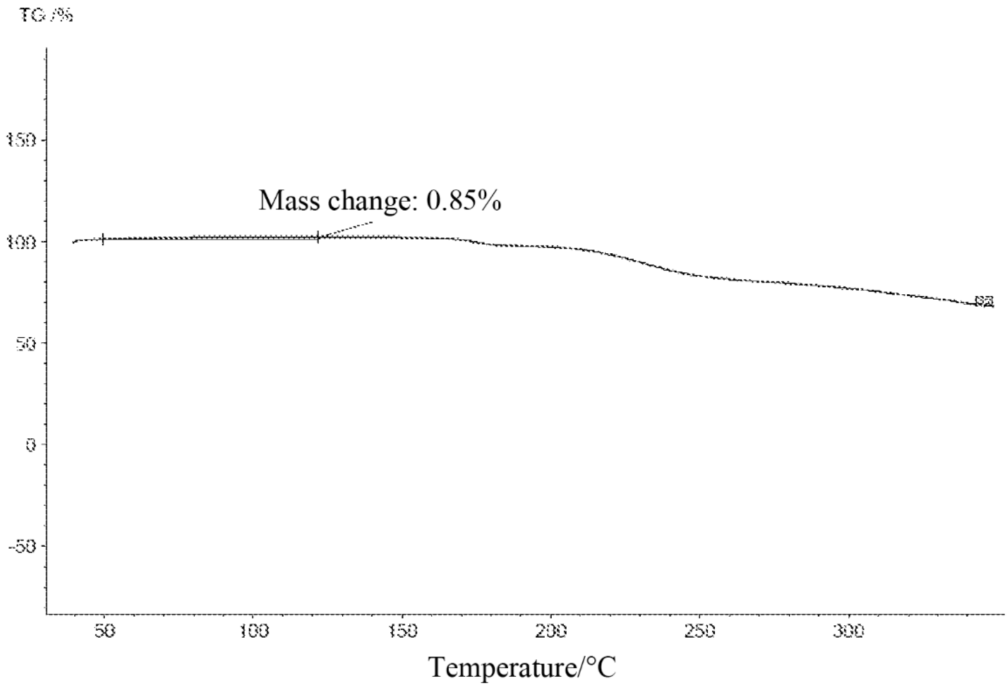
TG /%
150
100
50
0
-50
Mass change: 0.85%
50
100
150
200
250
300
Temperature/°C

FIG. 19

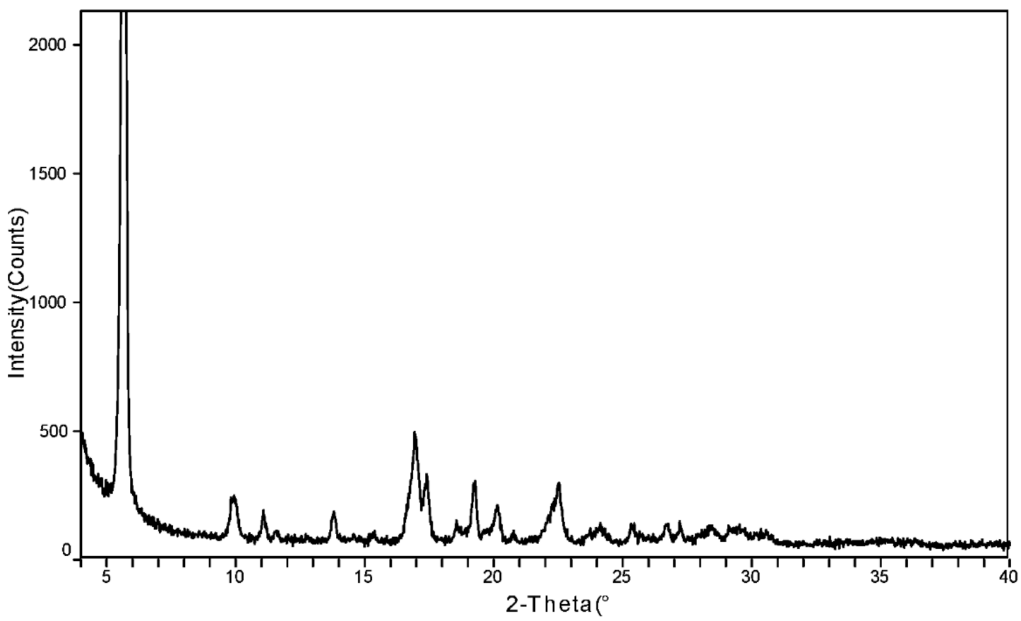
Intensity(Counts)
2000
1500
1000
500
0
5
10
15
20
25
30
35
40
2-Theta(°

FIG. 20

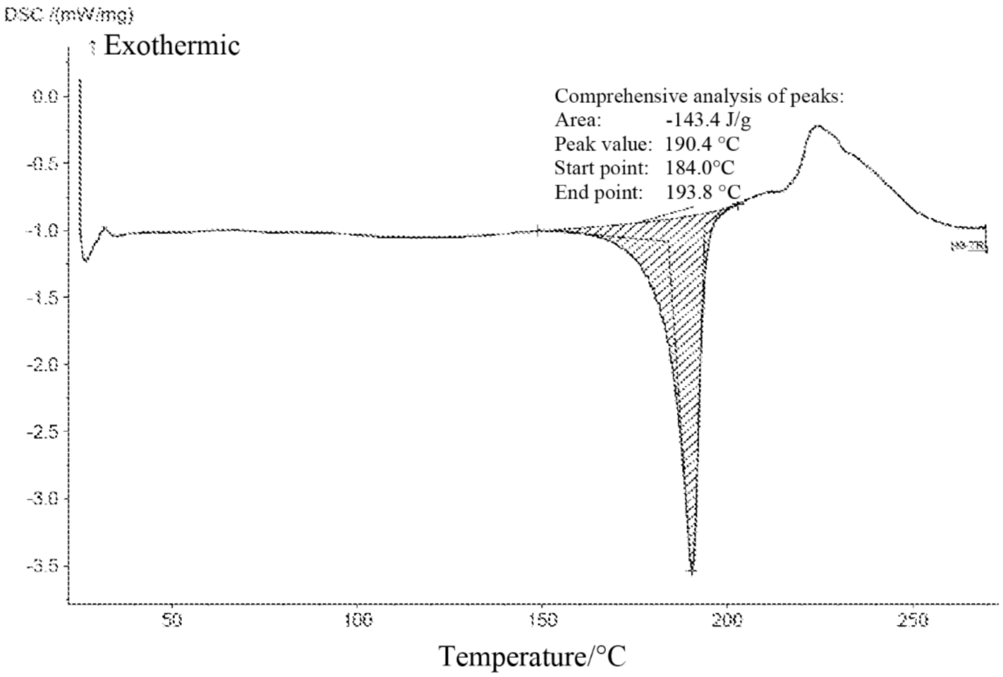
DSC /(mW/mg)
↑ Exothermic
Comprehensive analysis of peaks:
Area: -143.4 J/g
Peak value: 190.4 °C
Start point: 184.0°C
End point: 193.8 °C
0.0
-0.5
-1.0
-1.5
-2.0
-2.5
-3.0
-3.5
50
100
150
200
250
Temperature/°C

FIG. 21

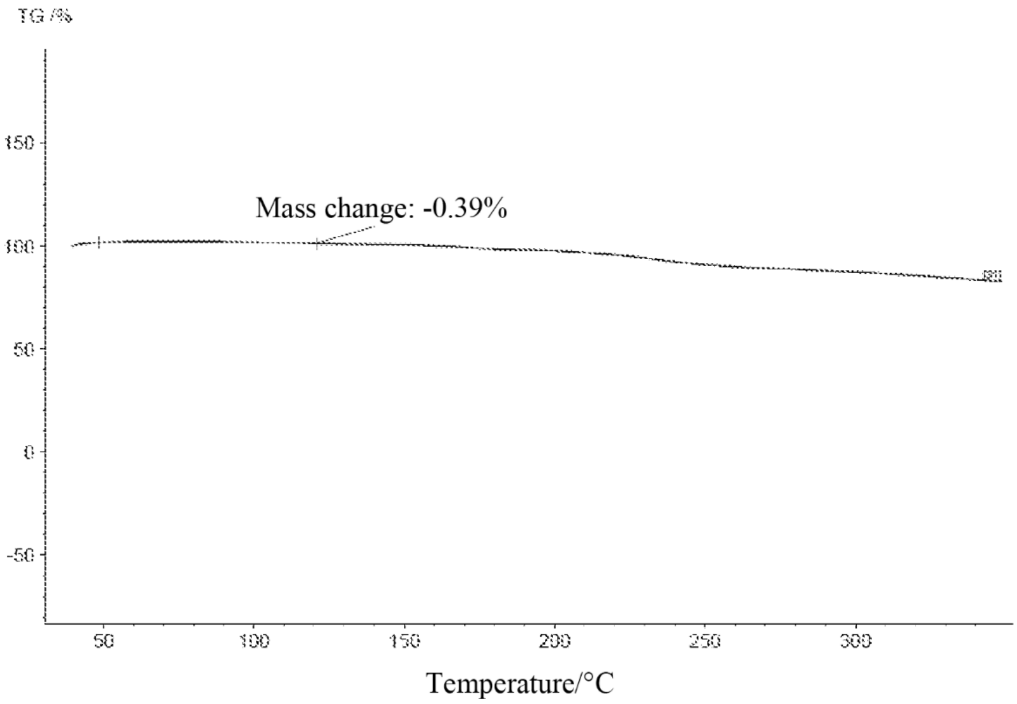
TG /%
150
100
50
0
-50
Mass change: -0.39%
50
100
150
200
250
300
Temperature/°C

FIG. 22

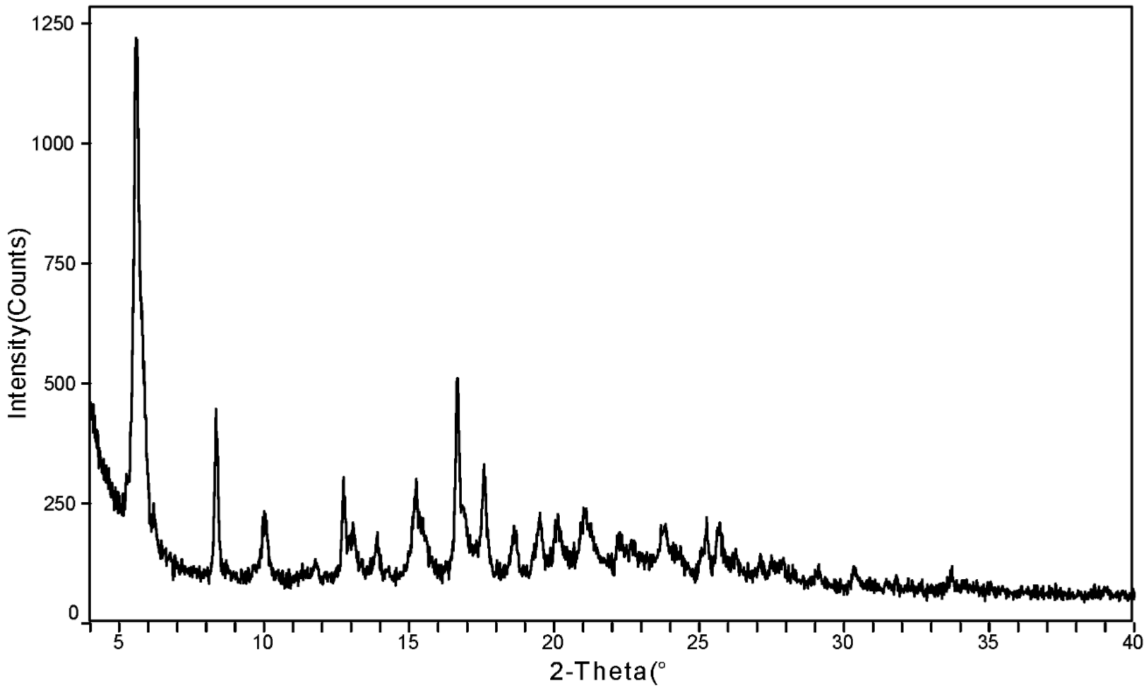
1250
1000
750
500
250
0
Intensity(Counts)
5
10
15
20
25
30
35
40
2-Theta(°

FIG. 23

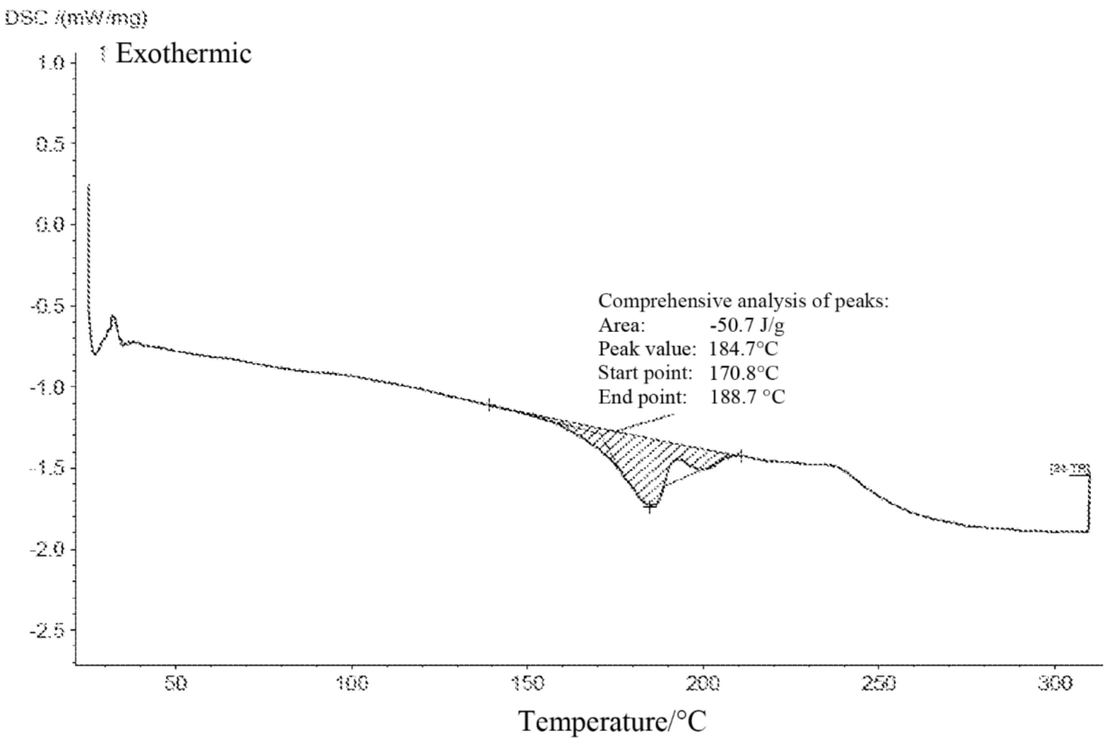
DSC /(mW/mg)
↑ Exothermic
1.0
0.5
0.0
-0.5
-1.0
-1.5
-2.0
-2.5
Comprehensive analysis of peaks:
Area: -50.7 J/g
Peak value: 184.7°C
Start point: 170.8°C
End point: 188.7 °C
50
100
150
200
250
300
Temperature/°C

FIG. 24

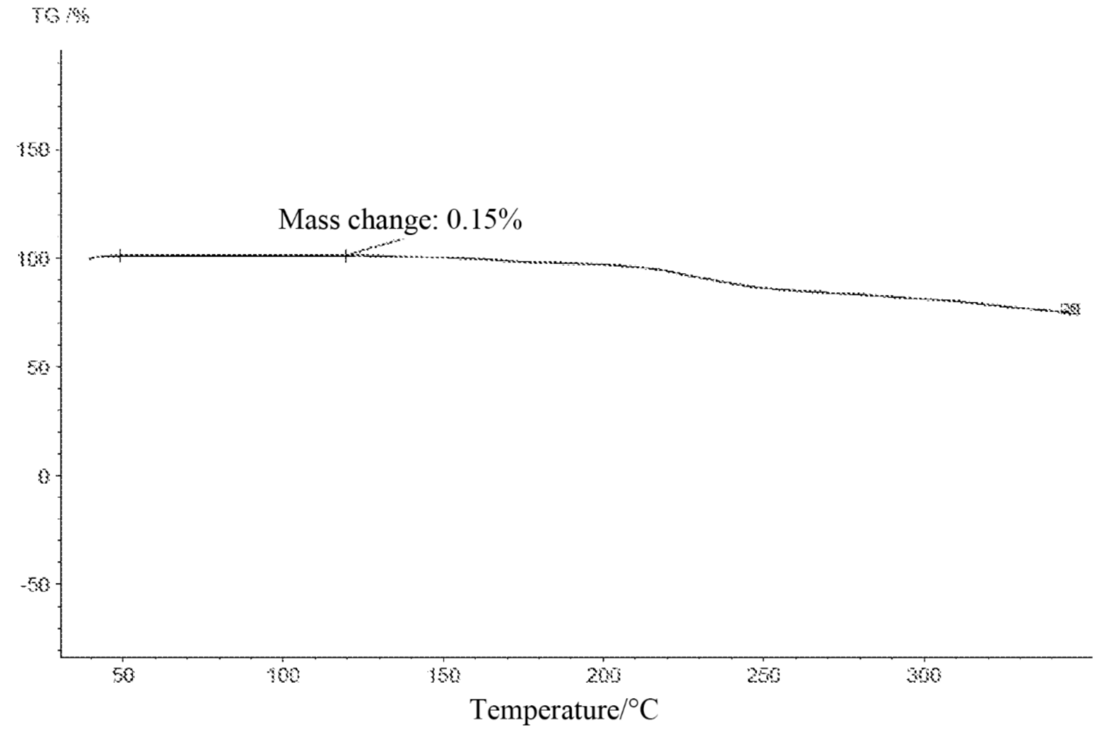
TG /%
Mass change: 0.15%
150
100
50
0
-50
50
100
150
200
250
300
Temperature/°C

FIG. 25

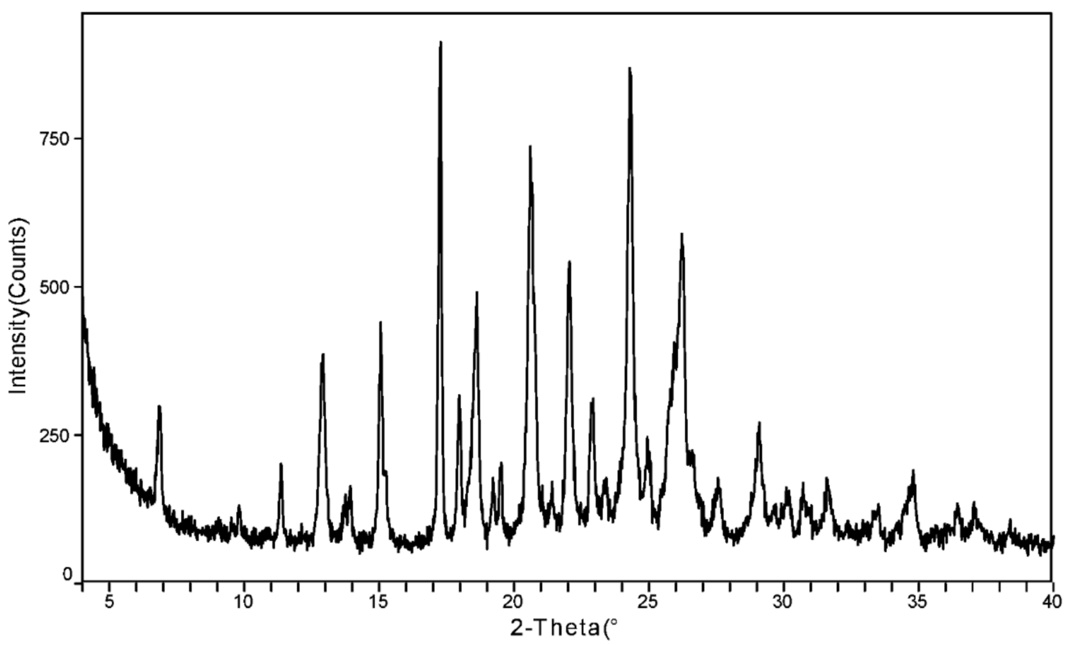
Intensity(Counts)
750
500
250
0
5
10
15
20
25
30
35
40
2-Theta(°

FIG. 26

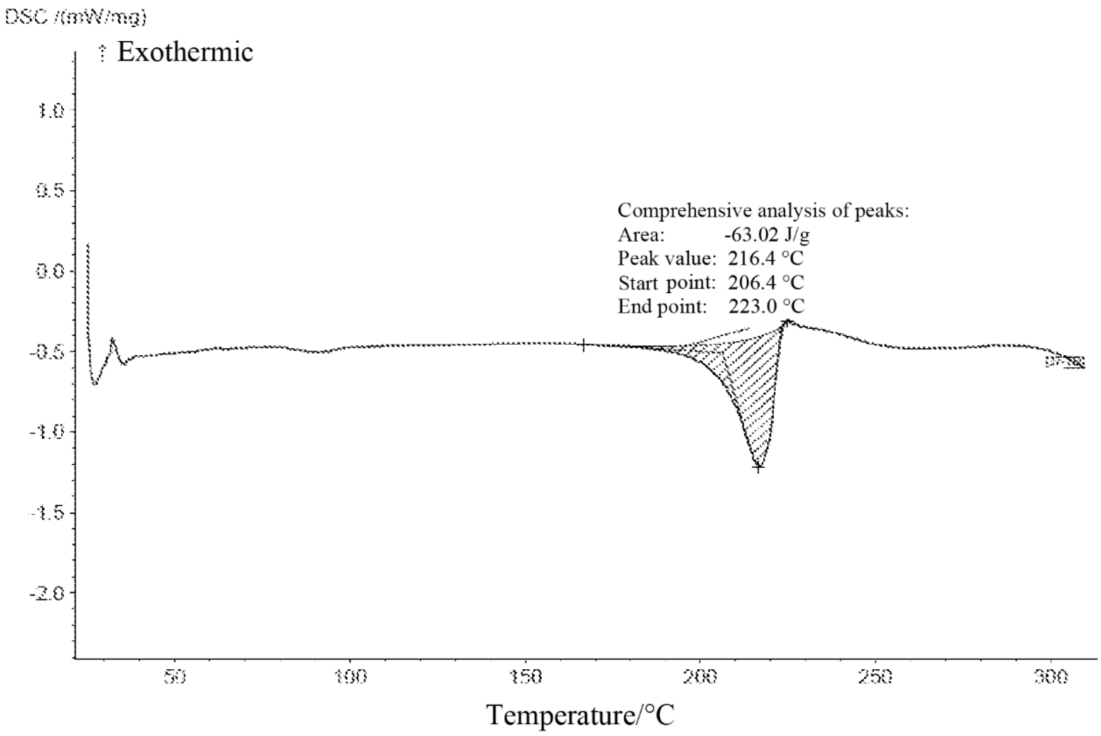
DSC /(mW/mg)
↑ Exothermic
Comprehensive analysis of peaks:
Area: -63.02 J/g
Peak value: 216.4 °C
Start point: 206.4 °C
End point: 223.0 °C
Temperature/°C

FIG. 27

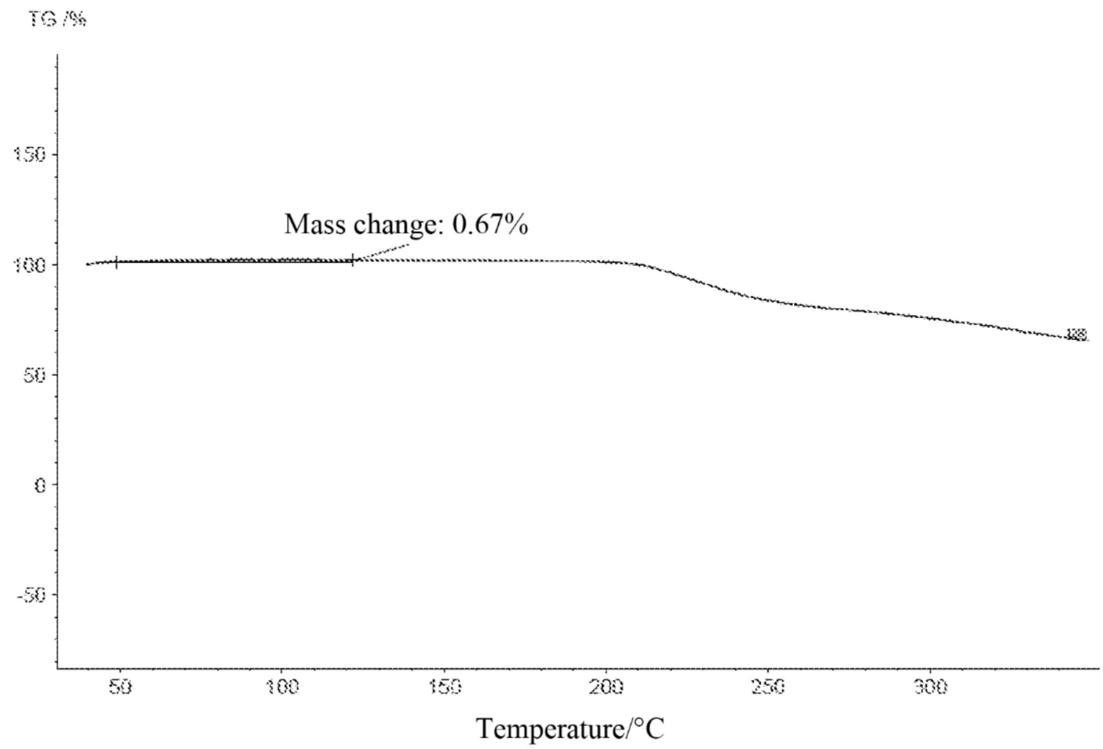
TG /%
Mass change: 0.67%
Temperature/°C

FIG. 28

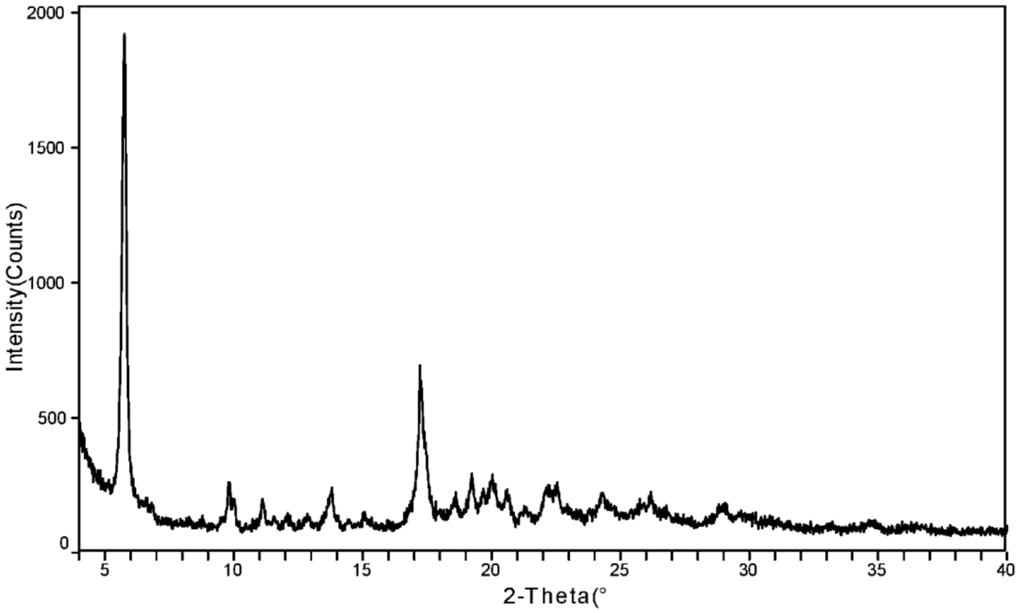
2000
1500
1000
500
0
Intensity(Counts)
5
10
15
20
25
30
35
40
2-Theta(°

FIG. 29

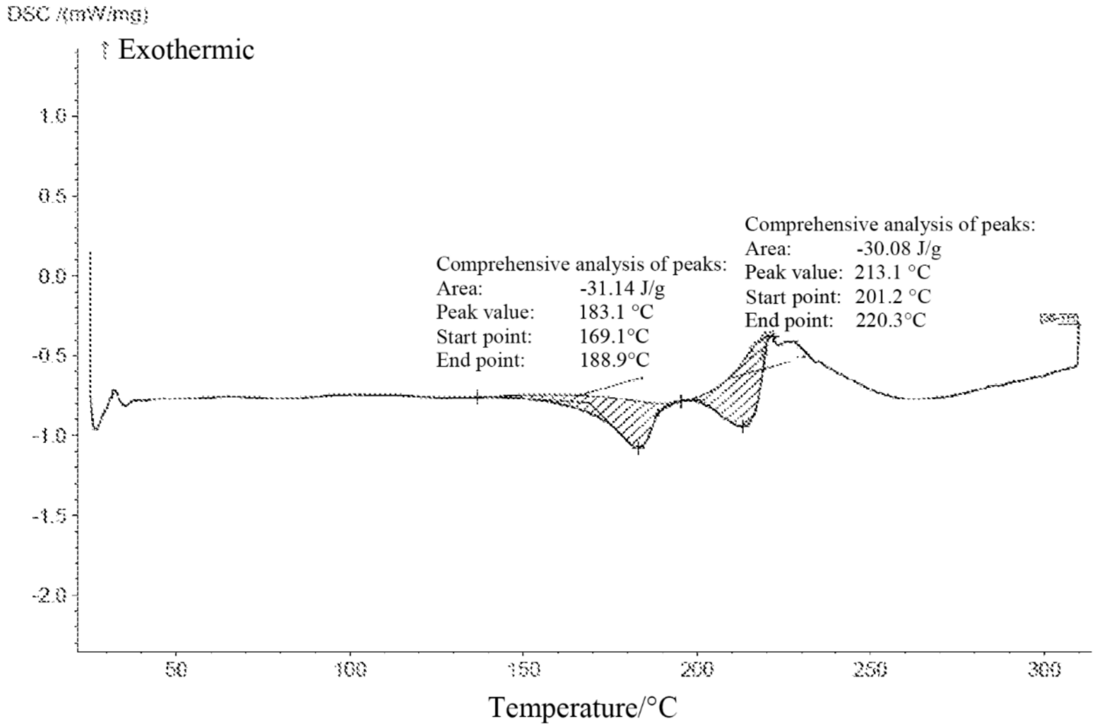
DSC /(mW/mg)
↑ Exothermic
1.0
0.5
0.0
-0.5
-1.0
-1.5
-2.0
Comprehensive analysis of peaks:
Area: -31.14 J/g
Peak value: 183.1 °C
Start point: 169.1°C
End point: 188.9°C
Comprehensive analysis of peaks:
Area: -30.08 J/g
Peak value: 213.1 °C
Start point: 201.2 °C
End point: 220.3°C
50
100
150
200
250
300
Temperature/°C

FIG. 30

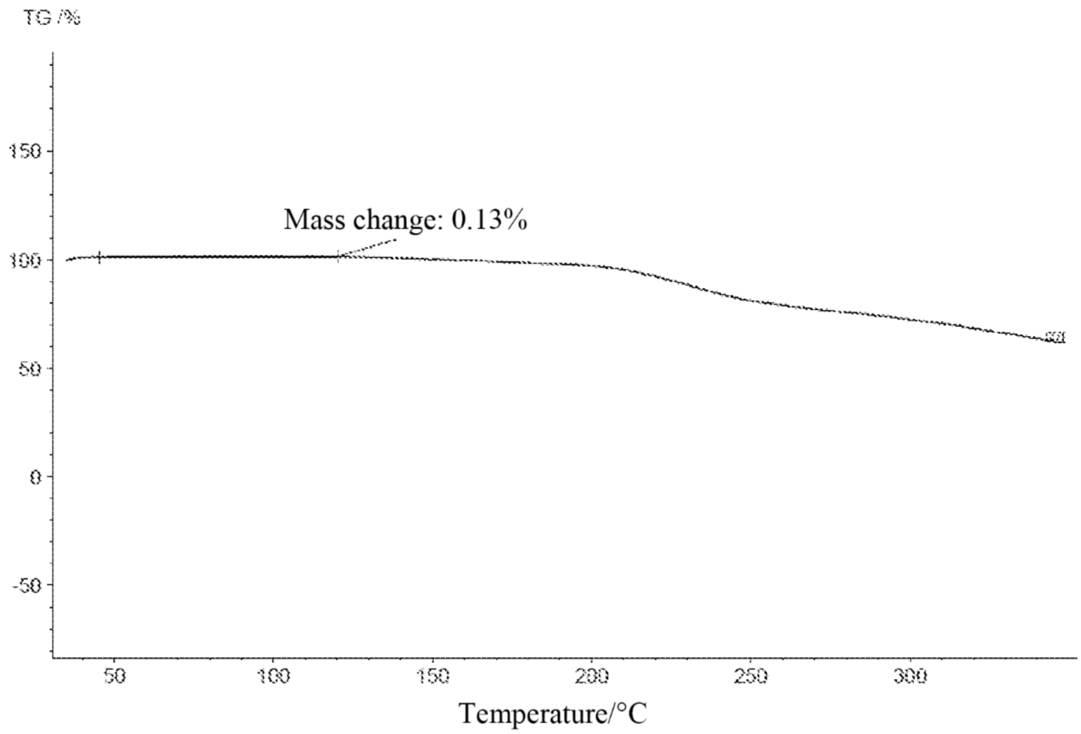
TG /%
150
100
50
0
-50
Mass change: 0.13%
50
100
150
200
250
300
Temperature/°C

FIG. 31

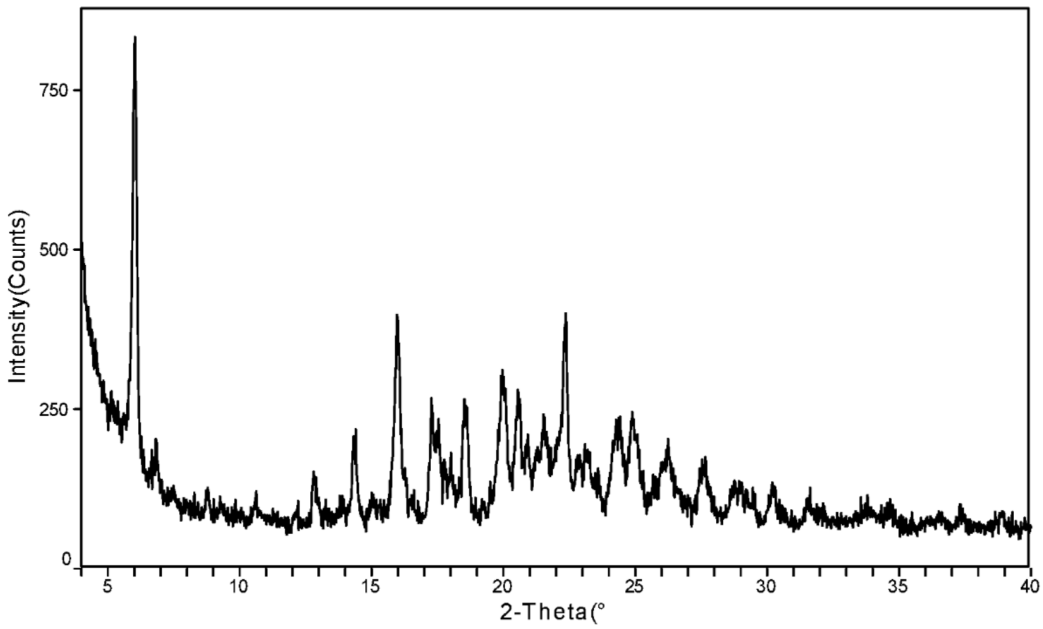
750
500
250
0
Intensity(Counts)
5
10
15
20
25
30
35
40
2-Theta(°

FIG. 32

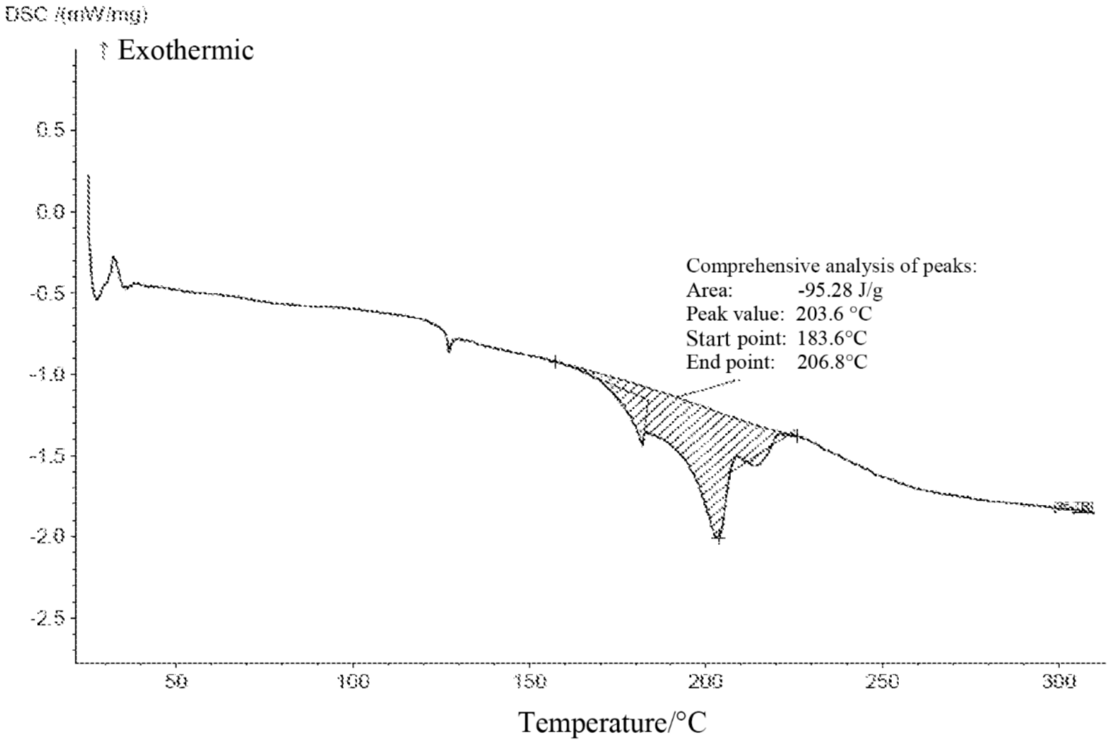
DSC /(mW/mg)
↑ Exothermic
0.5
0.0
-0.5
-1.0
-1.5
-2.0
-2.5
Comprehensive analysis of peaks:
Area: -95.28 J/g
Peak value: 203.6 °C
Start point: 183.6°C
End point: 206.8°C
50
100
150
200
250
300
Temperature/°C

FIG. 33

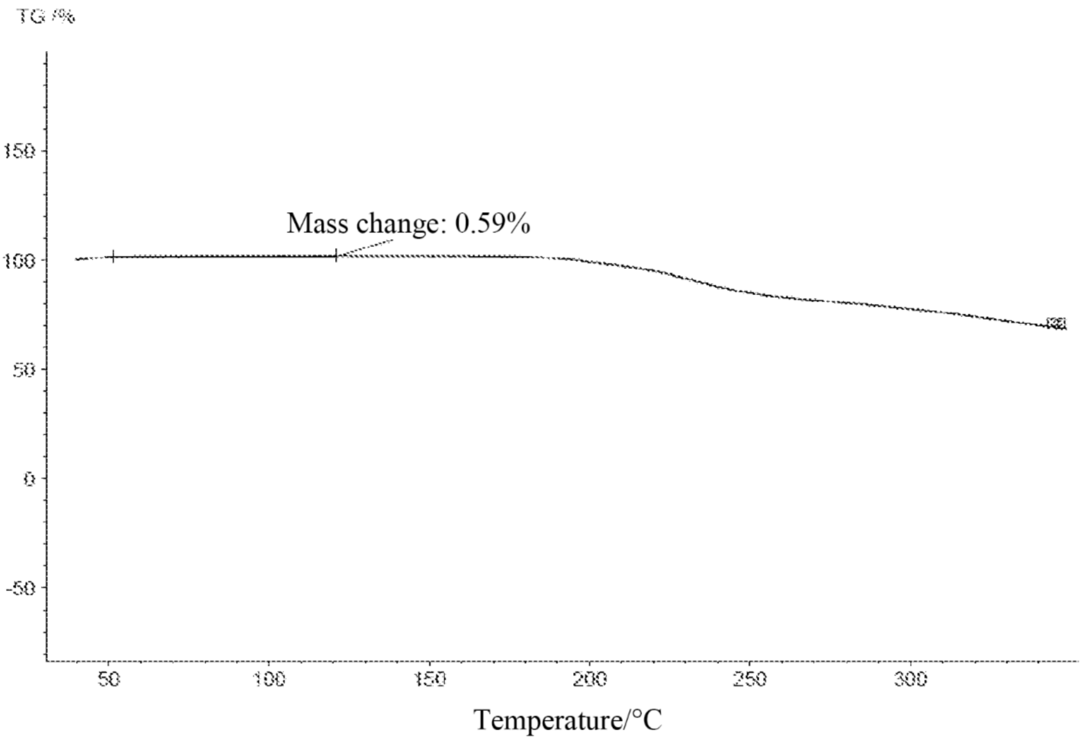
TG /%
150
100
50
0
-50
Mass change: 0.59%
50
100
150
200
250
300
Temperature/°C

FIG. 34

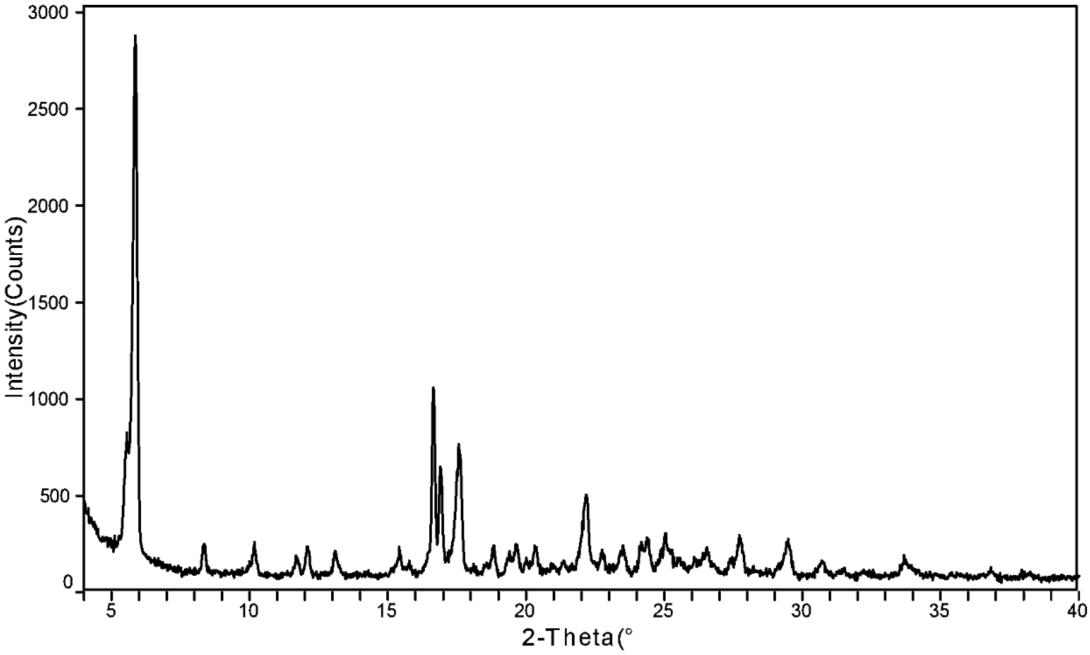
Intensity(Counts)
2-Theta(°

FIG. 35

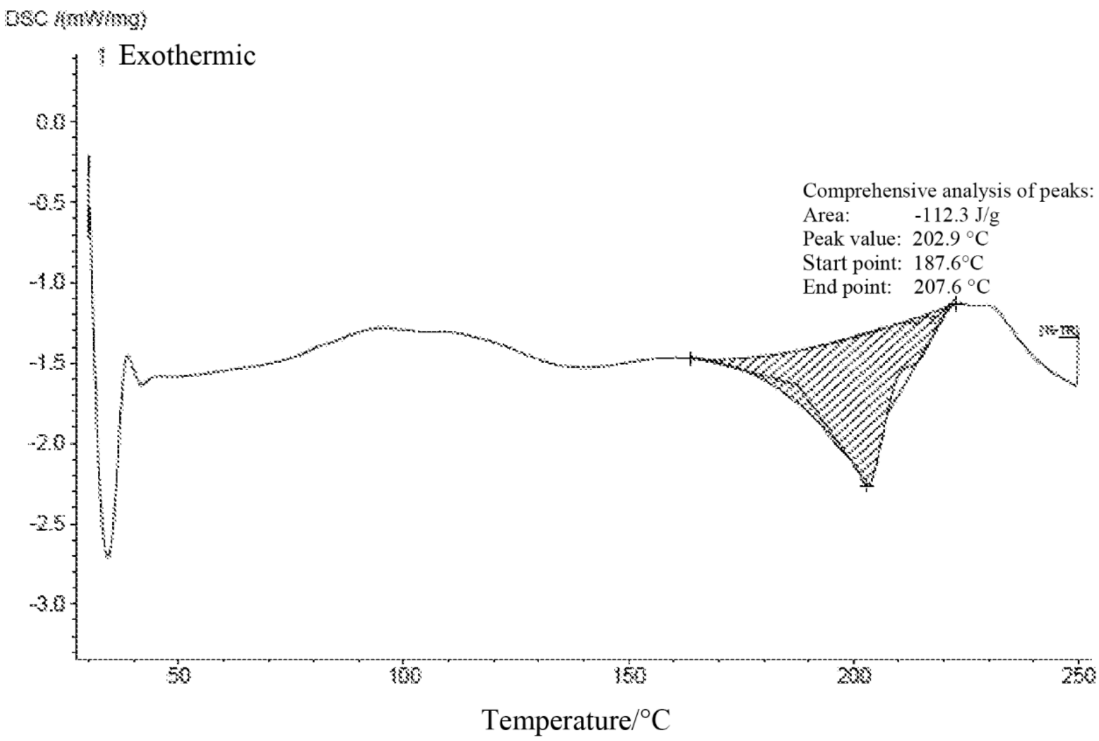
DSC /(mW/mg)
↑ Exothermic
Comprehensive analysis of peaks:
Area: -112.3 J/g
Peak value: 202.9 °C
Start point: 187.6°C
End point: 207.6 °C
Temperature/°C

FIG. 36

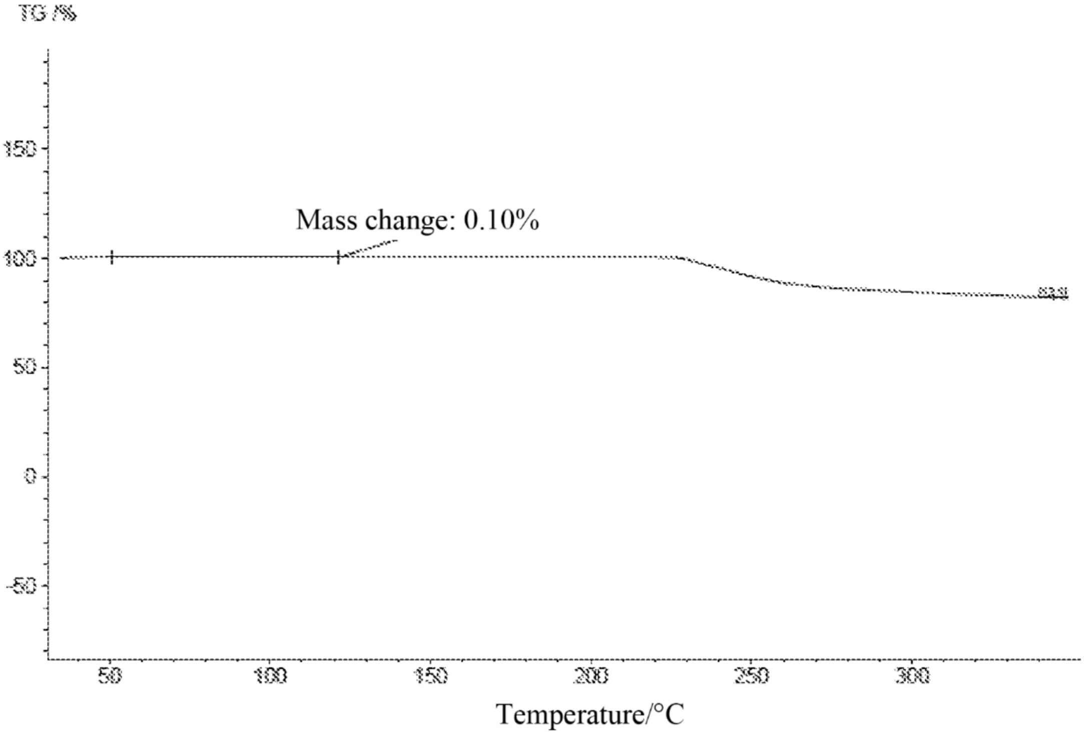
TG /%
150
100
50
0
-50
Mass change: 0.10%
50
100
150
200
250
300
Temperature/°C

FIG. 37

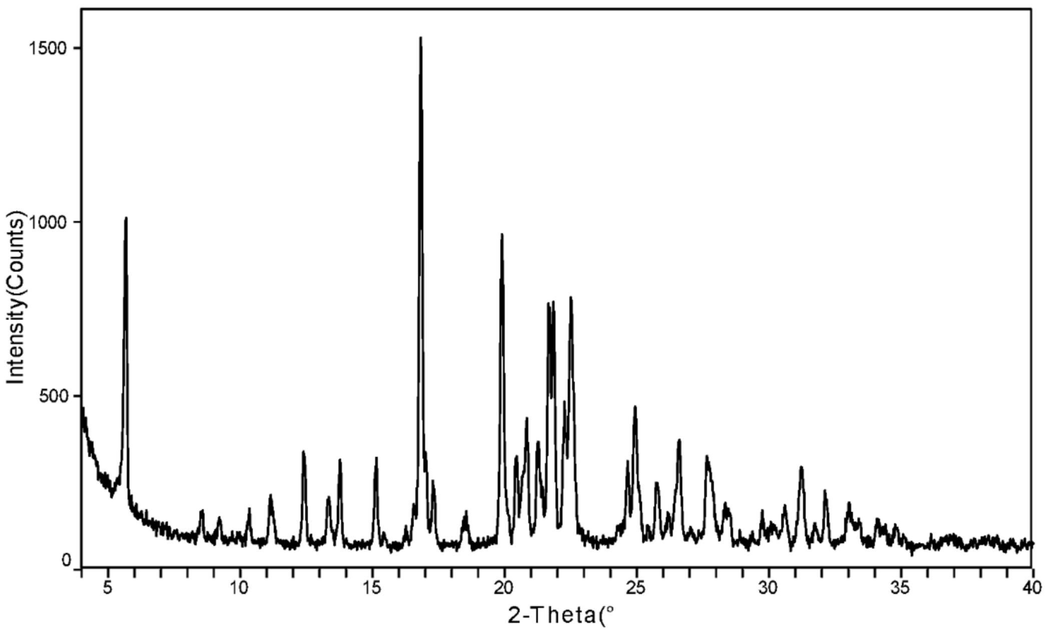
1500
1000
500
0
Intensity(Counts)
5
10
15
20
25
30
35
40
2-Theta(°

FIG. 38

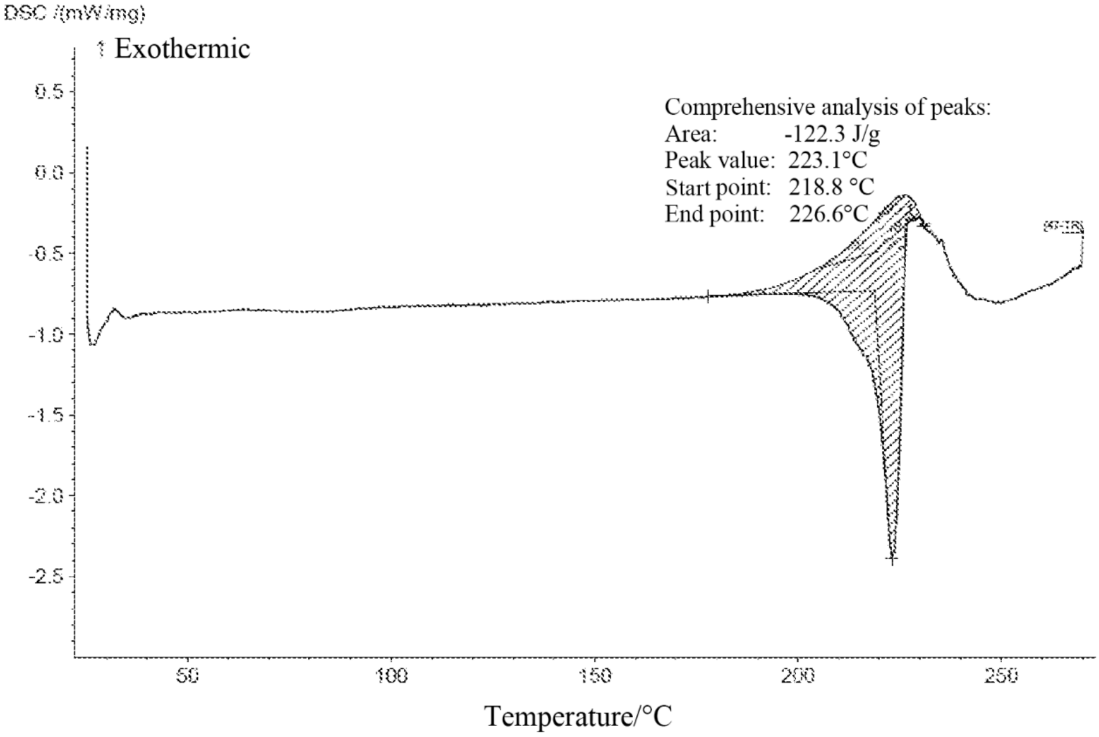
DSC /(mW/mg)
↑ Exothermic
0.5
0.0
-0.5
-1.0
-1.5
-2.0
-2.5
Comprehensive analysis of peaks:
Area: -122.3 J/g
Peak value: 223.1°C
Start point: 218.8 °C
End point: 226.6°C
50
100
150
200
250
Temperature/°C

FIG. 39

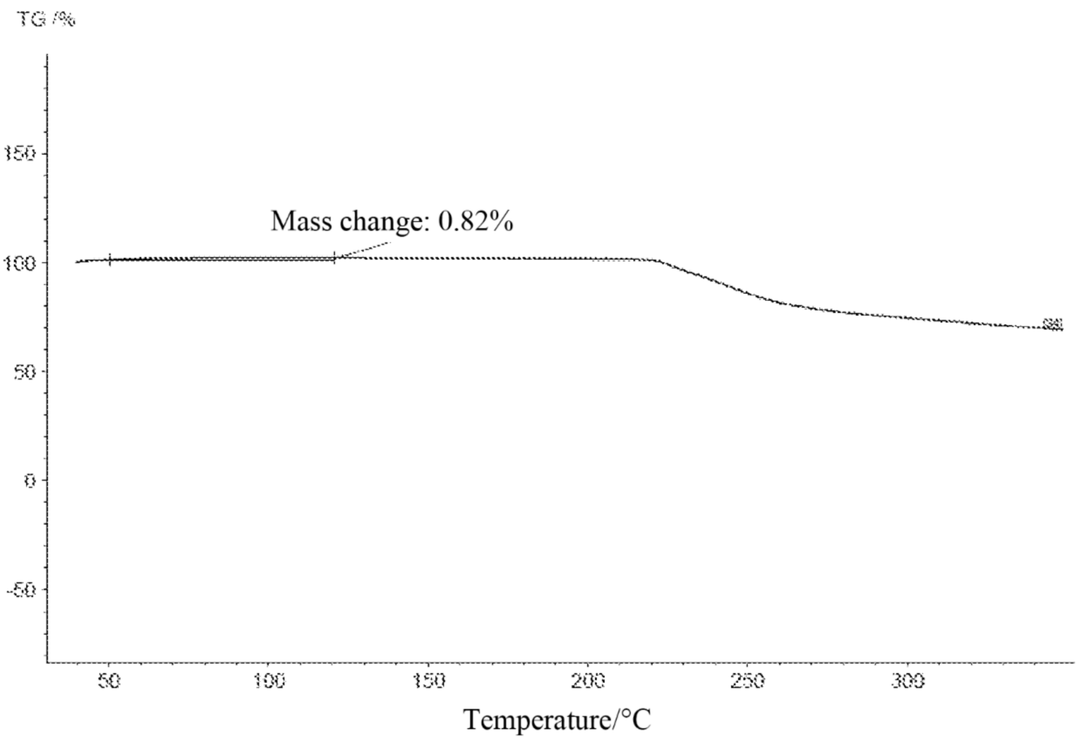
TG /%
150
100
50
0
-50
Mass change: 0.82%
50
100
150
200
250
300
Temperature/°C

FIG. 40

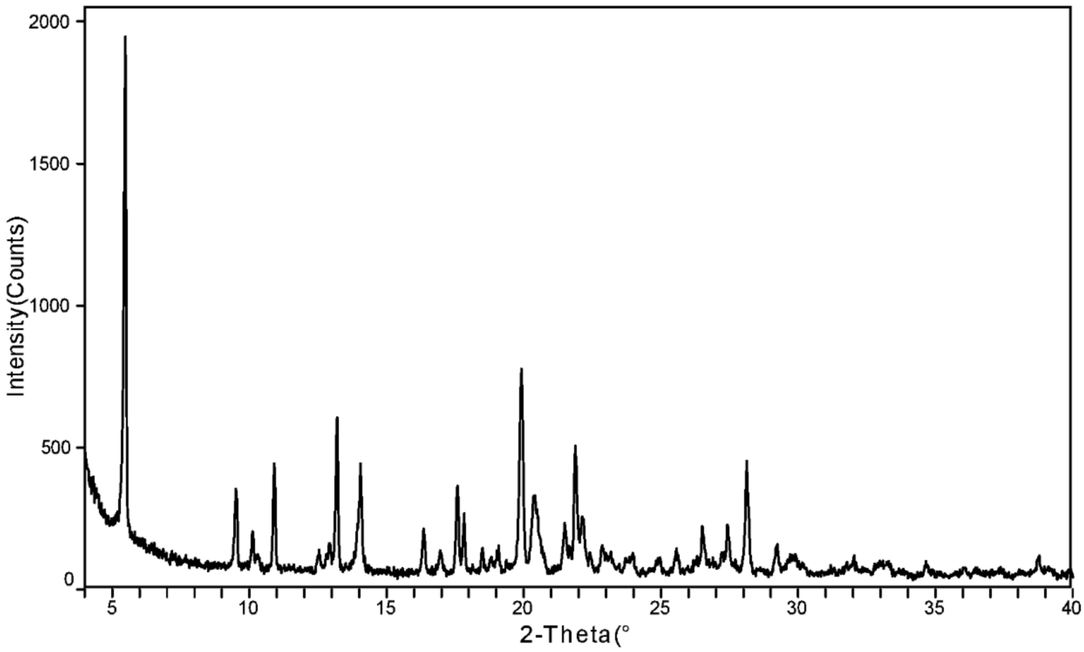
2000
1500
1000
500
0
Intensity(Counts)
5
10
15
20
25
30
35
40
2-Theta(°

FIG. 41

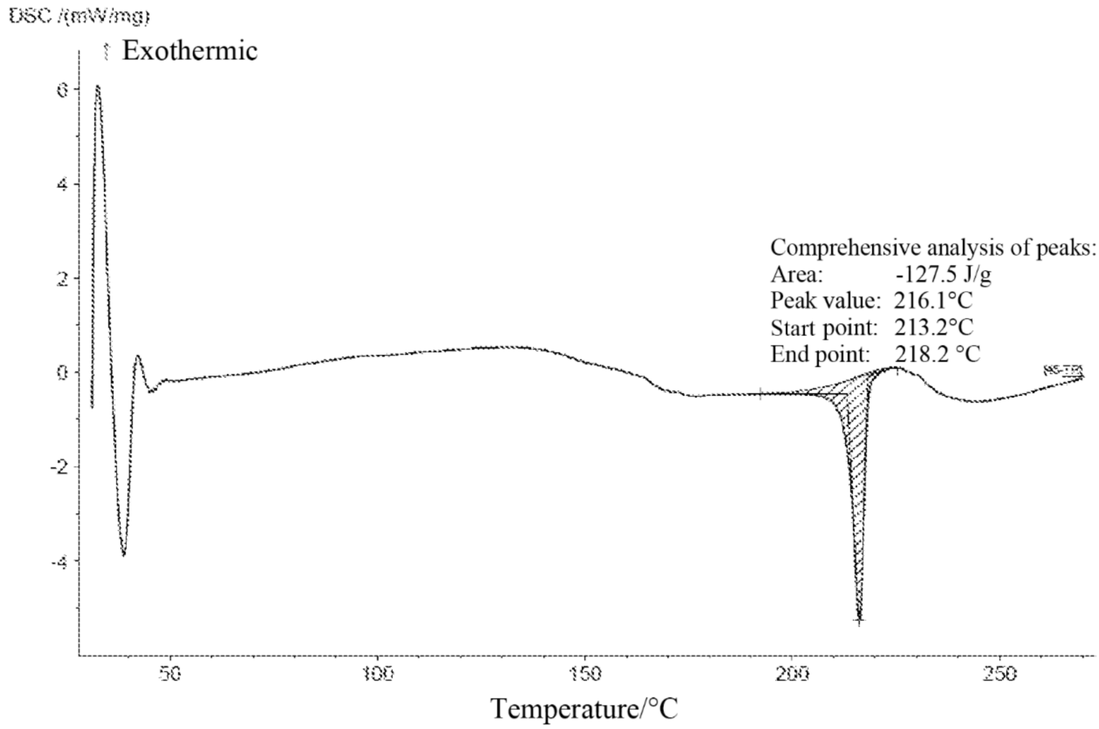
DSC /(mW/mg)
↑ Exothermic
6
4
2
0
-2
-4
Comprehensive analysis of peaks:
Area: -127.5 J/g
Peak value: 216.1°C
Start point: 213.2°C
End point: 218.2 °C
50
100
150
200
250
Temperature/°C

FIG. 42

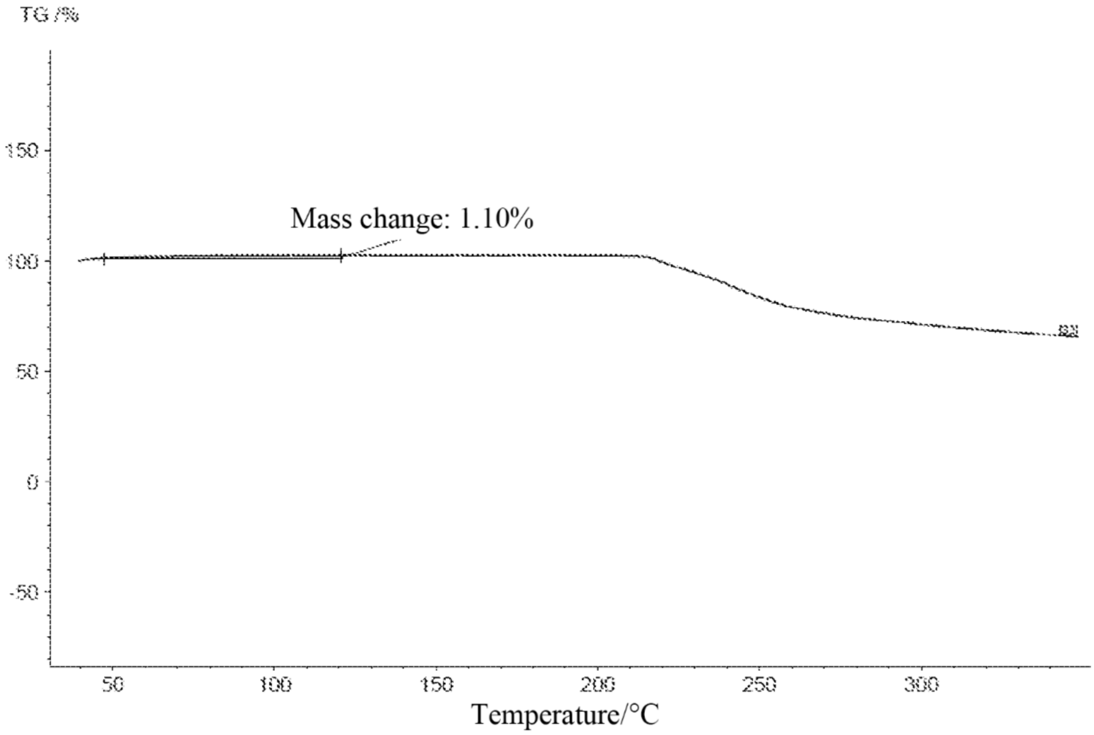
TG /%
150
100
50
0
-50
Mass change: 1.10%
50
100
150
200
250
300
Temperature/°C

FIG. 43

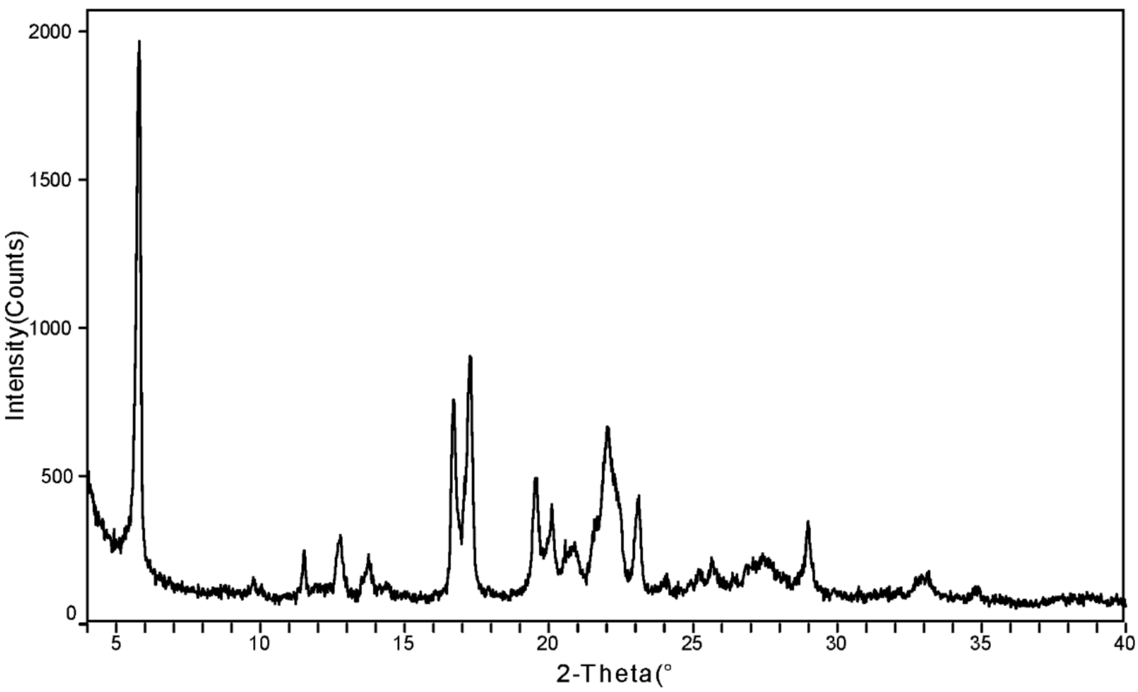
2000
1500
1000
500
0
Intensity(Counts)
5
10
15
20
25
30
35
40
2-Theta(°

FIG. 44

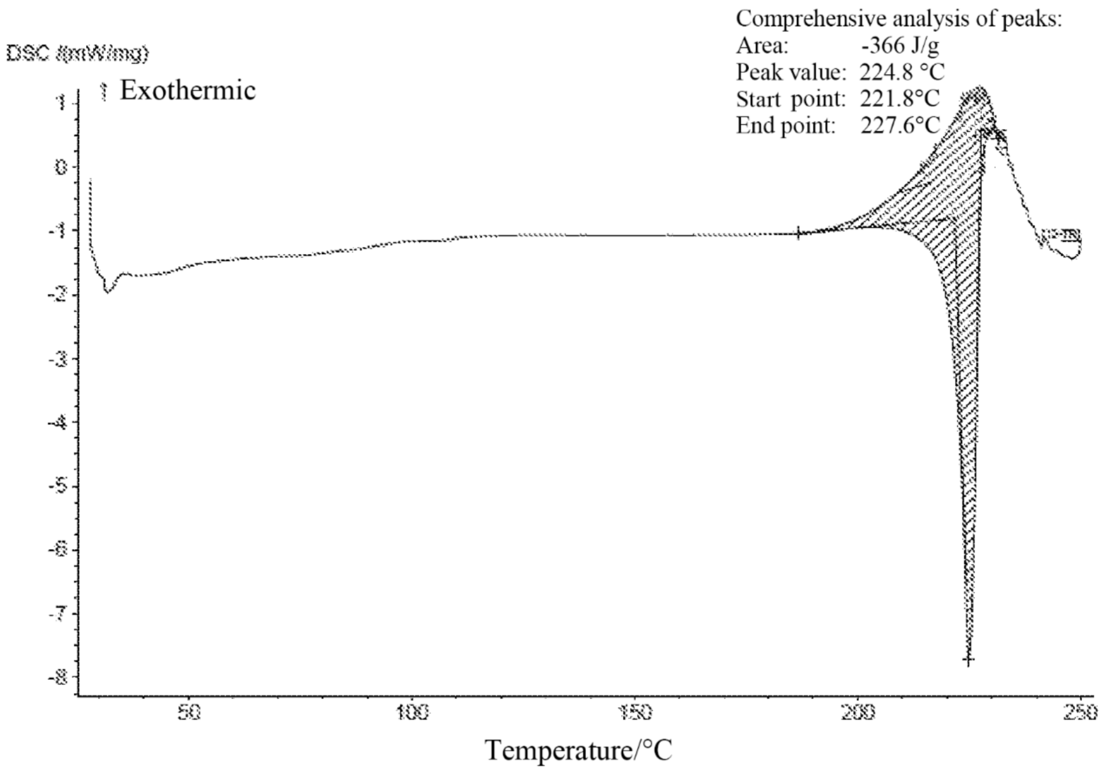
Comprehensive analysis of peaks:
Area: -366 J/g
Peak value: 224.8 °C
Start point: 221.8°C
End point: 227.6°C
DSC /(mW/mg)
↑ Exothermic
1
0
-1
-2
-3
-4
-5
-6
-7
-8
50
100
150
200
250
Temperature/°C

FIG. 45

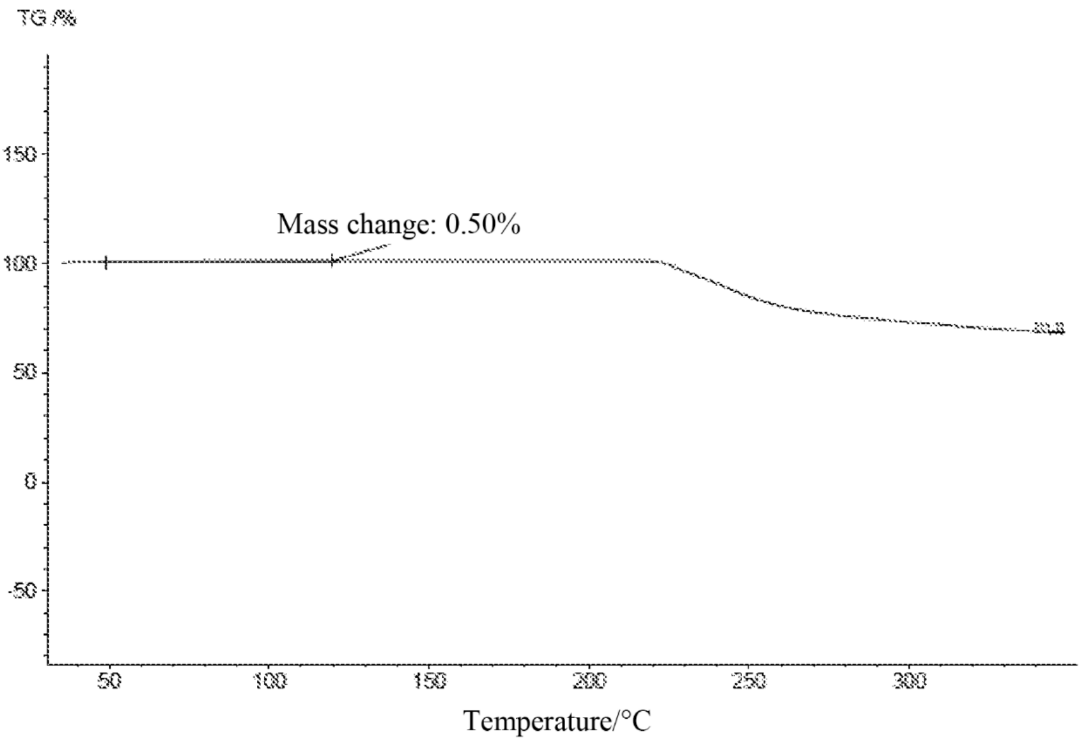
TG /%
150
100
50
0
-50
Mass change: 0.50%
50
100
150
200
250
300
Temperature/°C

FIG. 46

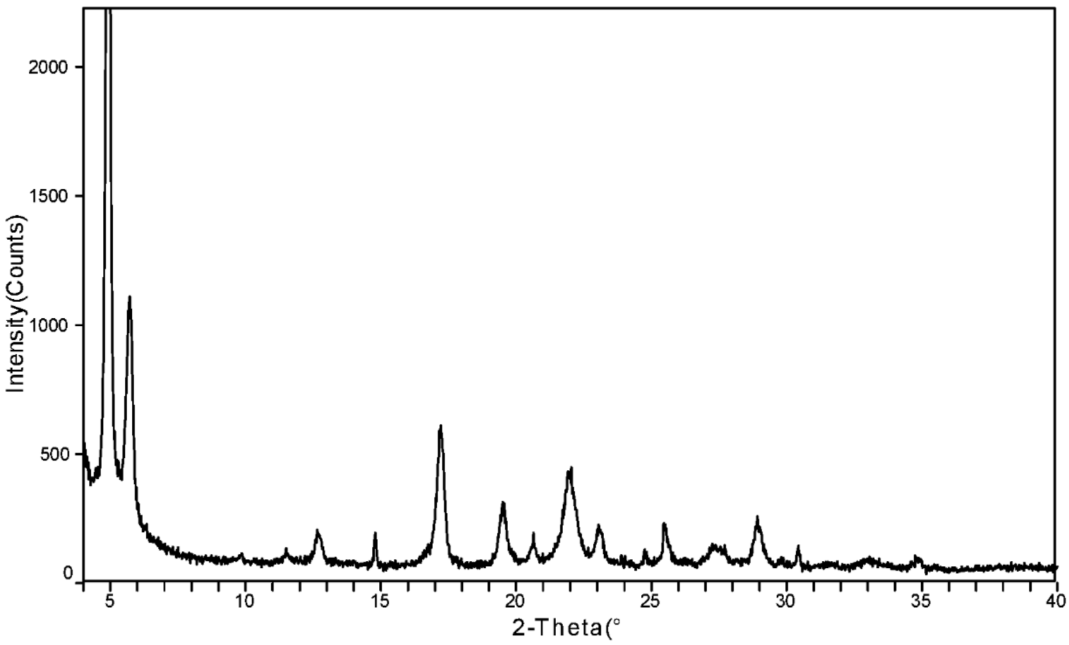
Intensity(Counts)
2000
1500
1000
500
0
5
10
15
20
25
30
35
40
2-Theta(°

FIG. 47

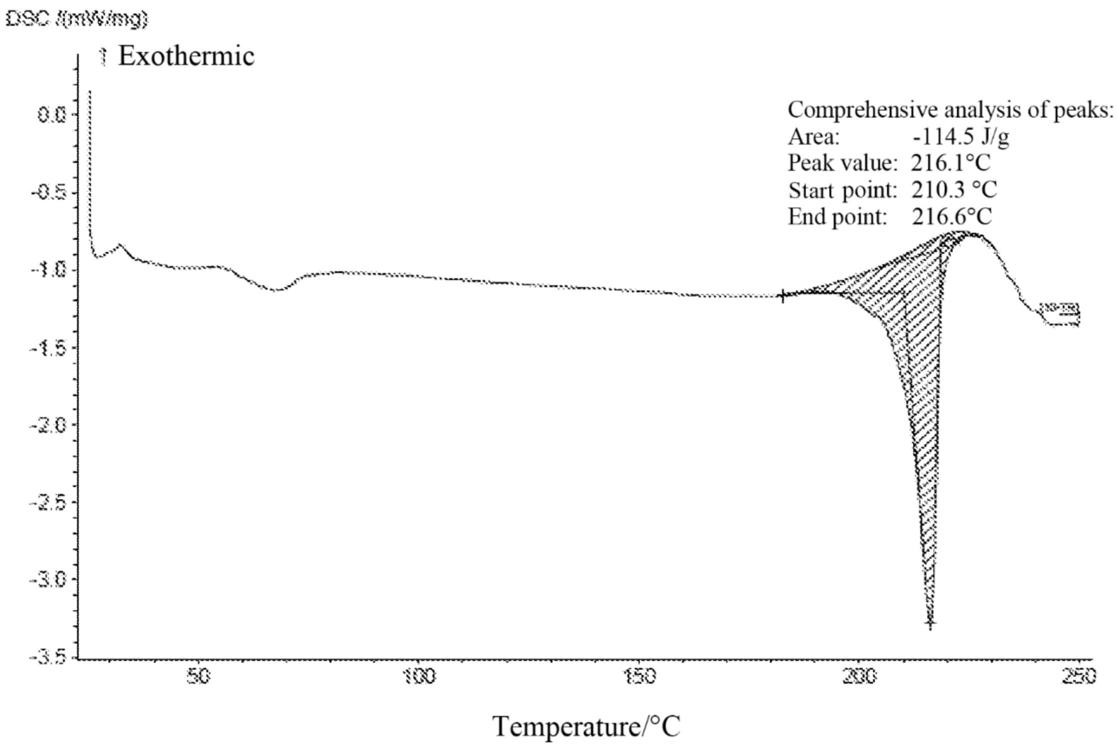
DSC /(mW/mg)
↑ Exothermic
Comprehensive analysis of peaks:
Area: -114.5 J/g
Peak value: 216.1°C
Start point: 210.3 °C
End point: 216.6°C
0.0
-0.5
-1.0
-1.5
-2.0
-2.5
-3.0
-3.5
50
100
150
200
250
Temperature/°C

FIG. 48

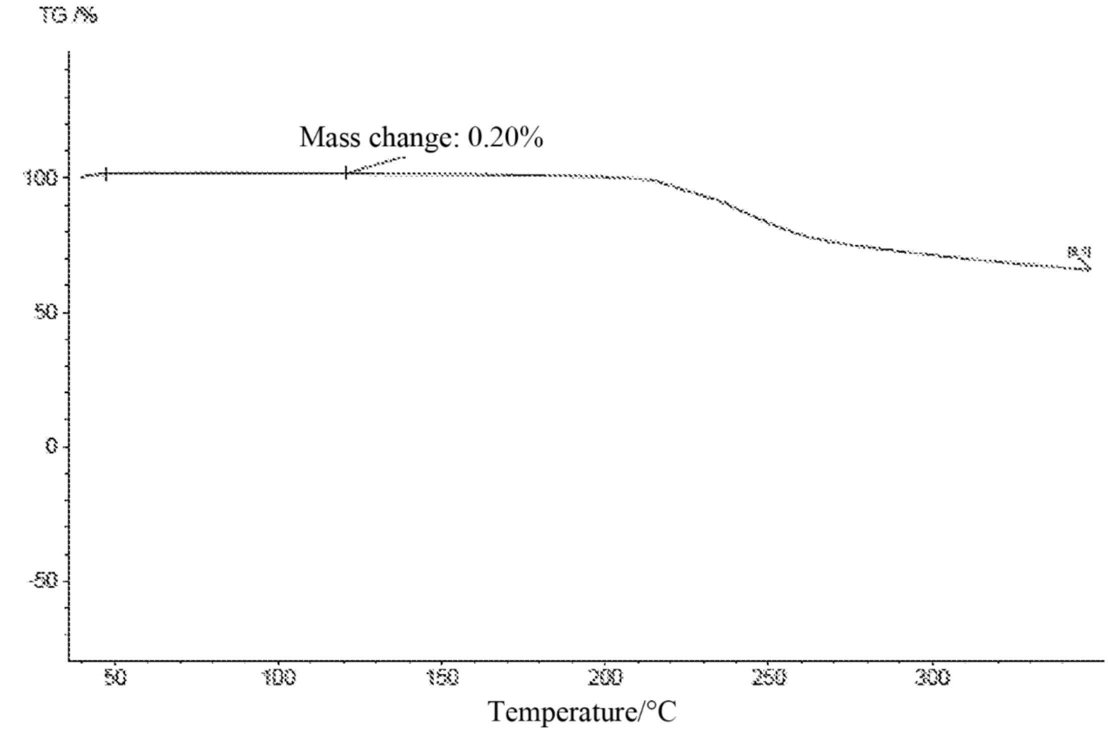
TG /%
Mass change: 0.20%
100
50
0
-50
50
100
150
200
250
300
Temperature/°C

FIG. 49

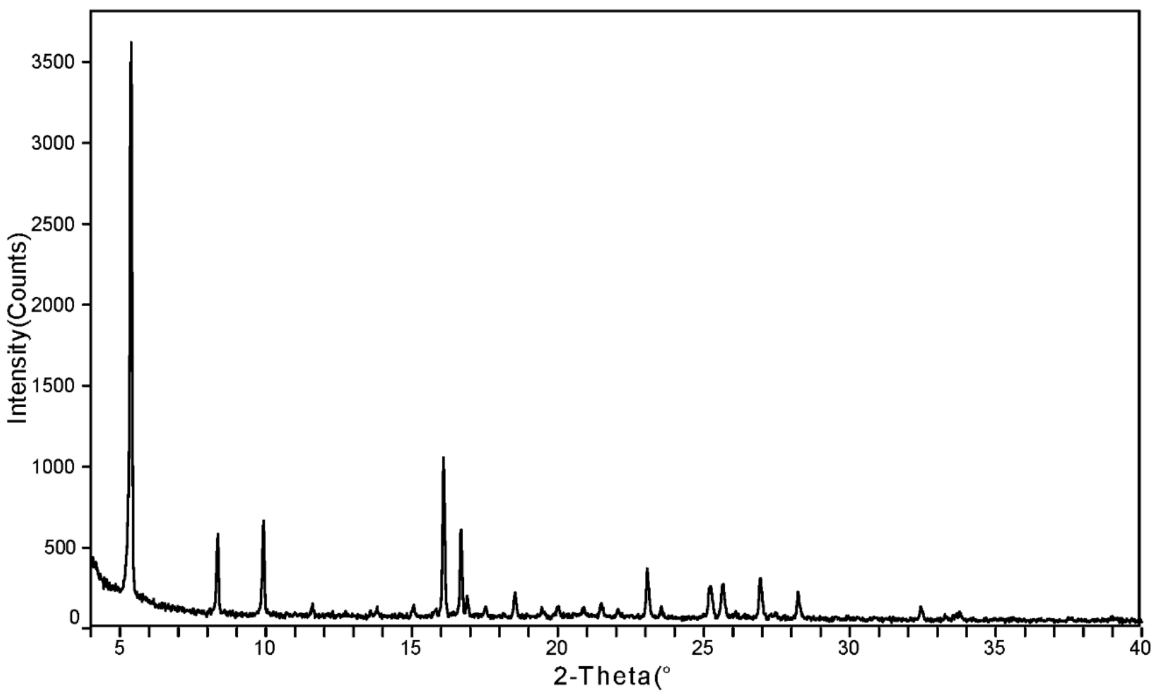
3500
3000
2500
2000
1500
1000
500
0
Intensity(Counts)
5
10
15
20
25
30
35
40
2-Theta(°

FIG. 50

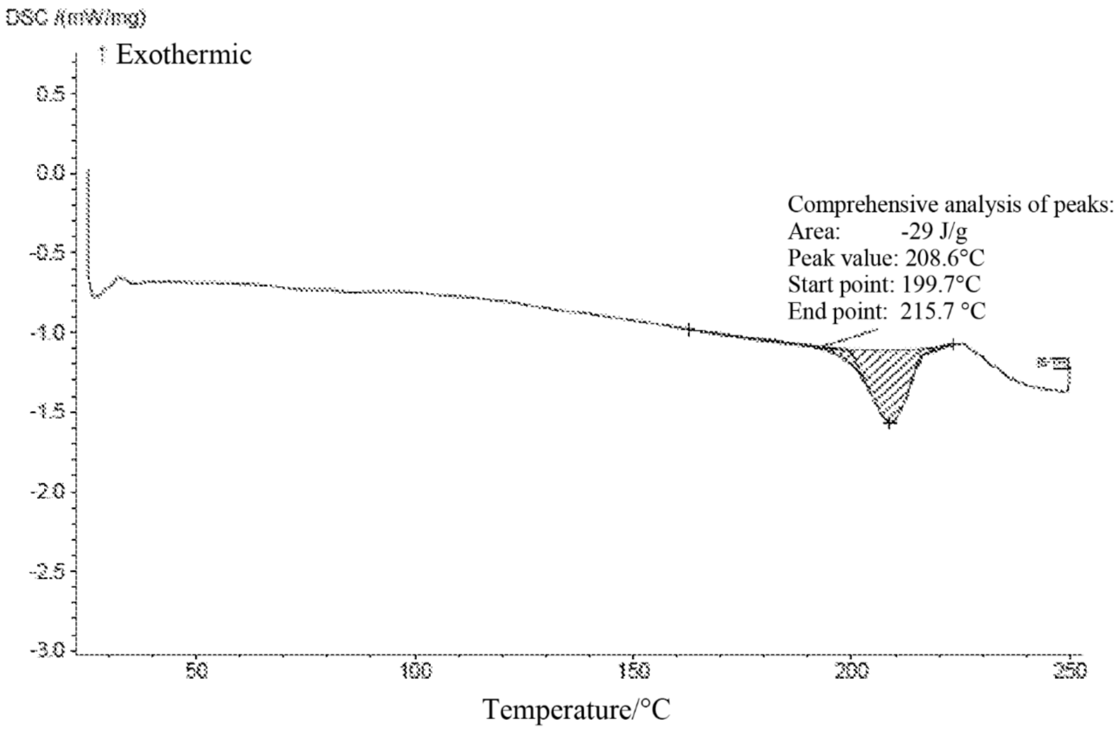
DSC /(mW/mg)
↑ Exothermic
0.5
0.0
-0.5
-1.0
-1.5
-2.0
-2.5
-3.0
Comprehensive analysis of peaks:
Area: -29 J/g
Peak value: 208.6°C
Start point: 199.7°C
End point: 215.7 °C
50
100
150
200
250
Temperature/°C

FIG. 51

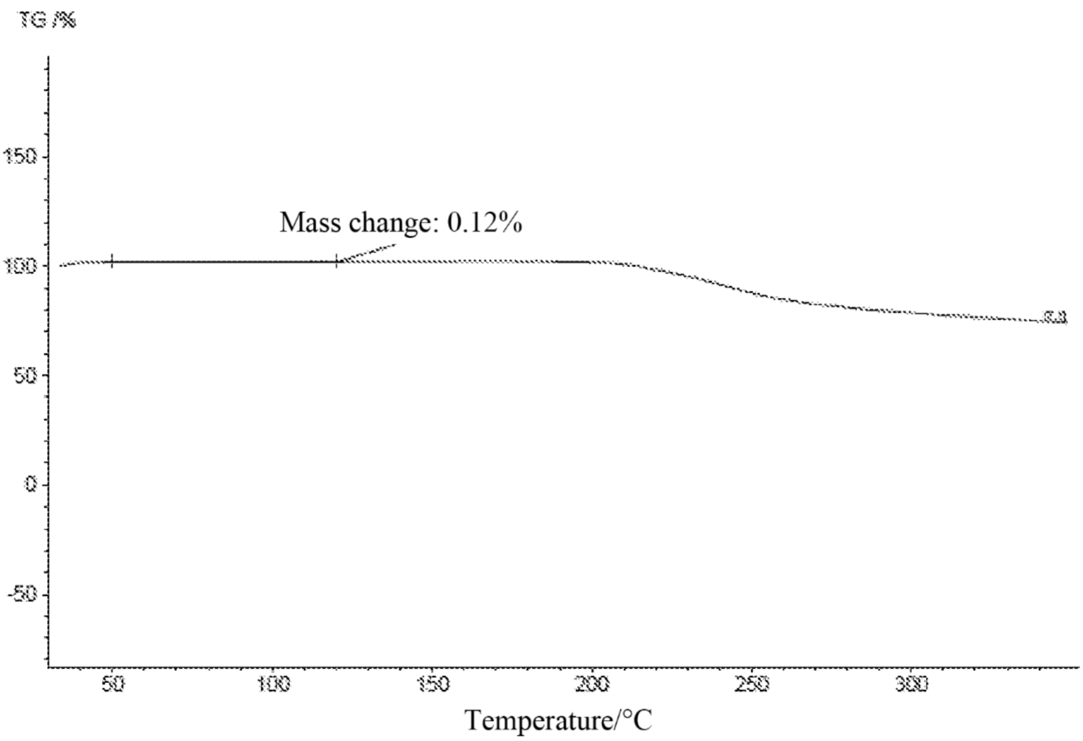
TG /%
150
100
50
0
-50
Mass change: 0.12%
50
100
150
200
250
300
Temperature/°C

FIG. 52

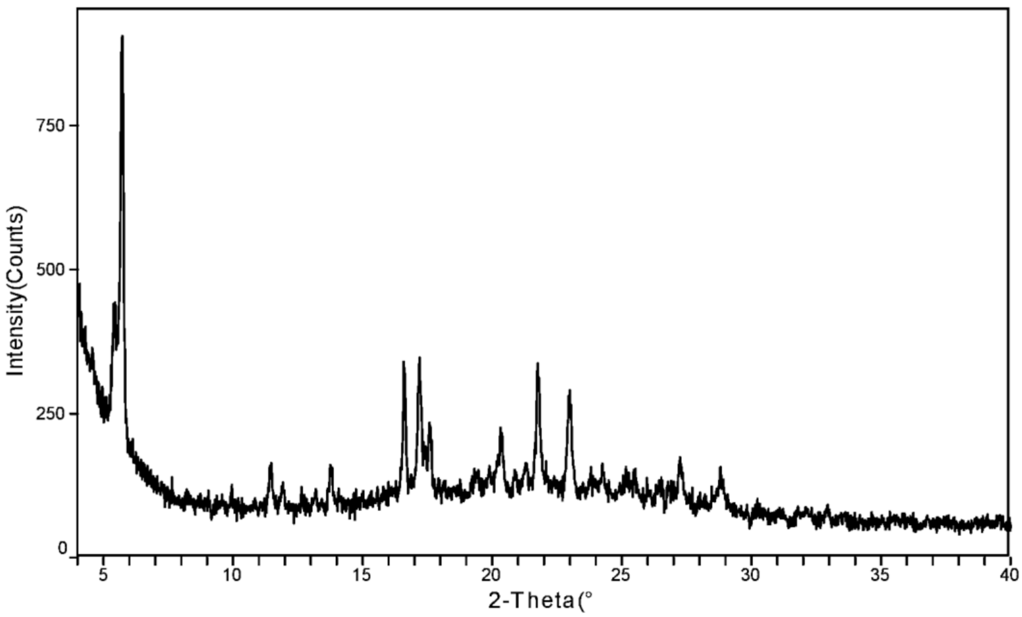
Intensity(Counts)
750
500
250
0
5
10
15
20
25
30
35
40
2-Theta(°

FIG. 53

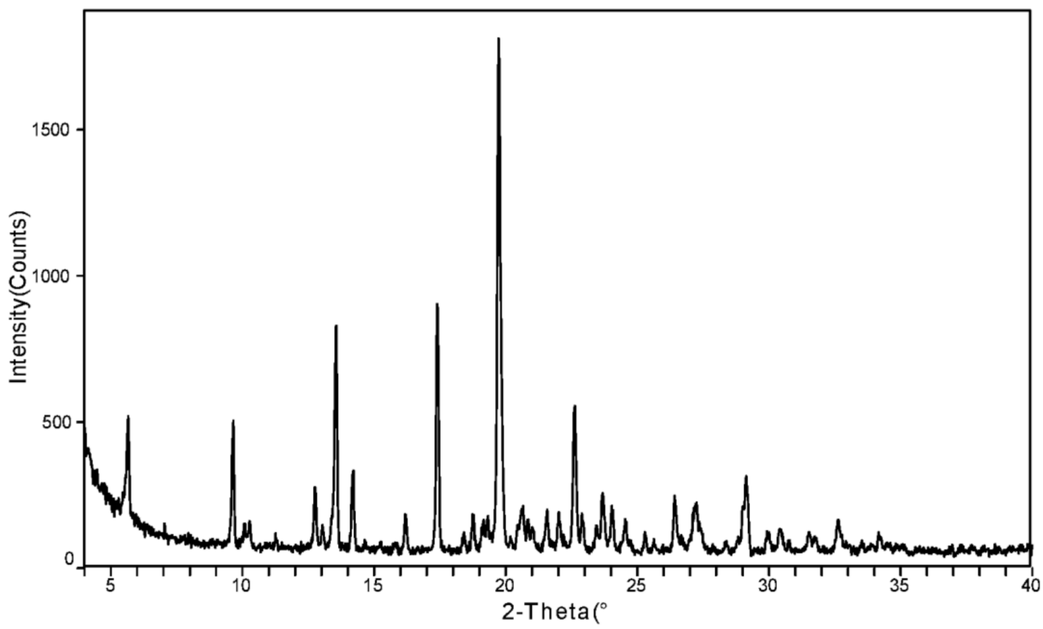
Intensity(Counts)
1500
1000
500
0
5
10
15
20
25
30
35
40
2-Theta(°

FIG. 54

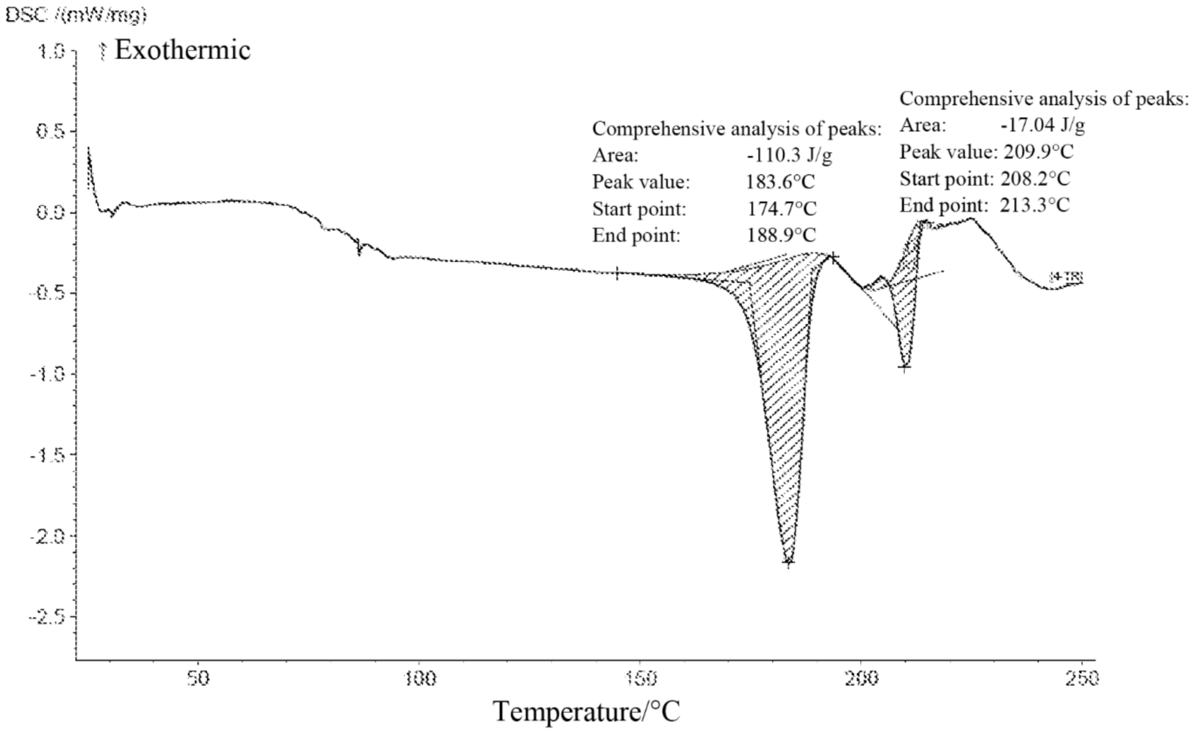
DSC /(mW/mg)
↑ Exothermic
Comprehensive analysis of peaks:
Area: -110.3 J/g
Peak value: 183.6°C
Start point: 174.7°C
End point: 188.9°C
Comprehensive analysis of peaks:
Area: -17.04 J/g
Peak value: 209.9°C
Start point: 208.2°C
End point: 213.3°C
1.0
0.5
0.0
-0.5
-1.0
-1.5
-2.0
-2.5
50
100
150
200
250
Temperature/°C

FIG. 55

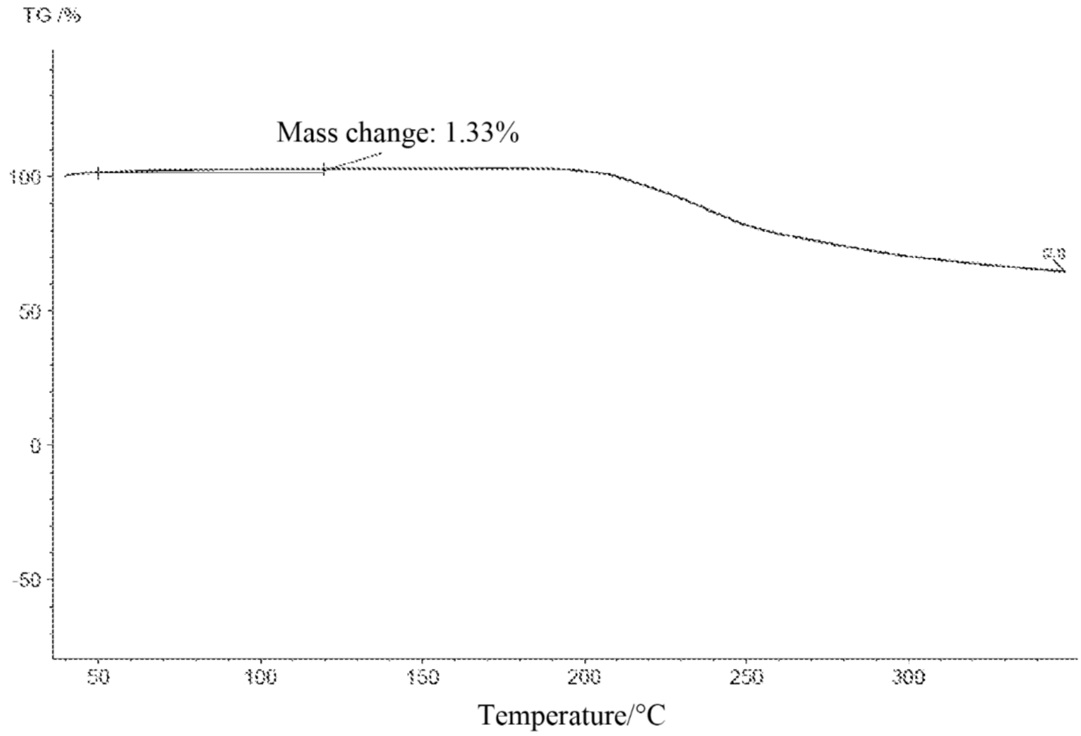
TG /%
Mass change: 1.33%
100
50
0
-50
50
100
150
200
250
300
Temperature/°C

FIG. 56

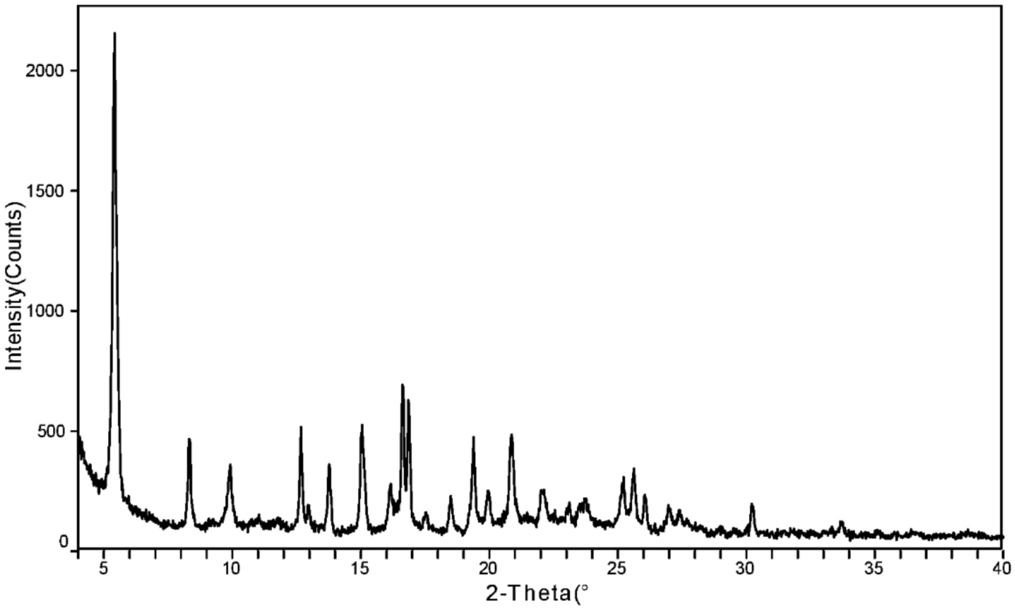
Intensity(Counts)
2000
1500
1000
500
0
5
10
15
20
25
30
35
40
2-Theta(°

FIG. 57

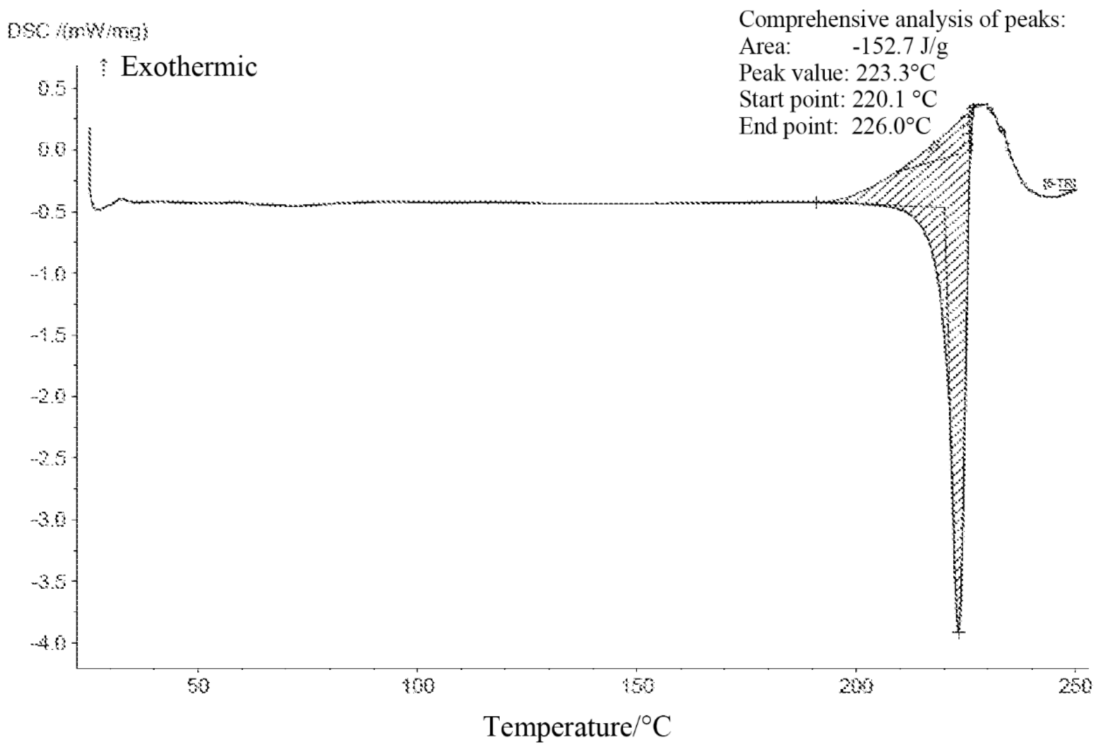
DSC /(mW/mg)
↑ Exothermic
Comprehensive analysis of peaks:
Area: -152.7 J/g
Peak value: 223.3°C
Start point: 220.1 °C
End point: 226.0°C
0.5
0.0
-0.5
-1.0
-1.5
-2.0
-2.5
-3.0
-3.5
-4.0
50
100
150
200
250
Temperature/°C

FIG. 58

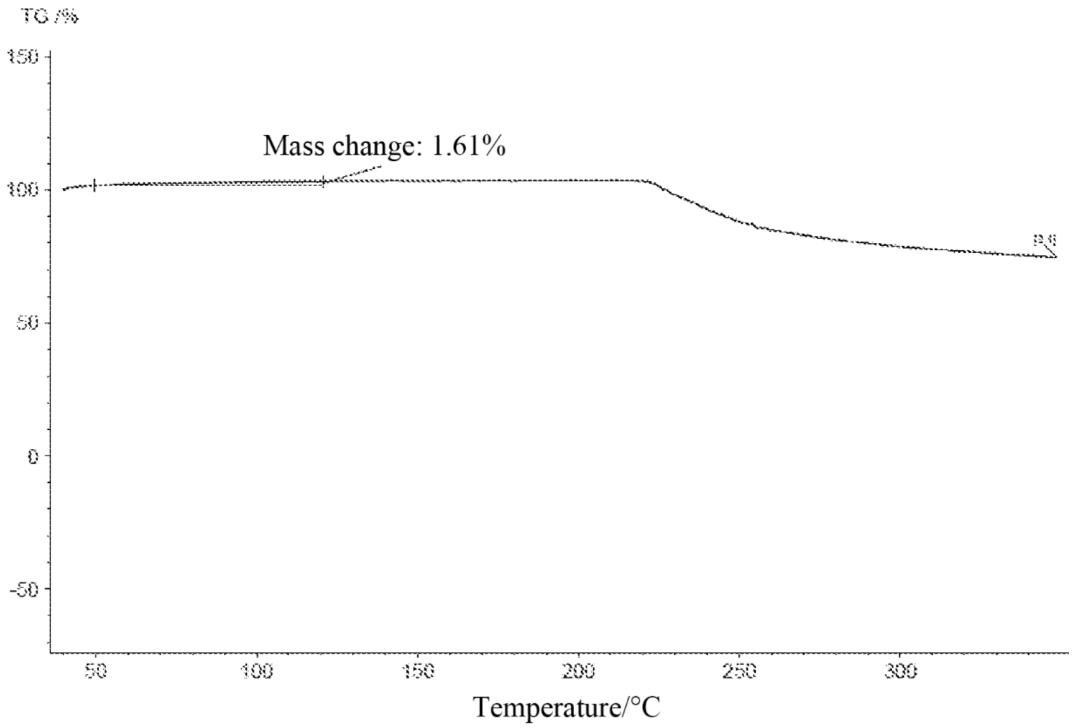
TG /%
150
100
50
0
-50
Mass change: 1.61%
50
100
150
200
250
300
Temperature/°C

FIG. 59

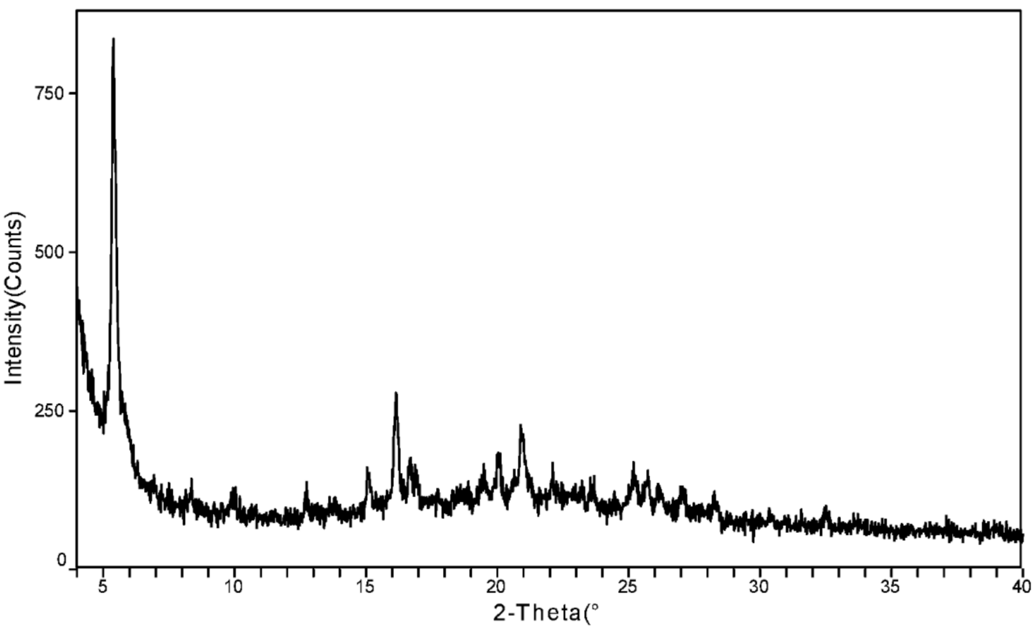
750
500
250
0
Intensity(Counts)
5
10
15
20
25
30
35
40
2-Theta(°

FIG. 60

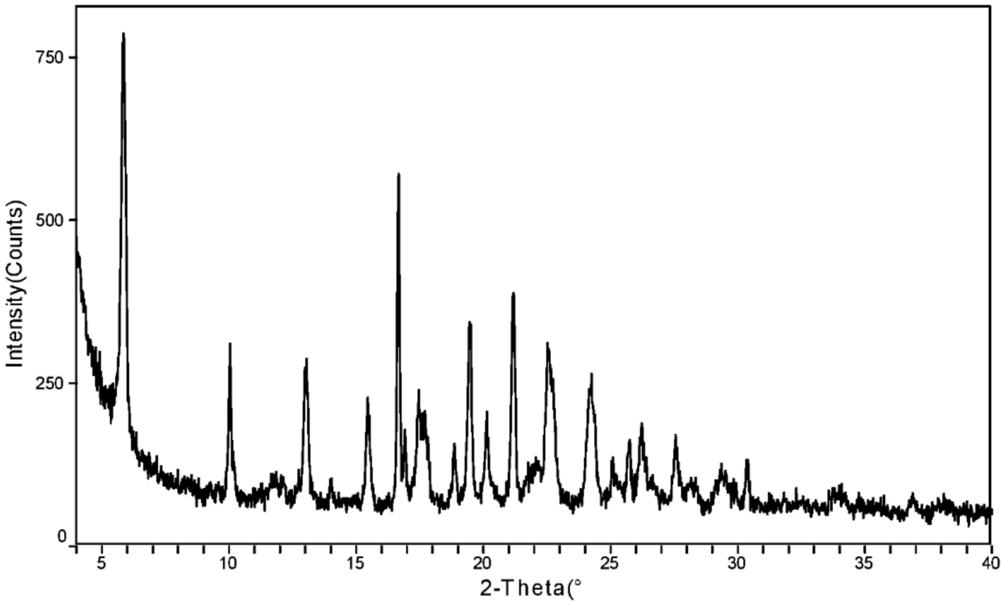
Intensity(Counts)
750
500
250
0
5
10
15
20
25
30
35
40
2-Theta(°

FIG. 61

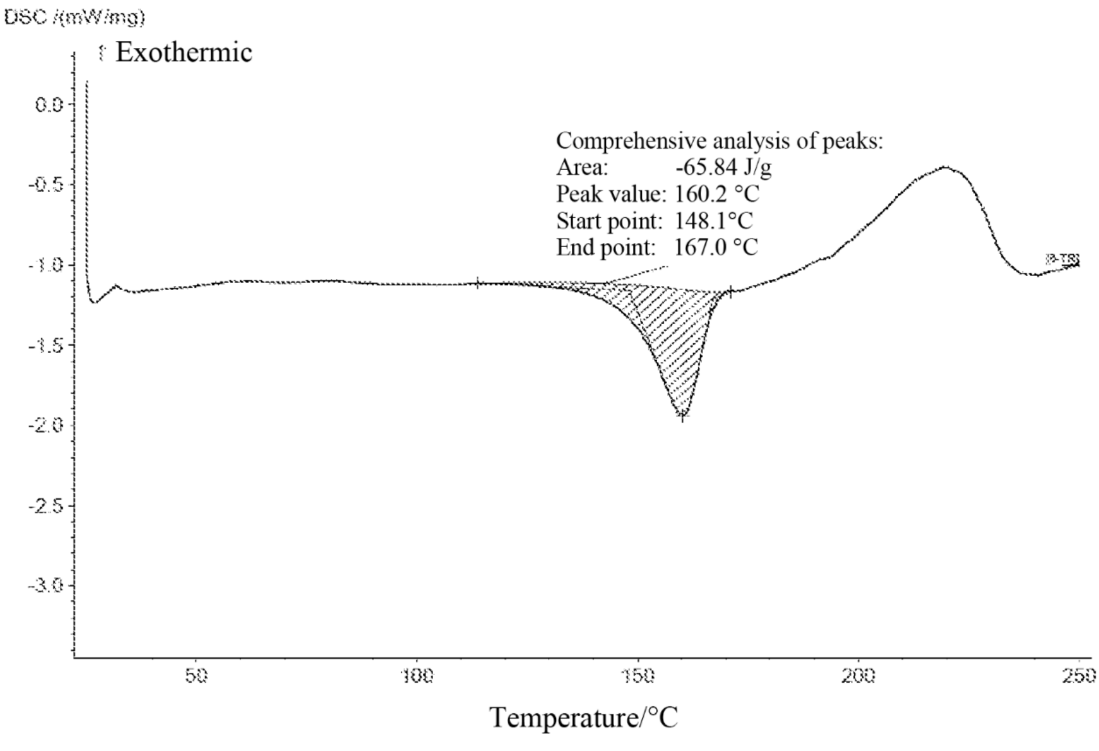
DSC /(mW/mg)
↑ Exothermic
0.0
-0.5
-1.0
-1.5
-2.0
-2.5
-3.0
Comprehensive analysis of peaks:
Area: -65.84 J/g
Peak value: 160.2 °C
Start point: 148.1°C
End point: 167.0 °C
50
100
150
200
250
Temperature/°C

FIG. 62

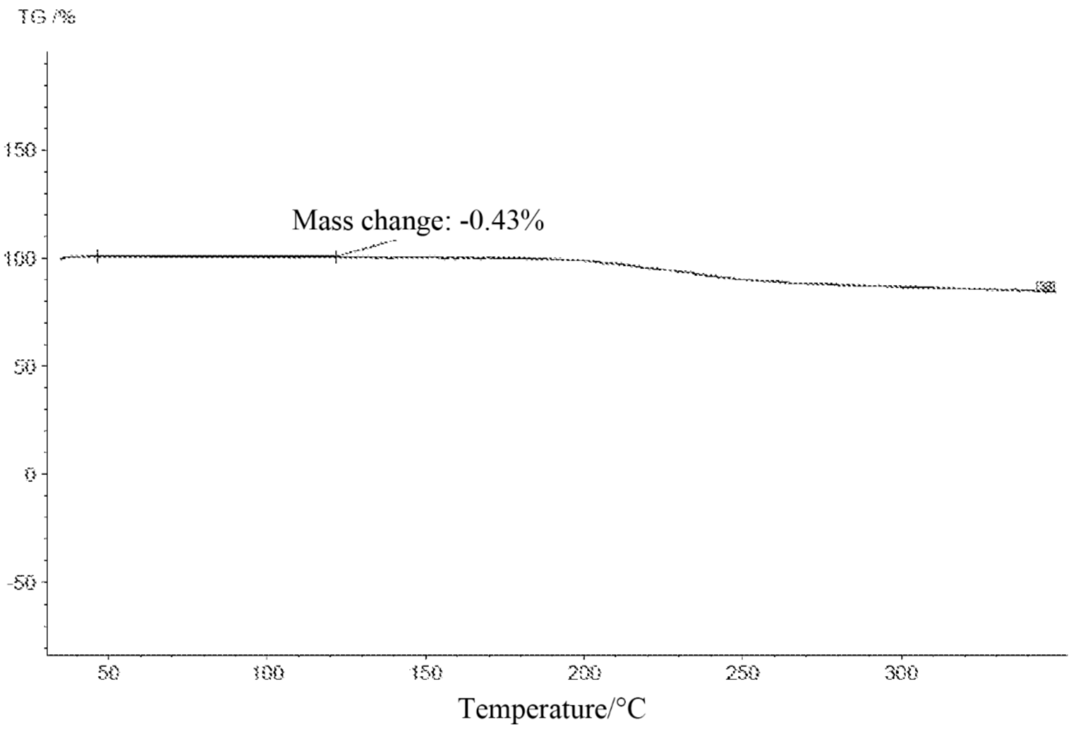
TG /%
150
100
50
0
-50
Mass change: -0.43%
50
100
150
200
250
300
Temperature/°C

FIG. 63

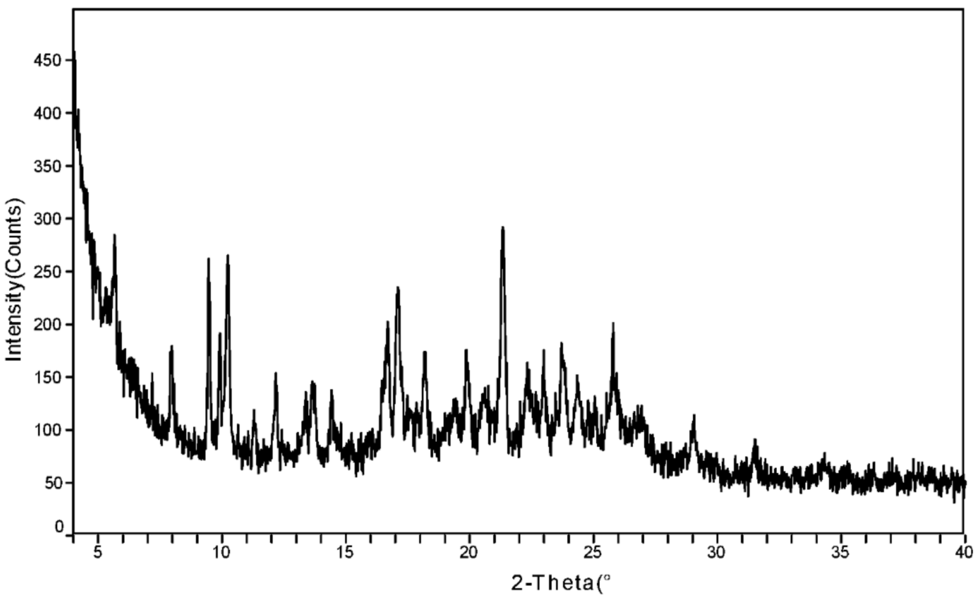
Intensity(Counts)
450
400
350
300
250
200
150
100
50
0
5
10
15
20
25
30
35
40
2-Theta(°

FIG. 64

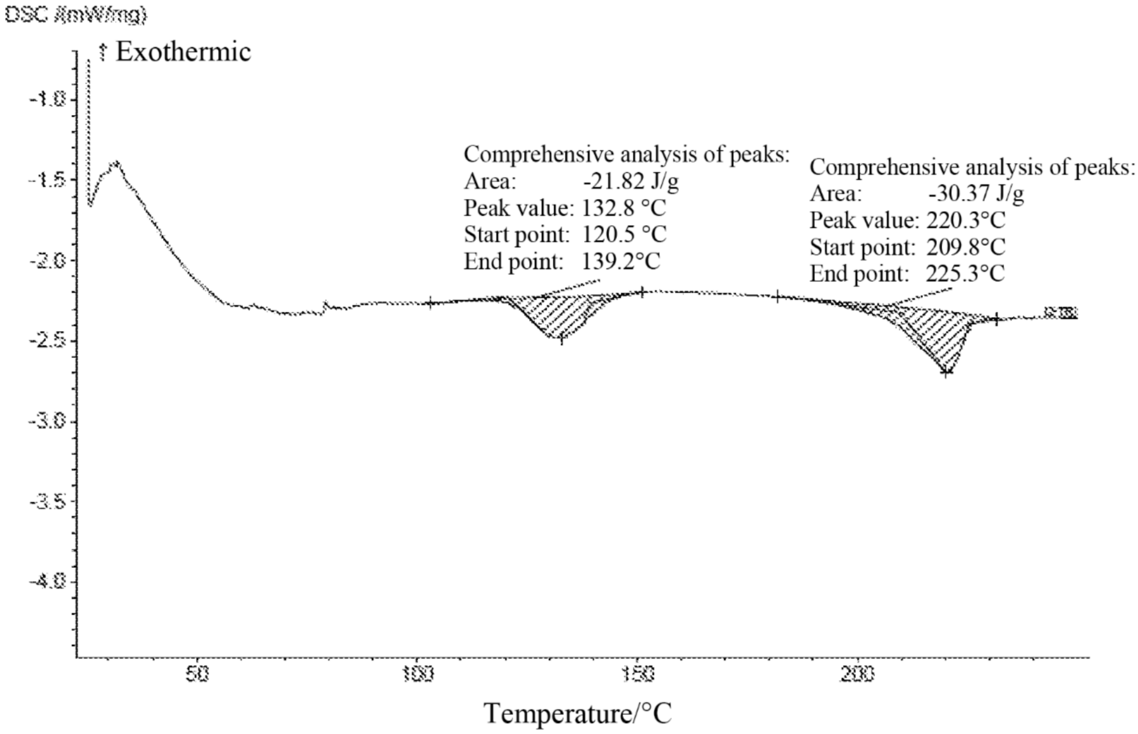
DSC /(mW/mg)
↑ Exothermic
-1.0
-1.5
-2.0
-2.5
-3.0
-3.5
-4.0
Comprehensive analysis of peaks:
Area: -21.82 J/g
Peak value: 132.8 °C
Start point: 120.5 °C
End point: 139.2°C
Comprehensive analysis of peaks:
Area: -30.37 J/g
Peak value: 220.3°C
Start point: 209.8°C
End point: 225.3°C
50
100
150
200
Temperature/°C

FIG. 65

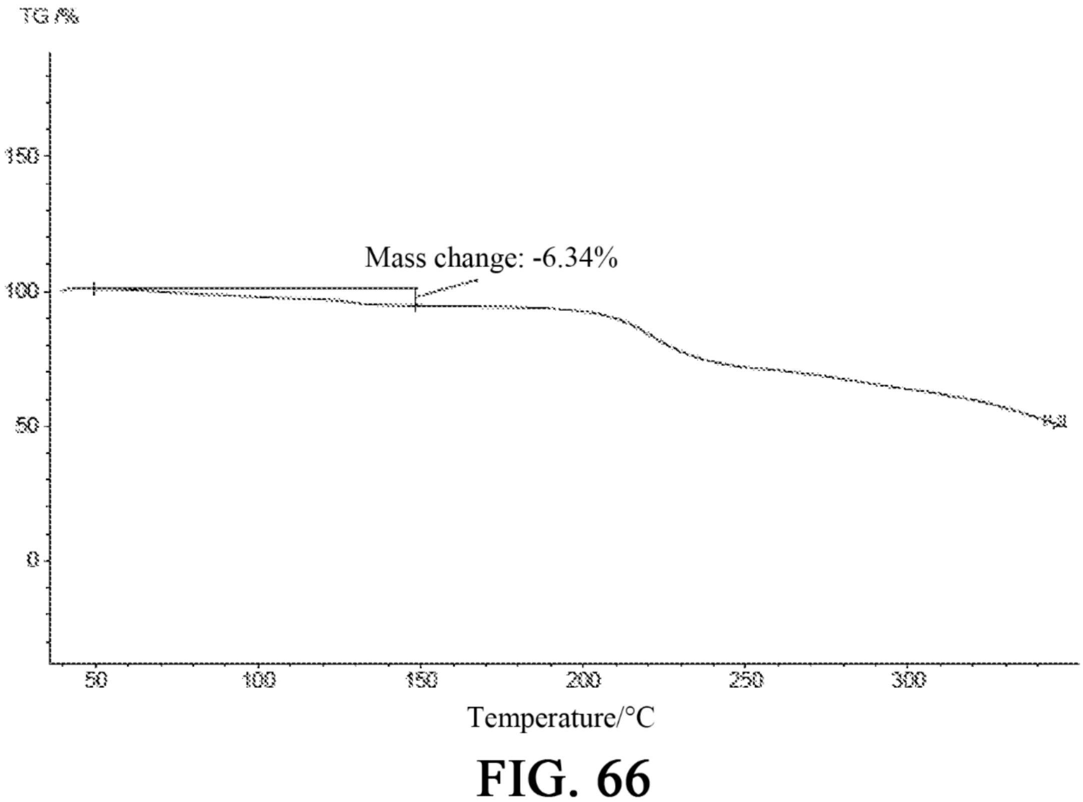
TG /%
150
100
50
0
Mass change: -6.34%
50
100
150
200
250
300
Temperature/°C

FIG. 66

**INTERNATIONAL SEARCH REPORT**

International application No.
**PCT/CN2024/084821**

**A. CLASSIFICATION OF SUBJECT MATTER**

C07D498/16(2006.01)i; C07D267/22(2006.01)i; C07D267/16(2006.01)i; A61K31/33(2006.01)i; A61K31/55(2006.01)i; A61P35/02(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; WPABS; DWPI; ENTXTC; CNKI; ISI_web of Science; CNKI; STN; 万方, WANFANG: 豪森, 翰森, 李媛媛, 吕临松, 酪氨酸, 蛋白, 激酶, 白血病, 环辛, 茚, tyrosine, kinase, abelson, ABL, BCR-ABL, leukemia, cyclooctane, indene, structural formula search

**C. DOCUMENTS CONSIDERED TO BE RELEVANT**

| Category* | Citation of document, with indication, where appropriate, of the relevant passages | Relevant to claim No. |
|---|---|---|
| A | US 2021101872 A1 (THE USA, AS REPRESENTED BY THE SECRETARY, DEPT. OF HEALTH AND HUMAN SERVICES et al.) 08 April 2021 (2021-04-08)<br>description, paragraphs [0066], [0111]-[0120] and [0160] | 1-13 |
| A | CN 103113355 A (WUXI ALLNATURE BIOLOGICAL SCIENCE & TECHNOLOGY CO., LTD.) 22 May 2013 (2013-05-22)<br>description, paragraphs [0004]-[0017], and embodiments 1-7 | 1-13 |
| A | CN 109790144 A (ASTAR BIOTECH LLC) 21 May 2019 (2019-05-21)<br>description, paragraphs [0016] and [0020]-[0037], and embodiments 001-067 | 1-13 |
| A | WO 2013033093 A1 (BIOCRYST PHARMACEUTICALS, INC. et al.) 07 March 2013 (2013-03-07)<br>embodiments 1-89, and page 122 | 1-13 |
| A | WO 2022232318 A1 (MERCK SHARP & DOHME CORP.) 03 November 2022 (2022-11-03)<br>pages 98-113 and 245-387 | 1-13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 July 2024** | **09 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/084821**

| Patent document cited in search report | Publication date (day/month/year) | Patent family member(s) | Publication date (day/month/year) |
|---|---|---|---|
| US 2021101872 A1 | 08 April 2021 | US 2023303501 A1 | 28 September 2023 |
| | | WO 2019173761 A1 | 12 September 2019 |
| | | CL 2020002317 A1 | 08 January 2021 |
| | | US 11649218 B2 | 16 May 2023 |
| CN 103113355 A | 22 May 2013 | None | |
| CN 109790144 A | 21 May 2019 | JP 2019515932 A | 13 June 2019 |
| | | EP 3448852 A1 | 06 March 2019 |
| | | EP 3448852 A4 | 10 April 2019 |
| | | WO 2017186148 A1 | 02 November 2017 |
| WO 2013033093 A1 | 07 March 2013 | None | |
| WO 2022232318 A1 | 03 November 2022 | EP 4329749 A1 | 06 March 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022122536 W